(19) Europäisches Patentamt
European Patent Office
Office européen des brevets

(11) EP 1 073 891 B1

(12) # EUROPEAN PATENT SPECIFICATION

(45) Date of publication and mention of the grant of the patent: **09.08.2006 Bulletin 2006/32**

(21) Application number: **99914190.6**

(22) Date of filing: **26.03.1999**

(51) Int Cl.: ***G01N 1/00*** *(2006.01)* ***G01N 33/74*** *(2006.01)* ***C07K 7/00*** *(2006.01)*

(86) International application number: **PCT/US1999/006664**

(87) International publication number: **WO 1999/054728 (28.10.1999 Gazette 1999/43)**

(54) **METHOD OF PREDICTING RECEPTOR MODULATING ACTIVITY**

VORHERSAGE ÜBER DIE REZEPTOR-MODULIERENDE WIRKUNG VON VERBINDUNGEN

METHODE PERMETTANT DE PREVOIR LA CAPACITE DE COMPOSES DE MODULER L'ACTIVITE BIOLOGIQUE DE RECEPTEURS

(84) Designated Contracting States:
**AT BE CH CY DE DK ES FI FR GB GR IE IT LI LU MC NL PT SE**

(30) Priority: **23.04.1998 US 82756 P**
**09.09.1998 US 99656 P**
**08.01.1999 US 115345 P**

(43) Date of publication of application: **07.02.2001 Bulletin 2001/06**

(73) Proprietors:
- **Duke University Durham, North Carolina 27710 (US)**
- **KARO BIO AB 141 57 Huddinge (SE)**

(72) Inventors:
- **PAIGE, Lisa, A. Rougemont, NC 27572 (US)**
- **FOWLKES, Dana, M. Chapel Hill, NC 27516 (US)**
- **MCDONNELL, Donald, P., Duke Univ. Medical Center Durham, NC 27710 (US)**
- **CHRISTENSEN, Dale, J. Apex, NC 27502 (US)**
- **NORRIS, John D. Raleigh, North Carolina 27613 (US)**
- **CHANG, Ching-Yi Durham, North Carolina 27713 (US)**

(74) Representative: **Elsy, David et al**
**Withers & Rogers LLP**
**Goldings House,**
**2 Hays Lane**
**London SE1 2HW (GB)**

(56) References cited:
**WO-A-97/15586** **WO-A-97/31646**

- **MCDONNELL, D.P. ET AL.: "Analysis of Estrogen Receptor Function in Vitro Reveals Three Distinct Classes of Antiestrogens" MOLECULAR ENDOCRINOLOGY, vol. 9, no. 6, June 1995 (1995-06), pages 659-669, XP002113863**
- **NORRIS, JOHN D. ET AL: "Peptide antagonists of the human estrogen receptor" SCIENCE (WASHINGTON, D. C.) (1999), 285(5428), 744-746, XP002113864**
- **PAIGE, LISA A. ET AL: "Estrogen receptor (ER) modulators each induce distinct conformational changes in ER.alpha. and ER.beta." PROC. NATL. ACAD. SCI. U. S. A. (MARCH 1999), 96(7), 3999-4004, XP002113865**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 073 891 B1

## Description

Cross-Reference to Related Applications

Thorp, Serial No. 08/904,842 (published as WO99/06839), METHOD OF IDENTIFYING AND DEVELOPING DRUG LEADS WHICH MODULATE THE ACTIVITY OF A TARGET PROTEIN, discloses several methods of identifying drug leads. In essence a protein of interest, in one or more states, is characterized by (a) its chemical reactivity with one or more characterizing reagents, and/or (b) its binding to one or more aptamers (especially nucleic acids), generating an array of descriptors by which it may be characterized as more or less similar for reference proteins for which an equivalent array of descriptors have been generated, and for which one or more activity-mediating reference drugs are known. Suitable drug leads for the protein of interest are those analogous to the reference drugs for the more similar reference proteins.

Fowlkes, *et al,* PCTIUS97/19638 (WO98/19162), 08/740,671, 09/050,359 and 09/069,827 (US 6,617,114), IDENTIFICATION OF DRUGS USING COMPLEMENTARY ; COMBINATORIAL LIBRARIES, disclose the use of a first combinatorial library, e.g., of peptides, to obtain a sec or binding peptides that can serve as a surrogate for the natural ligand of a target protein. A small organic compound library (preferably combinatorial in nature) is then screened for compound which inhibit the binding of the surrogates to the target protein.

Paige, et al., Serial No. 60/082, 756, filed April 23, 1998, and Paige, et al., Serial No. 60/099,656, filed September 9, 1998, are predecessors of the instant application.

## BACKGROUND OF THE INVENTION

Field of the Invention

This invention relates to a method of identifying drugs which can mediate the biological activity of a target protein.

Description of the Background Art

*Protein Binding and Biological Activity*

Many of the biological activities of the proteins are attributable to their ability to bind specifically to one or more binding partners (ligands), which may themselves be proteins, or other biomolecules.

When the binding partrier of a protein is known, it is relatively straightforward to study how the interaction of the binding protein and its binding partner affects biological activity. Moreover, one may screen compounds for the ability of the compound to competitively inhibit the formation of the complex, or to dissociate an already formed complex. Such inhibitors are likely to affect the biological activity of the protein, at least if they can be delivered in vivo to the site of the interaction.

If the binding protein is a receptor, and the binding partner an effector of the biological activity, then the inhibitor will antagonize the biological activity. If the binding partner is one which, through binding, blocks a biological activity, then an inhibitor of that interaction will, in effect, be an agonist.

*Screening for Modulators of Receptor Activity*

The current state of the art for screening for modulators of receptor activity involves the displacement of a labeled ligand from the ligand binding pocket of the receptor. For example, a screen may be for displacement of radiolabeled estradiol from the estrogen receptor. This assay only provides information concerning the relative affinities of the compounds for the receptor and gives no indication of the activity of the compound on the receptor, that is whether it functions as an agonist or an antagonist of receptor activity. This is a major problem for pharmaceutical companies to overcome in screening for modulators of receptor activity.

The assays that have been developed to date that can distinguish between agonists and antagonists involve cell-based assays and reporter gene systems. McDonnell, et al., Molec. Endocrinol., 9:659 (1995). In these systems, the receptor and a reporter gene are co-transfected into cells in culture. The reporter gene is only activated in the presence of active receptor. The ability of a compound to modulate receptor activity is determined by the relative strength of the reporter gene activity. These assays are time consuming and can produce variable results in different cell lines or with different reporter genes or response elements. Thus, the data must be interpreted with caution

Methods have been developed that also take advantage of the different conformational states of receptors. Proteolytic digestion of the estrogen receptor in the presence of an agonist or antagonist produces distinct banding patterns on a denaturing polyacrylamide gel. In certain conformations, the receptor is protected from digestion at a particular site, while a different conformation may expose that site. Thus the banding patterns may indicate whether the receptor was complexed with an agonist or antagonist at the time of proteolytic digestion. This method requires copious amounts of receptor protein and is time consuming and expensive in that it requires a gel to be run for each sample. It is not suitable for screening numerous samples.

The following are examples of patents on cell based screening methods:

Patent US 5723291 - Methods for screening compounds for estrogenic activity
Patent US 5298429 - Bioassay for identifying ligands for steroid hormone receptors
Patent US 5445941 - Method for screening anti osteoporosis agents
Patent US 5071773 - Hormone receptor-related bioassays
Patent US 5217867 - Receptors: their identification, characterization, preparation and use

*Nuclear Receptors*

Nuclear receptors are a family of ligand activated transcriptional activators, see Evans and Hollenberg, Cell, 52:1-3 (1988), factors which include the receptors for steroid and thyroid hormones, retinoids, and vitamin D. The steroid receptor family is composed of receptors for glucocorticoids, mineralocorticoids, androgens, progestins, and estrogens. These receptors are organized into distinct domains for ligand binding, dimerization, transactivation, and DNA binding. Receptor activation occurs upon ligand binding, which induces conformational changes allowing receptor dimerization and binding of co-activating proteins. These co-activators, in turn, facilitate the binding of the receptors to DNA and subsequent transcriptional activation of target genes. In addition to the recruitment of co-activating proteins, the binding of ligand is also believed to place the receptor in a conformation that either displaces or prevents the binding of proteins that serve as co-repressors of receptor function. Lavinsky, et al., Proc. Nat. Acad. Sci. (USA), 95:2920 (1998).

The estrogen receptor is a member of the steroid family of nuclear receptors. Human ERα is a 595 amino acid protein composed of six functional domains or regions (A-F). The A/B region contains the transcription function AF-1, and the E domain contains the transcription function AF-2. These functions activate transcription in a cell- and promoter context-specfic manner. AF-1 is constitutively active, while AF-2 is induced by hormone binding to the receptor. The C region contains the DNA-binding domain and a dimerization domain. The DNA-binding domain binds the estrogen (receptor) response element (ERE) associated with a regulated gene. The DBD contains two zinc fingers. The C region may also be responsible for nuclear localization. The E region contains the hormone (ligand) binding domain.

The classical ERE is composed of two inverted hexanucleotide repeats, and ligand-bound ER binds to the ERE as a homodimer. The ER also mediates gene transcription from an AP1 enhancer element that requires ligand and the AP1 transcriptional factors Fos and Jun for transcriptional activation. Tamoxifen inhibits transcription of genes regulated by a classical ERE, but activates transcription of genes under the control of an AP1 element. See Paech, et al., Science, 277:1508-11 (1997).

In the absence of hormone, the estrogen receptor resides in the nucleus of target cells where it is associated with an inhibitory heat shock protein complex. (Smith, et al., (1993) Mol. Endocrinol., 7:4-11.) Upon binding ligand, the receptor is activated. This process permits the formation of stable receptor dimers and subsequent interaction with specific DNA response elements located within the regulatory region of target genes. (McDonnell, et al. (1991), Mol. Cell Biol., 11:4350-4355.) The DNA bound receptor can then either positively or negatively regulate target gene transcription. Although the precise mechanism by which the ER modulates RNA polymerase activity remains to be determined, it has been shown recently that agonist bound ER can recruit transcriptional adaptors, proteins that permit the receptor to transmit its regulatory information to the cellular transcriptional apparatus. (Onate, et al. (1995), Science, 270: 1354-1357; Norris, et al. (1998), J. Biol. Chem., 273:6679-6688; Smith, et al. (1997), Mol. Endocrinol., 11:657-666). Conversely, when occupied by antagonists, the DNA bound receptor actively recruits co-repressors, proteins that permit the cell to distinguish between agonists and antagonists. (Norris, et al. (1998); Smith, et al. (1997); Lavinsky, et al., (1998) Proc. Natl. Acad. Sci. USA, 95:2920-2925). Building on this complexity was the recent discovery of a second estrogen receptor, ERβ, whose mechanism of action appears to be similar, yet distinct from ERα. (Greene, et al. (1986), Science, 231:1150-1154; Kuiper, et al. (1996), Proc. Natl. Acad. Sci. USA, 93:5925-5930; Mosselman, et al. (1996), FEBS Lett., 392:49-53).

Thus, there are two forms of this receptor, α and β, presently known; other forms may exist. Both receptors activate transcription in response to estrogens, which are an important group of steroid hormones that not only influence the growth, differentiation, and functioning of the reproductive system, but also exert effects in the bone, brain and cardiovascular system. Estrogens can produce a broad range of effects in this diverse set of target tissues. These differential effects are believed mediated, in part, by tissue specific activation of the two different transactivation domains present at the amino-terminal and carboxy-terminal regions of the receptor. It is also likely that the two forms of the receptor (α and β) function in distinct tissues and thereby mediate the transactivation of different subsets of genes. (Paech, et al., Science, 277:1508, 1997; Kuiper and Gustafsson, FEBS Lett., 410:87, 1997; Nichols, et al., EMBO J.,

17:765, 1998; Montano, et al., Mol. Endo., 9:814, 1995.)

Drugs that target the estrogen receptor can exhibit a variety of effects in different target tissues. For example, tamoxifen is an ER antagonist in breast tissue, (Jordan, V.C., (1992) Cancer, 70:977-982), but an ER agonist in bone (Love, et al. (1992), New Engl. J. Med., 326:852-856) and uterine, (Kedar, et al. (1994), Lancet, 343:1318-1321) tissue. Raloxifene is also an ER antagonist in breast tissue; however, it exerts agonist activity in bone but not uterine tissue (Black, et al. (1994), J. Clin. Invest., 93:63-69). Indeed, one of the greatest challenges in understanding the pharmacology of the estrogen receptor is determining how different ER ligands produce such diverse biological effects.

Estrogens, in general, are stimulatory agents, resulting in increased gene expression and cell proliferation in target tissues. However, many molecules have been described that bind to the estradiol binding site on the receptor, but produce negative effects on gene expression and cell growth. These agents have historically been termed "antiestrogens", but this term has proven to be much too simplistic. (Tremblay, et al., Can. Res., 58:877, 1988; Katzenellenboge, et al., Breast Can Res. Treatm., 44:23, 1997; Howell, Oncology (suppl. 1), 11:59, 1997; Gallo and Kaufman, Sem. in Oncol. (Suppl. 1), 24:71, 1997). One of the most noteworthy of these agents is tamoxifen, which has been successfully used in the treatment of ER-positive breast cancer. Tamoxifen, a derivative of triphenylethylene, is metabolized in the cell to produce 4-OH tamoxifen, which has very high affinity for the estradiol binding pocket of the ER. Although this compound competes with estradiol for binding to the ER, it does not induce transcriptional activation in breast tissue, thus it does not promote cell growth and acts as a classic antiestrogen in this tissue. Tamoxifen, however, does have estrogen-like activities in other tissues. In the uterus, tamoxifen acts as an agonist of receptor activity, stimulating the growth of uterine tissue leading to an increased incidence of endometrial hyperplasia in treated patients. Tamoxifen also produces estrogenic effects in the bone and cardiovascular system. This activity generates beneficial effects such as reducing the risk of osteoporosis and lowering serum LDL levels. The numerous differential effects produced by compounds such as tamoxifen has led to the replacement of the term "antiestrogen" with "selective estrogen receptor modulators" or SERMs. SERMs may have both positive and negative effects on ER activity depending on the biology of receptor and the tissue in which it is being expressed.

A goal of current research is to develop SERMs that have agonistic or estrogenic effects on bone and the cardiovascular system and antagonistic or antiestrogenic effects in the breast and uterus. One SERM that has recently been approved for treatment of post-menopausal symptoms is Raloxifene. Raloxifene is a benzothiophene derivative that, like tamoxifen, binds in the ligand binding pocket of the ER. Clinical studies indicate that this compound lacks estrogenic activity in the breast and uterus, but produces estrogenic activity in the bone and perhaps the cardiovascular system. It is currently prescribed for prevention for osteoporosis in post-menopausal women. There are several additional SERMs in clinical trials, and a great deal of effort in the pharmaceutical industry is focused on the identification and characterization of additional SERMs.

The search for SERMs poses a major obstacle. In order to screen large libraries of compounds for SERMs, it is necessary to have a convenient assay for identifying which lead molecules have the desired effect(s). Currently, when a compound is identified that competes with estradiol for binding to the ER, a number of cell-based assays must be conducted to determine its activity. These studies are more laborious than in vitro assays and still do not absolutely predict the complete spectrum of biological activity of the SERM. Thus, studies often have to move into animal models or clinical trials before the selective modes of action of the SERM can be determined. A simple *in vitro* system to distinguish between agonist and antagonist activity of a SERM would be of great utility.

The development of such a system requires knowledge of the mechanisms that produce the broad effects of SERMs. There is evidence that SERMs are able to produce differential (agonistic and antagonistic) effects due to their ability to alter the conformation of the ER. In general, the receptor is thought of as having two conformations, active or inactive. These conformations are formed in the presence or absence of ligand, respectively. The SERM drives the receptor into a conformation that is neither fully active nor fully inactive. This intermediate conformation creates changes in the association patterns of co-activators, co-repressors, and other regulatory molecules with the receptor, thus producing variable effects. The broad range of effects produced by SERMs may also be due to selective tissue expression of ER alpha and beta as well as co-activators and co-repressors. It may also be due to different affinities of the SERM for the two receptors. *Traditional Drug Screening*

In traditional drug screening, natural products (especially those used in folk remedies) were tested for biological activity. The active ingredients of these products were purified and characterized, and then synthetic analogues of these "drug leads" were designed, prepared and tested for activity. The best of these analogues became the next generation of "drug leads", and new analogs were made and evaluated.

Both natural products and synthetic compounds could be tested for just a single activity, or tested exhaustively for any biological activity of the interest to the tester. Testing was originally carried out in animals, later, less expensive and more convenient model systems, employing isolated organs, tissue, or cells, or cell cultures, membrane extracts or purified receptors, were developed for some pharmacological evaluations.

Testing in whole animals and isolated organs typically requires large amounts of chemical compound to test. Since the quantity of a given compound within a collection of potential medicinal compounds is limited, this requires one to limit the number of screens executed.

Also, it is inherently difficult to establish structure/activity relationships (SAR) among compounds tested using whole animals, or isolated organs or tissues or, to a lesser extent, cultured cells. This is because the actual molecular target of any given compound's action may be quite different from that of other compounds scoring positive in the assay. By testing a battery of compounds on a very specific target, one can correlate the action of various chemical residues with the quantitative activity and use that information to focus ones search for active compounds among certain classes of compounds or even direct the synthesis of novel compounds having a composite of the properties shared by the active compounds tested.

Another disadvantage to whole animal, organ, tissue and cell based screening is that certain limitations may prevent an active compound from being scored as such. For instance, an inability to pass through the cellular membrane may prevent a potent inhibitor, within a tested compound library, from acting on the activated oncogene ras and giving a spurious negative score in a cell proliferation assay. However, if it were possible to test ras in an isolated system, that potent inhibitor would be scored as a positive compound and contribute to the establishment of a relevant SAR. Subsequent, chemical modifications could then be carried out to optimize the compound structure for membrane permeability. (In the case of cell-based assays, this problem can be alleviated to some degree by altering membrane permeability.)

*Drug Discovery.* The human genomics effort could yield gene sequences that code for as many as 70,000 proteins, each a potential drug target; microbial genomics will increase this number further. Unfortunately, since genomic studies identify genes, but not the biological activity of the corresponding proteins, it is likely that many of the genes will prove to encode proteins whose activation or inactivation has no effect on disease progression. (Gold, et al., J. Nature Biotech., 15:297, 1997). There is therefore a need for a method of determining which proteins are most likely to be productive targets for pharmacological intervention.

Even if one knew in advance the perhaps 10,000 proteins which could be considered interesting targets, there remains the problem of efficiently screening hundreds of thousands of possible drugs for a useful activity against these 10,000 targets.

Historically, acquiring chemical compound libraries has been a barrier to the entry of smaller firms into the drug discovery arena. Due to the large quantity of chemical required for testing on whole animals and even on cells in culture, it was a given that whenever a compound was synthesized it should be done in fairly large quantity. Thus, there was a synthesis and purification throughput of less than 50 compounds per chemist per year. Large companies maintained their immensely valuable collections as trade barriers. However, with the downsizing of targets to the molecular level and the automation of screens, the quantity of a given compound necessary for an assay has been reduced to very small amounts. These changes have opened the door for the utilization of so-called combinatorial chemistry libraries in lieu of the traditional chemical libraries. Combinatorial chemistry permits the rapid and relatively inexpensive synthesis of large numbers of compounds in the small quantities suitable for automated assays directed at molecular targets. Numerous small companies and academic laboratories have successfully engineered combinatorial chemical libraries with a significant range of diversity (reviewed in Doyle, 1995, Gordon *et al,* 1994a, Gordon *et al,* 1994b).

*Combinatorial Libraries.* In a combinatorial library, chemical building blocks are randomly combined into a large number (as high as 10E15) of different compounds, which are then simultaneously screened for binding (or other) activity against one or more targets.

Libraries of thousands, even millions, of random oligopeptides have been prepared by chemical synthesis (Houghten *et al.,* Nature, 354:84-6(1991)), or gene expression (Marks *et al.,* J Mol Biol, 222:581-97(1991)), displayed on chromatographic supports (Lam *et al.*, Nature, 354:82-4(1991)), inside bacterial cells (Colas *et al.*, Nature, 380: 548-550(1996)), on bacterial pili (Lu, Bio/Technology, 13:366-372(1990)), or phage (Smith, Science, 228:1315-7(1985)), and screened for binding to a variety of targets including antibodies (Valadon *et al.,* J Mol Biol, 261:11-22(1996)), cellular proteins (Schmitz *et al.,* J Mol Biol, 260:664-677(1996)), viral proteins (Hong and Boulanger, Embo J, 14:4714-4727 (1995)), bacterial proteins (Jacobsson and Frykberg, Biotechniques, 18:878-885(1995)), nucleic acids (Cheng *et al.,* Gene, 171:1-8(1996)), and plastic (Siani *et al.,* J Chem Inf Comput Sci, 34:588-593(1994)).

Libraries of proteins (Ladner, USP 4,664,989), peptoids (Simon *et al.*, Proc Natl Acad Sci U S A, 89:9367-71 (1992)), nucleic acids (Ellington and Szostak, Nature, 246:818(1990)), carbohydrates, and small organic molecules (Eichler *et al.*, Med Res Rev, 15:481-96(1995)) have also been prepared or suggested for drug screening purposes.

The first combinatorial libraries were composed of peptides or proteins, in which all or selected amino acid positions were randomized. Peptides and proteins can exhibit high and specific binding activity, and can act as catalysts. In consequence, they are of great importance in biological systems. Unfortunately, peptides *per se* have limited utility for use as therapeutic entities. They are costly to synthesize, unstable in the presence of proteases and in general do not transit cellular membranes. Other classes of compounds have better properties for drug candidates.

Nucleic acids have also been used in combinatorial libraries. Their great advantage is the ease with which a nucleic acid with appropriate binding activity can be amplified. As a result, combinatorial libraries composed of nucleic acids can be of low redundancy and hence, of high diversity. However, the resulting oligonucleotides are not suitable as drugs for several reasons. First, the oligonucleotides have high molecular weights and cannot be synthesized conveniently in large quantities. Second, because oligonucleotides are polyanions, they do not cross cell membranes. Finally, deoxy- and ribo-nucleotides are hydrolytically digested by nucleases that occur in all living systems and are therefore usually decomposed before reaching the target.

There has therefore been much interest in combinatorial libraries based on small molecules, which are more suited to pharmaceutical use, especially those which, like benzodiazepines, belong to a chemical class which has already yielded useful pharmacological agents. The techniques of combinatorial chemistry have been recognized as the most efficient means for finding small molecules that act on these targets. At present, small molecule combinatorial chemistry involves the synthesis of either pooled or discrete molecules that present varying arrays of functionality on a common scaffold. These compounds are grouped in libraries that are then screened against the target of interest either for binding or for inhibition of biological activity. Libraries containing hundreds of thousands of compounds are now being routinely synthesized; however, screening these large libraries for binding or inhibition with all 10,000 potential targets cannot be reasonably accomplished with present screening technologies, and there are numerous experimental and computational strategies under development to reduce the number of compounds that must be screened for each target.

*Information-intensive drug discovery.* As pointed out by Paterson, et al., J. Med. Chem., 39: 3049-59 (1996), medicinal chemistry advances through the dual processes of "lead discovery" and "lead optimization". In "lead discovery", the search objective is the discovery of an "activity island", a chemical class with a high frequency of active molecules. (this class may be defined mathematically as a volume within a multidimensional space defined by various molecular descriptors). In "lead optimization", the "activity island" is explored in detail. If each compound synthesized and tested can be considered as a probe of a "neighborhood" of similar compounds, in "lead discovery", it is inefficient to test substances whose neighborhoods overlap.

Coupled to the recent advancements in genomics and molecular biology has been a revolution in information technology, which includes relational databases, computer graphics, and neural networks (*13*). These capabilities permit the construction of databases of descriptors that describe either compounds or targets in quantitative terms, and these descriptors can be related to make predictions about the structures of compounds, their biological activities, and the targets they act on (*5-8*).

Structure descriptors can be based on a variety of structural features. These approaches provide arrays of molecular descriptors that can be used to assess the similarity of molecules in a library.

See Patterson, et al. , et al., J. Med. Chem., 39: 3049-59 (1996), Klebe and Abraham, J. Med. Chem., 36:70-80 (1993), Cummins, et al., J. Chem. Inf. Comput. Sci., 36:750-63 (1996), Matter, J. Med. Chem., 40:1219-29 (1997); Weinstein, et al., Science, 275:343-9 (1997).

For proteins, structural descriptors cannot be directly calculated from the amino acid sequence.

Compounds may be characterized by their activity rather than by structure. Kauvar, et al., Chemistry & Biology, 2: 107-118 (1995) "fingerprinted" over 5,000 compounds by the binding potency (concentration needed to inhibit 50% of the protein's activity) of each compound to each member of a reference panel of eight proteins. (These proteins were selected on the basis of readily assayable activity, broad cross-reactivity with small organic molecules, and low correlation between each other in binding patterns.) A screening library of 54 compounds was then selected based on the diversity in their "fingerprints" (inhibitory activity against the reference panel proteins).

This "training set" was used to evaluate the similarity of the ligand binding characteristics of a new protein to one of the reference panel proteins. By regression analysis, a computational surrogate (a weighted sum of two or more reference panel proteins) for the new protein is determined. The activity of all fingerprinted compounds to inhibit the activity of the new protein is predicted as the sum of their appropriately weighted inhibitory activities against the component reference proteins of the computational surrogate. Predictions may be improved by testing additional sets of compounds against the new protein. See also L. M. Kauvar, H. O. Villar. Method to identify binding partners. US Patent 5587293.

Weinstein, supra, in a study of the molecular pharmacology of cancer, took a similar approach. The "activity" database **(A)** contains the activities against 60 cell lines for 60,000 compounds that have been screened at NCI. The similarity in the activity profile against the panel of cell lines can then be calculated for any two compounds, and is generally assessed by a pairwise correlation coefficient (PCC), which is determined by an algorithm called COMPARE, which calculates the similarity of all of the compounds in the database to a user-supplied "seed" compound.

## SUMMARY OF THE INVENTION

The present invention is directed to a method for the more efficient identification of small organic molecules, preferably molecules having a molecular weight of less than 500 daltons, which are pharmaceutically acceptable and which are potent modulators of the biological activity of a protein.

This method provides a simple and consistent means for identifying and characterizing modulators of receptor activity, using oligomers (especially peptides) to probe receptor conformation. It can be used as both a tool in both primary and secondary screens for compounds that modulate the activity of a receptor. In some embodiments, the method is also completely in vitro so the activity of a compound can be assessed without using a cell based assay, let alone a whole animal assay.

We have explored the possibility that various ER ligands induce distinct conformational changes in the ER. These distinct conformations may, in turn, alter the interactions of the receptor with cell and tissue specific co-activating or co-repressing proteins or even estrogen response elements, thus leading to diverse biological effects. Using limited proteolysis, we and others have shown that the ER agonist estradiol and the ER antagonist Imperial Chemical Industries (ICI) 182,780 induced distinct ER conformations (McDonnell, et al. (1995), Mol. Endocrinol., 9:659-669; Beekman, et al. (1993), Mol. Endocrin., 7:1266-1274). However, the picture is much more complicated than this. There are a variety of ER ligands, namely, selective estrogen receptor modulators (SERMS), which are neither pure agonists nor antagonists. These ligands, which include tamoxifen and raloxifene, produce distinct tissue specific biological effects, yet conformational differences cannot be discerned in the protease digestion assay. It is likely that these compounds are also eliciting distinct conformational changes that affect ER activity, but the changes are too subtle to be detected by the protease digestion assay (Brzozowski, et al. (1997), Nature, 389:753-758; Shiau, et al. (1998), Cell, 95:927-937).

This invention is based on the observation that peptides isolated by screening a phage-displayed peptide library for binding to the estrogen receptor had dramatically different binding affinities depending on whether the receptor was unliganded, or complexed with an agonist or an antagonist. Thus, the peptide binding appears to be a barometer of protein conformation, and hence of whether a compound which is complexed to the receptor is acting as an agonist or an antagonist.

In essence, a panel of "BioKeys" (typically peptides) which alter the conformation of a receptor in distinctly different ways, are used to obtain a "fingerprint" of how a compound of interest interact with that receptor in its various BioKey-modified conformations, each element of the fingerprint being a measure of the strength of interaction of the compound with the receptor in the presence of a given BioKey. Once fingerprints are obtained for a reasonable number of reference compounds with known biological activities, as measured by a "gold standard" (whole animal, or isolated organ or tissue) assay, the similarity of the fingerprint of a new compound to that of the reference compounds may be calculated, and used to predict the bioactivity of the new compound.

The invention has advantages over the whole animal-based systems described above in that 1) the same technology can be applied to a variety of different receptors, 2) the system can be used for high throughput screening and compound characterization, and 3) the system gives very distinct patterns for agonists and antagonists of receptor activity using very little protein.

In the "molecular braille" (MB) embodiment of the present invention, the reference and test fingerprints are based on in vitro (cell-free) assays.

In the "cellular-braille" (CB) embodiment of the present invention, the reference and test fingerprints are based on cellular assays (but not on assays of whole multicellular organisms, or their organs or tissues).

The advantages of "molecular braille" are

- gives information about affinity, and, based on a fingerprint, bioactivity in a single assay
- can be faster and less expensive if the protein is a) inexpensive to purchase or b) easy to express and purify
- gives information about structure-activity relationships
- peptide/receptor interactions may be more sensitive because there will not be anything extraneous to get in the way

Its disadvantages are

- protein may not be properly folded, modified, or be in the presence of cofactors it needs to be active
- doesn't give much of the information given by CB

In contrast, the advantages of "cellular braille" are

- If in yeast it can be cheaper than MB
- Bioactivity (including dose:effect) information
- gives closer indication of how a whole animal might respond
- you may get active metabolites
- no need for protein purification

Its disadvantages are

- compounds that cannot get into the cell will automatically be selected against
- does not give affinity information directly
- throughput likely to be lower than with MB, although still better than whole animal assay.

Both "molecular braille" and "cellular braille" are faster and less expensive than whole animal bioassays, and more readily automated for high throughput, and their use as preliminary screens helps minimize experimentation on animals, which itself is an ethical goal of society.

It will be appreciated that both techniques may be used, either sequentially or simultaneously. For example, MB may be used as a first screen and CB as a second screen of the first round positives. Or compounds may be screened by both MB and CB, and compounds earmarked by either screen given further attention. Similarities may be calculated separately from in vitro and cell-based assays, or the results of these two types of assays may be combined into a single fingerprint for each reference or test compound.

In a preferred embodiment, this method uses phage display to isolate peptides (BioKeys) that map the sites of biological interaction on both the active and the inactive receptor. These BioKeys are probes for alterations in receptor conformation, and can readily distinguish between active, inactive and partially active receptor. The patterns of binding obtained with the peptides provides a fingerprint of the receptor conformation. The binding of the individual peptides will increase or decrease in the presence of an agonist or an antagonist of receptor activity. Such activity may or may not be tissue-specific. In some cases, whether a molecule is an agonist or an antagonist will depend on the tissue in question (e.g. for SERMs), or on other environmental factors. Therefore, the peptides may be used to classify compounds, not only as pure agonists or antagonists, but also more complexly. The method has the following applications:

1) One or more of these peptides can be used in a competitive displacement assay to identify modulators of receptor activity in a high-throughput (in vitro or simple cell) screen.
2) The peptides can be used to fingerprint modulators of receptor activity and classify them as agonists or antagonists of receptor activity.
3) Peptides identified for orphan receptors may be used to identify the natural ligand of these receptors.
4) This method may be used for nuclear receptors as well as other receptors such as G-protein coupled receptors.
5) Method can be applied to any protein that undergoes a conformational change upon ligand/substrate binding.

In a particular preferred embodiment, the invention is used to predict SERM activity against nuclear receptors, such as the estrogen receptor.

In order to characterize SERM activity at the estrogen receptor, we have developed a system that utilizes peptides to mimic the binding of various ER associated proteins to ER $\alpha$ and $\beta$ in an *in vitro* setting. The peptides bind preferentially to either the active or inactive conformation of the receptor, and will distinguish between different conformational changes in the ER that result from the binding of a SERM. The system will also allow the comparison of effects of the SERM on ER $\alpha$ and $\beta$. This assay provides a simple procedure to determine the relative agonist/antagonist activity of a newly identified SERM. The technology may also be applied to the analysis of selective modulators of any receptor.

We have developed an *in vitro* system for identifying, characterizing and classifying modulators of receptor activity. The technique was developed using the estrogen receptor and is based on mapping sites of biological interaction on the active and inactive receptor using phage displayed peptide libraries. The peptides that bind to these sites appear to mimic proteins that bind preferentially to the active or inactive estrogen receptor. Certain sites on the receptor are only available for binding when an agonist is bound to the ER. Other sites are more readily available for binding with a SERM complexed ER. The relative binding affinities of these peptides on an estrogen complexed receptor, or a SERM complexed receptor relative to an unliganded receptor provides a fingerprint that is indicative of the agonist/antagonist activity of the SERM. The system has been tested on the ER using several known agonists and SERMs. Agonists of receptor function and SERMs produced distinct fingerprints in our system indicative of their distinct in vivo functions. This system may be used as a primary screening tool to identify hits, to classify lead compounds from a drug screen, to characterize SERMs in terms of agonist and antagonist function and to predict possible clinical effects of SERMs such as tissue and receptor specificity. This method can also be applied to the fractionation of mixtures of SERMs to determine which components are producing agonistic and antagonistic activity. This method may also be used with other receptors (e.g., progesterone, androgen, glucocorticoid, thyroid, vitamin D, beta-adrenergic, dopamine, epidermal growth factor, etc.), to identify, characterize and classify modulators of receptor activity.

While peptides have been identified for use as probes to modify receptor conformation, to help screen compound libraries, certain of these peptides may be useful in their own right as drugs or diagnostics.

In addition, nonpeptide mimetics or other analogues of the aforementioned peptides may be useful as drugs or diagnostics.

The screened compounds, and their analogues, are also of interest.

## BRIEF DESCRIPTION OF THE DRAWINGS

Figure 1 shows the effect of seven drugs which modulate estrogen receptor activity, in five sites of action.
Figure 2 maps the binding site for interaction of the four peptides with ERα (or moieties thereof) as influenced by estradiol or 4-OH tamoxifen.
Figure 3 shows that different ligands induce different structural alterations in ER alpha and ER beta, as shown by differences in the binding of 11 different peptides. (The data in this figure is tabulated in table 14.)
Figure 4 compares the effects of estradiol (an agonist) and raloxifene (an antagonist) on ERalpha conformation.
Figure 5 shows how the data in Fig. 3 and Table 14A is used to calculate similarities.
Figure 5A duplicates Table 14A.
Figure 5B shows the raw Euclidean distances.
Figure 5C shows the calculated similarities, after scaling:

$$\text{similarity} = \frac{\text{maximum dist - actual dist}}{\text{maximum dist}}$$

With 8 descriptors (BioKeys) and scores 0-7, the maximum distance is SQRT (7*7*8), or 19.79899.
Figure 5D is a 3D bar graph corresponding to 5C.
Figure 5E is a 2D bar graph isolating the similarity data for estradiol and 4-OH tamoxifen.
Figure 6 shows, in a similar manner, the calculation of similarities based on the ERbeta data.
Figure 7 analyzes the interaction of seven drugs with ERα and four different peptides (AB1, A2, AB3, AB5) using a mammalian two-hybrid assay system.
Figure 8 analyzes the specificity of interaction of various drugs with four more nuclear receptors and the same four peptides using the same assay system.
Figure 9 explores the interaction of the four peptides with mutant receptors (impaired AF-2 function) as influenced by seven different drugs.
Figure 10 studies the disruption of ER mediated transcriptional activity as a function of peptide concentration.
Figure 11 shows that the A2 disruption of tamoxifen-activated ER is not promoter-dependent.
Figure 12 explores disruption of ER transcriptional activity as mediated by the AP-1 pathway.
Figure 13 is a schematic model of potential mechanisms of action of peptides which block tamoxifen partial agonist activity.
Figure 14 shows the normalized luciferase activity of a two-hybrid mammalian system for ER AF2 in presence of estradiol (E2), 4-OH tamoxifen, ICI, DES, GW 7604, estrone, equilin and D8,9DHE.
Figure 15 shows the binding of various peptides to both wild-type and mutant ER.
Figure 16A shows the disruption of Eα transcriptional activation function in mammalian cells as a result of the action of LXXLL-containing peptides. B. shows the synergistic interaction of two copies of LXXLL motif function to compete with endogenous coactivators.
Figure 17 shows that LXXLL containing peptides disrupt AF2 functions in HepG2 cells.
Figure 18 shows that nuclear receptors have distinct preferences for different peptides with LXXLL motifs.
Figure 19 shows a similarity analysis of the data pictured in Figure 17.

## DETAILED DESCRIPTION OF THE PREFERRED EMBODIMENTS OF THE INVENTION

### Receptor-Mediated Pharmacological Activity

Many pharmacologically active substances elicit a specific physiological response by interacting with an element, known as a receptor, of the target cell. A receptor is a component, usually macromolecular, of an organism with which a chemical agent interacts in some specific fusion to case an action which leads to an observable biological effect. For purposes of the present invention, antibodies are not considered receptors.

The substances which are able to elicit the response, by specific interaction with a receptor site, are known as agonists. Typically, increasing the concentration of the agonist at the receptor site leads to an increasingly larger response, until a maximum response is achieved. A substance able to elicit the maximum response is known as a full agonist, and one which elicits only, at most, a lesser (but discernible) response is a partial agonist.

A pharmacological antagonist is a compound which interacts with the receptor without eliciting a response, and by doing so inhibits the receptor from responding to agonists. A competitive antagonist is one whose effect can be overcome by increasing the agonist concentration; a noncompetitive antagonist is one whose action is unaffected by agonist concentration. A sequestering antagonist is one which inhibits a ligand: receptor interaction by binding to the ligand in such a way that it can no longer bind the receptor. A competitive sequestering antagonist competes with the receptor for the ligand, whereas a competitive pharmacological antagonist competes with the ligand for the receptor.

Ligands are substances which bind to receptors, and thereby encompass both agonists and pharmacological antagonists. However, ligands exist which bind receptors, but which neither agonize nor antagonize the receptor. Ligands which activate (agonize) or inhibit (antagonize) the receptor are here collectively termed modulators. Some modulators change roles, acting as agonists or antagonists, depending on circumstances.

Natural ligands are those which, in nature, without human intervention, are responsible for agonizing or antagonizing a natural receptor. A natural ligand may be produced by the organism to which the receptor is native. A ligand native to a pathogen or parasite may bind to a receptor native to a host. Or a ligand native to a host may bind to a receptor native to a pathogen or parasite. All of these are natural ligands.

The clinical concept of drug antagonism is broader than the pharmacological concept, including phenomena that do not involve direct inhibition of agonist:receptor binding. A "physiological" antagonist could be a substance which directly or indirectly inhibits the production, release or transport to the receptor site of the natural agonist, or directly or indirectly facilitates its elimination (whether physical, or by modification to an inactive form) from the receptor site, or inhibits the production or increases the rate of turnover of the receptor, or interferes with signal transduction from the activated receptor.

A physiological antagonist of one receptor (e.g., an estrogen receptor) may be a pharmacological antagonist of another, e.g., a transcription factor. A physiological antagonist of one receptor may be a pharmacological agonist of another receptor, such as one which activates an enzyme which degrades the natural ligand of the first receptor.

Similarly, one may speak of a physiological agonist, which is a substance which directly or indirectly enhances the production, release or transport to the receptor site of the natural agonist, or directly or indirectly inhibits its elimination from the receptor site, or enhances the production or reduces the rate of turnover of the receptor, or in some way facilitates signal transduction from the activated receptor.

It follows that there are both "pharmacological" and "physiological" modulators.

A functional antagonist of a receptor is a substance which acts on a second receptor triggering a biological response which counteracts or inhibits the normal response to activation of the first receptor. Thus, a functional antagonist of one receptor may be a pharmacological agonist of another.

If a disease state is the result of inappropriate activation of a receptor, the disease may be prevented or treated by means of a physiological or pharmacological antagonist. Other disease states may arise through inadequate activation of a receptor, in which case the disease may be prevented by means of a suitable physiological or pharmacological agonist.

An important class of receptors are proteins embedded in the phospholipid bilayer of cell membranes. The binding of an agonist to the receptor (typically at an extracellular binding site) can cause an allosteric change at an intracellular site, altering the receptor's interaction with other biomolecules. The physiological response is initiated by the interaction with this "second messenger" (the agonist is the "first messenger") or "effector" molecule.

Enzymes are special types of receptors. Receptors interact with agonists to form complexes which elicit a biological response. Ordinary receptors then release the agonist intact. With enzymes, the agonists are enzyme substrates, and the enzymes catalyze a chemical modification of the substrate. Thus, enzyme substrates are "ligands". Enzymes are not necessarily integral membrane proteins; they may be secreted, or intracellular, proteins. Often, enzymes are activated by the action of a receptor's second messenger, or, more indirectly, by the product of an "upstream" enzymatic reaction.

Thus, drugs may also be useful because of their interaction with enzymes. The drug may serve as a substrate for the enzyme, as a coenzyme, or as an enzyme inhibitor. (An irreversible inhibitor is an "inactivator".) Drugs may also cause, directly or indirectly, the conversion of a proenzyme into an enzymes. Many disease states are associated with inappropriately low or high activity of particular enzymes.

The present invention may be used to identify both agonists and antagonists of receptors. It is not unusual for a relatively small structural change to convert an agonist into a pharmacological antagonist, or vice versa. Therefore, even if the drugs known to interact with a reference protein are all agonists, the drugs in question may serve as leads to the identification of both agonists and antagonists of the reference protein and of related proteins. Similarly, known antagonists may serve as drug leads, not only to additional antagonists, but to agonists as well.

Potency

The potency of an antagonist of a receptor may be expressed as an IC50, the concentration of the antagonist which causes a 50% inhibition of a receptor's binding or biological activity in an in vitro or in vivo assay system. A pharmaceutically effective dosage of an antagonist depends on both the IC50 of the antagonist, and the effective concentrations of the receptor and its clinically significant binding partner(s).

Potencies may be categorized as follows:

| Category | IC50 |
|---|---|
| Very Weak | >1 $\mu$ moles |
| Weak | 100 n moles to 1 $\mu$ mole |
| Moderate | 10 n moles to 100 n moles |
| Strong | 1 p mole to 10 n moles |
| Very Strong | <1 p mole |

Preferably, the antagonists identified by the present invention are in one of the four higher categories identified above, and are in any event more potent than any antagonist known for the protein in question at the time of filing of this application.

In a similar manner, the potency of an agonist may be quantified as the dosage resulting in 50% of its maximal effect on a receptor.

General Method

In the present invention, the biological activity of a test substance, as mediated by a particular receptor, in a particular organism, and thereof is predicted by:

(I) providing a panel of "Biokeys", the "Biokeys" having a differential ability to bind the receptor in the presence or absence of one or more ligands, said panel therefore being able to discriminate among two or more different receptor conformations,
(II) screening a set of two or more reference substances, which are known pharmacological agonists or antagonists of the receptor in one or more organisms and tissues, for the ability to alter the binding of the "Biokeys" to the receptor, thereby obtaining a reference "fingerprint", for each reference substance, which is an array of descriptors, each descriptor defining, qualitatively or quantitatively, the effect of the reference compound on the binding of a Biokey panel member to the receptor.
(III) The test compound is similarly screened for its ability to alter the binding of the "Biokeys" to the receptor, thereby obtaining a test fingerprint,
(IV) the similarity of the test fingerprint to each of the reference fingerprint to each of the reference fingerprints is determined, and
(V) the biological activity of the test substance in one or more target organisms, and in one or more target tissues thereof, is predicted on the basis of the biological activities of the reference substances therein, appropriately weighted by the similarity between the test substance and the reference substance.

The Biokey panel of step (I) is preferably obtained by screening the members of a combinatorial library for the ability to bind to (a) the unliganded receptor, and (b) a liganded receptor. In one embodiment, a combinatorial library is first screened against (a), and then either the whole library, or only the unliganded receptor-binding members, are screened against (b). In another embodiment, the whole library is screened against (a) and (b) simultaneously. It is also premissible to screen first against (b) and then against (a).

Preferably, the combinatorial library is an amplifiable combinatorial library, i.e., a library of nucleic acids or peptides. The members of the Biokey panel may be individual molecules, or mixtures of molecules with similar binding characteristics.

It will be appreciated that step (II) need only be performed once for a given receptor and that it is not necessary that all reference substances be fingerprinted simultaneously. Also, steps (II) and (III) may be interchanged.

In step (IV), similarity may be determined in a qualitative and subjective way, i.e., by "eyeballing" the fingerprints and judging from experience which is more similar, or in a quantitative and objective manner, using the similarity measures set forth infra.

Similarly, in step (V), the biological activity may be predicted in a qualitative and subjective way, or more quantitatively and objectively, by mathematically weighting each reference substance's activity scores by the calculated similarity of its fingerprint to the fingerprint of the test substance.

By way of example, peptides (BioKeys) that bind to the ER can be classified based on their ability to bind to the ER in the presence or absence of ER agonists. The different affinities of the peptides are due to alterations in receptor conformation following binding of an agonist. Since SERMs also uniquely alter receptor conformation, it is likely that they can affect the binding of the peptides from the different classes as well. Each agonist or SERM has associated pharmacological effects. For example, estrogen has stimulatory activity in breast and uterus, bone and the cardiovascular system. Likewise, tamoxifen is stimulatory in the uterus, bone and the cardiovascular system, but it has antagonistic effects in the breast. The pattern of BioKey binding to the ER in response to each compound could be matched with the pharmacological effect of each compound. Additionally, a comparison between BioKey fingerprints on ER α and β will supplement the information on agonist and antagonist activity and should be predictive of tissue specificity. New estrogen agonists and antagonists could then be screened and classified based on their BioKey binding pattern to ER α and β, and compounds with a desired tissue-specific activity could be more readily identified.

Hypothetical Table of a "BioKey Fingerprint" for a Hypothetical Nuclear Receptor

| | Compound A | Compound B | Compound C | Compound D | Compound E |
|---|---|---|---|---|---|
| BioKey 1 | + | + | + | + | + |
| BioKey 2 | + | + | + | + | - |
| BioKey 3 | + | + | + | - | - |
| BioKey 4 | + | + | - | - | - |
| BioKey 5 | + | - | - | - | - |

Hypothetical Table of Pharmacological Effects of Receptor Modulating Compounds

| | Breast | Uterus | Bone | Cardiovascular |
|---|---|---|---|---|
| Compound A | + | + | + | + |
| Compound B | - | + | - | + |
| Compound C | - | + | - | - |
| Compound D | + | + | - | - |
| Compound E | + | + | - | + |

For example, using the above tables, compounds with unknown pharmacological effects could be characterized by "BioKey fingerprinting" to predict their activity in various tissues. A compound X, that had a fingerprint similar to compound A, would be predicted to have pharmacological effects similar to compound A. The binding or lack of binding of a specific BioKey with the receptor could indicate activity in a specific tissue type. In the above examples, binding of BioKey 1 to the receptor in the presence of a compound could indicate activity in the uterus. Whereas binding of BioKey 5 to the receptor in the presence of compound could indicate activity in bone.

Substances

A "substance" may be either a pure compound, or a mixture of compounds. Preferably it is at least substantially pure, that is, sufficiently pure enough to be acceptable for clinical use. If it is a mixture, then it comprises at least an effective amount (i.e., able to give rise to a detectable biological response in a biological assay) of a biologically active compound, or it comprises a substantial amount of a compound which is suspected of being biologically active and is suitable as a drug lead if so active.

Test substances and Drug Leads

A test substance comprises an effective amount of a compound, which is a member of a structural class which is generally suitable, in terms of physical characteristics (e.g., solubility), as a source of drugs and which is not known to have the pharmacological activity of interest. A drug lead is a former test substance which has either been predicted to have desirable pharmacological activity, or in fact has been shown to have such activity, and which therefore could serve effectively as a starting point for the design of analogues and derivatives which are useful as drugs. The "drug lead" may be a useful drug in its own right, or it may be a substance which is deficient as a drug because of inadequate potency or undesirable side effects. In the latter case, analogues and derivatives are sought which overcome these deficiencies. In the former case, one seeks to improve the already useful drug.

Such analogues and derivatives may be identified by rational drug design, or by screening of combinatorial or noncombinatorial libraries of analogues and derivatives.

Preferably, a drug lead is a compound with a molecular weight of less than 1,000, more preferably, less than 750, still more preferably, less than 600, most preferably, less than 500. Preferably, it has a computed log octanol-water partition coefficient in the range of -4 to +14, more preferably, -2 to +7.5.

A small organic compound library is a library of compounds each of which has a molecular weight of less than 1000, and which are not peptides or nucleic acids.

SERMs from distinct structural classes may produce fingerprints unique to its class. In addition SERMs from different classes that have similar biological activities should produce similar fingerprints. Numerous SERMs that have been identified can be fingerprinted in our system. These include steroidal antiestrogens such as the ICI compounds 164,384 and 182,780, and non-steroidal compounds such as the benzothiophene derivative Raloxifene, and triphenylethylene derivatives Toremifene, Droloxifene, TAT-59 and Idoxifene. We have found that the steroidal SERMs will produce fingerprints distinct from the non-steroidal SERMs (see Example 2). Steroidal compounds such as the ICI compounds have been categorized as pure anti-estrogens, in that there is no well documented evidence of any estrogenic effects in response to these compounds. These fingerprints may be similar to the unliganded (inactive) receptor, or they may indicate that a co-repressor is bound more tightly or that a co-activator is completely inhibited from binding.

The fingerprinting system should be useful for identifying agonistic and antagonistic components from complex mixtures. The prescription drug Premarin is used for the treatment of post-menopausal symptoms. It is a complex mixture derived from the urine of pregnant mares. The active components of this mixture are not known. Fractionation of Premarin followed by fingerprinting of the individual components would indicate which of the components play an active role in modulating estrogen receptor function. It is also likely that components of Premarin interact with other nuclear receptors such as the progesterone receptor. The effect of these components could be determined as well.

## Reference Ligands

A reference ligand is a substance which is a ligand for a target receptor. Preferably, it is a pharmacological agonist or antagonist of a target receptor protein in one or more target tissues of a target organism. However, a reference ligand may be useful, even if it is not an agonist or antagonist, if it alters the conformation of its receptor, e.g., such that at least some Biokeys which bound the unliganded receptor do not bind as well, or bind better, the liganded receptor. Preferably, a reference ligand has a differential effect on Biokeys, so that Biokeys may be differentiated on the basis of their interaction with the receptor in the presence of the reference ligand. A reference ligand may be an agonist of one receptor and an antagonist of another. It may also be agonist of a receptor in one tissue and an antagonist of the same receptor in another tissue, or in another organism.

The reference ligand may be, but need not be, a natural ligand of the receptor.

The reference ligands may, but need not, satisfy some or all of the desiderata set forth above for test substances and drug leads.

If a test substance from one screening becomes a drug lead, and that compound, or an analogue thereof, is ultimately found to mediate the biological activity of at least one receptor in at least one tissue of at least one organism, it may be used as a reference ligand in subsequent screenings of other test substances, and in redefining the Biokey panel.

## Reference Conformation

When a target receptor is in an unliganded state, it has a particular conformation, i.e., a particular 3-D structure. When the receptor is complexed to a ligand, the receptor's conformation changes. If the ligand is a pharmacological agonist, the new conformation is one which interacts with other components of a biological signal transduction pathway, e.g.; transcription factors, to elicit a biological response in the target tissue. If the ligand is a pharmacological antagonist, the new conformation is one in which the receptor cannot be activated by one or more agonists which otherwise could activate that receptor.

Each of the conformations of a target receptor which is used as a binding target in a binding array is considered a reference conformation.

It may be that two different ligands will coincidentally cause a receptor to assume the same conformation. However, for the purpose of this invention, those will be considered different reference conformations because different ligands are involved.

## Biokeys

For the purpose of the present invention, Biokeys are substances whose ability to bind to a target receptor in the presence or absence of one or more reference ligands for that receptor can be used to differentiate the reference ligands, and ultimately to calculate the degree of similarity between a test substance (having an assayable effect on the binding of the Biokeys to the target receptor protein) and reference substances (likewise having an assayable effect as such binding, but whose effect on biological activity of the receptor protein in target organisms and tissues of interest is also known).

Preferably, Biokeys are members of a combinatorial library, and in particular an amplifiable combinatorial library such as a peptide or nucleic acid library. The library may then be screened for binding to various receptor conformations.

Biokeys need not themselves be suitable as drug leads.

Biokey Panel

For the purpose of fingerprinting the reference and test substances, a representative selection of Biokeys are collected into a panel. If only a single reference ligand is known for a receptor, the panel could include one or more representative members of each of at least two of the following binding classes:

| Class | Binds UL-R | Change in Binding (Effect of Ligand) |
|---|---|---|
| A | + | + |
| B | + | - |
| C | + | 0 |
| D | - | 0 |
| E | - | + |

Thus classes A, B and C bind unliganded receptor (UL-R), but the ligand increases the binding of A, decreases the binding of B, and has no effect on the binding of C. Classes D and E do not bind the UL-R. The ligand causes E, but not D, to bind the receptor.

Instead of only two of the above, the panel can include representative members of three, four or all five of the classes, if Biokeys having the appropriate properties can be identified.

The above classes look at binding in only a qualitative manner. However, it would be possible to differentiate between strong and weak binders of UL-R, and between large and small changes in binding as the result of the ligand. If desired, one could draw even finer divisions, e.g.; strong vs. moderate vs. weak, etc.

If more than one ligand is available, the combinatorial possibilities are increased, and, if suitable Biokeys can be identified, the panel can be expanded appropriately.

For example, with two ligands, the following possibilities could exist

| Biokey | UL-R | Ligand A | Ligand B |
|---|---|---|---|
| Z | + | + | + |
| Y | + | + | 0 |
| X | + | + | - |
| W | + | 0 | + |
| V | + | 0 | 0 |
| U | + | 0 | - |
| T | + | - | + |
| S | + | - | 0 |
| R | + | - | - |
| Q | - | 0 | 0 |
| P | - | + | 0 |
| O | - | 0 | + |
| N | - | + | + |

And one could discriminate further, e.g., for Z-1, the effect of A is greater than that of B, for Z-2, the reverse, and for Z-3, the effects are equal.

Preferably, one, two, three, four, five or more reference ligands are used to define the Biokey panel.

It is not necessary that a particular binding class be represented by only a single Biokey. Instead, it may be represented by a mixture of two or more Biokeys, and indeed the mixture may correspond to all of the Biokeys in the Biokey library which satisfied the binding criteria for the class in question.

The members of the Biokey panel are chosen with a view to maximizing the discriminatory power of the panel. For example, to take an extreme case, if two members of the panel have identical binding properties, vis-a-vis, all the available reference conformations of the receptor, then one of these members is redundant. While including it in the panel does no harm, it needlessly increases the costs of the screening.

The similarity of any pair of potential panel members may be determined using the similarity measures set forth infra. The overall diversity of a given panel may be determined by computing all of the pairwise dissimilarities. For a given size panel, extracted from a given library, one may seek to maximize the overall diversity of effect on biological activity. Or one may seek to determine, for a set of binding members from a library, what is the size and composition of the subject which maximizes the ratio of the overall diversity to the number of members.

The number of panel-based descriptors in the fingerprint will normally be equal to the number of members in the panel. The optimal number of members depends on the number of reference substances, and the ability of the panel to differentiate them. The larger the number of reference substances, and the larger the number of target organisms and tissues in which the biological activity of the reference substance is to be predicted, the larger the panel should be. Typically, there will be 2, 3, 4, 5, 6, 7, 8, 9, or 10 panel members. More members may be used, but the cost of the assay increases, without necessarily providing a commensurate increase in the predictive power of the data.

Reference substances

Reference substances are known pharmacological agonists or antagonists for the receptor in question, and have a known or ascertainable biological activity in one or more organisms and/or tissues.

Typically, for a given receptor, one, two, three, four, five or more reference substances will be fingerprinted.

"Fingerprinting" of Test and Reference Substances

Each test substance will be characterized by a plurality of descriptors (the "fingerprint") by which it may be compared to reference substances.

These reference substances may be the particular reference ligands used to define the Biokey panel, but are not limited to those reference ligands. Thus, in example 1, only estradiol was used to define the five classes of peptides, but the reference substances were estradiol, estriol, tamoxifen, nafoxidine and clomiphene. The use of estradiol was not critical; the reference substances need not include any of the reference ligands used to define the BioKey panel.

The reference substances must be pharmacological agonists or antagonists in at least one organism and tissue, while the reference ligands are not so limited.

For the purpose of the present invention, a plurality of descriptors must refer to the effect of the test substance on the binding of a member of the Biokey panel to a reference conformation, e.g., unliganded receptor X, receptor X/ligand A, receptor X/ligand B, unliganded receptor Y, receptor Y/ligand C, etc. Note that in this context, the term "member" may refer to a mixture of Biokeys of the same binding class. The descriptor may be qualitative (binds vs. nonbinds; increases vs. decreases vs. no effect, etc.) or quantitative. Preferably, at least 2-10 Biokey-based descriptors are used.

The test substance may additionally be characterized by other descriptors, such as structural descriptors, known in the art. Preferably, at least 5-10 different reference substances are "fingerprinted".

The reference substances will be characterized in a similar manner to the test substances, so that their descriptors may be "paired" with the test substance descriptors in such a manner that the degree of similarity may be calculated.

When fingerprinting a given reference or test substance, it may be screened simultaneously against all panel members, or individual panel members (or subsets of panel members) may be tested separately. Also, all reference substances may be screened simultaneously against a given receptor/panel member combination, or the reference substances may be screened individually. The same is true of the screening of the test substances. The test substances may be screened after, before or simultaneously with the reference substances.

Descriptors

A "descriptor" (also known as a parameter, character, variable, or variate) is a numerically expressed characteristic of a compound (which may be a protein, or a protein ligand), which helps to distinguish that compound from others. A descriptor value need not be absolutely specific to a compound to be useful. The characteristics may be pure structural characteristics (as in a "structural descriptor") or they may refer to the compound's interaction with other compounds. "Paired Descriptors" are descriptors of the same property as measured in two different molecules. A "descriptor array", "list", or "set" is an array, list or set whose elements are different descriptors for the same molecule. Such an array, list or set is referred to herein as a "fingerprint".

A plurality of paired descriptors for two compounds may be used to calculate a similarity between the two compounds.

Similarity Measures

A similarity measure or coefficient quantifies the relationship between two individuals (compounds), given the values of a set of variates (descriptors) common to both. Similarity coefficients are usually defined to take values in the range of 0 to 1.

One commonly used measure of similarity is the product moment correlation coefficient. Its correlation is unity whenever two profiles are parallel, regardless of how far apart they are in level. Two profiles may have correlation of +1 even if they are not parallel, provided that the two sets of scores are linearly related.

For binary descriptors, the simplest measure of similarity is the simple matching coefficient

$$s_{ij} = \frac{\text{number of matches}}{\text{number of comparisons}}$$

The Jaccard or Sneath coefficient modifies the simple matching coefficient by ignoring bits which in both i and j are zero, i.e., by ignoring negative matches (mutual absences). In other words, it is obtained by dividing the number of bits which are set in both descriptor bit strings, and dividing by the total number of bits set in either descriptor string. It is also called the unweighted Tanimoto coefficient.

The weighted Tanimoto coefficient for descriptors k and individuals i and j is:

$$S_{ij} = \frac{\sum_k w_k x_{ik} x_{jk}}{\sum_k w_k x_{ik} + \sum_k w_k x_{jk} - \sum_k w_k x_{ik} x_{jk}}$$

Gower has defined a general similarity coefficient which can be used for binary, qualitative, and quantitative data:

$S_{ij}=\sum^{p} s_{ijk} / \sum^{p} w_{ijk}$ for individuals i and j and descriptor k.

k=1 k=1

$W_{ijk}$ is set to 1 if the comparison is valid for variable k, and to 0 otherwise. If $w_{ijk}$=0, then $s_{ijk}$ is 0. For binary data, $w_{ijk}$ and $s_{ijk}$ are both 0 if the variable is negative in both individuals. The $s_{ijk}$ is positive only if the binary variable is positive for both individuals. For qualitative data, $s_{ijk}$=1 if the individuals are the same for the kth character, and $s_{ijk}$=0 if they differ. For quantitative data, $s_{ijk}=1-|X_{ik}-X_{jk}|/R_k$ where $X_{ik}$ is the value of descriptor k for individual i, and $R_k$ is the total range of variable k.

Descriptors may be quantitative or qualitative. Quantitative descriptors may be integers or real numbers. Qualitative descriptors divide the data into categories which may be, but need not be, expressible as having relative magnitudes. Binary descriptors are a special case of qualitative descriptors, in which there are just two categories, typically representing the presence or absence of a feature. Qualitative data for which the variates have several levels may be treated like binary data with each level of a variate being regarded as a single binary variable (i.e., an eight level variate expressed as eight bits). Or the levels may be numbered sequentially (i.e., an eight level variable expressed as three bits).

A set of n-descriptors defines an n-dimensional descriptor space; each compound for which a descriptor set is available may be said to occupy a point in descriptor space. The dissimilarity of two compounds may be expressed as a distance between the two points which they occupy in descriptor space.

A distance measure is a similarity measure which is also a metric, i.e., satisfies the conditions (i) d(x,y) ≥0; and d(x,y)=0 if x=y; (ii) d(x,y)+d(y,x); and (iii) d (x, z) +d (y, z) ≥d (x, y) (the metric or triangular inequality) . Of course, the greater the distance, the less the similarity.

Distances may be calculated on the basis of any of a variety of distance measures known in the statistical arts.

The most commonly used distance measure is the Euclidean metric:

$$d_{ij} = \left(\sum_k (X_{ik} - X_{jk})^2\right)^{½}$$

It corresponds most closely to our intuitive sense of distance.

The absolute, city block, or Manhattan metric is

$$d_{ij}=\sum_{k}|X_{ik}-X_{jk}|$$

Its rationale is that if the variables have scale units of equal value, the entities should have the same distance whether two units apart on each of two variables, or one unit apart on one and three on the other.
The "cosine theta" distance is the cosine of the angle between the vector from the origin to point $X_{ik}$ and the vector from the origin to point $X_{jk}$.
A generalized distance measure is the Minkowski metric:

$$d_{ij}=(\sum_{k}|X_{ik}-X_{jk}|^{r})^{1/r}$$

which is a Euclidean metric for r=2 and a city block metric for r=1.
The Mahalonobis distance measure ($D^2$) is of the form

$$d_{ij}=(X_i-X_j)'\ \Sigma^{-1}(X_i-X_j)$$

where Σ is the pooled-within-groups variance-covariance matrix, and $X_i$ and $X_j$ are the vectors of scores for entities i and j. The Mahalanobis distance allows for correlations between variables; if the variables are uncorrelated, $D^2$ is equivalent to Euclidean distance measured using standard variables.
The Canberra metric, given below, has the advantage of being unaffected by the range of the variable:

$$d(i,j) = \sum_{k} (|X_{jk} - X_{ik}|)/(X_{ik} + X_{jk})).$$

A modified form, which accommodates negative states, is

$$d(i,j) = \sum_{k} (|X_{jk} - X_{ik}|/(|X_{ik}| + |X_{jk}|)).$$

The Calhoun distance uses only rank orders; for molecules i and j, the distance is the proportion of the entire set (excluding i and j) that have descriptor states intermediate between that for i and that for j for one or more of the descriptors k.
A distance measure may be transformed into a similarity measure by any of a variety of transformations that convert a non-negative number to the range 0..1, e.g.,

$$S_{ij}=1/(1+d_{ij})$$

A similarity measure may be converted into a distance by, e.g., $d_{ij} = 1-s_{ij}$.
If there is a theoretical maximum distance ($d_{tmax}$), based on the theoretically possible ranges for each of the component descriptors, the similarity may be expressed as

$$S_{ij}=1-(d_{ij}/d_{tmax})$$

Alternatively, one may calculate the distances between all pairs, and then use the actual maximum distance ($d_{amax}$) :

$$S_{ij}=1-(d_{ij}/d_{amax})$$

Instead of using the ratio of the actual distance to the actual or theoretical maximum distance, one may express $S_{ij}$ as the fraction of the pairs for which the distance is greater than or equal to $d_{ij}$. This is a measure of relative similarity.

Descriptors may be weighted (or otherwise transformed) for any of several reasons, including:

(a) to reflect the perceived value of the descriptor for determining whether two proteins will be modulated by structurally similar drugs;
(b) to reflect the perceived reliability of the descriptor data;
(c) to correct for differences in scale between descriptors, so that a descriptor does not dominate a similarity or distance calculation merely because its values are of higher magnitude or are spread over a greater range; and
(d) to correct for correlations between descriptors.

The raw descriptor values may be, but need not be, transformed prior to use in calculating distances. Typical transformations are (a) presence (1)/absence (0), (b) 1n(x+1), (c) frequency in sample, (d) root, and (e) relative range, i.e., (value-min) / (max-min).

The raw descriptor values may be standardized (normalized) to have zero mean ($x'=x-\mu_x$) and/or unit variance ($x'=x/\sigma_x$)' possibly both ($x'=(x-\mu_x)/\sigma x$) or they be standardized (unitized) to fall into the range 0 to 1.

Descriptor weights may be adjusted empirically on the basis of specially designed test sets. A training set of proteins is identified. Descriptors are evaluated for each protein in the set. A training set of compounds, including are also tested against each compound in the set. These compounds are chosen so that, for any protein in the set, there is at least one compound which is an agonist or antagonist for it. A neural net, with the descriptor weights as inputs, is used to predict the activity of each compound against each protein, using the calculated protein similarities. For example, it will calculate the similarity of protein x to all other proteins, then treat the activities of the compounds against the other proteins as "knowns" and use it to predict the activity of the compounds against protein x. This is done repeatedly, with each protein taking on the role of protein x, in turn.

The coefficient of variation may be useful in comparing descriptors; it is the standard deviation divided by the mean. If there is no information available about the ultimate significance of a descriptor, one may give a greater weight to descriptors which have a larger CV and hence a more uniform distribution.

It must be emphasized that we do not require use of weighted descriptors, let alone of any particular method of deriving weights.

It is likely that some degree of correlation will exist among the descriptors. Standard mathematical methods, such as cluster analysis, principal components analysis, or partial least squares analysis, may be used to determine which descriptors are strongly correlated and to replace them with a new descriptor which is a weighted sum of the original correlated descriptors. One may alternatively choose (perhaps randomly) one of each pair of highly correlated descriptors and simply prune it, thereby reducing the amount of data which must be collected.

One way of correcting for correlation among the descriptors is for each descriptor m, calculate the average of its squared correlation coefficients with all descriptors n (including m=n, for which the coefficient is necessarily unity), and subtract this number from one to obtain a weight representing the fraction of the variation in descriptor m which is not explained by the "average" descriptor n. With this "average $r^2$" method, if we have four descriptors, and two are perfectly correlated to each other, and the descriptors are otherwise completely uncorrelated, the correlated descriptors will have weights of 0.5 each, and the other two will have weights of 1.0 each.

The diversity of a set of compounds, as measured by a set of descriptors, may be calculated in several ways.

A purely geometric method involves assuming that each compound sweeps out a hypersphere in descriptor space, the hypersphere having a radius known as the similarity radius. The total hypervolume in descriptor space of points within a unit similarily radius of one or more of the compounds is calculated. This is compared to the hypervolume achievable if none of hypersphere's overlap; i.e., to n * volume of a single hypersphere, where n is the number of compounds in the set. The swept hypervolume may be determined exactly, or by Monte Carlo methods. The ratio of the swept hypervolume to the maximum hypervolume is a measure of compound set diversity, ranging from 1 (maximum) to 1/n (minimum).

Another approach is to calculate all of the pairwise distances between compounds in descriptor space. The mean distance is a measure of diversity. If desired, this can be scaled by calculating the ratio of the mean distance to the maximum theoretical distance.

A third approach is to apply cluster analysis to the set of compounds. The method used should be one which does not set the number of clusters arbitrarily, but rather decides the number based on some goodness-of-fit criterion. The resulting number of cluster is a measure of diversity, as is the ratio of the number of clusters to the number of compounds.

One may calculate a measure of disorder for a descriptor as

$$H(k) = - \sum_{g=1}^{m_k} P_{kg} \ln P_{kg}$$

where $m_k$ is the number of different states in descriptor k, and $P_{kg}$ is the observed proportion of individuals exhibiting state g for descriptor k. For uncorrelated descriptors, the sum of H(k) for all k is a measure of overall diversity. Standard techniques may be used to correct for correlation.

Target Receptor

The target receptor may be a naturally occurring substance, or a subunit or domain thereof, from any natural source, including a virus, a microorganism (including bacterial, fungi, algae, and protozoa), an invertebrate (including insects and worms), or the normal or cancerous cells of a vertebrate (especially a mammal, bird or fish and, among mammals, particularly humans, apes, monkeys, cows, pigs, goats, llamas, sheep, rats, mice, rabbits, guinea pigs, cats and dogs). (Usually it is a protein; it may be a nucleic acid. References to proteins apply, mutatis mutandis, to nucleic acids, lipids, carbohydrates and other macromolecules which can act as receptors.) Alternatively, the receptor protein may be a modified form of a natural receptor. Modifications may be introduced to facilitate the labeling or immobilization of the target receptor, or to alter its biological activity (An inhibitor of a mutant receptor may be useful to selectively inhibit an undesired activity of the mutant receptor and leave other activities substantially intact). In the case of a protein, modifications include mutation (substitution, insertion or deletion of a genetically encoded amino acid) and derivatization (including glycosylation, phosphorylation, and lipidation).

A target receptor may be, inter alia, a glyco-, lipo-, phospho-, or metalloprotein. It may be a nuclear, cytoplasmic, membrane, or secreted protein. It may, but need not, be an enzyme.

The target receptor, instead of being a protein, may be a macromolecular nucleic acid, lipid or carbohydrate. If a nucleic acid, it may be a ribo- or a deoxribonucleic acid, and it may be single or double stranded. It may, but need not, have enzymatic activity.

The target receptor need not be a single macromolecule, rather, it may be a complex of a macromolecule with one or more additional molecules, especially macromolecules. Examples includes ribosomes (RNA:protein complexes), polysomes (mRNA:ribosome complexes), and chromatin (DNA:protein complexes). For use of polysomes as binding molecules (or as display systems), see Kawasaki, USP 5,643,768 and 5,658,754; Gersuk, et al., Biochem. Biophys. Res. Comm. 232:578 (1997); Mattheakis, et al., Proc. Nat. Acad. Sci. USA, 91:9022-6 (1994).

The known binding partners (if any) of the target receptor may be, inter alia, proteins, oligo- or polypeptides, nucleic acids, carbohydrates, lipids, or small organic or inorganic molecules or ions.

The functional groups of the receptor which participate in the ligand-binding interactions together form the ligand binding site, or paratope, of the receptor. Similarly, the functional groups of the ligand which participate in these interactions together form the epitope of the ligand.

In the case of a protein, the binding sites are typically relatively small surface patches. The binding characteristics of the protein may often be altered by local modifications at these sites, without denaturing the protein.

While it is possible for a chemical reaction to occur between a functional group on a receptor and one on a ligand, resulting in a covalent bond, receptor protein-ligand binding normally occurs as a result of the aggregate effects of several noncovalent interactions. Electrostatic interactions include salt bridges, hydrogen bonds, and van der Waals forces.

What is called the hydrophobic interaction is actually the absence of hydrogen bonding between nonpolar groups and water, rather than a favorable interaction between the nonpolar groups themselves. Hydrophobic interactions are important in stabilizing the conformation of a receptor protein and thus indirectly affect ligand binding, although hydrophobic residues are usually buried and thus not part of the binding site.

The receptor may have more than one paratope and they may be the same or different. Different paratopes may interact with epitopes of different binding partners. An individual paratope may be specific to a particular binding partner, or it may interact with several different binding partners. A receptor can bind a particular binding partner through several different binding sites. The binding sites may be continuous or discontinuous (e.g., vis-a-vis the primary sequence of a receptor protein).

A list of agonists, antagonists, radioligands and effectors for many different receptors appears in Appendix I of King, Medicinal Chemistry: Principles and Practice, pp. 290-294 (Royal Soc'y Chem. 1994). Appendix II lists blockers for various ion channels (which are another special type of receptor). Some receptors, and their agonists and/or antagonists, are listed in Table A.

Any nuclear receptor, such as receptors for progestins, androgens, glucocorticoids, thyroid hormones, retinoids, vitamin D3 and mineralocorticoids could be used in this fingerprinting system. Affinity selection of peptide libraries could be used to identify peptide sequences that bind in the presence or absence of agonist as described above. The peptides could then be used in the manner described above to classify and characterize modulators of the receptor's activity. As described above, components of Premarin are likely to interact with the progesterone receptor. A system for fingerprinting the progesterone receptor may be developed to test for active components of Premarin.

As an example of a non-protein receptor, we cite DNA. DNA can undergo conformational changes when it is bound for example, by a transcription factor or small molecule. For example, the antitumor agent cisplatin binds to and alters the structure of DNA. The altered structure attracts a cellular protein containing an HMG box (high mobility group). The protein is believed to sterically block the repair of the cisplatin lesion on the DNA and contribute to the effectiveness of cisplatin in the treatment of certain types of cancer. BioKeys could be identified that bind specifically to DNA in certain conformations. These BioKeys could be used to identify conformational changes that take place in the DNA upon binding of a small molecule or protein.

## Target Organism

A purpose of the present invention is to predict the biological activity in one or more target tissues, as hereafter defined, of a target organism.

The target organism may be a plant, animal, or microorganism.

In the case of a plant, it may be an economic plant, in which case the drug may be intended to increase the disease, weather or pest resistance, alter the growth characteristics, or otherwise improve the useful characteristics or mute undesirable characteristics of the plant. Or it may be a weed, in which case the drug may be intended to kill or otherwise inhibit the growth of the plant, or to alter its characteristics to convert it from a weed to an economic plants. The plant may be a tree, shrub, crop, grass, etc. The plant may be an algae (which are in some cases also microorganisms), or a vascular plant, especially gymnosperms (particularly conifers) and angiosperms. Angiosperms may be monocots or dicots. The plants of greatest interest are rice, wheat, corn, alfalfa, soybeans, potatoes, peanuts, tomatoes, melons, apples, pears, plums, pineapples, fir, spruce, pine, cedar, and oak.

If the target organism is a microorganism, it may be algae, bacteria, fungi, or a virus (although the biological activity of a virus must be determined in a virus-infected cell) . The microorganism may be human or other animal or plant pathogen, or it may be nonpathogenic. It may be a soil or water organism, or one which normally lives inside other living things.

If the target organism is an animal, it may be a vertebrate or a nonvertebrate animal. Nonvertebrate animals are chiefly of interest when they act as pathogens or parasites, and the drugs are intended to act as a biocidic or biostatic agents. Nonvertebrate animals of interest include worms, mollusks, and arthropods.

The target organism may also be a vertebrate animal, i.e., a mammal, bird, reptile, fish or amphibian. Among mammals, the target animal preferably belongs to the order Primata (humans, apes and monkeys), Artiodactyla (e.g., cows, pigs, sheep, goats, horses), Rodenta (e.g., mice, rats) Lagomorpha (e.g., rabbits, hares), or Carnivora (e.g., cats, dogs). Among birds, the target animals are preferably of the orders Anseriformes (e.g., ducks, geese, swans) or Galliformes (e.g., quails, grouse, pheasants, turkeys and chickens). Among fish, the target animal is preferably of the order Clupeiformes (e.g., sardines, shad, anchovies, whitefish, salmon).

## Target Tissues

The term "target tissue" refers to any whole animal, physiological system, whole organ, part of organ, miscellaneous tissue, cell, or cell component (e.g., the cell membrane) of a target animal in which biological activity may be measured.

Routinely in mammals one would chose to compare and contrast the biological impact on virtually any and all tissues which express the subject receptor protein. The main tissues to use are: brain, heart, lung, kidney, liver, pancreas, skin, intestines, adrenal glands, breast, prostate, vasculature, retina, cornea, thyroid gland, parathyroid glands, thymus, bone marrow etc.

Another classification would be by cell type: B cells, T cells, macrophages, neutrophils, eosinophils, mast cells, platelets, megakaryocytes, erythrocytes, bone marrow stomal cells, fibroblasts, neurons, astrocytes, neuroglia, microglia, epithelial cells (from any organ, e.g. skin, breast, prostate, lung, intestines etc), cardiac muscle cells, smooth muscle cells, striated muscle cells, osteoblasts, osteocytes, chondroblasts, chondrocytes, keratinocytes, melanocytes, etc.

The "target tissues" include those set forth in Table B. Of course, in the case of a unicellular organism, there is no distinction between the "target organism" and the "target tissue".

In Vitro vs. In Vivo Assays

The term "in vivo" is descriptive of an event, such as binding or enzymatic action, which occurs within a living organism. The organism in question may, however, be genetically modified. The term "in vitro" refers to an event which occurs outside a living organism. Parts of an organism (e.g., a membrane, or an isolated biochemical) are used, together with artificial substrates and/or conditions. For the purpose of the present invention, the term in vitro excludes events occurring inside or on an intact cell, whether of a unicellular or multicellular organism.

In vivo assays include both cell-based assays, and organismic assays. The term cell-based assays includes both assays on unicellular organisms, and assays on isolated cells or cell cultures derived from multicellular organisms. The cell cultures may be mixed, provided that they are not organized into tissues or organs. The term organismic assay refers to assays on whole multicellular organisms, and assays on isolated organs or tissues of such organisms.

Biological Assays

While a major purpose of the invention is to minimize the need for biological assays, they cannot be altogether avoided. In order to predict the biological activity of a substance, one must know the biological activities of a reasonable number of reference substances.

A biological assay measures or detects a biological response of a biological entity to a substance. The present invention is concerned with responses which are, at least in part, mediated by a receptor.

The biological entity may be a whole organism, an isolated organ or tissue, freshly isolated cells, an immortalized cell line, or a subcellular component (such as a membrane; this term should not be construed as including an isolated receptor). The entity may be, or may be derived from, an organism which occurs in nature, or which is modified in some way. Modifications may be genetic (including radiation and chemical mutants, and genetic engineering) or somatic (e.g., surgical, chemical, etc.). In the case of a multicellular entity, the modifications may affect some or all cells. The entity need not be the target organism, or a derivative thereof, if there is a reasonable correlation between bioassay activity in the assay entity and biological activity in the target organism.

The entity is placed in a particular environment, which may be more or less natural. For example, a culture medium may, but need not, contain serum or serum substitutes, and it may, but need not, include a support matrix of some kind, it may be still, or agitated. It may contain particular biological or chemical agents, or have particular physical parameters (e.g., temperature), that are intended to nourish or challenge the biological entity.

There must also be a detectable biological marker for the response. At the cellular level, the most common markers are cell survival and proliferation, cell behavior (clustering, motility), cell morphology (shape, color), and biochemical activity (overall DNA synthesis, overall protein synthesis, and specific metabolic activities, such as utilization of particular nutrients, e.g., consumption of oxygen, production of $CO_2$, production of organic acids, uptake or discharge of ions) .

The direct signal produced by the biological marker may be transformed by a signal producing system into a different signal which is more observable, for example, a fluorescent or clorimetric signal.

The entity, environment, marker and signal producing system are chosen to achieve a clinically acceptable level of sensitivity, specificity and accuracy.

Reference substances should be tested in the appropriate assays relevant to the tissue distribution of the targeted receptor. For instance, for the estrogen receptor which is expressed in breast epithelium, liver mesenchymal cells, osteoclasts and uterine epithelium (among others) appropriate assays would include, among others, breast and uterine epithelial cell proliferation, osteoclast apoptosis, and hepatocyte production of lipids such as triglycerides and cholesterol and lipoproteins such as high density lipoproteins and low density lipoproteins.

If one were to utilize the androgen receptor which is expressed in, among others, prostate epithelium, hepatocytes, striated muscle cells, then one would might chose to carry out assays of the reference substance set for, among others, prostate hypertrophy, hyperplasia or prostate epithelial cell proliferation, muscle cell hyperplasia or hypertrophy and heptotoxicity etc.

As another example, if one were to utilize the beta-2-adrenergic receptor, which is expressed in, among others, the heart, brain and peripheral vasculature, then one may chose to test reference substances in cardiac function assays (such as cardiac rate and eletrocardiographic changes), assays for their impact on blood pressure and assays to evaluate their impact on neuronal activity within the central nervous system.

Preliminary Screening Assays

The invention contemplates three occasions for preliminary screening:

(a) screening for potential "BioKeys", using a known receptor and one or more known pharmacological modulators of the receptor (see General Method step (I)),

(b) screening reference compounds, having a known receptor-mediated bioactivity using a known receptor and an established BioKey panel, to obtain reference fingerprints (see General Method, step (II), and

(c) screening test compounds for their ability to alter the binding of a panel of BioKeys to the receptor, thereby obtaining a test fingerprint (see General Method, step (III)).

The same or different screening methods may be used on each occasion.

Preliminary, screening assays will typically be either in vitro (cell-free) assays (for binding to an immobilized receptor) or cell-based assays (for alterations in the phenotype of the cell). They will not involve screening of whole multicellular organisms, or isolated organs. The comments on biological assays apply mutatis mutandis to preliminary screening cell-based assays.

In a preferred cell-based assay, the receptor is functionally connected to a signal (biological marker) producing system, which may be endogenous or exogenous to the cell.

"Zero-Hybrid" Systems

In these systems, the binding of a peptide to the target protein results in a screenable or selectable phenotypic change, without resort to fusing the target protein (or a ligand binding moiety thereof) to an endogenous protein. It may be that the target protein is endogenous to the host cell, or is substantially identical to an endogenous receptor so that it can take advantage of the latter's native signal transduction pathway. Or sufficient elements of the signal transduction pathway normally associated with the target protein may be engineered into the cell so that the cell signals binding to the target protein.

"One-Hybrid" Systems

In these systems, a chimera receptor, a hybrid of the target protein and an endogenous receptor, is used. The chimeric receptor has the ligand binding characteristics of the target protein and the signal transduction characteristics of the endogenous receptor. Thus, the normal signal transduction pathway of the endogenous receptor is subverted.

Preferably, the endogenous receptor is inactivated, or the conditions of the assay avoid activation of the endogenous receptor, to improve the signal-to-noise ratio.

See Fowlkes USP 5,789,184 for a yeast system.

Another type of "one-hybrid" system combines a peptide: DNA-binding domain fusion with an unfused target receptor that possesses an activation domain.

"Two-Hybrid" System

In a preferred embodiment, the cell-based assay is a two hybrid system. One member of a peptide ligand: receptor binding pair is expressed as a fusion to a DNA-binding domain (DBD) from a transcription factor (this fusion protein is called the "bait"), and the other is expressed as a fusion to a transactivation domain (TAD) (this fusion protein is called the "fish", the "prey", or the "catch"). The transactivation domain should be complementary to the DNA-binding domain, i.e., it should interact with the latter so as to activate transcription of a specially designed reporter gene that carries a binding site for the DNA-binding domain. Naturally, the two fusion proteins must likewise be complementary.

This complementarity may be achieved by use of the complementary and separable DNA-binding and transcriptional activator domains of a single transcriptional activator protein, or one may use complementary domains derived from different proteins. The domains may be identical to the native domains, or mutants thereof. The assay members may be fused directly to the DBD or TAD, or fused through an intermediated linker.

The target DNA operator may be the native operator sequence, or a mutant operator. Mutations in the operator may be coordinated with mutations in the DBD and the TAD. An example of a suitable transcription activation system is one comprising the DNA-binding domain from the bacterial repressor LexA and the activation domain from the yeast transcription factor Gal4, with the reporter gene operably linked to the LexA operator.

It is not necessary to emply the intact target receptor; just the ligand-binding moiety is sufficient.

The two fusion proteins may be expressed from the same or different vectors. Likewise, the activatable reporter gene may be expressed from the same vector as either fusion protein (or both proteins), or from a third vector.

Potential DNA-binding domains include Gal4, LexA, and mutant domains substantially identical to the above.

Potential activation domains include E. coli B42, Gal4 activation domain II, and HSV VP16, and mutant domains substantially identical to the above.

Potential operators include the native operators for the desired activation domain, and mutant domains substantially identical to the native operator.

The fusion proteins may comprise nuclear localization signals.

The assay system will include a signal producing system, too. The first element of this system is a reporter gene operably linked to an operator responsive to the DBD and TAD of choice. The expression of this reporter gene will result, directly or indirectly, in a selectable or screenable phenotype (the signal). The signal producing system may include, besides the reporter gene, additional genetic or biochemical elements which cooperate in the production of the signal. Such an element could be, for example, a selective agent in the cell growth medium. There may be more than one signal producing system, and the system may include more than one reporter gene.

The sensitivity of the system may be adjusted by, e.g., use of competitive inhibitors of any step in the activation or signal production process, increasing or decreasing the number of operators, using a stronger or weaker DBD or TAD, etc.

When the signal is the death or survival of the cell in question, or proliferation or nonproliferation of the cell in question, the assay is said to be a selection. When the signal merely results in a detectable phenotype by which the signalling cell may be differentiated from the same cell in a nonsignalling state (either way being a living cell), the assay is a screen. However, the term "screening assay" may be used in a broader sense to include a selection. When the narrower sense is intended, we will use the term "nonselective screen".

Various screening and selection systems are discussed in Ladner, USP 5,198,346.

Screening and selection may be for or against the peptide: target protein or compound:target protein interaction.

Preferred assay cells are Microbial (bacterial, yeast, algal, protozooal), invertebrate (esp. mammalian, particularly human). The best developed two-hybrid assays are yeast and mammalian systems.

Normally, two hybrid assays are used to determined whether a protein X and a protein Y interact, by virtue of their ability to reconstitute the interaction of the DBD and the TAD. However, augmented two-hybrid assays have been used to detect interactions that depend on a third, non-protein ligand.

For more guidance on two-hybrid assays, see Brent and Finley, Jr., Ann. Rev. Genet., 31:663-704 (1997); Fremont-Racine, et al., Nature Genetics, 277-281 (16 July 1997) ; Allen, et al., TIBS, 511-16 (Dec. 1995); LeCrenier, et al., BioEssays, 20:1-6 (1998); Xu, et al., Proc. Nat. Acad. sci. (USA), 94:12473-8 (Nov. 1992); Esotak, et al., Mol. Cell. Biol., 15:5820-9 (1995); Yang, et al., Nucleic Acids Res., 23:1152-6 (1995) ; Bendixen, et al. , Nucleic Acids Res., 22: 1778-9 (1994) ; Fuller, et al., BioTechniques, 25:85-92 (July 1998); Cohen, et al., PNAS (USA) 95:14272-7 (1998); Kolonin and Finley, Jr., PNAS (USA) 95:14266-71 (1998). See also Vasavada, et al., PNAS (USA), 88:10686-90 (1991) (contingent replication assay), and Rehrauer, et al., J. Biol. Chem., 271:23865-73 91996) (LexA repressor cleavage assay).

"Substantially Identical"

A mutant protein is substantially identical to a reference protein if (a) it has at least 10% of a specific binding activity or a non-nutritional biological activity of the reference protein, and (b) is at least 50% identical in amino acid sequence to the reference protein.

Percentage amino acid identity is determined by aligning the mutant and reference sequences according to a rigorous dynamic programming algorithm which globally aligns their sequences to maximize their similarity, the similarity being scored as the sum of scores for each aligned pair according to an unbiased PAM250 matirx, and a penalty for each internal gap of -12 for the first null of the gap and -4 for each additional null of the same gap. The percentage identity is the number of matches expressed as a percentage of the adjusted (i.e., counting inserted nulls) of the reference sequence.

A mutant DNA sequence is substantially identical to a reference DNA sequence if they are structural sequences, and encoding mutant and reference proteins which are substantially identical as described above.

If instead they are regulatory sequences, they are substantially identical if the mutant sequence has at least 10% of the regulatory activity of the reference sequence, and ias at least 50% identical in nucleotide sequence to the reference sequence. Percentage identity is determined as for proteins except that matches are scored +5, mismatches -4, the gap open penalty is -12, and the gap extension penalty (per null) is -4.

Preferably, sequence which are substantially identical exceed the minimum identity of 50% are, e.g., 51%, 66%, 75%, 80%, 85%, 90%, 95% or 99% identical in sequence.

DNA sequences may also be considered "substantially identical" if they hybridize to each other under stringent conditions, i.e., conditions at which the Tm of the heteroduplex of the one strand of the mutant DNA and the more complementary strand of the reference DNA is not in excess of 10°C. less than the Tm of the reference DNA homoduplex. Typically this will correspond to a percentage identity of 85-90%.

Combinatorial Libraries

The term "library" generally refers to a collection of chemical or biological entities which are related in origin, structure, and/or function, and which can be screened simultaneously for a property of interest.

The term "combinatorial library" refers to a library in which the individual members are either systematic or random combinations of a limited set of basic elements, the properties of each member being dependent on the choice and location of the elements incorporated into it. Typically, the members of the library are at least capable of being screened simultaneously. Randomization may be complete or partial; some positions may be randomized and others predetermined, and at random positions, the choices may be limited in a predetermined manner. The members of a combinatorial library may be oligomers or polymers of some kind, in which the variation occurs through the choice of monomeric building block at one or more positions of the oligomer or polymer, and possibly in terms of the connecting linkage, or the length of the oligomer or polymer, too. Or the members may be nonoligomeric molecules with a standard core structure, like the 1,4-benzodiazepine structure, with the variation being introduced by the choice of substituents at particular variable sites on the core structure. Or the members may be nonoligomeric molecules assembled like a jigsaw puzzle, but wherein each piece has both one or more variable moieties (contributing to library diversity) and one or more constant moieties (providing the functionalities for coupling the piece in question to other pieces ) .

The ability of one or more members of such a library to recognize a target molecule is termed "Combinatorial Recognition". In a "simple combinatorial library", all of the members belong to the same class of compounds (e.g., peptides) and can be synthesized simultaneously. A "composite combinatorial library" is a mixture of two or more simple libraries, e.g., DNAs and peptides, or benzodiazepine and carbamates. The number of component simple libraries in a composite library will, of course, normally be smaller than the average number of members in each simple library, as otherwise the advantage of a library over individual synthesis is small.

Oligonucleotide Libraries

An oligonucleotide library is a combinatorial library, at least some of whose members are single-stranded oligonucleotides having three or more nucleotides connected by phosphodiester or analogous bonds. The oligonucleotides may be linear, cyclic or branched, and may include non-nucleic acid moieties. The nucleotides are not limited to the nucleotides normally found in DNA or RNA. For examples of nucleotides modified to increase nuclease resistance and chemical stability of aptamers, see Chart 1 in Osborne and Ellington, Chem. Rev., 97: 349-70 (1997). For screening of RNA, see Ellington and Szostak, Nature, 346: 818-22 (1990).

There is no formal minimum or maximum size for these oligonucleotides. However, the number of conformations which an oligonucleotide can assume increases exponentially with its length in bases. Hence, a longer oligonucleotide is more likely to be able to fold to adapt itself to a protein surface. On the other hand, while very long molecules can be synthesized and screened, unless they provide a much superior affinity to that of shorter molecules, they are not likely to be found in the selected population, for the reasons explained by Osborne and Ellington (1997). Hence, the libraries of the present invention are preferably composed of oligonucleotides having a length of 3 to 100 bases, more preferably 15 to 35 bases. The oligonucleotides in a given library may be of the same or of different lengths.

Oligonucleotide libraries have the advantage that libraries of very high diversity (e.g., $10^{15}$) are feasible, and binding molecules are readily amplified in vitro by polymerase chain reaction (PCR). Moreover, nucleic acid molecules can have very high specificity and affinity to targets.

In a preferred embodiment, this invention prepares and screens oligonucleotide libraries by the SELEX method, as described in King and Famulok, Molec. Biol. Repts., 20: 97-107 (1994); L. Gold, C. Tuerk. Methods of producing nucleic acid ligands, US#5595877; Oliphant et al. Gene 44:177 (1986).

The term "aptamer" is conferred on those oligonucleotides which bind the target protein. Such aptamers may be used to characterize the target protein, both directly (through identification of the aptamer and the points of contact between the aptamer and the protein) and indirectly (by use of the aptamer as a ligand to modify the chemical reactivity of the protein).

Peptide Library

A peptide library is a combinatorial library, at least some of whose members are peptides having three or more amino acids connected via peptide bonds. Preferably, they are at least five, six, seven or eight amino acids in length. Preferably, they are composed of less than 50, more preferably less than 20 amino acids.

The peptides may be linear, branched, or cyclic, and may include nonpeptidyl moieties. The amino acids are not limited to the naturally occurring amino acids.

A biased peptide library is one in which one or more (but not all) residues of the peptides are constant residues. The individual members are referred to as peptide ligands (PL). In one embodiment, an internal residue is constant, so that the peptide sequence may be written as

$$(X_{aa})_m\text{-}AA_1\text{-}(X_{aa})_n$$

Where Xaa is either any naturally occurring amino acid, or any amino acid except cysteine, m and n are chosen independently from the range of 2 to 20, the Xaa may be the same or different, and $AA_1$ is the same naturally occurring amino acid for all peptides in the library but may be any amino acid. Preferably, m and n are chosen independently from the range of 4 to 9.

Preferably, $AA_1$ is located at or near the center of the peptide. More specifically, it is desirable that m and n are not different by more than 2; more preferably m and n are equal. Even if the chosen $AA_1$ is required (or at least permissive) of the target protein (TP) binding activity, one may need particular flanking residues to assure that it is properly positioned. If $AA_1$ is more or less centrally located, the library presents numerous alternative choices for the flanking residues. If $AA_1$ is at an end, this flexibility is diminished.

The most preferred libraries are those in which $AA_1$ is tryptophan, proline or tyrosine. Second most preferred are those in which $AA_1$ is phenylalanine, histidine, arginine, aspartate, leucine or isoleucine. Third most preferred are those in which $AA_1$ is asparagine, serine, alanine or methionine. The least preferred choices are cysteine and glycine. These preferences are based on evaluation of the results of screening random peptide libraries for binding to many different TPs.

Ligands that bind to functional domains tend to have both constant as well as unique features. Therefore, by using "biased" peptide libraries, one can ease the burden of finding ligands. Either "biased" or "unbiased" libraries may be screened to identify "BioKey" peptides for use in developing reactivity descriptors, and, optionally, peptide aptamer descriptors and additional drug leads.

Studies of Orphan Receptors

Orphan receptors have been identified by virtue of their sequence similarity to known non-orphan receptors, however, by definition, they do not have known natural ligands.

The first step in seeking to predict an orphan receptor-mediated biological activity of a compound is to identify at least one pharmacological agonist or antagonist of the orphan receptor. (Once such a compound is identified, the receptor is not longer strictly speaking an "orphan".) This ligand, which need not be a natural ligand of the receptor, is then used as a reference ligand to define a Biokey panel, etc.

To identify an agonist or antagonist, a combinatorial library is first screened for members which bind the receptor. Preferably, at least five, more preferably at least ten, distinct members are identified. Preferably, it should be demonstrable from competition experiments that more than one binding site is involved.

Compounds are then screened for the ability to inhibit the binding of one or more of the aforementioned library members to the orphan receptor. Those which do so are likely to have altered the receptor conformation. These putative ligands are then screened for agonist or antagonist activity. The biological activities examined preferably include the activities native to those of the cognate receptors by reference to which the orphan receptors were originally identified. They also preferably include assays for cell proliferation for each cell type in which said orphan receptor is known (by detection of the receptor or its corresponding mRNA) to be expressed.

The screened compounds may be small organic compounds, such as compounds from a suitable combinatorial or noncombinatorial library, or they may come from natural sources, such as serum, urine, cerebrospinal fluid, lymphatic fluid, or tissue extracts, which might harbor the natural ligand. Optionally, these natural source materials may be fractionated by conventional methods, and each fraction tested. The compounds may be known agonists or antagonists (or analogues thereof) of the cognate receptor, but need not be.

Small Organic Compound Library

The small organic compound library ("compound library", for short) is a combinatorial library whose members are suitable for use as drugs if, indeed, they have the ability to mediate a biological activity of the target protein.

Peptides have certain disadvantages as drugs. These include susceptibility to degradation by serum proteases, and difficulty in penetrating cell membranes. Preferably, all or most of the compounds of the compound library avoid, or at least do not suffer to the same degree, one or more of the pharmaceutical disadvantages of peptides.

In designing a compound library, it is helpful to bear in mind the methods of molecular modification typically used to obtain new drugs. Three basic kinds of modification may be identified: disjunction, in which a lead drug is simplified to identify its component pharmacophoric moieties; conjunction, in which two or more known pharmacophoric moieties, which may be the same or different, are associated, covalently or noncovalently, to form a new drug; and alteration, in which one moiety is replaced by another which may be similar or different, but which is not in effect a disjunction or conjunction. The use of the terms "disjunction", "conjunction" and "alteration" is intended only to connote the structural relationship of the end product to the original leads, and not how the new drugs are actually synthesized, although it is possible that the two are the same.

The process of disjunction is illustrated by the evolution of neostigmine (1931) and edrophonium (1952) from physostigmine (1925). Subsequent conjunction is illustrated by demecarium (1956) and ambenonium (1956).

Alterations may modify the size, polarity, or electron distribution of an original moiety. Alterations include ring closing or opening, formation of lower or higher homologues, introduction or saturation of double bands, introduction of optically active centers, introduction, removal or replacement of bulky groups, isosteric or bioisosteric substitution, changes in the position or orientation of a group, introduction of alkylating groups, and introduction, removal or replacement of groups with a view toward inhibiting or promoting inductive (electrostatic or conjugative (resonance) effects.

Thus, the substituents may include electron acceptors and/or electron donors. Typical electron donors (+I) include $-CH_3$, $-CH_2R$, $-CHR_2$, $-CR_3$ and $-COO^-$. Typical electron acceptors (-I) include $-NH_3+$, $-NR_3+$, $-NO_2$, -CN, -COOH, -COOR, -CHO, -COR, -COR, -F, -C1, -Br, -OH, -OR, -SH, -SR, $-CH=CH_2$, $-CR=CR_2$, and -C=CH.

The substituents may also include those which increase or decrease electronic density in conjugated systems. The former (+R) groups include $-CH_3$, $-CR_3$, -F, -Cl, -Br, -I, - OH, -OR, -OCOR, -SH, -SR, $-NH_2$, $-NR_2$, and -NHCOR. The later (-R) groups include $-NO_2$, -CN, -CHC, -COR, -COOH, -COOR, $-CONH_2$, $-SO_2R$ and $-CF_3$.

Synthetically speaking, the modifications may be achieved by a variety of unit processes, including nucleophilic and electrophilic substitution, reduction and oxidation, addition elimination, double bond cleavage, and cyclization.

For the purpose of constructing a library, a compound, or a family of compounds, having one or more pharmacological activities (which need not be related to the known or suspected activities of the target protein), may be disjoined into two or more known or potential pharmacophoric moieties. Analogues of each of these moieties may be identified, and mixtures of these analogues reacted so as to reassemble compounds which have some similarity to the original lead compound. It is not necessary that all members of the library possess moieties analogous to all of the moieties of the lead compound.

The design of a library may be illustrated by the example of the benzodiazepines. Several benzodiazepine drugs, including chlordiazepoxide, diazepam and oxazepam, have been used on anti-anxiety drugs. Derivatives of benzodiazepines have widespread biological activities; derivatives have been reported to act not only as anxiolytics, but also as anticonvulsants, cholecystokinin (CCK) receptor subtype A or B, kappa opioid receptor, platelet activating factor, and HIV transactivator Tat antagonists, and GPIIbIIa, reverse transcriptase and ras farnesyltransferase inhibitors.

The benzodiazepine structure has been disjoined into a 2-aminobenzophenone, an amino acid, and an alkylating agent. See Bunin, et al., Proc. Nat. Acad. Sci. USA, 91:4708 (1994). Since only a few 2-aminobenzophenone derivatives are commercially available, it was later disjoined into 2-aminoarylstannane, an acid chloride, an amino acid, and an alkylating agent. Bunin, et al., Meth. Enzymol., 267:448 (1996). The arylstannane may be considered the core structure upon which the other moieties are substituted, or all four may be considered equals which are conjoined to make each library member.

A basic library synthesis plan and member structure is shown in Figure 1 of Fowlkes, et al., U,S. Serial No. 08/740, 671 (WO98/19162). The acid chloride building block introduces variability at the $R^1$ site. The $R^2$ site is introduced by the amino acid, and the $R^3$ site by the alkylating agent. The $R^4$ site is inherent in the arylstannane. Bunin, et al. generated a 1, 4-benzodiazepine library of 11,200 different derivatives prepared from 20 acid chlorides, 35 amino acids, and 16 alkylating agents. (No diversity was introduced at $R^4$; this group was used to couple the molecule to a solid phase.) According to the Available Chemicals Directory (HDL Information Systems, San Leandro CA), over 300 acid chlorides, 80 Fmoc-protected amino acids and 800 alkylating agents were available for purchase (and more, of course, could be synthesized). The particular moieties used were chosen to maximize structural dispersion, while limiting the numbers to those conveniently synthesized in the wells of a microtiter plate. In choosing between structurally similar compounds, preference was given to the least substituted compound.

The variable elements included both aliphatic and aromatic groups. Among the aliphatic groups, both acyclic and cyclic (mono- or poly-) structures, substituted or not, were tested. (While all of the acyclic groups were linear, it would have been feasible to introduce a branched aliphatic). The aromatic groups featured either single and multiple rings, fused or not, substituted or not, and with heteroatoms or not. The secondary substitutents included $-NH_2$, -OH, -OMe, -CN, -C1, -F, and -COOH. While not used, spacer moieties, such as -O-, - S-, -OO-, -CS-, -NH-, and -NR-, could have been incorporated.

Bunin et al. suggest that instead of using a 1, 4-benzodiazepine as a core structure, one may instead use a 1, 4-benzodiazepine-2, 5-dione structure.

As noted by Bunin et al., it is advantageous, although not necessary, to use a linkage strategy which leaves no trace of the linking functionality, as this permits construction of a more diverse library.

Other combinatorial nonoligomeric compound libraries known or suggested in the art have been based on carbamates, mercaptoacylated pyrrolidines, phenolic agents, aminimides, N-acylamino ethers (made from amino alcohols, aromatic hydroxy acids, and carboxylic acids), N-alkylamino ethers (made from aromatic hydroxy acids, amino alcohols and aldehydes) 1, 4-piperazines, and 1, 4-piperazine-6-ones.

DeWitt, et al., Proc. Nat. Acad. Sci. (USA), 90:6909-13 (1993) describes the simultaneous but separate, synthesis of 40 discrete hydantoins and 40 discrete benzodiazepines. They carry out their synthesis on a solid support (inside a gas dispersion tube), in an array format, as opposed to other conventional simultaneous synthesis techniques (e.g., in a well, or on a pin). The hydantoins were synthesized by first simultaneously deprotecting and then treating each of five amino acid resins with each of eight isocyanates. The benzodiazepines were synthesized by treating each of five deprotected amino acid resins with each of eight 2-amino benzophenone imines.

Chen, et al., J. Am. Chem. Soc., 116:2661-62 (1994) described the preparation of a pilot (9 member) combinatorial library of formate esters. A polymer bead-bound aldehyde preparation was "split" into three aliquots, each reacted with one of three different ylide reagents. The reaction products were combined, and then divided into three new aliquots, each of which was reacted with a different Michael donor. Compound identity was found to be determinable on a single bead basis by gas chromatography/mass spectroscopy analysis.

Holmes, USP 5, 549, 974 (1996) sets forth methodologies for the combinatorial synthesis of libraries of thiazolidinones and metathiazanones. These libraries are made by combination of amines, carbonyl compounds, and thiols under cyclization conditions.

Ellman, USP 5,545,568 (1996) describes combinatorial synthesis of benzodiazepines, prostaglandins, beta-turn mimetics, and glycerol-based compounds. See also Ellman, USP 5,288,514.

Summerton, USP 5,506,337 (1996) discloses methods of preparing a combinatorial library formed predominantly of morpholino subunit structures.

Heterocylic combinatorial libraries are reviewed generally in Nefzi, et al., Chem. Rev., 97:449-472 (1997). One or more moieties of the following types may be incorporated into compounds of the library, as many drugs fall into one or more of the following categories:

acetals
acids
alcohols
amides
amidines
amines
amino acids
amino alcohols
amino ethers
amino ketenes
ammonium compounds
azo compounds
enols
esters
ethers
glycosides
guanidines
halogenated compounds
hydrocarbons
ketones
lactams
lactones
mustards
nitro compounds
nitroso compounds
organo minerals
phenones
quinones
semicarbazones
stilbenes
sulfonamides
sulfones
thiols
thioamides
thioureas

ureas
ureides
urethans

Without attempting to exhaustively recite all pharmacological classes of drugs, or all drug structures, one or more compounds of the chemical structures listed below have been found to exhibit the indicated pharmacological activity, and these structures, or derivatives, may be used as design elements in screening for further compounds of the same or different activity. (In some cases, one or more lead drugs of the class are indicated.)

hypnotics

higher alcohols (clomethiazole)
aldehydes (chloral hydrate)
carbamates (meprobamate)
acyclic ureides (acetylcarbromal)
barbiturates (barbital)
benzodiazepine (diazepam)

anticonvulsants

barbiturates (phenobarbital)
hydantoins (phenytoin)
oxazolidinediones (trimethadione)
succinimides (phensuximide)
acylureides (phenacemides)

narcotic analgesics

morphines
phenylpiperidines (meperidine)
diphenylpropylamines (methadone)
phenothiazihes (methotrimeprazine)

analgesics, antipyretics, antirheumatics

salicylates (acetylsalicylic acid)
p-aminophenol (acetaminophen)
5-pyrazolone (dipyrone)
3, 5-pyrazolidinedione (phenylbutazone)
arylacetic acid (indomethacin)
adrenocortical steroids (cortisone, dexamethasone, prednisone, triamcilone)
athranilic acids

neuroleptics

phenothiazine (chlorpromazine)
thioxanthene (chlorprothixene)
reserpine
butyrophenone (halopendol)

anxiolytics

propandiol carbamates (meprobamate)
benzodiazepines (chlordiazepoxide, diazepam, oxazepam)

antidipressants

tricyclics (imipramine)

muscle/relaxants

propanediols and carbamates (mephenesin)

CNS stimulants

xanthines (caffeine, theophylline)
phenylalkylamines (amphetamine)
(Fenetylline is a conjunction of theophylline and amphetamine)
oxazolidinones (pemoline)

cholinergics

choline esters (acetylcholine)
N,N-dimethylcarbamates

adrenergics

aromatic amines (epinephrine, isoproterenol, phenylephrine)
alicyclic amines (cyclopentamine)
aliphatic amines (methylhexaneamine)
imidazolines (naphazoline)

anti-adrenergics

indolethylamine alkaloids (dihydroergotamine)
imidazoles (tolazoline)
benzodioxans (piperoxan)
beta-haloalkylamines (phenoxybenzamine)
dibenzazepines (azapetine)
hydrazinophthalazines (hydralazine)

antihistamines

ethanolamines (diphenhydramine)
ethylenediamines (tripelennomine)
alkylamines (chlorpheniramine)
piperazines (cyclizine)
phenothiazines (promethazine)

local anesthetics

benzoic acid
esters (procaine, isobucaine, cyclomethycaine)
basic amides (dibucaine)
anilides, toluidides, 2, 6-xylidides (lidocaine)
tertiary amides (oxetacaine)

vasodilators

polyol nitrates (nitroglycerin)

diuretics

xanthines
thiazides (chlorothiazide)

- sulfonamides (chlorthalidone)

antihelmintics

- cyanine dyes

antimalarials

- 4-aminoquinolines
- 8-aminoquinolines
- pyrimidines
- biguanides
- acridines
- dihydrotriazines
- sulfonamides
- sulfones

antibacterials

- antibiotics
  - penicillins
  - cephalosporins
  - octahydronapthacenes (tetracycline)
- sulfonamides
- nitrofurans
- cyclic amines
- naphthyridines
- xylenols

antitumor

- alkylating agents
  - nitrogen mustards
  - aziridines
  - methanesulfonate esters
  - epoxides
- amino acid antagonists
- folic acid antagonists
- pyrimidine antagonists
- purine antagonists

antiviral

- adamantanes
- nucleosides
- thiosemicarbazones
- inosines
- amidines and guanidines
- isoquinolines
- benzimidazoles
- piperazines

For pharmacological classes, see, e.g., Goth, Medical Pharmacology: Principles and Concepts (C.V. Mosby Co.: 8th ed. 1976); Korolkovas and Burckhalter, Essentials of Medicinal Chemistry (John Wiley & Sons, Inc.: 1976). For synthetic methods, see, e.g., Warren, Organic Synthesis: The Disconnection Approach (John Wiley & Sons, Ltd.: 1982); Fuson, Reactions of Organic Compounds (John Wiley & Sons : 1966) ; Payne and Payne, How to do an Organic Synthesis (Allyn and Bacon, Inc.: 1969); Greene, Protective Groups in Organic Synthesis (Wiley-Interscience). For selection of substituents, see e.g., Hansch and Leo, Substituent Constants for Correlation Analysis in Chemistry and Biology (John Wiley & Sons: 1979).

The library is preferably synthesized so that the individual members remain identifiable so that, if a member is shown to be active, it is not necessary to analyze it. Several methods of identification have been proposed, including:

(1) encoding, i.e., the attachment to each member of an identifier moiety which is more readily identified than the member proper. This has the disadvantage that the tag may itself influence the activity of the conjugate.

(2) spatial addressing, e.g., each member is synthesized only at a particular coordinate on or in a matrix, or in a particular chamber. This might be, for example, the location of a particular pin, or a particular well on a microtiter plate, or inside a "tea bag".

The present invention is not limited to any particular form of identification.

However, it is possible to simply characterize those members of the library which are found to be active, based on the characteristic spectroscopic indicia of the various building blocks.

Solid phase synthesis permits greater control over which derivatives are formed. However, the solid phase could interfere with activity. To overcome this problem, some or all of the molecules of each member could be liberated, after synthesis but before screening.

Examples of candidate simple libraries which might be evaluated include derivatives of the following:

- Cyclic Compounds Containing One Hetero Atom
  - Heteronitrogen
    - pyrroles
      - pentasubstituted pyrroles
    - pyrrolidines
    - pyrrolines
    - prolines
    - indoles
    - beta-carbolines
    - pyridines
      - dihydropyridines
      - 1,4-dihydropyridines
      - pyrido[2,3-d]pyrimidines
      - tetrahydro-3H-imidazo[4,5-c] pyridines
    - Isoquinolines
      - tetrahydroisoquinolines
    - quinolones
    - beta-lactams
      - azabicyclo[4.3.0]nonen-8-one amino acid
  - Heterooxygen
    - furans
      - tetrahydrofurans
        - 2,5-disubstituted tetrahydrofurans

pyrans

hydroxypyranones
tetrahydroxypyranones

gamma-butyrolactones

Heterosulfur

sulfolenes

Cyclic Compounds with Two or More Hetero atoms

Multiple heteronitrogens

imidazoles
pyrazoles
piperazines

diketopiperazines
arylpiperazines
benzylpiperazines

benzodiazepines
1,4-benzodiazepine-2,5-diones
hydantoins

5-alkoxyhydantoins

dihydropyrimidines
1,3-disubstituted-5,6-dihydopyrimidine-2,4-diones
cyclic ureas
cyclic thioureas
quinazolines

chiral3-substituted-quinazoline-2,4-diones

triazotes

1,2,3-triazoles

purines

Heteronitrogen and Heterooxygen

dikelomorpholines
isoxazoles
isoxazolines

Heteronitrogen and Heterosulfur

thiazolidines

N-axylthiazolidines

dihydrothiazoles

2-methylene-2,3-dihydrothiazates

2-aminothiazoles

thiophenes

3-amino thiophenes

4-thiazolidinones

4-melathiazanones

benzisothiazolones

For details on synthesis of libraries, see Nefzi, et al., Chem. Rev., 97:449-72 (1997), and references cited therein.

Amino Acids and Peptides

Amino acids are the basic building blocks with which peptides and proteins are constructed. Amino acids possess both an amino group ($-NH_2$) and a carboxylic acid group (-COOH) . Many amino acids, but not all, have the structure $NH_2$-CHR-COOH, where R is hydrogen, or any of a variety of functional groups.

Twenty amino acids are genetically encoded: Alanine, Arginine, Asparagine, Aspartic Acid, Cysteine, Glutamic Acid, Glutamine, Glycine, Histidine, Isoleucine, Leucine, Lysine, Methionine, Phenylalanine, Proline, Serine, Threonine, Tryptophan, Tyrosine, and Valine. Of these, all save Glycine are optically isomeric, however, only the L-form is found in humans. Nevertheless, the D-forms of these amino acids do have biological significance; D-Phe, for example, is a known analgesic.

Many other amino acids are also known, including: 2-Aminoadipic acid; 3-Aminoadipic acid; beta-Aminopropionic acid; 2-Aminobutyric acid; 4-Aminobutyric acid (Piperidinic acid); 6-Aminocaproic acid; 2-Aminoheptanoic acid; 2-Aminoisobutyric acid, 3-Aminoisobutyric acid; 2-Aminopimelic acid; 2,4-Diaminobutyric acid; Desmosine; 2,2'-Diaminopimelic acid; 2,3-Diaminopropionic acid; N-Ethylglycine; N-Ethylasparagine; Hydroxylysine; allo-Hydroxylysine; 3-Hydroxyproline; 4-Hydroxyproline; Isodesmosine; allo-Isoleucine; N-Methylglycine (Sarcosine); N-Methylisoleucine; N-Methylvaline; Norvaline; Norleucine; and Ornithine.

Peptides are constructed by condensation of amino acids and/or smaller peptides. The amino group of one amino acid (or peptide) reacts with the carboxylic acid group of a second amino acid (or peptide) to form a peptide (-NHCO-) bond, releasing one molecule of water. Therefore, when an amino acid is incorporated into a peptide, it should, technically speaking, be referred to as an amino acid <u>residue.</u>

A peptide is composed of a plurality of amino acid residues joined together by peptidyl (-NHCO-) bonds. A biogenic peptide is a peptide in which the residues are all genetically encoded amino acid residues; it is not necessary that the biogenic peptide actually be produced by gene expression.

The peptides of the present invention include peptides whose sequences are disclosed in this specification, or sequences differing from the above solely by no more than one nonconservative substitution and/or one or more conservative substitutions, preferably no more than a single conservative substitution. The substitutions may be of non-genetically encoded (exotic) amino acids, in which case the resulting peptide is nonbiogenic.

A conservative substitution is a substitution of one amino acid for another of the same exchange group, the exchange groups being defined as follows

I Gly, Pro, Ser, Ala (Cys) (and any nonbiogenic, neutral amino acid with a hydrophobicity not exceeding that of the aforementioned a.a.'s)
II Arg, Lys, His (and any nonbiogenic, positively-charged amino acids)
III Asp, Glu, Asn, Gln (and any nonbiogenic negatively-charged amino acids)
IV Leu, Ile, Met, Val (Cys) (and any nonbiogenic, aliphatic, neutral amino acid with a hydrophobicity too high for I above)
V Phe, Trp, Tyr (and any nonbiogenic, aromatic neutral amino acid with a hydrophobicity too high for I above).

Note that Cys belongs to both I and IV.

A highly conservative substitution, which is preferred, is Arg/Lys/His, Asp/Glu, Asn/Gln, Leu/Ile/Met/Val, Phe/Trp/Tyr, or Gly/Ser/Ala.

Additional peptides witin the present invention may be identified by systematic mutagenesis of the lead peptides, e.g.

(a) separate synthesis of all possible single substitution (especially of genetically encoded AAs) mutants of each lead peptide, and/or

(b) simultaneous binomial random alanine-scanning mutagenesis of each lead peptide, so each amino acids position may be either the original amino acid or alanine (alanine being a semi-conservative substitution for all other amino acids), and/or

(c) simultaneous random mutagenesis sampling conservative substitutions of some or all positions of each lead peptide, the number of sequences in total sequences space for a given experiment being such that any sequence, if active, is within detection limits (typically, this means not more than about $10^{10}$ different sequences).

The mutants are tested for activity, and, if active, are considered to be within "peptides of the present invention". Even inactive mutants contribute to our knowledge of structure-activity relationships and thus assist in the design of peptides, peptoids, and peptidomimetics.

Preferably, substitutions of exotic amino acids for the original amino acids take the form of

(I) replacement of one or more hydrophilic amino acid side chains with another hydrophilic organic radical, not more than twice the volume of the original side chain, or

(II) replacement of one or more hydrophobic amino acid side chains with another hydrophobic organic radical, not more than twice the volume of the original side chain.

The exotic amino acids may be alpha or non-alpha amino acids (e.g., beta alanine). They may be alpha amino acids with 2 R groups on the Ca, which groups may be the same or different. They may be dehydro amino acids (HOOC-$C(NH_2)=CHR$).

For further information on synthesis of peptides including exotic amino acids, see:


1. Bielfeldt, T., Peters, S., Meldal, M., Bock, K. and Paulsen, N.A. new strategy for solid-phase synthesis of O-glycopeptides. *Angew. Chem. (Engl)* 31:857-859, 1992.

2. Gurjar, M.K. and Saha, U.K. Synthesis of the glycopeptide-O-(3,4-di-O-methyl-2-0-[3,4-di-O-methyl-α-L-rhamnopyranosyl]-α-L-rhamnophyranosyl)-L-alanilol: An unusual part structure in the glycopeptidolipid of Mycobacterium fortuitum. *Tetrahedron* 48:4039-4044, 1992.

3. Kessler, H., Wittmann, V., Kock, M. and Kottenhahn, M. Synthesis of *C*-glycopeptides via free radical addition of glycosyl bromides to dehydroalanine derivatives. *Angew. Chem. (Engl.)* 31:902-904, 1992.

4. Kraus, J.L. and Attardo, G. Synthesis and biological activities of new *N*-formylated methionyl peptides containing an α-substituted glycine residue. *European Journal of Medicinal Chemistry* 27:19-26, 1992.

5. Mhaskar, S.Y. Synthesis of N-lauroyl dipeptides and correlation of their structure with surfactant and antibacterial properties. *J. Am. Oil Chem. Soc.*69:647-652, 1992.

6. Moree, W.J., Van der Marel, G.A. and Liskamp, R.M.J. Synthesis of peptides containing the β-substituted aminoethane sulfinamide or sulfonamide transition-state isostere derived from amino acids. *Tetrahedron Lett.* 33: 69-6392, 1992.

7. Paquet, A. Further studies on the use of 2,2,2-trichloroethyl groups for phosphate protection in phosphoserine peptide synthesis. *International Journal* of *Peptide and Protein Research* 39:82-86, 1992.

8. Sewald, N., Riede, J., Bissinger, P. and Burger, K. A new convenient synthesis of 2-trifluoromethyl substituted aspartic acid and its isopeptides. Part 11. *Journal of the Chemical Society. Perkin Transactions 1* 1992:267-274, 1992.

9. Simon, R.J., Kania, R.S., Zuckermann, R.N., Huebner, V.D., Jewell, D.A., Banville, S., Ng, S., Wang, L., Rosenberg, S., Marlowe, C.K., Spellmeyer, D.C., Tan, R., Frankel, A.D., Santi, D.V., Cohen, F.E. and Bartlett, P.A. Peptoids: A modular approach to drug discovery. *Proc. Natl, Acad. Sci. USA* 89:9367-9371, 1992.

10. Tung, C.-H., Zhu, T., Lackland, H. and Stein, S. An acridine amino acid derivative for use in Fmoc peptide synthesis. *Peptide Research* 5:115-118, 1992.

11. Elofsson, M. Building blocks for glycopeptide synthesis: Glycosylation of 3-mercaptopropionic acid and Fmoc amino acids with unprotected carboxyl groups. *Tetrahedron* Lett. 32:7613-7616, 1991.

12. McMurray, J.S. Solid phase synthesis of a cyclic peptide using Fmoc chemistry. *Tetrahedron Letters* 32: 7679-7682, 1991.

13. Nunami, K.-I., Yamazaki, T. and Goodman, M. Cyclic retro-inverso dipeptides with two aromatic side chains. I. Synthesis. *Biopolymers* 31:1503-1512, 1991.

14. Rovero, P, Synthesis of cyclic peptides on solid support. *Tetrahedron Letters* 32:2639-2642, 1991.

15. Elofsson, M., Walse, B. and Kihlberg, J. Building blocks for glycopeptide synthesis: Glycosylation of 3-mercaptopropionic acid and Fmoc amino acids with unprotected carboxyl groups. *Tetrahedron Letter,* 32:7613-7616, 1991.

16. Bielfeldt, T., Peter, S., Meldal, M., Bock, K. and Paulsen, H. A new strategy for solid-phase synthesis of O-glycopeptides. *Agnew. Chem (Engl)* 31:857-859, 1992.

17. Luning, B., Norberg, T. and Tejbrant, J. Synthesis of glycosylated amino acids for use in solid phase glycopeptide synthesis, par 2:N-(9-fluorenylmethyloxycarbonyl)-3-O-[2,4,6-tri-O-acetyl-α-D-sylopyranosyl)-β-D-glucopyranosyl]-L-serine. *J. Carbohydr. Chem.* 11:933-943, 1992.

18. Peters, S., Bielfeldt, T., Meldal, M., Bock, K. and Paulsen, H. Solid phase peptide synthesis of mucin glycopeptides. *Tetrahedron Lett.* 33:6445-6448, 1992.

19. Urge, L., Otvos, L., Jr., Lang, E., Wroblewski, K., Laczko,I. and Hollosi, M. Fmoc-protected, glycosylated asparagines potentially useful as reagents in the solid-phase synthesis of N-glycopeptides, *Carbohydr. Res.* 235:83-93, 1992.

20. Gerz, M., Matter, H. and Kessler, H., S-glycosylated cyclic peptides, *Angew. Chem. (Engl.)* 32:269-271, 1993.

*Cyclic Peptides*

Many naturally occurring peptide are cyclic. Cyclization is a common mechanism for stabilization of peptide conformation thereby achieving improved association of the peptide with its ligand and hence improved biological activity. Cyclization is usually achieved by intra-chain cystine formation, by formation of peptide bond between side chains or between N- and C-terminals. Cyclization was usually achieved by peptides in solution, but several publications have appeared recently that describe cyclization of peptides on beads (see references below) .

1. Spatola, A.F., Anwer, M.K. and Rao, M.N. Phase transfer catalysis in solid phase peptide synthesis. Preparation of cycle [Xxx-Pro-Gly-Yyy-Pro-Gly] model peptides and their conformational analysis. *Int. J. Pept. Protein Res.* 40: 322-332, 1992.

2. Tromelin, A., Fulachier, M.-H., Mourier, G. and Menez, A. Solid phase synthesis of a cyclic peptide derived from a curaremimetic toxin. *Tetrahedron Lett.* 33:5197-5200, 1992.

3. Trzeciak, A. Synthesis of 'head-to-tail' cyclized peptides on solid supports by Fmoc chemistry. *Tetrahedron Lett.* 33:4557-45560, 1992.

4. Wood, S. J. and Wetzel, R. Novel cyclization chemistry especially suited for biologically derived, unprotected peptides, *Int. J. Pept. Protein Res.* 39:533-539, 1992.

5. Gilon, C., Halle, D., Chorev, M., Selinger, Z. and Byk, G. Backbone cyclization: A new method for conferring conformational constraint on peptides. *Biopolymers* 31:745-750, 1991.

6. McMurray, J. S. Solid phase synthesis of a cyclic peptide using Fmoc chemistry. *Tetrahedron Letters* 32: 7679-7682, 1991.

7. Rovero, P. Synthesis of cyclic peptides on solid support. *Tetrahedron Letters* 32:2639-2642, 1991.

8. Yajima, X. Cyclization on the bead via following Cys Acm deprotection. *Tetrahedron* 44:805, 1988.

Peptoid

A peptoid is an analogue of a peptide in which one or more of the peptide bonds are replaced by pseudopeptide bonds, which may be the same or different.

Such pseudopeptide bonds may be:

Carba $\Psi(CH_2-CH_2)$
Depsi $\Psi$(CO-O)
Hydroxyethylene $\Psi(CHOH-CH_2)$
Ketomethylene $\Psi$ $(CO-CH_2)$
Methylene-ocy $CH_2$-O-
Reduced $CH_2$-NH
Thiomethylene $CH_2$-S-
Thiopeptide CS-NH
N-modified -NRCO-

See also

1. Corringer, P.J., Weng, J.H., Ducos, B., Durieux, C., Boudeau, P., Bohme, A. and Roques, B.P. CCK-B agonist or antagonist activities of structurally hindered and peptidase-resistant Boc-$CCK_4$ derivatives. *J. Med. Chem.* 36: 166-172, 1993. Amino acids reported: aromatic naphthylalaninimide (Nal-NH2); N-methyl amino acids.

2. Beylin, V.G., Chen, H.G., Dunbar, J., Goel, O.P., Harter, W., Marlatt, M. and Topliss, J.G. Cyclic derivatives of 3,3-diphenylalanine (Dip) (II), novel α-amino acids for peptides of biological interest. *Tetrahedron Lett.* 34:953-956, 1993.

3. Garbay-Jaureguiberry, C., Ficheux, D. and Roques, B.P. Solid phase synthesis of peptides containing the non-gydrolysable analog of (O)phosphotyrosine, p($CH_2PO_3H_2$) Phe. Application to the synthesis of 344-357 sequences of the $\beta_2$ adrenergic receptor. *Int. J. Pept. Protein Res.* 39:523-527, 1992.

4. Lüning, B., Norberg, T. and Tejbrant, J. Synthesis of glycosylated amino acids for use in solid phase glycopeptide synthesis, part 2: *N*-(9-fluorenylmethyloxycarbonyl)-3-*O*-[2,4,6-tri-*O*-acetyl-3-*O*-(2,3,4-tri-*O*-acetyl-α-D-xylopyranosyl)-β-D-glucopyranosyl]-L]serine. *J. Carbohydr. Chem.* 11:933-943, 1992.

5. Tung, C.H., Zhu, T., Lackland, H. and Stein, S. An acridine amino acid derivative for use in Fmoc peptide synthesis. *Peptide Research* 5:115-118, 1992.

6. Eric Frerot, PyBOP and PyBroP: Two reagents for the difficult coupling of the alpha,alpha-dialkyl amino acid Aib. *Tetrahedron* 47:259-270, 1991.

7. Moree, W.J., Van der Marel, G.A. and Liskamp, R.M.J. Synthesis of peptides containing the β-substituted aminoethane sulfinamide or sulfonamide transition-state isostere derived from amino acids. *Tetrahedron Lett.* 33: 6389-6392, 1992.

8. Rana, T.M. Synthesis of a metal-binding amino acid suitable for solid phase assembly of peptides. *Tetrahedron Lett.* 33:4521-4524, 1992.

9. Urge, L., Otvos, L., Jr., Lang, E., Wroblewski, K., Laczko, I. and Hollosi, M. Fmoc-protected, glycosylated asparagines potentially useful as reagents in the solid-phase synthesis of *N*-glycopeptides. *Carbohydr. Res.* 235:83-93, 1992.

10. Pavone, V., DiBlasio, B., Lombardi, A., Maglio, O., Isernia, D., Pedone, C., Benedette, E., Altmann, E. and Mutter, M. Non coded $C^{\alpha,\alpha}$-disubstituted amino acids. X-ray diffraction analysis of a dipeptide containing (S)-α-methylserine. *Int. J. Pept. Protein Res.* 41:15-20, 1993.

11. Nishino, N., Mihara, H., Kiyota, H., Kobata, K. and Fujimoto, T. Aminoporphyrinic acid as a new template for polypeptide design. *J. Chem. Soc. Chem. Commun.* 1993:162-163, 1993.

12. Sosnovsky, G., Prakash, I. and Rao, N.U.M. In the search for new anticancer drugs. XXIV: Synthesis and anticancer activity of amino acids and dipeptides containing the 2-chloroethyl- and [*N'*-nitroso]-aminocarbonyl groups. *J. Pharm. Sci.* 82:1-10, 1993.

13. Berti, F., Ebert, C. and Gardossi, L. One-step stereospecific synthesis of α,β-dehydroamino acids and dehydropeptides. *Tetrahedron Lett.* 33:8145-8148, 1992.

Peptidomimetic

A peptidomimetic is a molecule which mimics the biological activity of a peptide, by substantially duplicating the pharmacologically relevant portion of the conformation of the peptide, but is not a peptide or peptoid as defined above. Preferably the peptidomimetic has a molecular weight of less than 700 daltons.

Designing a peptidomimetic usually proceeds by:

(a) identifying the pharmacophoric groups responsible for the activity;
(b) determining the spatial arrangements of the pharmacophoric groups in the active conformation of the peptide; and
(c) selecting a pharmaceutically acceptable template upon which to mount the pharmacophoric groups in a manner which allows them to retain their spatial arrangment in the active conformation of the peptide.

Step (a) may be carried out by preparing mutants of the active peptide and determining the effect of the mutation on activity. One may also examine the 3D structure of a complex of the peptide and the receptor for evidence of interactions, e.g., the fit of a side chain of the peptide into a cleft of the receptor; potential sites for hydrogen bonding, etc.).

Step (b) generally involves determining the 3D structure of the active peptide, in the complex, by NMR spectroscopy or X-ray diffraction studies. The initial 3D model may be refined by an energy minimization and molecular dynamics simulation.

Step (c) may be carried out by reference to a template database, see Wilson, et al. Tetrahedron, 49:3655-63 (1993). The templates will typically allow the mounting of 2-8 pharmacophores, and have a relatively rigid structure. For the latter reason, aromatic structures, such as benzene, biphenyl, phenanthrene and benzodiazepine, are preferred. For orthogonal protection techniques, see Tuchscherer, et al., Tetrahedron, 17:3559-75 (1993).

For more information on peptoids and peptidomimetics, see USP 5, 811, 392, USP 5,811,512, USP 5,578,629, USP 5,817,879, USP 5,817,757, USP 5,811,515.

Analogues

Also of interest are analogues of the disclosed peptides, and other compounds with activity of interest.

Analogues may be identified by assigning a hashed bitmap structural fingerprint to the compound, based on its chemical structure, and determining the similarity of that fingerprint to that of each compound in a broad chemical database. The fingerprints are determined by the fingerprinting software commercially distributed for that purpose by Daylight Chemical Information Systems, Inc., according to the software release current as of January 8, 1999. In essence, this algorithm generates a bit pattern for each atom, and for its nearest neighbors, with paths up to 7 bonds long. Each pattern serves as a seed to a pseudorandom number generator, the output of which is a set of bits which is logically ored to the developing fingerprint. The fingerprint may be fixed or variable size.

The database may be SPRESI' 95 (InfoChem GmbH), Index Chemicus (ISI), MedChem (Pomona/Biobyte), World Drug Index (Derwent), TSCA93(EPA) May bridge organic chemical catalog (Maybridge), Available Chemicals Directory (MDLIS Inc.), NCI96 (NCI), Asinex catalog of organic compounds (Asinex Ltd.), or IBIOScreen SC and NP (Inter BioScreen Ltd.), or an inhouse database.

A compound is an analogue of a reference compound if it has a daylight fingerprint with a similarity (Tanamoto coefficient) of at least 0.85 to the Daylight fingerprint of the reference compound.

A compound is also an analogue of a reference compound id it may be conceptually derived from the reference compound by isosteric replacements.

Homologues are compounds which differ by an increase or decrease in the number of methylene groups in an alkyl moiety.

Classical isosteres are those which meet Erlenmeyer's definition: "atoms, ions or molecules in which the peripheral layers of electrons can be considered to be identical". Classical isosteres include

| Monovalents | Bivalents | Trivalents | Tetra | Annular |
|---|---|---|---|---|
| F, OH, $NH_2$, $CH_3$ | -O- | -N= | =C=<br>=Si= | -CH=CH- |
| Cl, SH, $PH_2$ | -S- | -P= | -N+= | -S- |
| Br | -Se- | -As- | =P+= | -O- |
| i | -Te- | -Sb-<br>-CH= | =As+=<br>=Sb+= | -NH- |

Nonclassical isosteric pairs include -CO- and $-SO_2-$, -COOH and $-SO_3H$, $-SO_2NH_2$ and $-PO(OH)NH_2$, and -H and -F, -OC (=O) - and C (=O) O-, -OH and $-NH_2$.

Pharmaceutical Methods and Preparations

The preferred animal subject of the present invention is a mammal. By the term "mammal" is meant an individual belonging to the class Mammalia. The invention is particularly useful in the treatment of human subjects, although it is intended for veterinary uses as well. Preferred nonhuman subjects are of the orders Primata (e.g., apes and monkeys), Artiodactyla or Perissodactyla (e.g., cows, pigs, sheep, horses, goats), Carnivora (e.g., cats, dogs), Rodenta (e.g., rats, mice, guinea pigs, hamsters), Lagomorpha (e.g., rabbits) or other pet, farm or laboratory mammals.

The term "protection", as used herein, is intended to include "prevention," "suppression" and "treatment." "Prevention" involves administration of the protein prior to the induction of the disease (or other adverse clinical condition). "Suppression" involves administration of the composition prior to the clinical appearance of the disease. "Treatment" involves administration of the protective composition after the appearance of the disease.

It will be understood that in human and veterinary medicine, it is not always possible to distinguish between "preventing" and "suppressing" since the ultimate inductive event or events may be unknown, latent, or the patient is not ascertained until well after the occurrence of the event or events. Therefore, it is common to use the term "prophylaxis" as distinct from "treatment" to encompass both "preventing" and "suppressing" as defined herein. The term "protection," as used herein, is meant to include "prophylaxis." It should also be understood that to be useful, the protection provided need not be absolute, provided that it is sufficient to carry clinical value. An agent which provides protection to a lesser degree than do competitive agents may still be of value if the other agents are ineffective for a particular individual, if it can be used in combination with other agents to enhance the level of protection, or if it is safer than competitive agents. The drug may provide a curative effect, an ameliorative effect, or both.

At least one of the drugs of the present invention may be administered, by any means that achieve their intended purpose, to protect a subject against a disease or other adverse condition. The form of administration may be systemic or topical. For example, administration of such a composition may be by various parenteral routes such as subcutaneous, intravenous, intradermal, intramuscular, intraperitoneal, intranasal, transdermal, or buccal routes. Alternatively, or concurrently, administration may be by the oral route. Parenteral administration can be by bolus injection or by gradual perfusion over time.

A typical regimen comprises administration of an effective amount of the drug, administered over a period ranging from a single dose, to dosing over a period of hours, days, weeks, months, or years.

It is understood that the suitable dosage of a drug of the present invention will be dependent upon the age, sex, health, and weight of the recipient, kind of concurrent treatment, if any, frequency of treatment, and the nature of the effect desired. However, the most preferred dosage can be tailored to the individual subject, as is understood and determinable by one of skill in the art, without undue experimentation. This will typically involve adjustment of a standard dose, e.g., reduction of the dose if the patient has a low body weight.

Prior to use in humans, a drug will first be evaluated for safety and efficacy in laboratory animals. In human clinical studies, one would begin with a dose expected to be safe in humans, based on the preclinical data for the drug in question, and on customary doses for analogous drugs (if any). If this dose is effective, the dosage may be decreased, to determine the minimum effective dose, if desired. If this dose is ineffective, it will be cautiously increased, with the patients monitored for signs of side effects. See, e.g., Berkow et al, eds., *The Merck Manual,* 15th edition, Merck and Co., Rahway, N.J., 1987; Goodman et al., eds., *Goodman and Gilman's The Pharmacological Basis of Therapeutics,* 8th edition, Pergamon Press, Inc., Elmsford, N.Y., (1990); *Avery's Drug Treatment: Principles and Practice of Clinical Pharmacology and Therapeutics,* 3rd edition, ADIS Press, LTD., Williams and Wilkins, Baltimore, MD. (1987), Ebadi, *Pharmacology,* Little, Brown and Co., Boston, (1985), which references and references cited therein, are entirely incorporated herein by reference.

The total dose required for each treatment may be administered by multiple doses or in a single dose. The protein may be administered alone or in conjunction with other therapeutics directed to the disease or directed to other symptoms thereof.

The appropriate dosage form will depend on the disease, the protein, and the mode of administration; possibilities include tablets, capsules, lozenges, dental pastes, suppositories, inhalants, solutions, ointments and parenteral depots. See, e.g., Berker, supra, Goodman, supra, Avery, *supra* and Ebadi, *supra*, which are entirely incorporated, herein by reference, including all references cited therein.

In the case of peptide drugs, the drug may be adminstered in the form of an expression vector comprising a nucleic acid encoding the peptide, such a vector, after in corporation into the genetic complement of a cell of the patient, directs synthesis of the peptide. Suitable vectors include genetically engineered poxviruses (vaccinia), adenoviruses, adeno-associated viruses, herpesviruses and lentiviruses which are or have been rendered nonpathogenic.

In addition to at least one drug as described herein, a pharmaceutical composition may contain suitable pharmaceutically acceptable carriers, such as excipients, carriers and/or auxiliaries which facilitate processing of the active compounds into preparations which can be used pharmaceutically. See, e.g., Berker, supra, Goodman, supra, Avery, *supra* and Ebadi, *supra.*

Anti-Cancer Utility

One utility of certain ER-binding peptides of the present invention, and related peptoids, peptidomimetics and analogues, and compounds fingerprinted as sensitive to the interaction of such peptides with ER, is in circumventing tamoxifen resistance in breast cancer.

It is now estimated that the lifetime risk among American women of being diagnosed with breast cancer is about one in eight. Although this figure represents a doubling of the incidence of this disease over the past fifty years, it is counterbalanced by the observation that mortality from this disease has decreased slightly over the same period. In the recent NSAPB-B14 trial it was demonstrated that the 10 year survival rate in breast cancer patients who were node negative at time of diagnosis was greater than 80%. It is likely that this favorable response is due in large part to advances in early detection which has had the effect of decreasing the number of women who present with metastatic disease to more manageable early stage malignancies. In addition to early detection however, the strategic use of the antiestrogen tamoxifen for the treatment of metastatic disease and as an adjuvant chemotherapeutic has had a positive impact on survival in breast cancer patients. One of the most dramatic benefits of tamoxifen is that it reduces the incidence of contralateral primary tumors in patients by greater than 50%. It was this finding, combined with the results of the NSABP-P1 chemoprevention trial, which led recently to the approval of tamoxifen for use as a breast cancer chemopreventative in women who are at an elevated risk for breast cancer. Clearly, tamoxifen is an extremely successful pharmaceutical. As with most drugs however, the effectiveness of tamoxifen as a chemotherapeutic agent decreases with time. In the metastatic setting, it has been observed that most tamoxifen responsive breast cancers eventually become resistant to its antiestrogenic actions. A decrease in effectiveness over time in the adjuvant setting is also inferred from the results of the NSABP-B14 trial which demonstrated that the overall survival rate of breast cancer patients who were asking tamoxifen for 10 years was no better, and possibly even worse, than women who took this drug for only five years. The latter result has led to the suggestion that the tumors in patients who were on tamoxifen for extended periods of time may loose the ability to recognize this drug as an antiestrogen and may in fact change in some manner to respond to the drug as an estrogen. The observation that some patients display a withdrawal response when tamoxifen administration is discontinued supports this hypothesis. Consequently, there has been a tremendous amount of interest in understanding the process by which breast tumors fail tamoxifen and in the application of this knowledge to the development of novel antiestrogens with improved therapeutic benefits.

Several years ago it was considered unlikely that the estrogen receptor (ER) would be a useful target in those cells which have failed tamoxifen. However, the emergence of pure antiestrogens, like ICI182,780, which have been used successfully to treat tamoxifen refractory breast cancers has validated ER as a target in this stage of the disease. However, since they non-selectively block estrogen action in all target organs they will have a negative impact in the skeletal and cardiovascular systems and consequently will not be suitable for use as adjuvant chemotherapeutics. There is an unmet medical need therefore, for novel antiestrogens which are mechanistically distinct from tamoxifen in the breast but which retain the positive estrogenic actions of tamoxifen in the bond and the cardiovascular systems.

Tamoxifen was developed originally as an antiestrogen which could be used to block the actions of estrogen at the receptor level in breast cancer cells. Thus, it was generally held that resistance to this agent occurred as a consequence of ER mutations, selective extrusion of the compound from cells or as a result of inactivating metabolic processes. However, it now appears that these mechanisms only explain tamoxifen resistance in a small percentage of cases. Other mechanisms are now being considered. We favor a model in which epigenetic changes occur within target cells affecting their ability to recognize tamoxifen as an antagonist and may in fact permit them to recognize the drug as an estrogenic ligand. This hypothesis stems from the observation that tamoxifen is in fact a selective estrogen receptor modulator (SERM) which can function as an ER antagonist, or an agonist, depending on the cell background in which it is studied. Thus, we believe that in breast the selective pressure of tamoxifen promotes the outgrowth of a population of cells, through accommodation or selection, which recognize tamoxifen as an agonist. Consequently, we and others, have focused on defining the molecular basis for the cell selective actions of tamoxifen, and other SERMs, with a view to understanding tamoxifen resistance and the eventual development of novel antiestrogens. These studies have revealed that upon binding ligand, ER undergoes a conformational change, the nature of which is influenced by the structure of the bound ligand. The significance of these conformational changes was revealed when it was determined that ER contains two activation domains, AF-1 located at the amino terminus and AF-2 contained within the hormone binding domain, the activity of which is influenced by both cell and promoter context. In most cells both AFs are required for maximal transcriptional activity. Accordingly, it has been shown that estradiol functions as an ER agonist in all cells as it facilitates the interaction of both AFs with the transcription apparatus. It has now been determined that tamoxifen alters ER structure in a manner which inhibits AF-2 function. Thus, in all contexts where AF-2 is required, tamoxifen manifests antagonist activity. In cell contexts where AF-1 alone is sufficient for ER transcriptional activity we have determined that tamoxifen can function as a partial agonist. This finding led us to hypothesize that the residual agonist activity of tamoxifen, observed in AF-1 dominant environments, may be linked to the failure of this drug as an antiestrogen in breast cancer. Thus, we searched for compounds which did not activate AF-1 and evaluated their ability to inhibit tamoxifen partial agonist activity. This work led to the discovery of a novel antiestrogen, GW5638, which when assayed in vitro, inhibits tamoxifen partial agonist activity under all conditions examined and effectively inhibited the growth of MCF-7 cell xenografts in A-thymic nude mice. Because of these properties, GW5638 will soon enter clinical trails for evaluation as a treatment of tamoxifen refractory breast cancer.

One of the surprising properties of the novel antiestrogen, GW5638, is that although it is devoid of AF-1 and AF-2 agonist activity it is not a pure antagonist when assayed in vivo. Unlike tamoxifen, it does not display uterotrophic activity. However, like tamoxifen, it functions as an estrogen in bone and the cardiovascular system. These results indicate that the ability to differentially activate AF-1 and AF-2 may be important but that the pharmacology of this class of antiestrogens is more complex than we anticipated. Consequently, we have focused recently on defining the molecular mechanism(s) by which cells distinguish between tamoxifen and GW5638. Although still ongoing, it has led to the development of a novel approach to inhibit the partial agonist activity of tamoxifen. Specifically, using phage display technology we have identified small peptides whose interaction with ER is influenced by the nature of the bound ligand. Peptides have been found which interact with ER in the presence of any ligand, in the presence of any agonist, in the presence of any antagonist and more importantly, we have identified peptides which interact with ER only in the presence of tamoxifen. With respect to the development of strategies to treat tamoxifen refractory breast cancer, the latter peptides are the most interesting as we have shown in vitro that these peptides efficiently inhibit tamoxifen partial agonist activity. Mapping of the sites on ER with which these peptides interact will help in determining if they mimic specific coactivator interactions. Regardless however, this work has defined several sites on ER that will serve as targets for new drug discovery. Although peptides do not generally serve as good starting places for drug development, there has been a tremendous amount of progress of late in generating small molecules which modulate protein-protein interactions. Consequently, we are now in the process of screening for small molecules which interact with the target sites implicated by the novel peptides and additionally are in the process of defining smaller peptides which in themselves may be useful, if suitably formulated, as drugs.

Binding Molecule

For the purpose of the discussion of diagnostic methods and agents which follows, the "binding molecule" is the peptide, peptoid or peptidomimetic of the present invention. The analyte is a target protein.

In Vitro Diagnostic Methods and Reagents

The in vitro assays of the present invention may be applied to any suitable analyte-containing sample, and may be qualitative or quantitative in nature. In order to detect the presence, or measure the amount, of an analyte, the assay must provide for a signal producing system (SPS) in which there is a detectable difference in the signal produced, depending on whether the analyte is present or absent (or, in a quantitative assay, on the amount of the analyte). The detectable signal may be one which is visually detectable, or one detectable only with instruments. Possible signals include production of colored or luminescent products, alteration of the characteristics (including amplitude or polarization) of absorption or emission of radiation by an assay component or product, and precipitation or agglutination of a component or product. The term "signal" is intended to include the discontinuance of an existing signal, or a change in the rate of change of an observable parameter, rather than a change in its absolute value. The signal may be monitored manually or automatically.

The component of the signal producing system which is most intimately associated with the diagnostic reagent is called the "label". A label may be, e.g., a radioisotope, a fluorophore, an enzyme, a co-enzyme, an enzyme substrate, an electron-dense compound, an agglutinable particle. One diagnostic reagent is a conjugate, direct or indirect, or covalent or noncovalent, of a label with a binding molecule of the invention.

The radioactive isotope can be detected by such means as the use of a gamma counter or a scintillation counter or by autoradiography. Isotopes which are particularly useful for the purpose of the present invention are $^{3}H$, $^{125}I$, $^{131}I$, $^{35}S$, $^{14}C$, and, preferably, $^{121}I$.

It is also possible to label a compound with a fluorescent compound. When the fluorescently labeled antibody is exposed to light of the proper wave length, its presence can then be detected due to fluorescence. Among the most commonly used fluorescent labelling compounds are fluorescein isothiocyanate, rhodamine, phycoerythrin, phycocyanin, allophycocyanin, o-phthaldehyde and fluorescamine.

Alternatively, fluorescence-emitting metals such as $^{125}Eu$, or others of the lanthanide series, may be attached to the binding protein using such metal chelating groups as diethylenetriaminepentaacetic acid (DTPA) of ethylenediaminetetraacetic acid (EDTA).

The binding molecules also can be detectably labeled by coupling to a chemiluminescent compound. The presence of the chemiluminescent compound is then determined by detecting the presence of luminescence that arises during the course of a chemical reaction after a suitable reactant is provided. Examples of particularly useful chemiluminescent labeling compounds are luminol, isolumino, theromatic acridinium ester, imidazole, acridinium salt and oxalate ester.

Likewise, a bioluminescent compound may be used to label the binding molecule. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

Enzyme labels, such as horseradish peroxidase and alkaline phosphatase, are preferred. When an enzyme label is used, the signal producing system must also include a substrate for the enzyme. If the enzymatic reaction product is not itself detectable, the SPS will include one or more additional reactants so that a detectable product appears.

Assays may be divided into two basic types, heterogeneous and homogeneous. In heterogeneous assays, the interaction between the affinity molecule and the analyte does not affect the label, hence, to determine the amount or presence of analyte, bound label_must be separated from free label. In homogeneous assays, the interaction does affect the activity of the label, and therefore analyte levels can be deduced without the need for a separation step.

In general, a target-binding molecule of the present invention may be used diagnostically in the same way that a target-binding antibody is used. Thus, depending on the assay format, it may be used to assay the target, or by competitive inhibition, other substances which bind the target. The sample will normally be a biological fluid, such as blood, urine, lymph, semen, milk, or cerebrospinal fluid, or a fraction or derivative thereof, or a biological tissue, in the form of, e.g., a tissue section or homogenate. However, the sample conceivably could be (or derived from) a food or beverage, a pharmaceutical or diagnostic composition, soil, or surface or ground water. If a biological fluid or tissue, it may be taken from a human or other mammal, vertebrate or animal, or from a plant. The preferred sample is blood, or a fraction or derivative thereof.

In one embodiment, the binding molecule is insolubilized by coupling it to a macromolecular support, and target in the sample is allowed to compete with a known quantity of a labeled or specifically labelable target analogue. (The conjugate of the binding molecule to a macromolecular support is another diagnostic agent within the present invention.)

The "target analogue" is a molecule capable of competing with target for binding to the binding molecule, and the term is intended to include target itself. It may be labeled already, or it may be labeled subsequently by specifically binding the label to a moiety differentiating the target analogue from authentic target. The solid and liquid phases are separated, and the labeled target analogue in one phase is quantified. The higher the level of target analogue in the solid phase, i.e., sticking to the binding molecule, the lower the level of target analyte in the sample.

In a "sandwich assay", both an insolubilized target-binding molecule, and a labeled target-binding molecule are employed. The target analyte is captured by the insolubilized target-binding molecule and is tagged by the labeled target-binding molecule, forming a tertiary complex. The reagents may be added to the sample in either order, or simultaneously. The target-binding molecules may be the same or different, and only one need be a target-binding molecule according to the present invention (the other may be, e.g., an antibody or a specific binding fragment thereof). The amount of labeled target-binding molecule in the tertiary complex is directly proportional to the amount of target analyte in the sample.

The two embodiments described above are both heterogeneous assays. However, homogeneous assays are conceivable. The key is that the label be affected by whether or not the complex is formed.

A label may be conjugated, directly or indirectly (e.g., through a labeled anti-target-binding molecule antibody), covalently (e.g., with SPDP) or noncovalently, to the target-binding molecule, to produce a diagnostic reagent. Similarly, the target binding molecule may be conjugated to a solid-phase support to form a solid phase ("capture") diagnostic reagent. Suitable supports include glass, polystyrene, polypropylene, polyethylene, dextran, nylon, amylases, natural and modified celluloses, polyacrylamides, agaroses, and magnetite. The nature of the carrier can be either soluble to some extent or insoluble for the purposes of the present invention. The support material may have virtually any possible structural configuration so long as the coupled molecule is capable of binding to its target. Thus the support configuration may be spherical, as in a bead, or cylindrical, as in the inside surface of a test tube, or the external surface of a rod. Alternatively, the surface may be flat such as a sheet, test strip, etc.

In Vivo Diagnostic Uses

Analyte-binding molecules can be used for in vivo imaging.

Radio-labelled binding molecule may be administered to the human or animal subject. Administration is typically by injection, e.g., intravenous or arterial or other means of administration in a quantity sufficient to permit subsequent dynamic and/or static imaging using suitable radio-detecting devices. The preferred dosage is the smallest amount capable of providing a diagnostically effective image, and may be determined by means conventional in the art, using known radio-imaging agents as a guide.

Typically, the imaging is carried out on the whole body of the subject, or on that portion of the body or organ relevant to the condition or disease under study. The radio-labelled binding molecule has accumulated. The amount of radio-labelled binding molecule accumulated at a given point in time in relevant target organs can then be quantified.

A particularly suitable radio-detecting device is a scintillation camera, such as a gamma camera. A scintillation camera is a stationary device that can be used to image distribution of radio-labelled binding molecule. The detection device in the camera senses the radioactive decay, the distribution of which can be recorded. Data produced by the imaging system can be digitized. The digitized information can be analyzed over time discontinuously or continuously. The digitized data can be processed to produce images, called frames, of the pattern of uptake of the radio-labelled binding protein in the target organ at a discrete point in time. In most continuous (dynamic) studies, quantitative data is obtained by observing changes in distributions of radioactive decay in target organs over time. In other words, a time-activity analysis of the data will illustrate uptake through clearance of the radio-labelled binding molecule by the target organs with time.

Various factors should be taken into consideration in selecting an appropriate radioisotope. The radioisotope must be selected with a view to obtaining good quality resolution upon imaging, should be safe for diagnostic use in humans and animals, and should preferably have a short physical half-life so as to decrease the amount of radiation received by the body. The radioisotope used should preferably be pharmacologically inert, and, in the quantities administered, should not have any substantial physiological effect.

The binding molecule may be radio-labelled with different isotopes of iodine, for example $^{123}I$, $^{125}I$, or $^{131}I$ (see for example, U.S. Patent 4,609,725). The extent of radio-labeling must, however be monitored, since it will affect the calculations made based on the imaging results (i.e. a diiodinated binding molecule will result in twice the radiation count of a similar monoiodinated binding molecule over the same time frame).

In applications to human subjects, it may be desirable to use radioisotopes other than $^{125}I$ for labelling in order to decrease the total dosimetry exposure of the human body and to optimize the detectability of the labelled molecule (though this radioisotope can be used if circumstances require). Ready availability for clinical use is also a factor. Accordingly, for human applications, preferred radio-labels are for example, $^{99m}Tc$, $^{67}Ga$, $^{68}Ga$, $^{90}Y$, $^{111}In$, $^{113m}In$, $^{123}I$, $^{186}Re$, $^{188}Re$ or $^{211}At$.

The radio-labelled binding molecule may be prepared by various methods. These include radio-halogenation by the chloramine - T method or the lactoperoxidase method and subsequent purification by HPLC (high pressure liquid chromatography), for example as described by J. Gutkowska et al in "Endocrinology and Metabolism Clinics of America: (1987) 16 (1) :183. Other known method of radio-labelling can be used, such as IODOBEADS™.

There are a number of different methods of delivering the radio-labelled binding molecule to the end-user. It may be administered by any means that enables the active agent to reach the agent's site of action in the body of a mammal. If the molecule is digestible when administered orally, parenteral administration, e.g., intravenous, subcutaneous, or intramuscular, would ordinarily be used to optimize absorption.

Other Uses

The binding molecules employed in the present invention may also be used to purify target from a fluid, e.g., blood. For this purpose, the target-binding molecule is preferably immobilized on a solid-phase support. Such supports include those already mentioned as useful in preparing solid phase diagnostic reagents.

Peptides, in general, can be used as molecular weight markers for reference in the separation or purification of peptides by electrophoresis or chromatography. In many instances, peptides may need to be denatured to serve as molecular weight markers. A second general utility for peptides is the use of hydrolyzed peptides as a nutrient source. Hydrolyzed peptide are commonly used as a growth media component for culturing microorganisms, as well as a food ingredient for human consumption. Enzymatic or acid hydrolysis is normally carried out either to completion, resulting in free amino acids, or partially, to generate both peptides and amino acids. However, unlike acid hydrolysis, enzymatic hydrolysis (proteolysis) does not remove non-amino acid functional groups that may be present. Peptides may also be used to increase the viscosity of a solution.

The peptides employed in the present invention may be used for any of the foregoing purposes, as well as for therapeutic and diagnostic purposes as discussed further earlier in this specification.

EXAMPLES

*Example 1*

*Initial Studies Relating to the Estrogen Receptor*

The estrogen receptor (ER) is a member of the steroid family of nuclear receptors. Like other nuclear receptors, the ER is a ligand dependent transcriptional activator. R. C. J. Ribeiro, P. J. Kushner, J. D. Baxter, *Ann. Rev. Med.* **46**, 443, (1995); J.-M. Wurtz et al., *Nat. Struct. Biol.* **3**, 87 (1996); D. Moras and H. Gronemeyer, *Curr. Opin. Cell Biol.* **10**, 384 (1998). Two distinct estrogen receptors have been described, ER $\alpha$ and ER $\beta$, which may play distinct roles in gene regulation. K. Paech et al., *Science* **277**, 1508 (1997); G. G. J. M. Kuiper and J.-Å Gustafsson, *FEBS Lett.* **410,** 87 (1997); J. T. Moore et al., *Biochem. Biophys. Res. Comm.* **247**, 75 (1998); V. Giguére, A. Tremblay, G. B. Tremblay, *Steroids* **63**, 335 (1998). In addition to the natural ligand, estradiol, the activity of the estrogen receptor is regulated by the association/dissociation of accessory proteins collectively termed co-activators and co-repressors. J. Torchia et al., Nature **387,** 677 (1997); C. K. Glass, D. W. Rose, M. G. Rosenfeld, *Curr. Opin. Cell Biol.* **9**, 222 (1997); J. Torchia, C. Glass, M. G. Rosenfeld, *ibid.* **10**, 373 (1998). Upon binding estradiol, the ER undergoes a conformational change that exposes sites for the association of co-activating proteins. This change may also conceal the binding sites for co-repressors or other molecules that are associated with the inactive receptor, thus preventing their association.

The estrogen receptor is a therapeutic target for diseases such as breast and ovarian cancer, and it is also the target for drugs that ameliorate symptoms and effects of menopause including osteoporosis. While effective, compounds that target the estrogen receptor can exhibit a variety of effects in different target tissues. For example, tamoxifen is an estrogen receptor antagonist in breast tissue and is effective in slowing the growth of ER positive breast tumors. However, tamoxifen can have agonist effects on uterine cell growth. M. A. Gallo and D. Kaufman, *Seminars in Oncology* **24 (suppl. 1)**, S1-71 (1997). Because of their wide range of effects, estrogen receptor targeted drugs cannot be classified as strict agonists or antagonists, but are more appropriately called selective estrogen receptor modulators or SERMS. H. U. Bryant and W. H. Dere, *Proc . Soc. Exp . Biol. Med .* **217**, 45 (1998). SERMs appear to drive the receptor into conformations that are neither fully active nor inactive. Distinguishing between these various intermediate conformations in an *in vitro* environment has been a difficult task at best. We have developed peptidic probes that allow distinction between ER conformations induced by different SERMs. Each SERM, which has a distinct biological effect, also produces a unique pattern in the fingerprint assay. These probes should provide valuable tools for both research and drug discovery, and may provide a link between receptor conformation and biological activity.

In this example, peptides were identified which bind to the unliganded estrogen receptor $\alpha$ (ex. 1.1; table 1) or to the estradiol-activated receptor (ex 1.2, table 2). These Er$\alpha$-binding peptides were then classified (ex 1.3) into five arbitrary classes on the basis of their ability to bind to Erα or Erβ in the presence or absence of estradiol. (Naturally, they all bound either the apo-ERα or the estradiol-activated Era.) Finally, representative peptides of each class were used to "fingerprint" the known ER SERMs estradiol, estriol, nafoxidine, tamoxifen or clomifene (ex. 1.4).

*Example 1.1: Identification of peptides that bind to the unliganded (unactivated) estrogen receptor α*

ER alpha (Panvera Corp.) was immobilized on Immulon 4 plastic plates (Dynatech) for the phage affinity selection as described in patent application Fowlkes, 09/050,359. Peptide sequences obtained for binding to the unliganded (unactivated) receptor are listed below (Table 1).

*Example 1.2: Identification of peptides that bind to the estradiol activated estrogen receptor α*

Estrogen receptor was immobilized as described above and incubated with 100 μM estradiol for 15 minutes prior to the addition of phage for affinity selection. Sequences obtained in the presence of estradiol are listed in Table 2.

In the presence of estradiol, numerous sequences were isolated which contain the consensus LXXLL. This motif, which is found in nuclear receptor co-activators, has previously been shown to be necessary and sufficient for their association with nuclear receptors. This association is accomplished via a helical region found in the ligand binding domain of the ER that is exposed upon binding of estradiol. Crystallographic studies indicated that this region is not properly positioned in the presence of some SERMS, thus preventing co-activator association at this site. See generally D. M. Heery, E. Kalkhoven, S. Hoare, M. G. Parker, Nature **387**, 73 (1997); M. Nichols, J. M. J. Rientjes, A. F. Stewart, *EMBO J.* **17**, 765 (1998); W. Feng et al., *Science* **280**, 1747 (1998); A. M. Brzozowski et al., *Nature* **389**, 753 (1997).

Consistent with this, peptide sequences containing the LXXLL motif were not isolated during affinity selection on the apo-receptor or in the presence of 4-OH tamoxifen.

*Example 1.3: Classification of peptide sequences*

a.) Comparison of phage vis-a-vis binding to the ER α and β in the presence or absence of estradiol

Phage expressing distinct peptide sequences were classified according to a number of different parameters. Initial studies measured the relative binding of each of the phage to ER α and β in the absence or presence of estradiol. ER α and β were immobilized on Immulon 4 plates and treated for 15 minutes with 100 μM estradiol or buffer alone prior to the addition of phage supernatant from a fresh overnight culture. Bound phage were detected using an anti-M13 antibody coupled to HRP. From these results, 12 phage were selected for further study. Sequences were selected that bound preferentially to ER α or ER β and that bound preferentially in the absence or presence of estradiol.

b) Competition of phage with a peptide containing an LXXLL motif.

The co-activating proteins that that have been identified to date, interact with nuclear receptors via a leucine rich region on the coactivator with the consensus LXXLL, where L is leucine and X is any amino acid. Co-activators containing this consensus motif bind to the ER at helix 12 in the AF2 domain (the C-terminal transactivation domain). This helical region is exposed when the receptor is activated. Many of the peptide sequences that were isolated for the activated receptor were leucine rich and a great number contained the LXXLL motif. All of these sequences bound preferentially to the activated ER. A peptide containing an LXXLL motif was synthesized and used in competition assays with phage to determine if the binding of the LXXLL peptide to the ER would affect the binding of the phage. The peptide sequence corresponds to peptide #4 that was isolated in the presence of estradiol: SSNHQSSRLIELLSRSGSGK-biotin.

ER α and β were immobilized as described above and preincubated in the presence of 100 μM LXXLL peptide, 100 μM estradiol, buffer alone, or a combination of 100 μM estradiol and 100 μM peptide for 20 min prior to adding phage supernatant from a fresh overnight culture. Bound phage were detected as described above. All of the phage expressing an LXXLL containing peptide were competed by the peptide, and several other phage that do not contain the LXXLL motif were also competed by the peptide. These phage may express sequences that mimic the LXXLL motif, or they may be allosterically affected by the binding of the peptide. There were also phage that do not contain an LXXLL motif that did not compete with peptide.

Based on these data, the peptide sequences were divided into 5 classes listed below, as seen in Table 3 and 4. Table 3 lists peptides of each class, while Table 4 defines the classes. In a comparison of binding to unliganded ER α and β, class 1 and class 5 peptides have higher affinity for ER β. Class 2, 3 and 4 peptides have higher affinity for ER α. Ligand (estradiol) increases the affinity of class 1 peptides for both ER α and β, and decreases the binding of class 5 peptides to both receptors. Ligand has no effect on the binding of class 2 peptides to either receptor. Ligand increases the binding of class 3 peptides to ER α, while having no effect on ER β, and ligand decreases the binding of class 4 peptides to ER a while also having no effect on ER β. A peptide containing an LXXLL motif, described above, was able to compete with phage from class 1 on both ER α and β, and with phage from classes 4 and 5 on ER a only. Phage from classes 2 and 3 did not compete with the LXXLL peptide on either receptor.

*Example 1.4: Fingerprinting estrogen receptor agonists and SERMs*

There are many known agonists and SERMs for the estrogen receptor. For initial testing of the fingerprinting system, two agonists, 17-β estradiol and estriol, and three SERMs, 4-OH tamoxifen, nafoxidine and clomiphene, were selected. All three SERMs are derivatives of triphenylethylene. All reagents were purchased from Sigma.

The effect of agonists and SERMs on the binding of phage from each of the 5 classes described above was investigated. To do this, immobilized ER α or ER β was incubated with 100 μM estradiol, estriol, nafoxidine, tamoxifen or clomifene in TBST or with TBST alone for 20 minutes prior to adding the phage supernatant from a fresh overnight culture. Following a 1 hour incubation, the wells were washed five times with TBST and the bound phage were visualized using an anti-M13 antibody coupled to HRP.

The following fingerprints were identified (Table 6). The data are based on the relative change in binding (as determined by an increase or decrease in absorbance) compared to the unliganded receptor. The number of + or - signs indicates the degree and the direction of the change in signal; +/- indicates no significant change.

The agonists (estradiol and estriol) produce fingerprints that are distinct from those of the SERMs (tamoxifen, nafoxidine and clomiphene). In addition, the fingerprints are different for ER α and ER β. As predicted, the agonists, which have similar biological effects, produce fingerprints that are similar on each receptor. The SERMs are all from the same class of triphenylethylene derivatives and have similar yet distinct biological effects. The fingerprinting analysis readily distinguishes them from pure agonists and also indicates that they may have similar yet distinct *in vivo* activities.

If an increase in the binding of a class 1 peptide indicates agonist activity, then the fingerprint suggests that tamoxifen produces low levels of agonist activity on ER a and no agonist activity on ER β. Similarly, if the reduction in the binding of a class 4 peptide indicates agonist activity, then the fingerprint suggests that tamoxifen has antagonist activity on both ER α α and β. The combination of the signals with each peptide class creates a fingerprint for the SERM that provides information on the relative levels of agonist and antagonist activity it produces. The differential changes in the signals on ER α and ER β may indicate the tissue specificity of the alteration in receptor activity in response to the SERM.

*Example 2 Further Investigations with Estrogen Receptors*

Affinity selection of phage displayed peptide libraries (Sparks, et al. (1996), Phage Display of Peptides and Proteins, A Laboratory Manual, pp. 227-253) was conducted on both ERα and β under conditions that were predicted to place the ER in different conformations: apo-ER, estradiol bound ER and 4-OH tamoxifen bound ER. Unique sets of high affinity peptides were identified under each condition. Most notably, affinity selection of peptides in the presence of estradiol revealed a number of sequences containing an LXXLL motif (Table 100A). This motif, which is found in nuclear receptor co-activators (Table 100B), has been shown to be necessary and sufficient for their association with nuclear receptors (Heery, et al. (1997), Nature, 387:733-736). Studies have shown that the association of the LXXLL motif with the ER is accomplished via a helical region in the ligand binding domain of the receptor that is exposed upon binding estradiol. Structural studies using X-ray crystallography have shown that this region is not properly positioned in the presence of raloxifene (Brzozowski, et al. (1997)) or 4-OH tamoxifen (Shiau, et al. (1998)), thus preventing the interaction of the co-activator LXXLL motif. The identification of these sequences in the presence of estradiol indicate that the ER is undergoing conformational changes in response to ligand in vitro consistent with the changes that are predicted to occur in vivo.

Materials

Estrogen receptor α and β were purchased from PanVera Corporation, Madison, WI. Immulon 4 96-well plates were from Dynatech. Streptavidin, 17-β estradiol, 4-OH tamoxifen, nafoxidine, clomiphene, diethylstilbestrol, progesterone, 16-α OH estrone, and estriol were purchased from Sigma. Premarin is a product of Wyeth-Ayerst. Raloxifene is a product of Eli Lilly Corporation. ICI 182,780 was purchased from Tocris Cookson Inc., Ballwin, MO. Anti-M13 antisera was purchased from Pharmacia. Sequencing of single strand M13 DNA was conducted by Sequetech Corp., Mountain View, CA. Peptide synthesis was conducted by AnaSpec, San Jose, CA.

*Example 2.1:*

Additional phage affinity selections were made of peptides which bound plastic-immobilized Erα in the presence of the SERMs 4-OH Tamoxifen, ICI 182,780, or both simultaneously (see Table 7).

*Example 2.2:*

Further phage affinity selections were made with ERα or Erβ conjugated to ERE (estrogen response element), which in turn was immobilized. For Era, selections were carried out with no ligand present (apo-receptor), or in the presence of 17-β estradiol, 4-OH Tamoxifen, Raloxifen, or ICI 182,780.

The methodology is described in more detail below. Affinity selection of phage for the various conformations of the estrogen receptor was conducted essentially as described (Sparks, et al. (1996)). Selections were conducted with the estrogen receptor in TBST (10nM Tris-HC1, pH 8.0, 150 nM NaCl, 0.05% Tween 20), or in TBST containing 1 μM 17-β estradiol, or 4-OH tamoxifen. Immulon 4 96-well plates were coated with streptavidin in 0.1 sodium bicarbonate. The plates were then incubated for 1 h with 2 pmol biotinylated, vitellogenin estrogen response element (ERE) per well (Anderson (1998), Biochemistry, 37:17287-17298), followed by incubation for 1 h with 3 pmol (monomer) ERα or ERβ per well. Oligonucleotides corresponding to the vitellogenin ERE, biotin-GATCTAGGTCACAGTGACCTGCG (forward) and biotin-GATCCGCAGGTCACTGTGACCTA (reverse), were synthesized by Genosys. The sequenced active peptides are shown in Table 8. For ERβ, selections were carried out with no ligand present, or in the presence of estradiol or tamoxifen. The resulting active peptides are shown in Table 9.

*Example 2.3*

All of the phage were classified based on their ability to bind to ER a and ER β, in the presence or absence of SERMs. These assays were conducted by phage ELISA. In essence, plastic plates were coated with streptavidin (sigma). Biotinylated-EREs (see above) were conjugated to the solid-phase streptavidin, and ER to the ERE. Bound phage were detected using horseradish peroxidase-labeled anti-(M13 phage) antibodies.

The ER was then incubated with 100μl TBST or TBST containing 1 μM of the appropriate modulator. Phage (40μl), from a 5 hour culture grown in DH5αF' cells, was added directly to the wells and incubated 30 minutes at room temperature. Unbound phage were then removed by 5 washes with TBST. Bound phage were detected using an anti-M13 antibody coupled to horseradish peroxidase (HRP). Assays were developed with 2,2'-azinobis(3-ethylbenzothiazoline)-6 sulfonic acid (ABTS) and hydrogen peroxide for 10 minutes and then stopped by the addition of 1% SDS. Absorbance was measured at 405 nm in a Molecular Devices microplate reader.

The results are shown in Tables 11-13, as follows:

Table 11, Binding of ERα-Selected Peptides to ERα Receptor;

Table 12, Binding of ERα-Selected Peptides to ERβ Receptor;

Table 13, Binding of ERβ-Selected Peptides to ERα or ERβ Receptors.

The binding activity is indicated on a semiquantitative scale of 0 to 7+.

*Example 2.4*

Selection and Characterization of Panel Peptides

All of the affinity selected phage were evaluated by phage ELISA for binding to apo- ERα and β, and to ERα and β in the presence of estradiol or 4-OH tamoxifen as described above. Many phage showed distinct preferential binding. Some sequences bound more strongly to the apo-receptor, while others exhibited preferential binding to the estradiol activated or the 4-OH tamoxifen activated receptor. Based on this analysis, eleven phage expressing different peptide sequences and showing distinct binding preferences, were chosen for further use as conformational probes.

Five of these probes bound to both ER α and ER β(α/β I-V), three were specific for ER α (α I-III), and three were specific for ER β (β I-III) (see Table 10). One may view this either as defining a three class panel, with several representatives in each class, or as an eleven class panel, with one member per class. The identification of distinct classes of peptides, some of which recognized both ERα and ERβ, and others that were receptor specific is consistent with the primary structures of the two receptors being similar yet distinct.

The binding sites of the probes, α/β I-V and α I-III, were mapped on ER a using ERα ligand binding domain (residues 282-595) fused to glutathione-S-transferase (GST), an Erα amino terminal domain (1-184) fused to GST, and the full length ER. Assays were conducted using the format described in Example 2.3, except that the domains were directly immobilized on the plastic surface of the well. Assays were conducted as for phage ELISA (Ex. 2.3). Results are shown in Fig. 2.

All of the probes except α I bound to the ligand binding domain. The α I probe, which binds only to the full length protein, may be binding to a site that is created by the tertiary structure formed by the interaction between receptor domains.

The probes were used to fingerprint the interaction of ER α and ER β with a variety of different SERMs by the assay method previously described (using ERE). Next, we evaluated the binding of each of the probes to ERα and ERβ in the presence of a variety of ER ligands that have distinct biological activities. The goal was to determine if each of the ligands would induce a conformational change in the ER that would alter the binding pattern of the probes, thus producing a "fingerprint" for each compound. The ligands used for this study include the ER agonists estradiol, estriol, and diethylstilbestrol (DES); the SERMs 4-OH tamoxifen, nafoxidine, clomiphene, and raloxifene; the antagonist ICI 182,780; and the estradiol metabolite 16-α-OH estrone. Premarin, the mixture of conjugated estrogens used as estrogen replacement therapy, was also included, but it should be noted that many of the components of Premarin must be metabolically activated. Thus, their action may not be detected in this *in vitro* assay. Buffer only (apo-receptor) and progesterone were included as controls. Information on the structures and biological effects of the SERMs used in this study may be found in the following papers and reviews: B. S. Katzenellenbogen, M. M. Montano, K. Ekena, M. E. Herman, E. M. McInerney, *Breast Can. Res. Treat.* **44**, 23 (1997) ;J. I. Macgregor and V. C. Jordan, *Pharmacological Rev.* **50**, 151 (1998); B. T. Zhu and A. H. Conney, *Carcinogenesis* **19**, 1 (1998); M. T. R. Subbiah, *Proc. Soc. Exp. Biol. Med.* **217**, 23 (1998); Sulistiyani, S. J. Adelman, A. Chandrasekaran, J. Jayo, R.W. St. Clair, *Arteriosclerosis, Thrombosis, and Vascular Biology* **15**, 837 (1995) ; B. R. Bhavnani and A. Cecutti, *J. Clin. Endocrinol. And Metab.* **78**, 197 (1994); B. Bhavnani, *Proc. Soc. Exp. Biol. Med.* **217**, 6 (1998); T.A. Grese et al., *Proc. Natl. Acad. Sci. USA* **94**, 14105 (1997); T. A. Grese et al., *J. Med. Chem.* **41**, 1272 (1998); A Howell, *Oncology 11,* **suppl 1**, 59 (1997).

As shown in Table 14, each of the ligands tested did indeed alter the binding pattern of the probes, producing a distinct fingerprint for each, whereas the pattern produced by progesterone was indistinguishable from that produced by buffer.

The unique ligand dependent binding patterns of the probes indicates that each ligand induces a receptor conformational change that exposes different peptide binding surfaces. The binding patterns for estradiol and ICI 182,780 are distinct or both ERα and β, confirming the conformational change illustrated by the earlier protease digestion studies. The protease digestion assay, which relies on the location of cleavage sites for detection of conformational changes, could distinguish between conformational changes induced by estradiol and 4-OH tamoxifen or estradiol and ICI 182,780. However, it was unable to distinguish between changes induced by 4-OH tamoxifen and other ER modulators such as ICI 182,780. The fingerprint assay, however, clearly indicates that unique peptide binding surfaces are exposed on both ERα and β in the presence of 4-OH tamoxifen that are not exposed in the presence of ICI 182,780. Tamoxifen, nafoxidine and clomiphene contain the same triphenylethylene core structure. These three compounds, although similar in structure, produce distinct biological effects. Therefore, it might be predicted that these compounds would induce similar, yet distinct, conformational changes in the receptors. The fingerprint assay shows that the probes α/β III, IV and V, which have high affinity for the ER in the presence of 4-OH tamoxifen, have lower affinity for the ER complexed with nafoxidine and clomiphene, indicating that these peptide binding surfaces differ in the presence of these compounds. The a III probe more clearly differentiates these three compounds. The fingerprint assay also differentiates 4-OH tamoxifen and raloxifene. The probes α/β III, IV and V have reduced affinity for both ER a and β in he presence of raloxifene compared to 4-OH tamoxifen. The probes α/β II, β I and β III further distinguish ER β conformational changes induced by these two compounds. The fingerprint pattern produced by Premarin is distinct compared to other agonists; however, Premarin's activities are due to a mixture of components. It would be interesting to assess the binding patterns of the probes in the presence of each of the purified, activated components of Premarin.

The probe α/β I contains an LXXLL motif. The binding of estradiol to the ER strongly enhanced the binding of this probe to both ER a and ER β. However, estriol, Premarin and DES, which are also considered ER agonists failed to activate the binding of this probe to ER α to the same extent as estradiol. On ER β, the binding of the probe was enhanced significantly with all of the agonists. The SERMs, 4-OH tamoxifen, nafoxidine, clomiphene, raloxifene and ICI 182,780 prevented the binding of this probe to both ER α and β and appeared to reduce the binding to a level below that which is observed in buffer alone.

The probes α/β III-V show enhanced binding in the presence of SERMs, particularly 4-OH tamoxifen, indicating that a new binding surface is exposed on the ER in the presence of these compounds. The binding patterns of these three probes along with the probes α/β II, α III, β I and β III illustrate differences in the receptor conformation induced by 4-OH tamoxifen, nafoxidine, clomiphene, and raloxifene. Since the binding of the probes to the ER in the presence of these SERMs may be altered but not abrogated, subtle changes in receptor conformation can be visualized. **This is the first *in vitro* assay that distinguishes between these four compounds.** The probe α II is also unique in that it binds to ER α in the presence of any compound that binds to the estrogen receptor, indicating that while some receptor conformational changes are unique to the modulator, others may be more universal. Overall, these probes allow the detection of both subtle and distinct conformational changes that are induced by many different modulators of ER activity.

To confirm that the binding of the probes to the ER was dependent upon the peptide expressed on the surface of the phage, biotinylated peptides corresponding to the sequences were synthesized with biotin attached to a carboxy-terminal lysine. The peptides were coupled to europium labeled streptavidin and binding studies were conducted using time resolved fluorescence spectroscopy (TRF).

Time resolved fluorescence (TRF) assays were performed at room temperature as follows: Costar high-binding 384 well plates were coated with streptavidin in 0.1 M sodium bicarbonate and blocked with bovine serum albumin. Biotinylated ERE (2 pmol) was added to each well. Following a 1 h incubation, biotin was added to check any remaining binding sites. The plates were washed and 2 pmol ERα was added to each well. Following a 1h incubation, the plates were washed and the ER modulators were added at a range of concentrations, from picomolar to micromolar. Following a 30 min incubation with the modulators, 2 pmol of a europium labeled streptavidin (Wallace)-biotinylated peptide conjugate (prepared as described below) was added and incubated for 1 h. The plates were then washed and the europium enhancement solution was added. Fluorescent readings were obtained with a POLARstar fluorimeter (BMG Lab Technologies) using a <400 nm excitation filter and a 620 nm emission filter. The europium labeled streptavidin-biotinylated peptide conjugate was prepared by adding 8 pmol biotinylated peptide to 2 pmol labeled streptavidin. After incubation on ice for 30 min, the remaining biotin binding sites were blocked with biotin prior to addition to the ER coated plate.

The binding of the probes to the ER was measured. The results, shown in Table 15, indicate that the peptides are indeed conferring the binding specificity. Comparison of the fluorescence values obtained from the TRF binding assays and the signals obtained in the phage ELISA fingerprint indicates that the two methods produce similar patterns. However, the binding assay also provides an indication of the potency of each compound to induce the conformational change required for peptide binding. Taken together, these results indicate that conversion of the fingerprint assay from phage to peptides will provide an even more sensitive assay for detecting conformational change.

One of the most notable observations from the TRF binding assays is that the binding of the β I probe to ER β is enhanced in the presence of the SERM 4-OH tamoxifen and reduced in the presence of other SERMs such as raloxifene, nafoxidine, and clomiphene. The reduction in binding observed with these compounds is similar to the reduction observed with agonists such as estradiol, estriol, and DES.

We have identified peptides that serve as conformational probes of the estrogen receptor α and β. Many probes bind to both receptors, while other probes bind preferentially to either the α or β receptor. Consistent with the two receptors having regions of high homology and other more divergent regions, these results indicate that the receptors have some binding surfaces in common, while others are unique. The implications of this are that both receptors may contact some of the same regulatory proteins in the cell, yet there may be additional proteins that specifically regulate either ER α or β action.

We have used our peptidic probes to show that both receptors undergo distinct conformational changes as a result of binding different ligands. The probes not only reveal receptor conformational changes by their relative changes in affinity, but they also identify unique binding surfaces on the two receptors. These binding surfaces may, in fact, be the surfaces that interact with various co-regulatory proteins in response to different ligands. For example, many peptides selected with the estradiol activated receptor contained sequences found in nuclear receptor co-activators, as illustrated by the peptides containing the LXXLL motif (Figure 1). These peptide probes are probably mimicking the interaction between the receptor and co-activating proteins. Potentially, these probes can be used to identify heretofore, unknown receptor-protein interactions.

Additional applications of the probes lie in the area of detection of ER modulators. One or more probes can be used to set up a high-throughput screen to identify modulators of ER activity. We anticipate that compounds that bind to the ER will alter receptor conformation and hence alter the binding patterns of the probes. The sites targeted by the screen may not be *bona fide* protein-protein interaction surfaces, but may represent sites exposed in the presence of a specific ligand, and thus serve as markers for specific conformations. The fingerprinting technique may also be applied to quickly classify hits from a screen into different categories such as agonist (resembling the estrogen pattern), antagonist (resembling the ICI 182,780 pattern), mixed (resembling the tamoxifen pattern) or novel effectors, prior to assessing them in a cell-based assay. Fingerprinting may also be used to determine structure activity relationships and to rapidly assess compounds following chemical modification during lead optimization.

This is the first technique described that can distinguish between estrogen receptor conformations induced by ligands both between and within ligand classes. The data gathered with this assay provide strong evidence that the biological activity of the estrogen receptor can be linked to the conformation induced upon binding ligand. A strength of this fingerprinting technique is that it is broadly applicable to any protein or receptor that undergoes structural changes upon binding of a ligand or substrate.

These studies confirm that the assays may readily be conducted with synthetic peptides in place of phage-bound and - expressed peptides.

*Example 2.5 Analysis of Known SERMS using Panel*

For fingerprint analysis of estrogen receptor modulators on ER α and ER β, estrogen receptor (3 pmol) was immobilized on 2 pmol biotinylated ERE. Immobilized ER was incubated with estradiol (1 μM) , estriol (1 μM), premarin (10 μM), 4-OH tamoxifen (1 μM), nafoxidine (10 μM), clomiphene (10 μM), raloxifene (1 μM), ICI 182,780 (1 μM), 16α-OH estrone (10 μM), DES (1 μM) or progesterone (1 μM) for 5 minutes prior to the addition of phage. Phage were amplified from plaques in DHSα F' for 5 hours. Bound phage were detected as described previously. Assays were developed with ABTS (2,2'-azinobis(3-ethylbenzthiazoline-sulfonic acid) for 10 minutes.

The results are shown in Tables 14A and 14B. Table 14A shows binding to the ERα receptor and Table 14B binding to the Erβ receptor. It is not necessary to list all 11 panel peptides in each table since some only bind the ERα and others only the ERβ. The binding activity is indicated on a semiquantitative scale of 0 to 7+.

*Example 2.6 Calculation of Similarity Between SERMs*

Based on Tables 14A and B, one may define a fingerprint for each SERM. This fingerprint is an array of descriptors, each of which is a value in the Table representing the binding affinity of a particular panel peptide for either ERα or ERβ in the presence of the SERM in question. The tables in question allow each fingerprint to be composed of 16 descriptors (one for each row in the tables). We obtain 12 fingerprints, one for each of the 11 SERMs, plus buffer.

We can therefore calculate the similarity of between each of the 12x12=144 possible pairings of these fingerprints. To begin with, we calculate the Euclidean distance between each fingerprint. This is the square root of the sum of the square of the differences between the respective column values in Tables 14A and 14B. For example, the distance between buffer and Estradiol is the square root of the sum of the square of the 16 descriptor pair differences, i.e., the square root of the sum of

(1-6)^2, 25;
(7-2)^2, 25;
(1-1)^2, 0;
(1-1)^2, 0;
(1-1)^2, 0;
(7-7)^*2, 0;
(1-6)^2, 25;
(5-2)^2, 9;
(2-7)^2, 25;
(7-2)^2, 25;
(2-1)^2, 1;
(1-1)^2, 0;
(6-3)^2, 9;
(1-5)^2, 16; and
(7-7)^2, 0;

for a total of 160, the square root of which is about 12.65.

The maximum possible distance in the present instance is the square root of 16*(7*7), which is 28. This is because each descriptor pair has a maximum possible difference of 7, and there are 16 descriptors in the fingerprint.

The distance may be converted into a similarity by

$$\text{current similarity} = (\text{maximum distance} - \text{current distance}) / \text{maximum distance},$$

which equals 1 when the current distance is 0, and 0 when the current distance equals the maximum distance. In the example above (buffer:estradiol) the similarity is 0.55.

In contrast, the fingerprints of cloniphene and raloxiphene are at a Euclidean distance of sqrt(40), which is 6.32, and therefore have a similarity of 0.77. 16α-OH estrone and DES are even more similar, with a Euclidean distance of sqrt(7), which is 2.645, and therefore a similarity of 0.91.

On the other hand, the fingerprints of estradiol and cloniphene are at a Euclidean distance of sqrt(210), which is 14.49. This corresponds to a similarity of 0.48.

It will be appreciated that we could have changed the choice and/or number of descriptors incorporated into the fingerprint, rescaled and/or weighted the descriptors in some way, used a different measure of distance, and/or converted distance into similarity by another method. We could also have determined similarity without first calculating a distance.

In the above text, we have lumped together the data for ERα and ERβ. We could have calculated separate fingerprints and similarities for each form of ER. This is shown in Figs. 5 and 6.

Thus, for buffer:estradiol, the distance between their fingerprints for ERα binding is SQRT(84), and for ERβ binding, SQRT(76), or 9.16 and 8.72. The maximum distance is SQRT (8*7*7), which is 19.8. So the similarities are 0.54 and 0.56 respectively, which aren't much different.

On the other hand, for ICI 182 780:16α-OH estrone, we calculate distances of SQRT(6) for ERα binding, and SQRT(76) for ERβ binding, corresponding to similarities of 0.88 and 0.56, respectively. So these compounds are more similar in how they bind ERα than in how they bind ERβ.

This fingerprinting technique provides a rapid and sensitive method to detect changes in protein conformation. We have applied this technique to ER α and β and demonstrated that these two receptors undergo different conformational shifts in response to various modulators of activity. Because the pattern of probe binding is unique for each modulator, the assay can be used to distinguish compounds both between and within modulator classes. The assay can also be used to identify modulators that have specificity for either the α or β form of the receptor.

One or more probes can be used to set up a high-throughput screen (HTS) to identify modulators of ER activity. Compounds that bind to the ER and alter receptor conformation will alter the binding patterns of the probes. This technique may also be applied to classify hits from a HTS as agonist (resembling the estrogen pattern) antagonist (resembling the ICI 182,780 pattern) or mixed (resembling the tamoxifen pattern) prior to assessing them in a cell-based assay. Fingerprinting may also be used for structure activity relationships. As chemical modifications are made to lead molecules, fingerprinting will provide a convenient method to quickly determine if the modification affects receptor conformation in a manner different than the parent compound.

All of the compounds used in this study are known to produce unique biological effects in vivo. Many of the differential effects are tissue specific, perhaps due to differential expression of regulatory proteins and/or the two forms of the receptor. Each of these compounds also produces a unique fingerprint pattern *in vitro,* derived from the conformation adopted by the receptors upon binding the modulator. Thus, fingerprinting conformational changes induced by SERMs *in vitro* is expected to be useful for predicting the *in vivo* biological activities of modulators.

*Example 3: Fingerprinting Using Yeast Two-Hybrid Cell-Based Assays*

The two hybrid methods of examining protein/protein interactions initially described by Fields and Song (Nature 340:245-246 (1989)) and later by Gyrius, et al (Cell 75:791-803 (1993)) utilize similar technologies. In both cases a yeast cell is provided as the host cell which carries a reporter gene operated by an upstream protein binding site (DNA binding site). The host cells carry a plasmid expressing peptide/protein fusions with the specific binding protein or domain (DNA binding domain). The host cell also carries a plasmid expressing a peptide/protein fusion with a transcriptional activation protein or domain (Activation domain). If the two peptide/protein fusions are capable of directed interactions within the cell, transcriptional activation of the reporter gene occurs. The level of reporter gene transcription is reflective of the strength of the interaction between the two protein fusions.

The LexA system that we employ utilizes a bacterial DNA binding protein domain, LexA, and a bacterially derived transcriptional activation sequence, B42. Proteins or peptides of interest are fused in frame with these domains and expressed using episomal plasmids in a yeast cell. The interactions between these proteins/peptides of interest are registered by monitoring the level of the reporter gene product, β-galactosidase, by an enzymatic assay. The differences in the levels of β-galactosidase activities reflect the relative strengths of the protein interactions.

We have tested the interactions of peptides F6 (an affinity-selected peptide with a high affinity for ERα), alpha2 (A2), alpha/beta 3 (AB3), and alpha/beta 5 (AB5) with estrogen receptor a using the LexA yeast two hybrid system in the presence of agonist or antagonist. These peptides were isolated previously from phage display libraries using estrogen receptor α (ERα) as a target. The interactions between these peptides and ERα are altered in the presence of agonist or antagonist in the in vitro phage display system. For example, peptide α2 was found to bind in the presence of estradiol and 4-OH-tamoxifen, but not in their absence; peptide α/β 3 binds to ERα only in the presence of 4-OH-tamoxifen, not estradiol or in the absence of any compound. We undertook the yeast two hybrid analysis to investigate whether these in vitro results could be recapitulated in vivo. The results from the yeast two hybrid system were qualitatively similar to those that were performed using phage display on purified ERα protein.

Yeast strains and genetic manipulations

References for plasmids and strain

Cloning vector pJG4-5

Genbank Accesion number: U89961

Reference: Gyuris,J., Golemis,E., Chertkov,H. and Brent,R. Cdi1, a human G1 and S phase protein phosphatase that associates with Cdk2. Cell 75 (4), 791-803 (1993)

Cloning vector pEG202 (pLexA), complete sequence.

Genbank Accession number: U89960

AUTHORS Golemis,E., Gyuris,J. and Brent,R.

TITLE Interaction trap/two-hybrid systems to identify interacting proteins

JOURNAL Unpublished

pJK103

Reference:. J. Kamens and R. Brent A yeast transcription assay defines distinct *rel* and *dorsal* DNA recognition sequences. New Biol. 3:1005-1013 (1991).

Yeast Strain EGY48

Reference: Gyuris,J., Golemis,E., Chertkov,H. and Brent, R. Cdi1, a human G1 and S phase protein phosphatase that associates with Cdk2. Cell 75 (4), 791-803 (1993)

The yeast strains used in this study was EGY48 (MAT a trp1 his3 ura3 leu2::61exAops-LEU2) purchased from OriGeneTechnologies for yeast two hybrid analysis. This strain contains 6 LexA operators upstream of the LEU2 gene in the yeast genome and provides high sensitivity in detecting protein-protein interactions in the LexA two hybrid system. The plasmids used in this study were pEG202 (LexA-DNA binding domain), pJG4-5 (B42-activation domain), and the plasmid containing a β-galactosidase reporter, pJK103 (OriGene Technologies). The full length estrogen receptor was subcloned in frame into the EcoRI and XhoI sites of pJG4-5 to generate an ERα-B42 activation domain fusion. ERα was subcloned in the activation domain plasmid because ERα was able to autoactivate reporters when fused to the LexA DNA binding domain.

The peptide sequences used in this study were generated from synthetic oligos filled in by T7 Sequenase (Life Science) and subcloned into the EcoRI-XhoI sites of pEG202. The synthetic oligos were:

F6, 5'-
GACTGTGCGAATTCGGTCATGAACCATTAACTTTATTAGAAAGATTATTAATGGATGATA
AACAAGCTGTTCTCGAGCGTGTCAG;

αII, 5'-GACTGTGCGAATTCTCTTCTTTAACTTCTAGAGATTTTGGTTCTTGGTATGCTTCTAGACTCGAGCGTGTCAG;

α/βIII, 5'-GACTGTGCGAATTCTCTTCTTGGGATATGCATCAATTTTTTTGGGAAGGTGTTTCTAGACTCGAGCGTGTCAG;

α/βV, 5'-GACTGTGCGAATTCTCTTCTCCAGGTTCTAGAGAATGGTTTAAAGATATGTTATCTAGACTCGAGCGTGTCAG.

The complementary synthetic oligo used to generate double stranded DNA was 3'XhoPrim, 5'-CTGACACGCTCGAG. Each 5'-oligo was annealed to the 3'-oligo by heating to 90 °C for 15 minutes and cooled slowly to 35 °C. T7 sequenase was added and the fill-in reaction allowed to proceed at 30 °C for 30 minutes. The reaction was terminated by heat denaturing the enzyme at 65 °C for 1 hour, restriction digests were performed and the resulting DNA fragments subcloned into pEG202 to generate peptide-LexA DNA binding domain fusions.

Yeast cells were transformed by the method of Ito et al. (J. Bacteriol. **153**: 163-168 (1983)) and grown on selective media.

β-galactosidase activity assays

10 ml cultures of yeast strain EGY48 containing pJG4-5 ERα pJK103 and pEG202-F6, -αII, -α/βIII, or -α/β V were grown overnight at 30 °C in selective media containing 100 nM estradiol, 4-OH tamoxifen, or tamoxifen citrate with galactose as the carbon source. The culture was diluted to ~2x10$^6$ cells/ml in the same media and allowed to grow at 30 °C until the cultures reached a density of ~1x10$^7$ cells/ml (~4 hours). The yeast cells were pelleted by centurifugation, washed with extraction buffer (60 mM $Na_2HPO_4$, 40 mM$NaH_2PO_4$, 10 mM KCl, 1 mM $MgSO_4$, 1mM PMSF, 7 mM 2-mercaptoethanol) and suspended in 200 μl of extraction buffer. 100 μl of acid-washed glass beads were added and cells were lysed by vigorous agitation for 10 minutes at 4 °C. Cellular debris was pelleted by centrifugation and the supernatant transferred to a clean tube. 10 μg of total cellular protein was diluted into complete Z buffer (60 mM $Na_2HPO_4$, 40 mM$NaH_2PO_4$ , 10 mM KCl, 1 mM $MgSO_4$, 7 mM 2-mercaptoethanol) to a volume of 100 μl in a 96-well microplate. 80 μg of o-nitrophenyl-β-D-pyranoside (ONPG) in 20 μl was added to each well to initiate color development. The reaction was stopped by the addition of 30 μl 1M $Na_2CO_3$ and the time for development was noted. β-galactosidase activity was determined by measuring the absorbance at 405 nm.

Yeast cultures were grown in the presence or absence of 100nM estradiol, 4-hydroxy-tamoxifen, or tamoxifen citrate and protein extracts prepared as described in methods. 10μg of each protein extract was assayed for β-galactosidase activity using o-nitrophenyl-b-D-pyranoside as substrate. Activity units are defied as 1000* $Abs_{405}$/minutes/mg/protein.

The results are shown in Table 99.

The peptides that were used in the two hybrid assay were originally isolated by phage display using ER alpha as the target protein. The isolation procedure was carried out in the presence of agonist or antaogonists (estradiol, 4-hydroxy tamoxifen,...) which generated a differing set of interacting peptides. The interactions of these peptides with ER alpha were investigated in vitro using diferent agonists and antagonists. The interaction profile generated by these in vitro studies allows us to use these peptides as probes for the physical state of the estrogen receptor. The two hybrid assay discerns whether these interactions can be maintained whithin the cell.

The results from the two hybrid experiments show a qualitatively similar interaction profile between the peptides and ER alpha as determined in vitro. Therefore, the effects of agonists and antagonists on the structure and availability of peptide binding sites on ER alpha is maintained in vitro and in vivo. These results allow interpretation of the structural state (activated or antagonized) of ER alpha in response to various compounds. The results using known activators and antagonists can be used to identify other unkown compounds as agonists or antagonists in a drug screen. The availability of more peptides and use of other known agonists and antagonists will generate better tools for identifying possible compounds or drug leads.

*Example 4 Use of Mammalian Two-Hybrid Assays to Explore ER Activation*

The estrogen receptor (ER) plays an important role in both normal and pathological processes of human development and disease. Clinically, ER antagonists such as tamoxifen have met with much success in the treatment of ER containing breast cancers. However, resistance to tamoxifen usually develops within 2-5 years after initial treatment. A potential mechanism of resistance may be the ability of tumors to switch from recognizing tamoxifen as an antagonist to responding to it as an agonist.

In this regard, several new tissue specific antiestrogens have been developed which may have clinical utility in the treatment of tamoxifen refractory breast cancers.

In an attempt to identify novel high affinity ligands which target the ER/tamoxifen complex, we have employed the use of phage display to screen for random peptides which will recognize the specific ER conformation induced by tamoxifen. We have isolated a series of 15mer peptides which can recognize this complex. Furthermore, these peptides are able to form complexes in vivo with ER as assessed in the mammalian two-hybrid system. Using various ER mutants, we have mapped the peptide interaction surface to the hormone binding domain. Importantly, we have demonstrated that expression of these peptides can block the partial agonist activity of tamoxifen in cells transfected with ER. Although the mechanism by which these peptides block ER/tamoxifen transcriptional activity remains unknown, it appears that DNA binding and ER stability are not affected by peptide expression. Therefore, it is possible that these peptides may be targeting a functionally active site present in the ER/tamoxifen conformation.

This makes possible a novel approach in the development of rational drug design for the treatment of tamoxifen refractory breast cancers. Traditionally, only molecules which interact with the ligand binding pocket have been considered for the development of novel ER antagonists. In addition to these traditional "hormonal" agents, we propose that the ability to target the specific receptor conformation induced by hormones will result in the development of therapeutically important novel pharmaceuticals. Furthermore, these findings may be applied to other nuclear receptors for which transcriptional interference may be clinically useful.

*Example 4.1*

Figure 7 shows the development of a cell-based assay to assess peptide-receptor interactions. Peptide sequences representing each class was fused to the DNA binding domain (DBD) of the yeast transcription factor Ga14. HepG2 cells were then transiently transfected with expression vectors for ERα-VP16 and the Gal4-peptide fusion proteins. In addition, a luciferase reporter construct under the control of 5 copies of a Ga14 upstream enhancer element was also transfected along with a pCMV-β galactosidase vector to normalize for transfection efficiency. Transfection of the Ga14 DBD alone is included as control. Cells were then induced with various ligands as indicated in the figure and assayed for luciferase activity and β galactosidase activity. Normalized response was obtained by dividing the luciferase activity by the β galactosidase activity.

Results. ERα does not interact with the Gal4 DBD alone under any condition. αβI interacts with ER in the presence of estradiol and somewhat with the apo-receptor. α II interacts with the receptor under all conditions with the apo-receptor and ICI 182,780 bound receptor showing the least activity. αβ III and αβ V interact almost exclusively with the tamoxifen bound receptor. This data in general confirms that obtained from the time resolved fluorescent study. Furthermore, the ability of these peptides to act as conformational detectors confirms in the cell earlier observations obtained from protease digestion and crystallization studies that the receptor undergoes distinct conformational changes when bound by different ligands.

*Example 4.2*

The specificity of peptide-nuclear receptor interaction was analyzed (Fig. 8) using the mammalian two-hybrid system. Experimental design is the same as in figure 2 except that either progesterone receptor (PRB-VP16), estrogen receptor beta (ERβ-VP16), glucocorticoid receptor (GR-VP16) or thyroid receptor beta (TRβ-VP16) was tested.

Results. All receptors tested, as expected, interact with the αβ I peptide in the presence of the appropriate agonist for that receptor. None of the receptors tested interact significantly with the α II or αβ III peptide. This was somewhat surprising considering that αβ III was originally isolated on ERβ. This suggests that the conformation of ERβ in the cell may be different from that of the purified receptor in vitro. Interestingly, αβ V was able to associate with both PRB and ERβ. This peptide bound ERβ only in the presence of tamoxifen but was able to associate with PRB in the presence of the PR antagonists RU 486 and ZK 98299. This suggest that αβ V is capable of recognizing the antagonist conformation of a subset of nuclear receptor family members.

*Example 4.3*

Figure 9 demonstrats that certain peptides which interact with the tamoxifen activated estrogen receptor do not require AF-2(Helix 12) of the receptor. Three ERα mutants were compared with wild-type ERα. ER-LL was characterized by mutations L540A/L541A. Mutant ER3X was characterized by mutations D538N/E542Q/D545N. Finally, mutant ER-535 STOP was truncated after residue V535. These mutations have been shown to significantly compromise ER AF-2 transcriptional activity and their interaction with several known coactivators. Mutant ER3X is partially AF-2 active and ERLL and ER535-stop are AF-2 inactive. Experimental design is the same as in figure 7 except that either (A) ER3X (B) ERLL or (C) ER535-stop was analyzed for binding to conformation sensitive peptides.

Results. αβ I peptide is unable to interact with the ERLL and ER535-stop mutant receptors in the presence of estradiol indicating that these mutations may abolish coactivator binding. αβ I peptide retains some ability to engage the ER3X mutant receptor in the presence of estradiol suggesting that these mutations significantly lower the affinity of the receptor for coactivators but does not destroy this interaction. These findings are consistent with the transcriptional properties of these receptors. α II peptide binding specificity is largely unaffected by any of the receptor mutations tested. Interestingly, the αβ III and αβ V peptides specificity of interaction is modified with each successive mutation resulting in a loss of the tamoxifen specificity and resulting in the ability of these peptides to engage the receptor in the presence of many of the ligands tested. These results suggest that although helix 12 is not required for the binding of peptides which recognize the conformation induced by tamoxifen that normal helix 12 structure is required for the specificity of interaction of αβ III and αβ V peptides.

*Example 4.4*

Figure 10 studies the disruption of ER mediated transcriptional activity by Gal4-peptide fusion proteins. HepG2 cells were transfected with the estrogen responsive C3-Luc reporter gene along with expression vectors for ERα and β galactosidase. Cells were induced with either estradiol or tamoxifen as indicated in the figure and analyzed for luciferase and β galactosidase activity (10A).

Then HepG2 cells were transfected as above except that expression vectors for Gal4-peptide fusions were included as indicated in 10B. Control represents the transcriptional activity of estradiol (10 nM) activated ER in the presence of the Gal4- DBD alone and is set at 100% activity. Increasing amounts of input plasmid for each Gal4-peptide fusion is also shown with the resulting transcriptional activity presented as % activation of control. Data is averaged from three independent experiments (each performed in triplicate) with error bars representing standard error of the mean, subfig. C is same as in (B) except that 4-OH tamoxifen was used to activate the receptor.

Results. Tamoxifen displays partial agonist activity in HepG2 cells. This activity is up to 30% of that exhibited by estrogen. αβ I and α II peptides are able to inhibit the ability of estradiol to activate transcription up to 50% under the conditions of this assay. It is not surprising that the αβ I peptide inhibits ER activity due to the fact that it probably competes for coactivator binding. The ability of a II peptide to disrupt ER transcriptional activity may suggest that this peptide recognizes some pocket in the receptor that is also important for coactivator binding. The inability of αβ III and αβ V to block estradiol mediated transcription correlates well with their inability to bind the receptor when bound by estradiol. Interestingly, α II, αβ III and αβ V are able to efficiently block the partial agonist activity of tamoxifen while αβ I is not. These findings are in agreement with the binding characteristics of these peptides and may suggest that the pocket(s) recognized by these peptides are important for the ability of tamoxifen to behave as a partial agonist.

*Example 4.5*

Figure 11 shows disruption of tamoxifen activated ER transcriptional activity by αII peptide is not promoter dependent. Experimental design is the same as in figure 6 except that the ability of αII peptide to inhibit tamoxifen (10nM) activated transcription was tested on several distinct promoters including 1x-ERE-Luc, 3X-ERE-Luc, TK-ERE-Luc and C3-Luc.

Results. The ability of α II peptide to block tamoxifen activated transcription is not dependent on the context of the promoter. This peptide blocks tamoxifen partial agonist activity from all promoters tested.

*Example 4.6*

Figure 12 shows disruption of ER mediated transcriptional activity through the AP-1 pathway by Gal4-peptide fusion proteins. (A) HepG2 cells were transfected with the AP-1 responsive collagenase reporter gene construct (pCOL-Luc) and expression vectors for ERα and β-galactosidase. Cells were then induced with either estradiol or tamoxifen as indicated in the figure and assayed for luciferase and β-galactosidase activity and normalized as detailed in figure 7. (B) Same as (A) except that Gal4-peptide fusion constructs were also transfected as indicated in the figure. Control represents the transcriptional activity of either estradiol or tamoxifen (100nM) activated ER in the presence of the Gal4 DBD alone and is set at 100% activity. The transcriptional activity of estradiol and tamoxifen is shown in the presence of each Gal4-peptide fusion with the resulting transcriptional activity presented as % activation of control. Data presented is from a single representative experiment.

Results. Both estradiol and tamoxifen are able to activate transcription from the AP-1 responsive collegenase reporter gene. This activity is manifest in the absence of an estrogen response element (ERE) and is believed to occur through some mechanism involving an interation between ER and the AP-1 proteins Fos and Jun. As with the C3-Luc reporter gene, each peptide is able to inhibit ER mediated transcriptional activity according to its ability to interact with the receptor in a ligand dependent manner. Those peptides which interact with the estradiol bound receptor inhibit estradiol mediated transcription while those which interact with the tamoxifen bound receptor inhibit tamoxifen mediated transcription.

*Example 4.7*

Figure 13 model of the potential mechanisms by which peptides block the partial agonist activity of tamoxifen. (A) Model of the activation pathway by which tamoxifen exhibits partial agonist activity. Upon binding tamoxifen (T), the receptor undergoes a conformational change which allows it to interact with soma as yet unidentified coactivator protein. This protein in turn transmits a signal to the general transcription machinery which results in activation of transcription. (B) In this model of inhibition, the receptor undergoes a conformational change when bound by tamoxifen but the coactivator protein is unable to engage the receptor due to competition for the same site by the peptide. (C) In this model of inhibition, the receptor undergoes a conformation change in the presence of tamoxifen which results in the formation of distinct pockets on the receptor. One pocket which is distal to the coactivator binding site interacts with the peptide. As a result of this interaction, an additional conformational change occurs precluding the interaction between the coactivator and the receptor.

*Example 5*

Figure 20 shows a similarity analysis of the data pictured in Figure 7. Each ligand has a five element footprint, the elements corresponding to the normalized transcriptional response which it induced in a mammalian two-hybrid system presening either the apo-receptor (control) or the receptor in the presence of one of the peptides αβ1, α2, αβ3 or αβ5.

*Example 101*

One of the distal steps in transcriptional activation by estrogen receptor (ER) is the recruitment by ligand-bound receptor of one of a number of coactivator proteins. This activity permits ER to interact with the general transcription machinery and exert its regulatory actions on target gene promoters. It has now emerged that one effect of agonist binding is to induce a conformational change within ER, permitting the interaction of ER helices 3 and 12, and the subsequent formation of a pocket which allows the coactivator proteins to dock. These observations suggest that receptor antagonists inhibit ER transcriptional activity by affecting the formation of the coactivator binding pocket and reducing the affinity of ER for coactivators. Although an ER-specific coactivator protein remains to be identified, several coastivaators have been identified which potentiate the transcriptional activity of ER and other members of the steroid receptor superfamily. Furthermore, the finding that these coactivators use a highly conserved LXXLL motif to interact with the receptors made it uncertain as to whether receptor-cofactor interactions were determined by simple competition or if there was some specificity built into the system.

In order to address these possibilities, we undertook a molecular approach to dissect the LXXLL-ER interaction and to evaluate the role of flanking sequences in influencing these interactions. We utilized phage display technology to screen 10 x $10^7$ variations of the core LXXLL motif. Using estradiol-activated ER as a target, we identified a number of phage which encoded high affinity ER-interacting peptides. Using the sequence information derived from these phage, we constructed a series of GAL4-peptide fusions and assessed their ability to interact with ERα, ERβ, GR and PR using a two-hybrid assay in mammalian cells. The results of this assay confirmed that the LXXLL motif was permissive for nuclear receptor binding but it also revealed that sequences flanking this motif were important determinants of specificity. Thus, as expected, not all LXXLL motifs are the same. This suggests that within a cell, specificity and not just mass action influences the ability of a nuclear receptor to find a required cofactor. In an effort to understand the mechanism underlying this observed specificity, we assayed the ability of these peptide fusions to interact with a series of ER helix-12 mutants. Using this approach we noticed that mutation of the conserved hydrophobic residues in this helix abolished ER-AF-2 function and blocked the interaction of all LXXLL peptides with ER. Disruption of helix 12 by mutating the three conserved charged residues (D538N/E542Q/D545N) prevented most peptides from binding and also abolished AF-2 function. However, a large number of the LXXLL-containing peptides studied were unaffected by this manipulation. This is an important observation since the latter mutation also blocks the interaction of ER with GRIP-1 and SRC-1. Cumulatively, our data indicate that the steroid receptors display distinct preferences for different classes of LXXLL motifs, suggesting a molecular basis for cofactor-receptor specificity. Importantly, however, they also indicate that Af-2 function and coactivator binding are not synonymous, a result which indicates that there are likely to be additional cofactors distinct from SRC-1 and GRIP-1 which remain to be discovered.

Plasmids: All the Gal4DBD-peptide fusions were constructed as follows: DNA sequences code for the peptides were excised from mBAX vector with XhoI and XbaI restriction enzymes and subcloned into pMsx vector derived from pM vector (Clontech) with a linker sequence to generate infram SalI and NheI sites for cloning. VP16ER-a construct was generated by polymerase chain reaction (PCR) of full length human ER-α cDNA with primers containing ECoRI flanking both 5' and 3' ends. The PCR product was then subcloned into pVP16 vector (Clontech) to generate the VP16-ERα fusion with VP16 located at the N-terminus of ERα cDNA, pVP16ER-β, pVP16-RARα, and pVP16-RXRα were generated in a similar fashion. pVP16VDR is a generous gift from J. W. Pike (University of Cincinnati, Cincinnati, OH); VP16TRβ expression plasmid (pCMX-VP-F-hTRβ was provided by D.D. More (Baylor College of Medicine, Houston, TX) ; VP16Gr, VP16PR-a, VP16PR-b, and VP16AR were gifts from J. Miner (GR), D.X. Wen (PR-a and PR-b), and K. Marschke (AR) (Ligand Pharmaceuticals, San Diego, CA). VP16-ER mutant constructs were generated by excision of mutant ER cDNAs from ER expression plasmids (ER-TAF1, ER-LL and ER-535 stop plasmids, Tzukerman et al. Mol. Endocrinol. 1994(8): 21-30 and Norris et al., J. Biol. Chem. (273):6679-6688, 1998) and sublconed into pVP16 vector. Mammalian expression plasmids for ERα, Erβ, and ER179C, as well as 3xERELuc receptor construct were described elsewhere (Tzukerman et al. Mol. Endocrinol. 1994 (8):21-30). 5xGal4Luc3 construct was modified from 5xGal4-TATALuc plasmid (a gift from X.F. Wang, Duke University, Durham, NC) where the luciferase gene was replaced by a modified version of luciferase cDNA from pGL3 basic vector (Promega), GRIP-1 and SRC-1 constructs were generated by subcloning PCR products corresponding to GRIP-1 a.a. 629-760 and SRC-1 a.a. 621-765 into pM vector (P.H. Giangrande, unpublished). All PCR products were sequenced to ensure the fidelity of the resultant constructs.

Example 101.1

A focused random peptide library ($X_7$-LXXLL-$X_7$ X=any AA, L=Leu) was constructed and displayed on M13 phage.

Baculovirus expressed full length ER-α was treated with $10^{-6}$ M of 17β estradiol and immobilized on 96-well Immulon-4 plates as selection targets. M13 phage-based random peptide libraries were incubated with target proteins in the wells, ER binding phage were retained while the unbound phage were washed away. Bound phage were eluted by low pH buffer, amplified in DH5αF' cells and subjected to subsequent round of selection. The selection processes were repeated 2-3 times to enrich for EB bonding phage. Individual phage were plaque purified, amplified and their binding characteristics were examined by ELISA. Phage that bound to ER only in the presence of estradiol were selected and the peptide sequences were deduced by DNA sequencing (Table 101).

The LXXL1 motif-containing peptides are major binding species in the affinity selection when estradiol activated ERα was used as a target. ER4 (Table 101) binds to agonist occupied ER but not partial agonist- or antagonist-occupied ER.

*Example 101.2*

The ability of ER4 peptide to interact with ER-α in mammalian cells was assayed in a mammalian two-hybrid system. The ER4 peptide sequence was fused to Gal4 DNA binding domain (GAL4DBD) while the full length ER was fused to the VP16 transactivation domain. The interaction between ER4 peptide and ERα is measured by the expression of 5xGal4Luc3 reporter gene. HepG2 cells were transiently transfected with (Fig. 14A) ERα expression vector and reporter 3xERELuc or (Fig. 14B) Gal4DBD-ER4, VP16-ERα and 5xGal4Luc3, and treated with different ER ligands. Luciferase activity was normalized to the activity of the cotransfected pCMVβgal. The ability of ER4 to interact with ER-α in the presence of different ER-agonists paralleled that of ER transactivation function as assayed with 3xERELuc reporter. However, partial agonists or antagonist inhibited this interaction (Fig. 14C) and (Fig. 14D). Therefore, the LXXLL containing peptides provide a sensitive probe for AF2 activation.

*Example 101.3*

To test whether different LXXLL motif peptides interact within the same region of ER, mammalian two hybrid assays were used. Selected peptide sequences and different ER mutants (ER-LL, ER-3X, ER-535 STOP) were expressed as fusion proteins to Gal4DBD and VP16 (TAD), respectively. The binding affinity of different peptides (ER4, F6, D47, C33, D22, D48) to wild type ER and the three ER mutants were measured by the expression of 5xGal4Luc3 reporter construct. GRIP-1* and SRC-1* constructs contain the center 3 copies of LXXLL motif (a.a. 629-761 for GRIP-1 and a.a. 622-765 for SRC-1) fused to Ga14DBD. The results are shown in Figure 15.

Example 101.4

HeLa cells were transfected with ERα expression plasmid (RST7ERα), 3xERELuc reporter, pCMVβgal along with different peptide-Gal4DBD fusion constructs. pM is the Gal4DBD control without peptide fusion. Luciferase activity was measured and normalized (Fig. 16). The ability of different peptides to disrupt ERα transcriptional activity correlates with the affinity of these peptides to ERα as measured in mammalian two-hybrid assays in [Figure 15(A)]. One exception is that the GRIP-1 construct although showed relatively weaker binding affinity to ER in mammalian two-hybrid assays, demonstrated to be an excellent candidate to disrupt ER transcriptional activity. Two copies of LXXLL motifs interact synergistically to disrupt ER transcriptional activity. 2XF6: two copies of F6 peptide was constructed in tandem with a 54-amino acid spacing linker that has the same sequence as in that in between GRIP-1 NR box II and NR box III. F6G: Ga14DBD-F6 fusion with only one copy of F6 peptide plus the linker. Transient transfection was performed in HeLa cells with 3xERELuc, RST7ERα, pCMVβgal and increasing amount of Gal4DBD-peptide fusion constructs as indicated in the X-axis.

Example 101.5

LXXLL containing peptides disrupted AF2 function in HepG2 cells, but did not totally abolish wtER transactivation function in HepG2 cells, where the AF-1 function is dominant (Fig. 17). However, in the same context, the transcriptional activity of a truncated form of ER (ER179C) that lacks the AF-1 domain was diminished by LXXLL containing peptides. HepG2 was transfected with either wtER or ER179C expression plasmids along with 3xERELus reporter, Gal4DBD-peptide fusion constructs, and pCMVβgal to normalize for transfection efficiency. After transfection, cells were induced with different concentrations of 17β-estradiol for 16 h before assaying.

Example 101.6

The interactions between different LXXLL motifs and different nuclear receptors were assayed in a mammalian two-hybrid system (Fig. 18). Full length receptors and selected peptides were expressed as VP16 and Gal4 DBD fusions, respectively. The strength of the interactions was measured by the activity of the 5xGa14Luc3 reporter gene. NH: no hormone; H: hormone treatments. Hormones used in this experiment: $10^{-7}$ M17β estradiol for ER-α and ER-β, $10^{-7}$ M progesterone for PR-a and PR-b, $10^{-7}$ M dexamethasone for GR, $10^{-7}$ M9-cis retinoic acid for RAR and RXR, $10^{-7}$ M T3 for TR, $10^{-7}$ M 1,25-dihydroxy Vit.D3 for VDR, and $10^{-6}$ M 5α-dihydrotestosterone for AR.

*Example 101.7*

Peptide #293 (ER beta 15e2, sequence SSIKDFPNLISLLSR) was affinity selected from phage display of estradiol activated ERβ. It contains the LXXLL motif. It showed selective interactions with ERβ, TRβ and RARα but not with other receptors tested as shown in Figure 7. Expression of this peptide did not interfere with the transcriptional activity of ERα but strongly disrupted the transcriptional activation by ERβ (Fig. 19). HeLa cells were transfected with either ERα or ERβ expression plasmids along with 3xERELuc reporter, pCMVβgal and peptide-DBD fusion constructs as indicated. Cells were treated with different concentrations of 17β-estradiol for 16h before assaying.

Conclusions:

- Peptides with LXXLL motifs have related but different activities. Flanking sequences determine:

    1. their affinity for nuclear receptors.

    2. the requirements for a functional AF2 in ER-α for interaction.

    3. their specificity of interaction with different nuclear receptors.

- LXXLL motifs can knock out the estradiol induced ER-α transcriptional function in HeLa cells where both AF1 and AF2 functions are required for activation. However, in the cell context where AF1 function is dominant (such as HepG2 cells), LXXLL motifs cannot totally abolish the estradiol activated transcriptional activity. This observation implies two possible explanations:

1. In HepG2 cells, the AF1 activity is due to a different coactivator that contacts primarily the AF1 region. Therefore, disruption of the interaction between ER-α and LXXLL-containing cofactors does not disrupt AF1 function.

2. A HepG2 specific cofactor contacts both AF1 and AF2 domains. Disruption of the AF2 binding site is not sufficient to knock out the interaction of cofactor-ER interaction.

- Peptides with Estrogen Receptor specific LXXLL-containing motifs can be obtained by phage display screening and, if active and pharmaceutically acceptable in humans, be used as receptor-specific antagonists.

*Example 201 Application of the Technology to G-Protein-Coupled Receptors and G α Subunits*

The in vitro drug identification system described above can also be extended to other classes of biological signal modulating proteins such as the serpentine receptors (also known as the G protein coupled receptors and seven transmembrane spanning receptors) and their cognate G proteins. We will refer to these as GPCRs. GPCRs have been extensively exploited as targets for drug discovery in many therapeutic areas such as gastrointestinal, cardiovascular and neurological diseases. The ability to rapidly and inexpensively identify drugs that activate or block GPCRs would be of great utility to the pharmaceutical industry.

All GPCRs have at least two functional domains. One is the ligand binding domain on the external surface and the other is the G protein binding domain that is on the intracellular surface.

In their quiescent state the G proteins that are activated by GPCRs exist as G protein (αβγ) heterotrimers containing guanine diphosphate (GDP) bound to Ga subunits. GPCRs activate their cognate G proteins by acting as guanine nucleotide exchange factors (GEFs). Upon GPCR activation, free GTP replaces GDP bound to the a subunit of the G protein. The GTP-bound Ga subunit and Gβγ then disassociate and regulate the function of second messenger enzymes and ion channels. GPCRs activate their cognate G proteins by acting as guanine nucleotide exchange factors (GEFs). Before GPCRs can activate G proteins, they must be switched from an inactive to an active state by the action of the appropriate ligand. GPCRs have little or no detectable affinity for their cognate G proteins until activated. Chemicals that mimic the action of GPCR ligands are known as agonists and induce a change in the GPCR such that it acquires selective affinity for its cognate G protein. Chemicals that block the action of the GPCR ligands are known as antagonists and prevent the induction of structural changes necessary for the GPCR to bind to the cognate G protein.

BioKeys (peptides and similar molecules) that probe GPCRs can be derived from both the GPCRs themselves or their cognate Ga subunits. The Ga subunits can indicate GPCR activation because when GPCRs are activated GTP is bound to the Ga subunit, and when they are inactive GDP is bound. For GPCRs these BioKeys will specifically recognize two basic functional domains, the ligand binding site and the activated receptor via the G protein-binding site. In the case of Ga subunits the BioKeys will specifically recognize the GDP or GTP bound forms. Thus, four classes of BioKeys can be identified, two each for Ga subunits and GPCRs. Such Biokeys can be of immense value for the identification of new therapeutic agents (drugs) using in vitro screening methods.

At present, drugs that act on GPCRs are generally identified in either of two ways. One (the cell based assay) is the use of whole cell assays that are very cumbersome and expense to carry out. The other (ligand displacement assay) is the use of labeled ligands to determine the ability of a test substance to compete for the binding of the ligand to the GPCR. While the latter is substantially less expensive and more convenient to carry out, it is not possible to distinguish agonists from antagonists and thus the usefulness of such an assay is limited.

Through the use of BioKeys specific to each of the basic functional domain of GPCRs, we can carry out simple, inexpensive in vitro screens for both agonists and antagonists to GPCRs and can distinguish the complete range of activities for such compounds from pure agonists, to partial agonists to complete antagonists.

Example A: Screen for agonists and antagonists to the beta-two adrenergic receptor (AR). AR is activated by ligands such as epinephrine and isoproterenol. These ligands are agonists and are useful for the treatment of diseases such as asthma and severe allergic reactions. Antagonists to AR are useful for regulating cardiac function and the treatment of hypertension and cardiac arrhymias.

Class I BioKeys to AR are identified by affinity selection of high affinity (better than 50 micromolar) peptides from BioKey libraries. AR can be produced using recombinant techniques known to those in the art using systems such as the baculovirus expression system in insect cells. AR containing membranes or purified AR can be used for the affinity selection of BioKeys to the ligand binding site. Selectivity for that site can be confirmed by the displacement of the BioKey by the agonist isoproterenol.

In a similar manner, Class II BioKeys can be identified to the G protein binding domain of activated AR. First, AR is pretreated with an excess of an agonist e.g. isoproterenol to induce the AR into an "active" conformation. BioKeys are selected as described herein and selectivity and specificity is confirmed by their ability to bind to agonist treated but not to untreated AR or antagonist treated AR.

Class III BioKeys can be identified to the GDP-bound form of Gsα subunits. Purified Gsα subunits are produced and purified using recombinant techniques and expression in bacterial cells. Purified Gsα subunits are pretreated with an excess of GDP to induce the "inactive" conformation or GDP-bound form of Gsα (GDP-Gsα). BioKeys are selected as described herein and selectivity and specificity is confirmed by their ability to bind to GDP treated, but not to GTP treated Gsα subunits.

In a similar manner, Class IV BioKeys can be identified to the GTP-bound form of Gsα subunits. Purified Gsα subunits are pretreated with an excess of GTP to induce the "active" conformation or GTP-bound form of Gsα (GTP-Gsα). BioKeys are selected as described herein and selectivity and specificity is confirmed by their ability to bind to GTP treated, but not to GDP treated Gsα subunits.

Representative BioKeys to all four classes can be labeled with a suitable moiety as described elsewhere herein such as europium labeled streptavidin and used in a drug screen using fluorescence measuring devices. Many other means of using BioKeys as surrogate ligands will be apparent to those skilled in the art of drug compound screening.

As can be seen from Table 5, it is very easy to distinguish three classes of compounds from a chemical compound collection. Inactive compounds do not bind to relevant functional domains on the AR and thus no change in signals from either BioKey is seen. For GPCRs, agonists are easily identified due to their ability to induce the AR into a conformational state such tha the G protein binding domain is capable of binding class II BioKeys that are surrogates for the AR's cognate G protein. Agonists that bind to the ligand binding site will also lead to a measurable decrease in class I BioKey binding. Antagonists are identified by their ability to bind to the ligand binding domain and hence are capable of blocking the natural ligand from binding. However, unlike agonists, they have no ability to induce the activation of the receptor; hence there is no change in the conformation of the AR's G protein binding domain and thus no change in ability to bind class II BioKeys.

Alternatively, screening for compound agonists or antagonists can be performed using AR-containing membranes, Gs and BioKeys specific for GTP-Gsα or GDP-Gsα. Agonists will activate the receptor and result in the formation of GTP-Gsα. The signal from BioKeys specific to GDP-Gsα will decrease if an agonist is binding the receptor. Likewise, the signal from BioKeys specific to GTP-Gsα will increase if an agonist is bound to AR. Antagonists are not capable of activating the receptor and therefore are not able to activate G proteins. The G protein will then remain a heterotrimer containing GDP bound to its Gsα subunit. Screen for antagonist using AR-containing membranes pretreated with an agonist. The signal from BioKeys specific to GTP-Gsα will decrease if an antagonist is binding the receptor. Likewise, the signal from BioKeys specific to GDP-Gsα will increase if an antagonist is bound to AR.

This system can be readily extended to other GPCRs for which one has access to a natural ligand or an agonist.

**Example 401 Fingerprinting of Modulators of the Glucocorticoid Receptor**

Peptide Sequences

F6: GHEPLTLLERLLMDDKQAV

α/βIII: SSWDMHQFFWEGVSR

α/βV : SSPGSREWFKDMLSR

αII: SSLTSRDFGSWYASR

Note that these peptides, while originally identified as peptides which bind the estrogen receptor, are usable in fingerprinting of modulators of the glucocorticoid receptor. The ER binding peptides work on the glucocorticoid receptor because nuclear receptors have structural similarities. The exact nature of these similarities are not known although there are sequence similarities. Identical coactivator proteins bind both receptor and contain LXXLL motifs. Thus it is not surprising that our LXXLL peptides might also bind both receptors in the presence of agonist. See McInerney, et al., Genes & Development, 12:3357-68 (1998); Nolte, et al., Nature, 395:134-143 (September 10, 1998).

Titration of GR vs. F6 with Deoxycorticosterone and Dexamethasone

Yeast strain EGY48 (MATα trp1 his3 ura 3 leu2::6LexAop-Leu2) was transformed with plasmids pJK103 (2μM, 2LexAop-LacZ), pJG4-5-F6 (2μM, LexADBD-F6 peptide), and pEG202-GR (2 μM, B42AD-Glucocorticoid Receptor a). The resulting transformed strain was grown overnight in media containing galactose as the sole carbon source to induce expression of GR. Deoxycorticosterone and dexamethasone were serially diluted into 100 μl of media in a 96-well microplate. 100 μl aliquots of the overnight yeast culture were added to the microplate wells and incubated at 30°C for 3 hours. To monitor the interaction of the F6 peptide with GR, a kinetic assay for β-galactosidase activity was performed. The cell density in each was determined by reading the $OD_{650}$. Yeast were pelleted by centrifugation for 5 minutes at 3000 rpm and the media removed. 20 μl of 1xZ buffer (60 mM $Na_2HPO_4$ 40 mM $NaH_2PO_4$ 10 mM KCl 1 mM $MgSO_4$ 7 mM 2-mercaptoethanol) containing 2.5% CHAPS detergent was added and briefly mixed by aggitation. Following a 5 minute incubation at room temperature, 100 μl of lxZ buffer containing 40 μg o-Nitrophenyl β-D-Galactopyranoside (ONPG) was added to each well. Color development was monitored by measuring the change in $OD_{405}$ referenced to $OD_{650}$ over 10 minutes (20 second intervals). β-galactodase activity is expressed as $\Delta(OD_{405}$-$OD_{650})$ /initial $OD_{650}$.

Interaction of GR with peptides

Yeast strain EGY48 (MATα trp1 his 3 ura3 leu2::6LexAop-Leu2) was transformed with plasmids pJK103 (2 μM, 2LexAop-LacZ), pEG202-GR(2 μM, LexADBD-F6, -α/βIII, -α/βV, or -αIi peptides). The resulting transformed strain was grown overnight in media containing galactose as the sole carbon source to induce expression of GR. The culture was diluted to an $OD_{600}$ of 0.1 in 10 ml of media and deoxycorticosterone dexamethasone, corticosterone, or β estradiol were added to a final concentration of 1 μM. The cultures were incubated at 30°C for 3 hours. Preparations of protein were made by lysing the yeast by aggitation with glass beads. The cellular debris was removed and the protein concentrated by precipitation with 50% ammonium sulfate for 30 minutes at 4°C. The protein pellet was suspended in storage buffer (100 mM HEPES 50 mM EDTA 40% glycerol 7 mM 2-mercaptoethanol, pH8) and protein concentrations determined. To determine the interaction of the peptides with GR, an end point assay for β-galactosidase activity was performed. 10 μg of protein extract was diluted into a final volume of 100 μl lxZ buffer and color development was initiated by the addition of 80 μg of ONPG. The reactions were stopped by addition of 30 μl of 1M $Na_2CO_3$ and the time of development noted. β-galactosidase activity is expressed as 1000*$OD_{405}$/min/mg protein.

Table A: List of Proteins for Fingerprinting Analysis:

| Receptors | Modulators of Activity |
|---|---|
| Nuclear receptors | |
| Estrogen Receptor α and β | Estradiol (agon), tamoxifen (antag), ICI 182,780 (antag), Raloxifene, (antag), |
| Progesterone | Progestins, estrogens (agon), RU486 (antag), ZX98299, (antag), onapristone (antag) |
| Androgen | Dihydroxytestosterone (agon), hydroxyflutamide (antag) |
| Glucocorticoid | Cortisone (agon), dexamethasone (agon) |
| mineralocorticoid | Aldosterone (agon), spironolactone (antag) |
| Retinoic acid | 9-cis retinoic acid (agon) |
| Thyroid | Thyroid hormone (agon) |
| Vitamin D3 | Vitamin D3 (agon) |
| PPAR(s) | Eicosinoids (agon), oxidized LDL (agon) |
| LXR | Oxidized cholesterol metabolites (agon) |
| FXR | Farnesoid metabolites (agon) |
| BXR | 3-aminoethyl benzoate (agon) |
| SXR | Steroids (agon), phytoestrogens(agon), xenobiotics (agon) |
| Orphan Nuclear Receptors | |
| Nurr1 | |
| Nor1 | |

| | |
|---|---|
| NGF1-B | |
| ERR1 | |
| SHP | |
| HNF-4 | |
| Coup-TF II | |
| Tyrosine Kinase Receptors | |
| Epidermal growth factor | EGF (agon), ATP |
| Insulin | Insulin(agon), ATP |
| Platelet derived growth factor | PDGF(agon), ATP |
| G-Protein Coupled Receptors | |
| β-adrenergic receptor | Isopreterenol (agon), alprenolol (antag) |
| Rhodopsin | |
| Dopamine D2 | Dopamine (agon), haloperidol (antag) |
| opiod | Leu-enkephalin (agon), Naltrindole (antag) |
| Endothelin | Endothelin 1(agon), BQ-123 (antag) |
| Erythropoietin receptor | Erythropoietin |
| FAS ligand receptor | FAS ligand |
| Interleukin receptor | Interferon (agon) IL-6 (agon) |
| Signal Transduction Proteins | |
| Kinases | |
| Protein Kinases | |
| Protein kinase C | diacylglycerol (agon), staurosporine (antag) |
| Tyrosine kinase | ATP, genistein (antag) |

| | |
|---|---|
| Serine kinase | ATP |
| Threonine kinase | ATP |
| Nucleotide kinase | ATP |
| Polynucleotide kinase | ATP, DNA, $PO_4$ |
| Phosphatase | |
| Protein Phosphatase | |
| Serine/threonine | |
| Tyrosine | |
| Nucleotide phosphatase | |
| Acid phosphatase | |
| Alkaline phosphatase | |
| pyrophosphatase | |
| Cell Cycle Regulators | |
| Cyclin CDK-2 | |
| CDC2 | |
| CDC25 | |
| p53 | |
| Retinoblastoma | |
| GTPases | |
| Large G proteins | |
| G$\alpha$s | suramin (antag), mastoparin (agon) |
| Small G Proteins | GAPs (ag), GEF (antag) |
| Rac | |
| Rho | |
| Rab | |
| Ras | |
| Proteases | |
| Endoprotease | |
| Exprotease | |
| Metalloprotease | |
| Serine protease | |
| Cysteine protease | |
| Nucleases | |
| Polymerases | |

Ion Channels

Chaperonins
- Heat shock Proteins

Viral Proteins

Deaminases

Nucleases
- Deoxyribonuclease
- Ribonuclease
- Endonucleases
- Exonucleases

Polymerases
- DNA dependent RNA polymerase
- DNA dependent DNA polymerase
- Telomerase
- Primase

Helicase

Dehydrogenase

Aminoacyl tRNA synthetases

Transferases
- Peptidyl transferase
- Transaminase
- Glycosyltransferase
- Ribosyltransferase
- Acetyl transferases
- Acyltransferases

Hydrolases

Carboxylases

Isomerases
- Dismutase
- Rotase
- Topoisomerase

Glycosidase
- Endoglycosidase
- Exoglycosidase

Deaminase

Lipases

Esterases
Sulfatases

Cellulase

Lyases

Reductases

Synthetase

DNA binding proteins

RNA binding proteins

Nuclear receptor coactivators

Ligases
- RNA
- DNA

Tumor suppressor

Adhesion molecule

Oxygenase
- Peroxidase

Transporters
- Electron transporters
- Protein transporters
- Peptide transport
- Hormone transport
  - Serotonin
  - DOPA
- Nucleic acid transport

Transcription factors
Neurotransmitters
Information carrier/storage
Antigen recognition protein
- MHC I complex
- MHC II complex
- **Antag=antagonist of receptor**
- **agon=agonist of receptor**

Table B: Target Tissues

Circulatory and Lymphatic Systems
- Heart
  - Walls
  - Valves
- Blood Vessels
- Blood Cells
  - Erythrocytes
  - Platelets
  - Leukocytes
- Lymph Nodes
- Lymphatic Vessels
- Spleen
- Thymus
- Tonsils

Respiratory System
- Lungs
  - Trachea
  - Bronchi
  - Bronchioles
  - Alveoli
  - Pleura
- Pharynx
- Larynx
- Trachea

Endocrine System
- Pituitary Gland
- Thyroid Gland
- Parathyroid Gland
- Adrenal Gland
- Adrenal Medulla
- Adrenal Cortex
- Pancreas
  - Islets of Langerhans
- Liver
- Gall Bladder

- Mammary Glands
- Central Nervous System
    - Brain
        - Neurons
        - Glial Cells
    - Spinal Cord
    - Nerves

- Peripheral Nervous System
    - Eye
        - Retina
        - Lens
    - Ear
        - Eardrum
        - Ampullae
        - Spiral organ of Corti
    - Nose
        - Olfactory bulbs
    - Tongue
        - taste buds

- Digestive System
    - Tongue
    - Salivary Gland
    - Pharynx
    - Esophagus
    - Stomach
    - Small Intestine
    - Large Intestine

- Urinary System
    - Kidney
        - nephrons
    - Bladder

- Male Reproductive System
    - testes
    - prostate gland

bulbourethral (Cowper's) glands
penis
sperm cells

Musculoskeletal System
- bones (various)
  - bone marrow
- joints (various)
- muscles (various)
- ligaments (various)

Female Reproductive System
- Ovaries
- Uterus
- Bartholin's Glands
- Paraurethral Glands
- Egg Cells

Integumentary System
- Skin
  - epidermis
  - dermis
  - hypodermis
- sweat glands
- sebaceous glands
- hair
- nails

**Table 1**

Peptides the Bind to the Unliganded (unactivated) Estrogen Receptor

| Sequence | Phage # |
|---|---|
| S R W E S P L G T W E W S R | 4 |
| S A A P R T I S H Y L M G G | 48 |
| S S W V R L S D F P W G V S R | 1 |
| S S W D R L S D F P W G V S R | 2 |

(continued)

Peptides the Bind to the Unliganded (unactivated) Estrogen Receptor

| Sequence | Phage # |
|---|---|
| S S W I R L R D L P W G E S R | 3 |
| S S W V L L R D L P W G S R | 31 |
| S S W V V L R D L P W G S R | 29 |
| S S C K W Y E K C S G L W S R | 7 |
| S S G I C F F W D G C F E S R | 35 |
| S R N L C F F W D D E Y C S R | 41 |
| H H H R H P A H P H T Y G G | 47 |

**Table 2**

Peptides that Bind to the Estradiol Activated Receptor

| Sequence | Phage # |
|---|---|
| S R A G L L S D L L E G K S R | 1/2 |
| S S R S L L R D L L M V D S R | 6 |
| S S N K L L Y N L L K M E S R | 22 |
| S S K S L L L N L L S T P S R | 23 |
| H S F P R E S L L V R L L Q G G | 42 |
| S R L E M L L R S E T D F S R | 3 |
| S R L E E L L K W G S V T S R | 11 |
| S R L E Q L L K E E F S Y S R | 21 |
| S R L E Q L L R S E P D F S R | 27 |
| S R L E D L L R A P F T T S R | 28 |
| S R L E S L L R F G Q L D S R | 29 |
| S S R L L S L L V G D F N S R | 19/20 |
| S R L E E L L L G T N R D S R | 30 |
| S R L K E L L L L P T D L S R | 15 |
| S R L E C L L E G R L N C S R | 34 |
| S S K L Y C L L D E S Y C S R | 35 |
| S R L S C L L M G F E D C S R | 36 |
| S S K L I R L L T S D E E L S R | 37 |
| S S R L M E L L Q E G Q G W S R | 40 |
| S S N H Q S S R L I E L L S R | 4 |
| S S R L W Q L L A S T D T S R | 16 |
| S S N S M L W K L L A A P S R | 13/14 |
| S S K T L W R L L E G E R S R | 17 |
| S R A G P V L W G L L S E S R | 32 |
| S S L T S R D F G S W Y A S R | 5 |
| S S W V R L S D F P W G V S R | 24/25 |
| S S E Y C F Y D S A H C S R | 33 |
| S R S L L E C H L M G N C S R | 7 |
| S S E L L R W H L T R D T S R | 8 |
| S R L E Y W L K W E P G P S R | 12 |
| S R S D S I L W R M L S E S R | 31 |
| S S K G V L W R M L A E P V S R | 38/39 |
| H S H G P L T L N L L R S S G G | 41 |
| S S A G G G A P A G S T P S R | 26 |

Other ER binding peptides include

SSKYSYSRSSEGHSR
SSYQWETHSDKWRSR
SSVTKKALTIAKDSR

The latter two are weak binders of ER in presence of estradiol.

**Table 3: Phage/Peptide Classification**

| Class 1 | # and isolation method |
|---|---|
| S S N H Q S S R L I E L L S R | #4 ER + estradiol |
| S R L K E L L L L P T D L S R | #15 ER + estradiol |
| S S K L Y C L L D E S Y C S R | #35 ER + estradiol |
| H G P L T L N L L R S S G G | #41 ER + estradiol |
| S R L E Y W L K W E P G P S R | #12 ER + estradiol |
| Class 2 | |
| S S C K W Y E K C S G L W S R | #7 ER |
| S S E Y C F Y W D S A H C S R | #33 ER + estradiol |
| S S W V L L R D L P W G S R | #31 ER |
| S S W V R L S D F P W G V S R | #24 ER + estradiol |
| Class 3 | |
| S S L T S R D F G S W Y A S R | #5 ER + estradiol |
| Class 4 | |
| S R T W E S P L G T W E W S R | #13 ER |
| Class 5 | |
| S A A C A T I S H Y L M G G | #48 ER |

**Table 4**

Characteristics of the 5 Phage Classes

| | Affinity for unliganded ER α | Affinity for unliganded ER β | Effect of Agonist (Estradiol) | Competition with LXXLL peptide |
|---|---|---|---|---|
| Class 1 | + | +++ | ↑ binding to α & β | + α + β + |
| Class 2 | +++ | ++ | No effect | - α - β |
| Class 3 | ++ | + | ↑ binding to α no effect on β | - α - β |
| Class 4 | +++ | ++ | ↓ binding to α no effect on β | + α - β |
| Class 5 | ++(+) | +++ | ↓ binding to α & β | + α - β |

**Table 5**

| | Agonist compound | Antagonist compound | Inactive compound |
|---|---|---|---|
| BioKey I * | No change or decreased signal | Decreased signal | No change |
| BioKey II @ | Increased signal | No change or decreased signal | No change |

* BioKey specific to the ligand binding site
@ BioKey specific to the G protein binding site on the activated receptor.

**Table 6A:Fingerprint Analysis for Agonists and SERMs on ER α, by Peptide Class**

| Class | Estradiol | estriol | 4-OH Tamoxifen | Nafoxidine | Clomiphene |
|---|---|---|---|---|---|
| 1 | +++ | ++++ | + | ++ | + |

(continued)

| Class | Estradiol | estriol | 4-OH Tamoxifen | Nafoxidine | Clomiphene |
|---|---|---|---|---|---|
| 2 | +/- | +/- | + | + | + |
| 3 | ++ | ++ | ++ | ++ | +++ |
| 4 | -- | -- | + | + | + |
| 5 | - | - | + | + | + |

**Table 6B:Fingerprint Analysis for Agonists and SERMs on ER β, by Peptide Class**

| Class | Estradiol | estriol | 4-OH Tamoxifen | Nafoxidine | Clomiphene |
|---|---|---|---|---|---|
| 1 | ++ | ++ | - | +/- | +/- |
| 2 | +/- | +/- | + | ++ | ++ |
| 3 | +/- | +/- | + | ++ | ++ |
| 4 | +/- | +/- | ++ | ++ | ++ |
| 5 | - | - | +/- | +/- | - |

**Table 7: New Erα Peptide Sequences Immobilized on Plastic**

| Peptide name | Peptide Sequence | Isolated in the presence of receptor form | SERM present when peptide was identified |
|---|---|---|---|
| 1PT | SRNLCFFWDDEYCSR | α | Tamoxifen & ICI 182,780 |
| 2PT | SWDMHQFFWEGVSR | α | Tamoxifen |
| 3PT | SRWHGTLFWQDEQSR | α | Tamoxifen |
| 4PT | SSCKWYEKCSGLWSR | α | Tamoxifen & ICI 182,780 |
| 5PT | SSRMGHVWYDWTFSR | α | Tamoxifen |
| 6PT | SSRLLGDFGGSWSR | α | Tamoxifen |
| 7PT | SSKYVFGFQVAGGSR | α | Tamoxifen |
| 8PT | SSWAGIKFGKPPHSR | α | Tamoxifen |
| 9PT | SSSWSYGKPTFLSSR | α | Tamoxifen |
| 10PT | SRDTGDMWWGRGGSR | α | Tamoxifen |
| 11PT | SSGRYDPFVLNAASR | α | Tamoxifen |
| 12PT | SSSPWWSFNLRDMSR | α | Tamoxifen |
| 13PT | SSWPYLPKREEWASR | α | Tamoxifen |
| 14PT | SSGWIEQKLRGSFSR | α | Tamoxifen |
| 15PT | SSSATSIKVQYQISR | α | Tamoxifen |
| 16PT | SSYLTLGKSMMAISR | α | Tamoxifen |
| 17PT | SSWHSRWDLALGFSR | α | Tamoxifen |
| 18PT | SSGYWGGWDYGAGSR | α | Tamoxifen |
| 19PT | SRDNCGAGLWAGCSR | α | Tamoxifen |
| 1PI | SSSTPGWWEWDWASR | α | ICI 182,780 |
| 2PI | SSYWDGSWRRKETCVSCSR | α | ICI 182,780 |
| 3PI | SSRTAEDYCFFADDYWCSR | α | ICI 182,780 |
| 4PI | SSRALALFPVGMESR | α | ICI 182,780 |
| 5PI | SSDCESLTSYPHLKALCSR | α | ICI 182,780 |
| 6PI | SSTATALRDRLAYSR | α | ICI 182,780 |
| 7PI | SSGKTREHYREGTSR | α | ICI 182,780 |

**Table 8: New ERα-ERE Peptide Sequence Information**

| Peptide name | Peptide Sequence | Isolated in the presence of receptor form | SERM present when peptide was identified |
|---|---|---|---|
| E1-1 | HSHNHHSPWLFRLLGG | α | Estradiol |

(continued)

| Peptide name | Peptide Sequence | Isolated in the presence of receptor form | SERM present when peptide was identified |
|---|---|---|---|
| E1-3 | HSHPHHSHLLYKLMGG | α | Estradiol |
| E1-4 | HSHPLPPLLSRLLTGG | α | Estradiol |
| E1-7 | SRLTCLLQSNGWDSEQCSR | α | Estradiol |
| I4-10 | SSLTSRDFGSWYASR | α | ICI |
| T3-1 | SRTLQLDWGTLYSR | α | Tamoxifen |
| T1-10 | SRLPPSVFSMCGSEVCLSR | α | Tamoxifen |
| T2-10 | SRFEIWKPEPGCVSSLENWE PGKRVCSR | α | Tamoxifen |
| T3-11 | SRVFGVSGGEVVLINGSSR | α | Tamoxifen |
| 1R | SRLCFGDWCMLGGVDVLSR | α | Raloxifen |
| 2R | SSLNMVVDTPWCGKWVCSR | α | Raloxifen |
| 3B | SSRPDAAFFGAKLSR | α | Buffer |
| 4B | SSRPSPSFWEKQLSR | α | Buffer |
| 5B | SSRPTAEWFRENLSR | α | Buffer |
| 6B | SRWWDTSWWLEELSR | α | Buffer |
| 1B | SSRIADLFWRLEPSR | α | Buffer |
| 7B | SRSYHGEWGVWTLSR | α | Buffer |
| 10B | SSDWCFGWGGWCASEAVSR | α | Buffer |
| 9B | SRNWDWAALELLPYPHPSR | α | Buffer |
| 1E | SSLTSRDFGSWYASR | α | Estradiol |
| 2E | SRSPILTHLLSLGSR | α | Estradiol |
| 3E | SSTGILWKLLTAESR | α | Estradiol |
| 9E | SSHGILWRLLSEGSR | α | Estradiol |
| 11E | SRSDSILWRMLSESR | α | Estradiol |
| 4E | SRLVALLKSPWSVSR | α | Estradiol |
| 5E | SRLEELLLMDFWRSR | α | Estradiol |
| 6E | SSKLWQLLSSPIDSR | α | Estradiol |
| 14E | SSKLYCLLDESYCSR | α | Estradiol |
| 7E | SRSLLMDMLMSDDYVTVSR | α | Estradiol |
| 8E | SSRLLACELMYEDADVCSR | α | Estradiol |
| 15E | HSHSPLLMALLAPPGG | α | Estradiol |
| 10E | SRLEYYLRLGTYESR | α | Estradiol |
| 13E | SSCLREILLYGACSR | α | Estradiol |
| 16E | SSRTAEDYCFFADDYWCSR | α | Estradiol |
| 17E | SSLRCYLSSSKVDQWACSR | α | Estradiol |
| 18E | SSYKPHSLLEWHLLGGTSR | α | Estradiol |

**Table 9: New ERβ-ERE Peptide Sequence Information**

| Peptide name | Peptide Sequence | Isolated in the presence of receptor form | SERM present when peptide was identified |
|---|---|---|---|
| 1B-β | SRLHCLLDSSYCSSR | β | Buffer |
| 2B-β | SRLHCLLDSSYCSSR | β | Buffer |
| 3B-β | SSWPNPTFWERQLSR | β | Buffer |
| 4B-β | SYSKEWFEERLNSR | β | Buffer |
| 5B-β | SSSMMREFFERELSR | β | Buffer |
| 6B-β | SSGLPPNFERMLKSR | β | Buffer |
| 7B-β | SSGPWLMHYLGGGSR | β | Buffer |
| 8B-β | SSTSWLHHYLMGTSR | β | Buffer |

(continued)

| Peptide name | Peptide Sequence | Isolated in the presence of receptor form | SERM present when peptide was identified |
|---|---|---|---|
| 9B-β | SRGGGECLGPWCLSR | β | Buffer |
| 12B-β | SSEACVGRWMLCEQLGVSR | β | Buffer |
| 14B-β | SSQVWPGPWRLVESR | β | Buffer |
| 16B-β | SSSLGPWRLSELESR | β | Buffer |
| 17B-β | SSSGPWRWGLSIESR | β | Buffer |
| 18B-β | SRECVGGWCLAELSR | β | Buffer |
| 19B-β | SSIPPRSWWLSQLSR | β | Buffer |
| 20B-β | SSWPGAEWFKEQLSR | β | Buffer |
| 21B-β | SSKLYCLLDESYCSR | β | Buffer |
| 23B-β | HSYSSHPLLLSYLWGG | β | Buffer |
| 24B-β | HSWLGPWRLSSIDLGG | β | Buffer |
| 25B-β | HSTDMGWLRPWRLLGG | β | Buffer |
| 1T-β | SSVFTIMDGKVALSR | β | Tamoxifen |
| 2T-β | SRPYCLGDVWCLDSR | β | Tamoxifen |
| 4T-β | SREWEDGFGGRWLSR | β | Tamoxifen |
| 5T-β | SSWNSREFFLSQLSR | β | Tamoxifen |
| 6T-β | SSTTMFDFFYERLSR | β | Tamoxifen |
| 7T-β | SSARPWWLQFEGSSR | β | Tamoxifen |
| 8T-β | SSQEEWLLPWRLASR | β | Tamoxifen |
| 9T-β | SRLPPSVFSMCGSEVCLSR | β | Tamoxifen |
| 10T-β | SSGPFYVGGMLWPADCLSR | β | Tamoxifen |
| 12T-β | SREGWMGPWRLADSR | β | Tamoxifen |
| 13T-β | SRNECIGPWCLTISR | β | Tamoxifen |
| 14T-β | SSPGSREWFKDMLSR | β | Tamoxifen |
| 15T-β | SSVASREWWVRELSR | β | Tamoxifen |
| 16T-β | SRMFQVCGDEVCLRSR | β | Tamokifen |
| 17T-β | SSDLHRDCLGVWCLSR | β | Tamoxifen |
| 18T-β | SRLNGVFCHDSSDLWVCSR | β | Tamoxifen |
| 20T-β | SRPGCLRGVWCLADTPPSR | β | Tamoxifen |
| 21T-β | SSRLVPHSFWLDGLMHGSR | β | Tamoxifen |
| 22T-β | SSISTYHMGEWFYAMLSSR | β | Tamoxifen |
| 23T-β | SSDLYSQMREFFQINLSR | β | Tamoxifen |
| 1E-β | SSRGLLWDLLTKDSR | β | Estradiol |
| 2E-β | SRHGILWDLLQGDSR | β | Estradiol |
| 3E-β | SRLHDLLLRDESPSR | β | Estradiol |
| 4E-β | SRDWRSGFLYELLSR | β | Estradiol |
| 5E-β | SSDTRSRLYELLSSSYTSR | β | Estradiol |
| 6E-β | SRLEELLRVGVLTSR | β | Estradiol |
| 7E-β | SRLEDLLRGDSKPQSR | β | Estradiol |
| 8E-β | SSPTGHRLLESLLLNSNSR | β | Estradiol |
| 9E-β | SSILERLLGGGSAETV | β | Estradiol |
| 10E-β | SRSPILWHLLQDGSR | β | Estradiol |
| 11E-β | SSRTPILFSLLETSR | β | Estradiol |
| 12E-β | SSIKDFPNLISLLSR | β | Estradiol |
| 13E-β | SSGSSAGRLMMLLQDGVSR | β | Estradiol |
| 14E-β | SREGLLMRLLIGDSR | β | Estradiol |
| 15E-β | SSHCHTRLCSLLTSR | β | Estradiol |
| 16E-β | SSRLLCLLDAGQCSR | β | Estradiol |
| 17E-β | SRNLLCLLDQEACSR | β | Estradiol |

(continued)

| Peptide name | Peptide Sequence | Isolated in the presence of receptor form | SERM present when peptide was identified |
|---|---|---|---|
| 18E-β | SSLKCLLNSNFCSR | β | Estradiol |
| 19E-β | SSLKCLLQSSPQKQPFCSR | β | Estradiol |
| 20E-β | SSRTLLEHYLLGGSR | β | Estradiol |
| 21E-β | SSAGLLEDMLRSRSR | β | Estradiol |
| 22E-β | SSRCSSLLCEMLIQTKESR | β | Estradiol |
| 23E-β | SSLQAGSWLMHYLRGGDSR | β | Estradiol |
| 24E-β | SRPEGSSWLLHYLSR | β | Estradiol |
| 25E-β | SSRTLLEHYLLGGSR | β | Estradiol |
| 26E-β | SRWWLDDHELLLYSSR | β | Estradiol |
| 27E-β | SSRTLYCHLTSSNPEWCSR | β | Estradiol |
| 28E-β | SSTRLMCWLGSADTSHCSR | β | Estradiol |
| 29E-β | SSYDWQCPSWYCPAPPSSR | β | Estradiol |
| 30E-β | SSTTWRCPEWYCGSR | β | Estradiol |
| 31E-β | SSWDFRVPWWYNNSR | β | Estradiol |
| 32E-β | SSQWQAPWWYIDASR | β | Estradiol |
| 33E-β | SSRPSFTIPWWFDDPSRSR | β | Estradiol |
| 34E-β | SSYEIPKWALQWLSR | β | Estradiol |
| 35E-β | SSLDLSQFPMTASFLRESR | β | Estradiol |

**Table 10: Panel Peptides for Example 2**

α/β I, SSNHQSSRLIELLSR (AB1) [17β-estradiol]
α/β II, SAPRATISHYLMGG (AB2) [no modulator]
α/β III, SSWDMHQFFWEGVSR (AB3) [4-OH tamoxifen]
α/β IV, SRLPPSVFSMCGSEVCLSR (AB4) [same]
α/β V, SSPGSREWFKDMLSR (AB5) [same]
α I, SSEYCFYWDSAHCSR (A1) [17β-estradiol]
α II, SSLTSRDFGSWYASR (A2) [17β-estradiol]
α III, SRTWESPLGTWEWSR (A3) [no modulator]
β I, SREWEDGFGGRWLSR (B1) [4-OH tamoxifen]
β II, SSLDLSQFPMTASFLRESR (B2) [17β-estradiol]
β III, SSEACVGRWMLCEQLGVSR. (B3) [no modulator]

Alternative name parenthesized. Modulator used to isolate peptide in brackets.

**Table 11: Erα Binding Activity, in Presence of SERMs or Buffer, of Peptides Isolated on ERα-ERE**

| SERM's / peptides | buffer | estra- | estriol | prem- | tamox- | nafox- | clomi- | ralox- | ICI | 16_α | DES | progest |
|---|---|---|---|---|---|---|---|---|---|---|---|---|
| 1R | 7+ | 7+ | 7+ | 7+ | 7+ | 7+ | 7+ | 7+ | 7+ | 7+ | 7+ | 7+ |
| 2R | 7+ | 7+ | 7+ | 7+ | 7+ | 7+ | 7+ | 7+ | 6+ | 7+ | 7+ | 5+ |
| 3B | 6+ | 2+ | 3+ | 3+ | 2+ | 2+ | 2+ | 2+ | 2+ | 2+ | 2+ | 4+ |
| 4B | 5+ | 3+ | 4+ | 4+ | 3+ | 3+ | 3+ | 3+ | 3+ | 3+ | 3+ | 4+ |
| 5B | 7+ | 4+ | 5+ | 4+ | 2+ | 2+ | 3+ | 3+ | 3+ | 3+ | 3+ | 5+ |
| 6B | 7+ | 7+ | 7+ | 7+ | 4+ | 5+ | 5+ | 4+ | 4+ | 7+ | 6+ | 7+ |
| 1B | 7+ | 7+ | 6+ | 7+ | 6+ | 6+ | 6+ | 5+ | 6+ | 6+ | 6+ | 6+ |
| 7B | 5+ | 3+ | 3+ | 4+ | 4+ | 3+ | 3+ | 3+ | 2+ | 3+ | 3+ | 4+ |
| 10B | 7+ | 7+ | 7+ | 7+ | 7+ | 7+ | 7+ | 7+ | 7+ | 7+ | 7+ | 7+ |
| 9B | 7+ | 6+ | 7+ | 7+ | 6+ | 7+ | 7+ | 5+ | 6+ | 6+ | 6+ | 7+ |
| 1E | 2+ | 5+ | 4+ | 5+ | 3+ | 3+ | 4+ | 3+ | 3+ | 3+ | 3+ | 1+ |
| 2E | 2+ | 4+ | 4+ | 5+ | 2+ | 2+ | 2+ | 2+ | 2+ | 3+ | 3+ | 2+ |
| 3E | 3+ | 5+ | 4+ | 5+ | 3+ | 2+ | 3+ | 2+ | 3+ | 4+ | 4+ | 3+ |
| 9E | 4+ | 7+ | 6+ | 7+ | 5+ | 5+ | 5+ | 5+ | 5+ | 6+ | 6+ | 5+ |
| 11E | 3+ | 6+ | 6+ | 4+ | 4+ | 3+ | 4+ | 4+ | 3+ | 4+ | 4+ | 4+ |
| 4E | 5+ | 7+ | 7+ | 7+ | 5+ | 4+ | 5+ | 5+ | 5+ | 6+ | 6+ | 5+ |
| 5E | 3+ | 5+ | 6+ | 6+ | 3+ | 3+ | 3+ | 3+ | 3+ | 4+ | 4+ | 3+ |
| 6E | 2+ | 4+ | 4+ | 4+ | 2+ | 2+ | 2+ | 2+ | 2+ | 3+ | 3+ | 2+ |

| | | | | | | | | | | | | |
|---|---|---|---|---|---|---|---|---|---|---|---|---|
| 14E | 3+ | 6+ | 6+ | 6+ | 2+ | 2+ | 2+ | 2+ | 2+ | 4+ | 4+ | 3+ |
| 7E | 2+ | 5+ | 4+ | 4+ | 2+ | 2+ | 3+ | 2+ | 2+ | 3+ | 3+ | 2+ |
| 8E | 4+ | 7+ | 6+ | 6+ | 4+ | 4+ | 4+ | 4+ | 4+ | 5+ | 4+ | 4+ |
| 15E | 2+ | 5+ | 4+ | 3+ | 2+ | 2+ | 2+ | 2+ | 2+ | 3+ | 3+ | 3+ |
| 10E | 6+ | 7+ | 7+ | 6+ | 6+ | 6+ | 6+ | 6+ | 6+ | 6+ | 7+ | 5+ |
| 13E | 3+ | 7+ | 5+ | 5+ | 3+ | 3+ | 3+ | 3+ | 3+ | 5+ | 5+ | 4+ |
| 16E | 7+ | 7+ | 7+ | 7+ | 7+ | 7+ | 7+ | 7+ | 7+ | 7+ | 7+ | 7+ |
| 17E | 2+ | 4+ | 3+ | 4+ | 2+ | 2+ | 2+ | 2+ | 2+ | 2+ | 2+ | 2+ |
| 18E | 3+ | 6+ | 5+ | 5+ | 3+ | 3+ | 3+ | 3+ | 3+ | 3+ | v | 3+ |
| E1-1 | 5+ | 7+ | 7+ | 6+ | 5+ | 6+ | 6+ | 5+ | 5+ | 5+ | 5+ | 5+ |
| E1-3 | 3+ | 7+ | 7+ | 6+ | 3+ | 3+ | 3+ | 3+ | 2+ | 3+ | 4+ | 3+ |
| E1-4 | 3+ | 6+ | 6+ | 4+ | 3+ | 3+ | 3+ | 3+ | 2+ | 3+ | 3+ | 3+ |
| E1-7 | 3+ | 6+ | 6+ | 5+ | 2+ | 2+ | 2+ | 2+ | 2+ | 2+ | 3+ | 2+ |
| I4-10 | 3+ | 6+ | 6+ | 6+ | 4+ | 5+ | 4+ | 4+ | 5+ | 3+ | 4+ | 4+ |
| T3-1 | 2+ | 2+ | 2+ | 2+ | 5+ | 4+ | 4+ | 3+ | 2+ | 2+ | 2+ | 2+ |
| T1-10 | 3+ | 3+ | 2+ | 3+ | 5+ | 4+ | 4+ | 3+ | 2+ | 2+ | 2+ | 2+ |
| T2-10 | 3+ | 3+ | 3+ | 3+ | 6+ | 4+ | 5+ | 4+ | 3+ | 3+ | 3+ | 3+ |
| T3-11 | 7+ | 5+ | 5+ | 6+ | 7+ | 6+ | 7+ | 5+ | 5+ | 6+ | 5+ | 5+ |

**Table 12: Er$\beta$ Binding Activity, in Presence of Buffer or SERMS, of Peptides Isolated on ER$\alpha$-ERE**

| SERM's > | buffer | estra-diol | estriol | prem-arin | tamox-ifen | nafox-idine | clomi-phene | ralox-ifene | ICI | HPTE | 16_$\alpha$ OH estr-one | 4-OH estr-one |
|---|---|---|---|---|---|---|---|---|---|---|---|---|
| peptides | | | | | | | | | | | | |
| 1R | 0 | 1+ | 0 | 2+ | 0 | 1+ | 2+ | 1+ | 1+ | 1+ | 2+ | 2+ |
| 2R | 0 | 0 | 0 | 1+ | 0 | 0 | 0 | 0 | 0 | 0 | 0 | 1+ |
| 3B | 0 | 0 | 0 | 1+ | 0 | 0 | 0 | 0 | 0 | 0 | 0 | 0 |
| 4B | 0 | 0 | 0 | 1+ | 0 | 0 | 0 | 0 | 0 | 0 | 0 | 0 |
| 5B | 0 | 0 | 0 | 1+ | 0 | 0 | 1+ | 1+ | 0 | 1+ | 1+ | 1+ |
| 6B | 6+ | 3+ | 5+ | 5+ | 1+ | 4+ | 1+ | 2+ | 1+ | 4+ | 4+ | 4+ |
| 1B | 0 | 0 | 0 | 2+ | 1+ | 1+ | 0 | 1+ | 1+ | 1+ | 2+ | 1+ |
| 7B | 0 | 0 | 0 | 2+ | 0 | 0 | 0 | 0 | 0 | 0 | 0 | 0 |
| 10B | 0 | 0 | 1+ | 1+ | 1+ | 2+ | 2+ | 2+ | 1+ | 2+ | 2+ | 2+ |
| 9B | 0 | 1+ | 1+ | 2+ | 2+ | 2+ | 2+ | 2+ | 2+ | 2+ | 2+ | 1+ |
| 1E | 0 | 0 | 0 | 2+ | 0 | 0 | 1+ | 1+ | 1+ | 1+ | 1+ | 1+ |
| 2E | 1+ | 7+ | 7+ | 5+ | 1+ | 2+ | 2+ | 2+ | 2+ | 2+ | 4+ | 2+ |
| 3E | 1+ | 7+ | 7+ | 5+ | 1+ | 2+ | 2+ | 2+ | 1+ | 2+ | 4+ | 2+ |
| 9E | 2+ | 6+ | 6+ | 5+ | 1+ | 1+ | 1+ | 1+ | 1+ | 2+ | 3+ | 2+ |
| 11E | 0 | 7+ | 6+ | 5+ | 2+ | 3+ | 2+ | 3+ | 2+ | 3+ | 5+ | 3+ |

| | | | | | | | | | | | | |
|---|---|---|---|---|---|---|---|---|---|---|---|---|
| 4E | 0 | 4+ | 4+ | 5+ | 2+ | 2+ | 2+ | 2+ | 2+ | 2+ | 3+ | 2+ |
| 5E | 0 | 5+ | 6+ | 5+ | 0 | 1+ | 1+ | 1+ | 1+ | 2+ | 3+ | 2+ |
| 6E | 0 | 5+ | 5+ | 4+ | 0 | 1+ | 1+ | 1+ | 0 | 1+ | 2+ | 1+ |
| 14E | 4+ | 7+ | 7+ | 7+ | 1+ | 2+ | 2+ | 1+ | 1+ | 3+ | 6+ | 4+ |
| 7E | 1+ | 7+ | 7+ | 6+ | 0 | 1+ | 1+ | 1+ | 1+ | 2+ | 4+ | 2+ |
| 8E | 2+ | 7+ | 7+ | 7+ | 1+ | 2+ | 2+ | 1+ | 1+ | 2+ | 4+ | 3+ |
| 15E | 1+ | 7+ | 7+ | 6+ | 0 | 1+ | 0 | 0 | 0 | 1+ | 3+ | 1+ |
| 10E | 0 | 7+ | 6+ | 5+ | 1+ | 1+ | 1+ | 1+ | 1+ | 1+ | 3+ | 2+ |
| 13E | 0 | 3+ | 3+ | 4+ | 1+ | 1+ | 0 | 1+ | 0 | 1+ | 2+ | 1+ |
| 16E | 0 | 1+ | 1+ | 2+ | 1+ | 1+ | 1+ | 1+ | 1+ | 1+ | 2+ | 1+ |
| 17E | 0 | 4+ | 3+ | 3+ | 0 | 0 | 0 | 0 | 1+ | 0 | 1+ | 0 |
| 18E | 1+ | 6+ | 6+ | 5+ | 0 | 1+ | 1+ | 1+ | 1+ | 1+ | 3+ | 2+ |
| E1-1 | 5+ | 7+ | 7+ | 7+ | 3+ | 6+ | 7+ | 4+ | 3+ | 5+ | 5+ | 5+ |
| E1-3 | 7+ | 7+ | 7+ | 7+ | 1+ | 4+ | 3+ | 2+ | 2+ | 4+ | 3+ | 4+ |
| E1-4 | 7+ | 7+ | 7+ | 7+ | 1+ | 4+ | 2+ | 2+ | + | 4+ | 4+ | 4+ |
| E1-7 | 7+ | 7+ | 7+ | 7+ | 2+ | 7+ | 4+ | 4+ | 2+ | 6+ | 6+ | 6+ |
| I4-10 | 1+ | 1+ | 2+ | 2+ | 2+ | 2+ | 2+ | 1+ | 1+ | 1+ | 1+ | 1+ |
| T3-1 | 2+ | 2+ | 2+ | 3+ | 3+ | 4+ | 4+ | 3+ | 3+ | 3+ | 3+ | 3+ |
| T1-10 | 5+ | 3+ | 3+ | 3+ | 7+ | 7+ | 7+ | 6+ | 2+ | 3+ | 3+ | 3+ |
| T2-10 | 3+ | 3+ | 3+ | 3+ | 7+ | 7+ | 7+ | 5+ | 2+ | 3+ | 3+ | 3+ |
| T3-11 | 4+ | 2+ | 3+ | 3+ | 7+ | 7+ | 7+ | 6+ | 2+ | 4+ | 3+ | 3+ |

**Table 13: Binding of New Erβ-ERE Peptides, in presence of Buffer or SERMS, to Erα or Erβ Receptors**

| | Receptor Form bound to and Modulator present | | | | | |
|---|---|---|---|---|---|---|
| | ERβ and buffer | ERβ and Estradiol | ERβ and tamoxifen | ERα and buffer | ERα and estradiol | ERα and tamoxifen |
| Peptide | | | | | | |
| 8b-β | 7+ | 7+ | 1+ | 1+ | 4+ | 2+ |
| 7b-β | 7+ | 7+ | 2+ | 2+ | 4+ | 3+ |
| 13b-β | 7+ | 7+ | 2+ | 2+ | 2+ | 2+ |

(continued)

| | Receptor Form bound to and Modulator present | | | | | |
|---|---|---|---|---|---|---|
| | ERβ and buffer | ERβ and Estradiol | ERβ and tamoxifen | ERα and buffer | ERα and estradiol | ERα and tamoxifen |
| Peptide | | | | | | |
| 14b-β | 7+ | 7+ | 7+ | 2+ | 2+ | 3+ |
| 17b-β | 5+ | 2+ | 4+ | 2+ | 2+ | 3+ |
| 24b-β | 7+ | 7+ | 7+ | 2+ | 2+ | 3+ |
| 23b-β | 7+ | 6+ | 7+ | 3+ | 3+ | 3+ |
| 11b-β | 7+ | 7+ | 7+ | 1+ | 1+ | 1+ |
| 16b-β | 7+ | 4+ | 5+ | 2+ | 3+ | 3+ |
| 18b-β | 7+ | 7+ | 7+ | 2+ | 2+ | 2+ |
| 19b-β | 7+ | 6+ | 7+ | 5+ | 4+ | 4+ |
| 20b-β | 7+ | 4+ | 5+ | 4+ | 3+ | 4+ |
| 3b-β | 5+ | 2+ | ++ | 5+ | 3+ | 3+ |
| 4b-β | 7+ | 3+ | 3+ | 4+ | 3+ | 3+ |
| 5b-β | 7+ | 6+ | 7+ | 6+ | 6+ | 7+ |
| 6b-β | 6+ | 2+ | 2+ | 5+ | 3+ | 3+ |
| 1b-β | 7+ | 7+ | 2+ | 4+ | 7+ | 3+ |
| 21b-β | 7+ | 7+ | 3+ | 6+ | 7+ | 3+ |
| 23b-β | 7+ | 7+ | 3+ | 3+ | 6+ | 3+ |
| 1t-β | 3+ | 2+ | 7+ | 3+ | 3+ | 7+ |
| 9t-β | 3+ | 2+ | 7+ | 3+ | 3+ | 6+ |
| 16t-β | 7+ | 5+ | 7+ | 7+ | 6+ | 7+ |
| 7t-β | 7+ | 7+ | 7+ | 2+ | 2+ | 2+ |
| 8t-β | 7+ | 5+ | 5+ | 2+ | 2+ | 2+ |
| 12t-β | 7+ | 7+ | 7+ | 1 + | 1+ | 1+ |
| 13t-β | 7+ | 7+ | 7+ | 3+ | 2+ | 4+ |
| 2t-β | 7+ | 6+ | 7+ | 2+ | 2+ | 3+ |
| 17t-β | 7+ | 7+ | 7+ | 1+ | 2+ | 2+ |
| 20t-β | 7+ | 6+ | 7+ | 2+ | 2+ | 3+ |
| 6t-β | 7+ | 4+ | 7+ | 4+ | 4+ | 6+ |
| 22t-β | 5+ | 4+ | 7+ | 3+ | 4+ | 6+ |
| 23t-β | 6+ | 6+ | 7+ | 4+ | 4+ | 6+ |
| 5t-β | 2+ | 2+ | 7+ | 3+ | 2+ | 5+ |
| 15t-β | 6+ | 2+ | 7+ | 3+ | 2+ | 5+ |
| 14t-β | 3+ | 2+ | 7+ | 2+ | 2+ | 7+ |
| 10t-β | 4+ | 2+ | 7+ | 4+ | 3+ | 6+ |
| 21t-β | 3+ | 3+ | 7+ | 3+ | 3+ | 6+ |
| 18t-β | 6+ | 3+ | 7+ | 7+ | 6+ | 7+ |
| 4t-β | 7+ | 7+ | 7+ | 2+ | 2+ | 3+ |
| | | | | | | |
| 1E-β | 5+ | 7+ | 3+ | 5+ | 7+ | 5+ |
| 2E-β | 4+ | 7+ | 2+ | 6+ | 7+ | 5+ |
| 3E-β | 3+ | 6+ | 1+ | 4+ | 5+ | 4+ |
| 4E-β | 5+ | 7+ | 3+ | 6+ | 5+ | 6+ |
| 5E-β | 5+ | 7+ | 2+ | 4+ | 6+ | 4+ |
| 6E-β | 3+ | 7+ | 1+ | 3+ | 5+ | 3+ |
| 7E-β | 5+ | 6+ | 1+ | 4+ | 6+ | 3+ |
| 8E-β | 5+ | 7+ | 1+ | 4+ | 6+ | 3+ |
| 9E-β | 4+ | 7+ | 1+ | 3+ | 4+ | ++ |
| 10E-β | 4+ | 7+ | 2+ | 5+ | 6+ | 4+ |

(continued)

| | Receptor Form bound to and Modulator present | | | | | |
|---|---|---|---|---|---|---|
| | ERβ and buffer | ERβ and Estradiol | ERβ and tamoxifen | ERα and buffer | ERα and estradiol | ERα and tamoxifen |
| Peptide | | | | | | |
| 11E-β | 5+ | 6+ | 4+ | 6+ | 5+ | 6+ |
| 12E-β | 4+ | 7+ | 1+ | 2+ | 2+ | 2+ |
| 13E-β | 2+ | 6+ | 1+ | 5+ | 4+ | 5+ |
| 14E-β | 5+ | 7+ | 2+ | 5+ | 7+ | 5+ |
| 15E-β | 4+ | 7+ | V | 6+ | 6+ | 5+ |
| 16E-β | 6+ | 7+ | 2+ | 3+ | 6+ | 3+ |
| 17E-β | 3+ | 7+ | 1+ | 5+ | 4+ | 4+ |
| 18E-β | 4+ | 7+ | 1+ | 5+ | 6+ | 5+ |
| 19E-β | 4+ | 7+ | 2+ | 4+ | 5+ | 3+ |
| 20E-β | 3+ | 7+ | 2+ | 5+ | 5+ | 4+ |
| 21E-β | 3+ | 7+ | 1+ | 4+ | 5+ | 3+ |
| 22E-β | 5+ | 7+ | 2+ | 4+ | 5+ | 3+ |
| 23E-β | 5+ | 7+ | 2+ | 6+ | 6+ | 5+ |
| 24E-β | 5+ | 7+ | 2+ | 5+ | 6+ | 4+ |
| 25E-β | 3+ | 7+ | 2+ | 5+ | 5+ | 4+ |
| 26E-β | 5+ | 7+ | 2+ | 6+ | 4+ | 5+ |
| 27E-β | 6+ | 7+ | 2+ | 4+ | 6+ | 3+ |
| 28E-β | 3+ | 7+ | 2+ | 4+ | 3+ | 3+ |
| 29E-β | 7+ | 7+ | 5+ | 3+ | 4+ | 3+ |
| 30E-β | 6+ | 7+ | 3+ | 4+ | 3+ | 3+ |
| 31E-β | 3+ | 7+ | 2+ | 3+ | 2+ | 3+ |
| 32E-β | 5+ | 7+ | 2+ | 5+ | 4+ | 4+ |
| 33E-β | 5+ | 7+ | 2+ | 3+ | 3+ | 2+ |
| 34E-β | 4+ | 6+ | 2+ | 5+ | 3+ | 4+ |
| 35E-β | 2+ | 6+ | 1+ | 2+ | 2+ | 2+ |

**Table 14A: Class Specific Fingerprint on ERα**

| Class | Peptide | Modulator (SERM) present during binding: buffer | Estra-diol | Es-triol | Prem-arin | 4-OH Tamoxifen | Nafoxidine | Clomiphene | Raloxifene | ICI 182,780 | 16a-OH Estrone | DES | Pro-gest-erone |
|---|---|---|---|---|---|---|---|---|---|---|---|---|---|
| α/β I | #4 | 1+ | 6+ | 4+ | 2+ | 1+ | 1+ | 1+ | 1+ | 1+ | 2+ | 2+ | 1+ |
| α/β_II | #48 ER | 7+ | 2+ | 4+ | 2+ | 1+ | 1+ | 1+ | 1+ | 1+ | 2+ | 2+ | 6+ |
| α/β_III | 2PT | 1+ | 1+ | 1+ | 2+ | 7+ | 4+ | 6+ | 4+ | 1+ | 2+ | 1+ | 2+ |
| α/β_IV | 9Tβ | 1+ | 1+ | 1+ | 1+ | 6+ | 4+ | 4+ | 2+ | 0 | 1+ | 1+ | 1+ |
| α/β_V | 14Tβ | 1+ | 1+ | 1+ | 1+ | 1+ | 1+ | 2+ | 1+ | 1+ | 1+ | 1+ | 1+ |
| α_I | #33 | 7+ | 7+ | 7+ | 6+ | 7+ | 7+ | 7+ | 7+ | 7+ | 6+ | 6+ | 6+ |
| α_II | #5 | 1+ | 6+ | 5+ | 6+ | 5+ | 4+ | 5+ | 4+ | 6+ | 5+ | 4+ | 1+ |
| α_III | #13 ER | 5+ | 2+ | 2+ | 2+ | 6+ | 2+ | 5+ | 2+ | 2+ | 2+ | 3+ | 4+ |

**Table 14B: Class Specific Fingerprint on ERβ**

| Class | Peptide | Modulator (SERM) present during binding: buffer | Estra-diol | Es-triol | Prem-arin | 4-OH Tamoxifen | Nafoxidine | Clomiphene | Raloxifene | ICI 182,780 | 16a-OH Estrone | DES | Pro-gest-eone |
|---|---|---|---|---|---|---|---|---|---|---|---|---|---|
| α/β I | #4 | 2+ | 7+ | 7+ | 6+ | 0 | 1+ | 0 | 0 | 0 | 5+ | 5+ | 1+ |
| α/β_II | #48 ER | 7+ | 2+ | 6+ | 4+ | 1+ | 4+ | 1+ | 4+ | 2+ | 3+ | 3+ | 6+ |
| α/β_III | 2PT | 2+ | 1+ | 1+ | 1+ | 7+ | 3+ | 5+ | 6+ | 1+ | 1+ | 1+ | 1+ |
| α/β_IV | 9Tβ | 2+ | 1+ | 1+ | 1+ | 7+ | 5 | 5+ | 4+ | 1+ | 1+ | 1+ | 1+ |
| α/β_V | 14Tβ | 1+ | 1+ | 1+ | 1+ | 7+ | 3+ | 5+ | 2+ | 0 | 1+ | 1+ | 1+ |
| βI | 4Tβ | 6+ | 3+ | 2+ | 7+ | 7+ | 4+ | 3+ | 4+ | 0 | 2+ | 4+ | 5+ |
| βI | 35Eβ | 1+ | 5+ | 6+ | 4+ | 0 | 0 | 0 | 0 | 0 | 3+ | 3+ | 0 |
| βIII | 12Bβ | 7+ | 7+ | 7+ | 7+ | 1+ | 5 | 3+ | 3+ | 1+ | 7+ | 7+ | 5+ |

Notes to Table 14:

Fingerprint analysis of estrogen receptor modulators on **(A)** ER $\alpha$ and **(B)** ER $\beta$. Immobilized ER was incubated with estradiol (1 $\mu$M), estriol (1 $\mu$M), premarin (10 $\mu$M), 4-OH tamoxifen (1 $\mu$M), nafoxidine (10 $\mu$M), clomiphene (10 $\mu$M), raloxifene (1 $\mu$M), ICI 182,780 (1 $\mu$M), 16$\alpha$-OH estrone (10 $\mu$M), DES (1 $\mu$M) or progesterone (1 $\mu$M). Phage ELISAs were conducted as described.

Table 15a Binding of the peptide probes to ERα in the presence of modulators

| Peptide Probe | α/βI | | α/βIII | | α/βIV | | α/βV | | αII | |
|---|---|---|---|---|---|---|---|---|---|---|
| | Equiv.[a] | EC50[b] | Equiv. | EC50 | Equiv. | EC50 | Equiv. | EC50 | Equiv. | EC50 |
| Buffer | 0 | | 0 | | 0 | | 0 | | 0 | |
| 17β-Estradiol | **100** | 8.0 | -66 | 18.0 | -43 | 8.1 | 0 | | **100** | 17.5 |
| 17α-Estradiol | 53 | 10.0 | -61 | 88.0 | -54 | 5.9 | 0 | | 80 | 9.6 |
| Estriol | 65 | 8.1 | -59 | 19.2 | -28 | 44.9 | 0 | | 62 | 11.8 |
| 4-OH Tamoxifen | 0 | | **100** | 54.9 | **100** | 59.6 | **100** | 30.9 | 38 | 41.7 |
| Nafoxidine | 0 | | 23 | 292.1 | 13 | 372.2 | 0 | | 32 | 39.0 |
| Clomiphene | 0 | | 37 | 143.2 | 19 | 708.5 | 19 | 282.1 | 56 | 118.9 |
| Raloxifene | 0 | | 51 | 49.2 | 0 | | 0 | | 44 | 41.7 |
| ICI 182,780 | 0 | | **-100** | 25.8 | **-100** | 24.7 | 0 | | 56 | 28.5 |
| Diethylstilbesterol | 71 | 13.4 | -53 | 29.7 | 0 | | 0 | | 69 | 15.8 |
| GW7604 | 0 | | 0 | | 0 | | 0 | | 35 | 8.4 |

[a]Equivalency may be positive or negative. These are both expressed in relative (percentage terms) but the positive and negative standards (100 and -100% marks) are set differently. Thus, the positive and negative values are scaled differently. Positive equivalency is defined as the maximum stimulation achieved with a given compound as a percentage of the maximum stimulation achieved with the positive modulator used for isolation of a given peptide probe (see Table 10). Negative values indicates that an increase in the concentration of a compound results in a reduction of the binding of the peptide probe as compared to the binding of the probe in buffer. These are expressed as a percentage of the reduction by ICI 182,780. For αII, ICI 182,780 acts as an agonist, and its equivalency is therefore stated as a percentage of the reference modulator β estradiol. Results for αIII were zero in all cases. [b]EC50 is defined as the concentration in nanomolar of a given compound required to achieve fifty percent of the maximal signal for that compound.

Table 15b Binding of the peptide probes to ERβ in the presence of modulators

| Peptide Probe | α/βI | | α/βIII | | α/βIV | | α/βV | | βI | | βIII | |
|---|---|---|---|---|---|---|---|---|---|---|---|---|
| | Equiv.[a] | EC50[b] | Equiv. | EC50 | Equiv. | EC50 | Equiv. | EC50 | Equiv. | EC50 | Equiv. | EC50 |
| Buffer | 0 | | 0 | | 0 | | 0 | | 0 | | 0 | |
| 17β-Estradiol | **100** | 21.8 | -71[c] | 5.7 | -84 | 26.7 | 0 | | -69 | 12.8 | **100** | 17.0 |
| 17α-Estradiol | 44 | 8.8 | -78 | 7.1 | -82 | 12.9 | 0 | | -74 | 10.1 | 42 | 6.7 |
| Estriol | 81 | 19.5 | -57 | 15.8 | -75 | 12.4 | 0 | | -96 | 20.7 | 77 | 11.7 |
| 4-OH Tamoxifen | | | **100** | 37.3 | **100** | 179.8 | **100** | 50.0 0 | **100** | 20.6 | -100 | 34.4 |
| Nafoxidine | | | 27 | 231.7 | 0 | | 0 | | -44 | 320.5 | 0 | |
| Clomiphene | | | 34 | 82.2 | 0 | | 13 | 149.8 | -62 | 135.1 | -61 | 122.5 |
| Raloxifene | | | 77 | 90.1 | 0 | | 0 | | -53 | 89.9 | -71 | 156.2 |
| ICI 182,780 | | | **-100** | 18.1 | **-100** | 35.3 | 0 | | **-100** | 28.9 | **-100** | 48.4 |
| Diethylstilbesterol | 68 | 33.9 | -78 | 14.5 | -96 | 17.8 | 0 | | -59 | 11.1 | 86 | 25.4 |
| GW 7604 | 0 | | -86 | 4.2 | 74 | 3050.1 | 0 | | 159 | 3.3 | -106 | 7.7 |

[a]Positive equivalency is defined as the maximum stimulation achieved with a given compound as a percentage of the maximum stimulation achieved with the modulator used for isolation of a given peptide probe. The equivalency numbers for these reference modulators are bolded. See also Table 10. Negative values indicate that an increase in the concentration of a compound results in a reduction of the binding of the peptide probe as compared to the binding of the probe in buffer. These negative values are expressed as a percentage of the reduction by ICI 182,780, so ICI 182,780 was scored -100 by definition, and is also bolded. Results for αβII were zero in all cases. [b]EC50 is defined as the concentration in nanomolar of a given compound required to achieve fifty percent of the maximal signal for that compound.

**Table 99**

| Peptide Interactions with ER Alpha | | | | | | | | |
|---|---|---|---|---|---|---|---|---|
| Peptide | No Addition | | Estradiol | | 4-hydroxy Tamoxifen | | Tamoxifen Citrate | |
| | value | standard deviation | value | standard deviation | value | standard deviation | value | standard deviation |
| F6 | 30983 | 7961 | 38883 | 2899 | 20267 | 3313 | 11947 | 460 |
| A2 | 1224 | 8 | 30983 | 2993 | 20100 | 2146 | 8933 | 1299 |
| AB3 | 381 | 16 | 605 | 64 | 3194 | 86 | 600 | 38 |
| AB5 | 548 | 16 | 648 | 44 | 4128 | 25 | 1005 | 36 |

Values are in beta-galactosidase units (MOD/min/mg)

Table 100

**A**

```
        S R A G L L S D L L E G K S R
        S S R S L L R D L L M V D S R
        S S N K L L Y N L L K M E S R
        S S K S L L L N L L S T P S R
H S F P R E S L L V R L L Q G G
              S R L E M L L R S E T D F S R
              S R L E E L L K W G S V T S R
              S R L E Q L L K E E F S Y S R
              S R L E Q L L R S E P D F S R
              S R L E D L L R A P F T T S R
              S R L E S L L R F G Q L D S R
            S S R L L S L L V G D F N S R
              S R L E E L L L G T N R D S R
              S R L E E L L L M D F W R S R
              S R L K E L L L L P T D L S R
              S R L E C L L E G R L N C S R
            S S K L Y C L L D E S Y C S R
              S R L S C L L M G F E D C S R
            S S K L I R L L T S D E E L S R
            S S R L M E L L Q E G Q G W S R
S S N H Q S S R L I E L L S R
            S S R L W Q L L A S T D T S R
            S S K L W Q L L S S P I D S R
              S R L V A L L K S P W S V S R
        S S N S M L W K L L A A P S R
          S S K T L W R L L E G E R S R
      S R A G P V L W G L L S E S R
        S R S P I L T H L L S L G S R
        S S T G I L W K L L T A E S R
        S S H G I L W R L L S E G S R
```

**B**

```
                K L V Q L L T T T A E
                I L H R L L Q E G S P
SRC1a           L L R Y L L D K D E K
                L L Q Q L L T E

CBP             Q L S E L L R G G S G
                Q L V L L L H A H K C

                Y L E G L L M H Q A A
                L L A S L L Q S E S S
                H L K T L L K K S K V
RIP140          Q L A L L L S S E A H
                L L L H L L K S Q T I
                L L Q L L L G H K N E
                V L Q L L L G N P K G
                L L S R L L R Q N Q D
                V L K Q L L L S E N C
```

Table 101

| | |
|---|---|
| ER4 | SSNHQSRLIELLSR |

(continued)

| | |
|---|---|
| D47 | HVYQHPLLLSLLSSEHESG |
| C33 | HVEMHPLLMGLLMESQWGA |
| D14 | QEAHGPLLWNLLSRSDTDW |
| F6 | GHEPLTLLERLLMDDKQAV |
| D22 | LPYEGSLLLKLLRAPVEEV |
| D48 | SGWENSILYSLLSDRVSLD |
| D43 | AHGESSLLAWLLSGEYSSA |
| D17 | GVFCDSILCQLLAHDNARL |
| D41 | HHNGHSILYGLLAGSDAPS |
| D26 | LGERASLLDMLLRQENPAW |
| D40 | SGWNESTLYRLLQADAFDV |
| D15 | PSGGSSVLEYLLTHDTSIL |
| D2 | GSEPKSRLLELLSAPVTDV |
| D30 | HPTHSSRLWELLMEATPTM |
| D11 | VESGSSRLMQLLMANDLLT |
| D10 | WEEHSQMLLHLLDTGEAVW |
| F4 | PVGEPGLLWRLLSAPVERE |

References

Anzick, S. L., Kononen, J., Walker, R. L., Azorsa, D. O., Tanner, M. M., Guan, X.-Y., Sauter, G., Kallioniemi, O.-P., Trent, J. M., and Meltzer, P. S. (1997) AIB1 a steroid receptor coactivator amplified in breast and ovarian cancer. Science 277, 965-968.

Chambraud, B., Berry, M., Redeuilh, G., Chambon, P., and Baulieu, E., (1990) Several regions of the human estrogen receptor are involved in the formation of receptor-heatshock protein 90 complexes. *J. Biol. Chem.* 265, 20686-20691.

Heery, D. M., Kalkhoven, E., Hoare, S., and Parker, M. G., (1997) A signature motif in transcriptional co-activators mediates binding to nuclear receptors. *Nature*, 387 733-736.

Kraus, W. L., McInerney, E. M., and Katzenellenbogen, B. S., (1995) Ligand-dependent, transcriptionally productive association of the amino-and carboxyl-terminal regions of a steroid hormone nuclear receptor. *Proc. Natl. Acad. Sci., USA* 92 12314-12318.

Montano, M. M., Muller, V., Trobaugh, A., and Katzenellenbogen, B. S., (1995) The carboxy-terminal F domain of the human estrogen receptor: role in the transcriptional activity of the receptor and the effectiveness of antiestrogens as estrogen antagonists. *Mol Endocrinol.,* 9 814-825.

Paech, K., Webb, P., Kuiper, G. G .J. M., Nilsson, S., Gustafsson, J.-A., Kushner, P. J., and Scanlan, T. S. (1997) Differential ligand activation of estrogen receptors ER α and ER β at AP1 sites. *Science* 277, 1508-1510.

**Claims**

1. A method of predicting the receptor-modulating activity of a compound which modulates the biological activity of a receptor which comprises:

   (a) providing a ligand for the receptor;
   (b) screening a first combinatorial library comprising a plurality of members for the ability to bind to a receptor in at least two different reference conformations,
   (c) based on said screening, providing a panel of first library members, said panel comprising members which differ with respect to their ability to binding to the receptor, depending on its conformation,
   (d) screening a plurality of reference substances known to modulate the biological activity of said receptor to determine their effect on the binding of each member of said panel to said receptor, thereby obtaining a reference fingerprint for each reference substance, said fingerprint comprising a plurality of panel-based descriptors, each panel-based descriptor characterizing the effect of the reference substance on the binding of a particular panel member to said receptor, said reference fingerprint's panel based descriptors collectively characterizing the effect of the reference substance on the binding of all of the panel members, individually, to said receptor,
(e) screening a test substance of unknown activity relative to said receptor to determine its effect on the binding of each member of said panel to said receptor, thereby obtaining a test fingerprint for said test substance,
(f) comparing the test fingerprint to the reference fingerprints, and
(g) predicting the biological activity of the test substance, based on the assumption that its biological activity will be similar to that of reference substances with similar fingerprints,

wherein screening steps (b), (d) and/or (e) are performed either in a cell-free in vitro assay or in a cell-based assay which is not an assay of a whole multicellular animal or tissues and organs isolated from such an animal.

**2.** The method of claim 1 in which at least one reference conformation is an unliganded conformation of the receptor.

**3.** The method of claim 2 in which at least one reference conformation is a liganded conformation of the receptor.

**4.** The method of claim 1 in which the conformations comprise a first liganded conformation induced by a first ligand and a second liganded conformation induced by a second and different ligand.

**5.** The method of claim 3 in which said panel comprises at least two of the following:

(i) a member which binds the ligand-bound receptor more strongly than it binds the unliganded receptor, and which detectably binds the unliganded receptor,
(ii) a member which binds tne ligand-bound receptor less strongly than it binds the unliganded receptor, and
(iii) a member which binds the ligand-bound receptor about as strongly as it binds the unliganded receptor, and detectably binds both.

**6.** The method of claim 1 wherein a plurality of different ligands are used in characterizing the panel.

**7.** The method of claim 1 in which the biological activity of the reference substances at said receptor is known for a plurality of different tissues, so that the biological activity of the test substance in said tissues is predicted.

**8.** The method of claim 1 in which the receptor is a nuclear receptor.

**9.** The method of claim 1 in which the receptor is an estrogen receptor.

**10.** The method of claim 1 in which the receptor is a G-protein coupled receptor, a G protein, or a G protein subunit.

**11.** The method of claim 1 in which at least one ligand is a pharmacological agonist or antagonist of the receptor.

**12.** The method of claim 1 in which at least one conformation is induced by a natural ligand of the receptor.

**13.** The method of claim 1 in which at least one conformation is induced by a ligand which is not a natural ligand of the receptor.

**14.** The method of claim 1 in which the first combinatorial library is an oligopeptide library.

**15.** The method of claim 1 in which the first combinatorial library is a nucleic acid library.

**16.** The method of claim 1 in which the test substances are provided and screened in the form of a combinatorial library.

**17.** The method of claim 1 in which the biologically active component of said test substance is an organic compound with a molecular weight of less than 500 daltons.

**18.** The method of claim 1 in which screening steps (b), (d) and (e) are performed in a cell-free in vitro assay.

**19.** The method of claim 1 in which screening steps (b), (d) and (e) are performed in a cell-based assay which is not an assay of a whole multicellular animal or tissues and organs isolated from such an animal.

20. The method of claim 19 in which screening steps (d) and (e) are performed in a two-hybrid assay system, and the members of the panel are peptides.

21. The method of claim 1 in which the receptor in a glucocorticoid receptor.

**Patentansprüche**

1. Verfahren zur Vorhersage der Rezeptor-modulierenden Aktivität einer Verbindung, welche die biologische Aktivität eines Rezeptors moduliert, welches umfasst:

(a) zur Verfügung stellen eines Liganden für den Rezeptor;
(b) Screenen einer ersten kombinatorischen Bibliothek umfassend eine Mehrzahl von Mitgliedern nach der Fähigkeit, einen Rezeptor in wenigstens zwei verschiedenen Referenzkonformationen zu binden,
(c) basierend auf diesem Screening das zur Verfügung stellen einer Auswahl von ersten Bibliotheksmitgliedern, wobei die Auswahl Mitglieder umfasst, welche sich bezüglich ihrer Fähigkeit, an den Rezeptor zu binden, abhängig von dessen Konformation unterscheidet,
(d) Screenen einer Mehrzahl von Referenzsubstanzen, von denen bekannt ist, dass sie die biologische Aktivität des Rezeptors modulieren, um deren Wirkung auf die Bindung jedes Mitglieds der Auswahl an den Rezeptor zu bestimmen, wobei ein Referenzfingerprint für jede Referenzsubstanz erhalten wird, wobei der Fingerprint eine Vielzahl von Auswahl-basierenden beschreibenden Eigenschaften umfasst, wobei jede Auswahl-basierende beschreibende Eigenschaft die Wirkung der Referenzsubstanz auf das Binden eines bestimmten Auswahlmitgliedes an den Rezeptor charakterisiert, wobei die Auswahl-basierenden beschreibenden Eigenschaften des Referenzfingerprints gemeinsam die Wirkung der Referenzsubstanz auf das Binden sämtlicher Auswahlmitglieder an den Rezeptor charakterisieren,
(e) Screenen einer Testsubstanz von unbekannter Aktivität relativ zu dem Rezeptor, um dessen Wirkung auf das Binden jedes Mitgliedes der Auswahl an den Rezeptor zu bestimmen, wobei ein Testfingerprint für die Testsubstanz erhalten wird,
(f) vergleichen des Testfingerprints mit den Referenzfingerprints, und
(g) Vorhersagen der biologischen Aktivität der Testsubstanz basierend auf der Annahme, dass dessen biologische Aktivität ähnlich zu derjenigen der Referenzsubstanzen mit ähnlichen Fingerprints sein wird,

wobei die Screeningschritte (b), (d) und/oder (e) entweder in einem zellfreien in vitro-Assay oder in einem zellbasierendem Assay ausgeführt werden, wobei der zellbasierende Assay kein Assay ist, der auf einem gesamten multizellulären Tier oder auf Geweben oder Organen, die aus einem solchen Tier isoliert worden, sind, basiert.

2. Verfahren gemäß Anspruch 1, wobei wenigstens eine Referenzkonformation eine Liganden-freie Konformation des Rezeptors ist.

3. Verfahren gemäß Anspruch 2, wobei wenigstens eine Referenzkonformation eine Konformation mit einem Liganden des Rezeptors ist.

4. Verfahren gemäß Anspruch 1, wobei die Konformationen eine erste Konformation mit Liganden umfasst, die durch einen ersten Liganden induziert wird und eine zweite Konformation mit einem Liganden umfassen, die durch einen zweiten, unterschiedlichen Liganden induziert wird.

5. Verfahren gemäß Anspruch 3, wobei die Auswahl wenigstens zwei der folgenden umfasst:

(i) ein Mitglied, welches den Liganden-gebundenen Rezeptor stärker bindet, als es den nicht durch einen Liganden gebundenen Rezeptor bindet, und welches den nicht durch einen Liganden gebundenen Rezeptor detektierbar bindet,
(ii) ein Mitglied, welches den Liganden-gebundenen Rezeptor weniger stark bindet, als es den nicht durch einen Liganden gebundenen Rezeptor bindet, und
(iii) ein Mitglied, welches den Liganden-gebundenen Rezeptor in etwa ebenso stark bindet, wie es den nicht durch einen Liganden gebundenen Rezeptor bindet, und beide detektierbar bindet.

6. Verfahren gemäß Anspruch 1, wobei eine Vielzahl von unterschiedlichen Liganden bei der Charakterisierung der Auswahl verwendet werden.

7. Verfahren gemäß Anspruch 1, wobei die biologische Aktivität der Referenzsubstanzen bei diesem Rezeptor für eine Vielzahl von unterschiedlichen Geweben bekannt ist, so dass die biologische Aktivität der Testsubstanzen in diesen Geweben vorhergesagt wird.

8. Verfahren gemäß Anspruch 1, wobei der Rezeptor ein nuklearer Rezeptor ist.

9. Verfahren gemäß Anspruch 1, wobei der Rezeptor ein Estrogenrezeptor ist.

10. Verfahren gemäß Anspruch 1, wobei der Rezeptor ein G-Protein-gekoppelter Rezeptor, ein G-Protein oder eine G-Proteinuntereinheit ist.

11. Verfahren gemäß Anspruch 1, wobei wenigstens ein Ligand ein pharmakologischer Agonist oder Antagonist des Rezeptors ist.

12. Verfahren gemäß Anspruch 1, wobei wenigstens eine Konformation durch einen natürlichen Liganden des Rezeptors induziert wird.

13. Verfahren gemäß Anspruch 1, wobei wenigstens eine Konformation durch einen Liganden induziert wird, welcher kein natürlicher Ligand des Rezeptors ist.

14. Verfahren gemäß Anspruch 1, wobei die erste kombinatorische Bibliothek eine Oligopeptid-Bibliothek ist.

15. Verfahren gemäß Anspruch 1, wobei die erste kombinatorische Bibliothek eine Nukleinsäure-Bibliothek ist.

16. Verfahren gemäß Anspruch 1, wobei die Testsubstanzen in der Form einer kombinatorischen Bibliothek zur Verfügung gestellt und gescreent werden.

17. Verfahren gemäß Anspruch 1, wobei der biologisch aktive Bestandteil der Testsubstanz eine organische Verbindung mit einem Molekulargewicht von weniger als 500 Dalton ist.

18. Verfahren gemäß Anspruch 1, wobei die Screeningschritte (b), (d) und (e) in einem zellfreien in-vitro Assay durchgeführt werden.

19. Verfahren gemäß Anspruch 1, wobei die Screeningschritte (b), (d) und (e) in einem zellbasierenden Assay durchgeführt werden, wobei der Assay kein Assay ist, der auf einem gesamten multizellulären Tier oder auf Geweben und Organen, die aus solche einem Tier isoliert wurden, basiert.

20. Verfahren gemäß Anspruch 19, bei welchem die Screeningschritte (d) und (e) in einem two-hybrid Assaysystem durchgeführt werden, und die Mitglieder der Auswahl Peptide sind.

21. Verfahren gemäß Anspruch 1, wobei der Rezeptor ein Glucokortikoidrezeptor ist.

## Revendications

1. Procédé de prédiction d'activité modulatrice de récepteur d'un composé qui module l'activité biologique d'un récepteur qui comprend :

(a) la mise en oeuvre d'un ligand pour le récepteur;
(b) le criblage d'une première banque combinatoire comprenant une pluralité d'éléments pour la capacité de se lier à un récepteur dans au moins deux conformations de référence différentes,
(c) la mise en oeuvre, sur la base dudit criblage, d'une gamme de premiers éléments de la banque, ladite gamme comprenant des éléments qui diffèrent dans leur capacité de liaison au récepteur en fonction de sa conformation,
(d) le criblage d'une pluralité de substances de référence connues pour moduler l'activité biologique dudit récepteur, en vue de déterminer leur effet sur la liaison de chaque élément de ladite gamme audit récepteur, ce qui fournit un profil de référence pour chaque substance de référence, ledit profil comprenant une pluralité de descripteurs basés sur la gamme, chaque descripteur basé sur la gamme caractérisant l'effet de la substance de référence sur la liaison d'un élément particulier de la gamme audit récepteur, lesdits descripteurs basés sur la gamme du profil de référence caractérisant collectivement l'effet de la substance de référence sur la liaison de tous les éléments de la gamme, individuellement, audit récepteur,
(e) le criblage d'une substance d'essai d'activité inconnue vis-à-vis dudit récepteur pour déterminer son effet sur la liaison de chaque élément de ladite gamme audit récepteur, ce qui permet d'obtenir ainsi un profil d'essai pour ladite substance d'essai,
(f) la comparaison du profil d'essai aux profils de référence, et
(g) la prédiction de l'activité biologique de la substance d'essai en se basant sur l'hypothèse que son activité biologique sera similaire à celle des substances de référence ayant des profils similaires,

dans lequel les étapes de criblage (b), (d) et/ou (e) sont effectuées dans un dosage acellulaire *in vitro* ou dans un essai cellulaire qui n'est pas un dosage sur un animal multicellulaire entier ou sur des tissus et des organes isolés de cet animal.

**2.** Procédé selon la revendication 1, dans lequel au moins une conformation de référence est une conformation du récepteur sans ligand.

**3.** Procédé selon la revendication 2, dans lequel au moins une conformation de référence est une conformation du récepteur avec un ligand.

**4.** Procédé selon la revendication 1, dans lequel les conformations comprennent une première conformation avec ligand induite par un premier ligand et une deuxième conformation avec ligand induite par un deuxième ligand différent.

**5.** Procédé selon la revendication 3, dans lequel ladite gamme comprend au moins deux des éléments suivants :

(i) un élément qui se lie au récepteur lié à un ligand plus fortement qu'il ne se lie au récepteur sans ligand et qui se lie de manière détectable au récepteur sans ligand,
(ii) un élément qui se lie au récepteur lié à un ligand moins fortement qu'il ne se lie au récepteur sans ligand et
(iii) un élément qui se lie au récepteur lié à un ligand à peu près aussi fortement qu'il se lie au récepteur sans ligand, et qui se lie de manière détectable dans les deux cas.

**6.** Procédé selon la revendication 1, dans lequel on utilise une pluralité de différents ligands pour caractériser la gamme.

**7.** Procédé selon la revendication 1, dans lequel l'activité biologique des substances de référence sur ledit récepteur est connue pour plusieurs tissus différents, si bien que l'activité biologique de la substance d'essai dans lesdits tissus est prédite.

**8.** Procédé selon la revendication 1, dans lequel le récepteur est un récepteur nucléaire.

**9.** Procédé selon la revendication 1, dans lequel le récepteur est un récepteur d'oestrogène.

**10.** Procédé selon la revendication 1, dans lequel le récepteur est un récepteur couplé à une protéine G, une protéine G ou une sous-unité de protéine G.

**11.** Procédé selon la revendication 1, dans lequel au moins un ligand est un agoniste ou un antagoniste pharmacologique du récepteur.

**12.** Procédé selon la revendication 1, dans lequel au moins une conformation est induite par un ligand naturel du récepteur.

**13.** Procédé selon la revendication 1, dans lequel au moins une conformation est induite par un ligand qui n'est pas un ligand naturel du récepteur.

**14.** Procédé selon la revendication 1, dans lequel la première banque combinatoire est une banque d'oligopeptides.

**15.** Procédé selon la revendication 1, dans lequel la première banque combinatoire est une banque d'acides nucléiques.

**16.** Procédé selon la revendication 1, dans lequel les substances d'essai sont fournies et criblées sous la forme d'une banque combinatoire.

**17.** Procédé selon la revendication 1, dans lequel le composant biologiquement actif de ladite substance d'essai , est un composé organique ayant un poids moléculaire de moins de 500 daltons.

**18.** Procédé selon la revendication 1, dans lequel les étapes de criblage (b), (d) et (e) sont effectuées dans un dosage acellulaire *in vitro.*

**19.** Procédé selon la revendication 1, dans lequel les étapes de criblage (b), (d) et (e) sont effectuées dans un dosage cellulaire qui n'est pas un dosage sur un animal multicellulaire entier ou sur des tissus et des organes isolés de cet animal.

**20.** Procédé selon la revendication 19, dans lequel les étapes de criblage (d) et (e) sont effectuées dans un système de dosage à double hybride et où les éléments de la gamme sont des peptides.

**21.** Procédé selon la revendication 1, dans lequel le récepteur est un récepteur de glucocorticoïde.

Figure 1

| Effect ⇨ / Compound Name | Breast | Bone | Uterus | Cardiovascular | Cholesterol |
|---|---|---|---|---|---|
| Estradiol | ↑ | ↑ | ↑ | ↑↑ | ↓↓ |
| Tamoxifen | ↓ | ↑ | ↕ | ↑ | ↓ |
| Raloxifene | ↓ | ↑ | ↓ | ↑ | ↓ |
| ICI 182,870 | ↓ | ↓ | ↓ | ↓ | ↑↑ |
| Nafoxidine | | ↑ | ↑ | | ↓ |
| Clomiphene | | ↑ | ↕ | | — |
| Premarin | ↕ | | ↑ | | |

Figure 2

Mapping the Binding Sites for
ER-Interacting Peptides

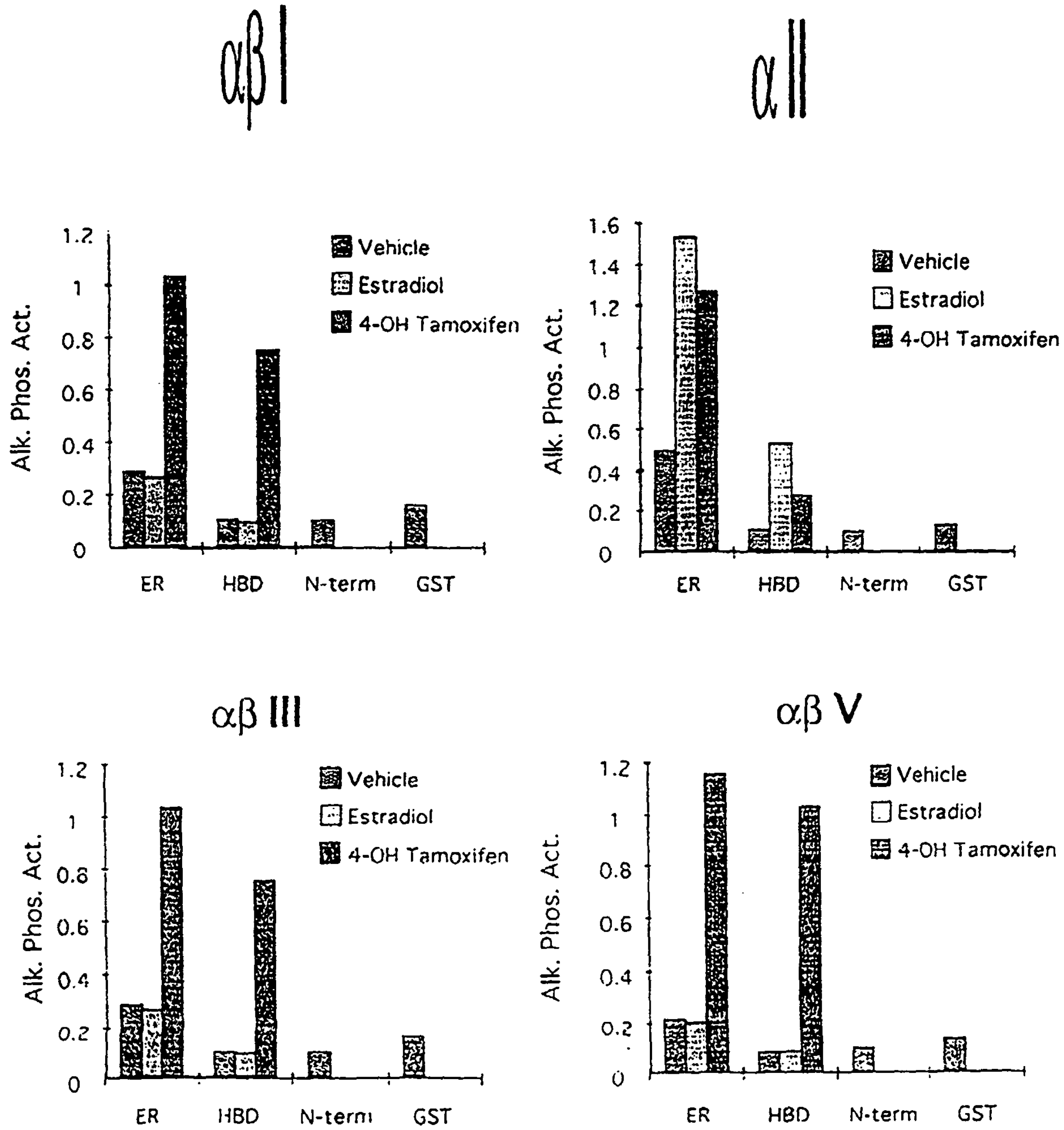

Figure 3

# Different Ligands Induce Different Structural Alterations in ERα and ERβ

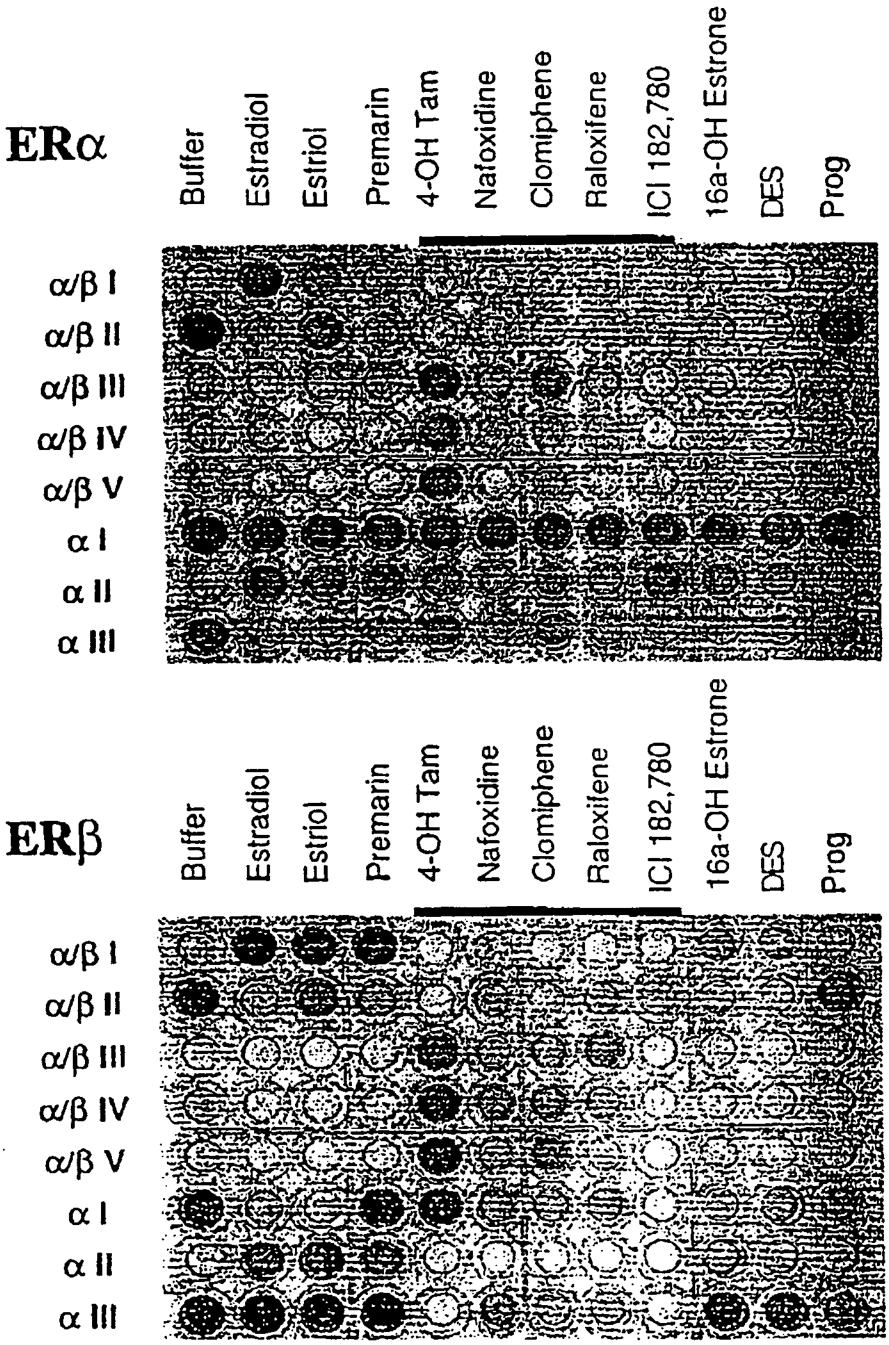

# Fig 4
# Agonists and Antagonists Induce Distinct Alterations in ERα Structure

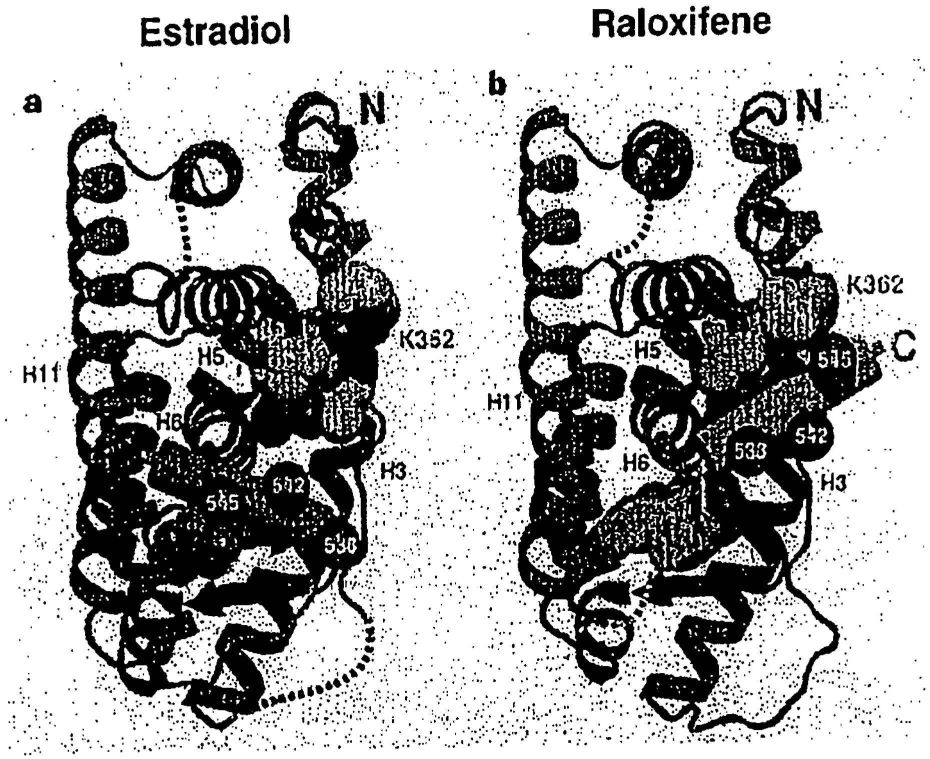


Brzozowski et al. Nature 1997, 389:753-758

Fig 5A

ER Alpha

| | buffer | estradiol | estriol | premarin | 4-OH Tamoxifen | nafoxidene | clomiphene | raloxifene |
|---|---|---|---|---|---|---|---|---|
| αβ I | 1 | 6 | 4 | 2 | 1 | 1 | 1 | 1 |
| αβ II | 7 | 2 | 4 | 2 | 1 | 1 | 1 | 1 |
| αβ III | 1 | 1 | 1 | 2 | 7 | 4 | 6 | 4 |
| α I | 1 | 1 | 1 | 1 | 6 | 4 | 4 | 2 |
| α II | 1 | 1 | 1 | 1 | 1 | 1 | 2 | 1 |
| α III | 7 | 7 | 7 | 6 | 7 | 7 | 7 | 7 |
| α VI | 1 | 6 | 5 | 6 | 5 | 4 | 5 | 4 |
| α V | 5 | 2 | 2 | 2 | 6 | 2 | 5 | 2 |

Fig 5B

| | | | | | | | | |
|---|---|---|---|---|---|---|---|---|
| Universal Max | 19.799 | 19.799 | 19.8 | 19.799 | 19.79898987 | 19.79899 | 19.79899 | 19.79899 |
| Max | 17.407 | 16.7033 | 15.46 | 15.6205 | 17.40689519 | 15.165751 | 15.748016 | 15.45962 |
| | | | | | | | | |
| buffer | 0 | 9.16515 | 6.557 | 7.87401 | 10.67707825 | 8.4852814 | 9.3273791 | 8 |
| estradiol | 9.1652 | 0 | 3 | 4.24264 | 10.19803903 | 6.9282032 | 8.4261498 | 6.324555 |
| estriol | 6.5574 | 3 | 0 | 3.31662 | 9.746794345 | 6.0827625 | 7.8740079 | 5.385165 |
| premarin | 7.874 | 4.24264 | 3.317 | 0 | 8.366600265 | 4.472136 | 6.244998 | 3.464102 |
| 4-OH Tamoxifen | 10.677 | 10.198 | 9.747 | 8.3666 | 0 | 5.4772256 | 2.6457513 | 6.480741 |
| nafoxidene | 8.4853 | 6.9282 | 6.083 | 4.47214 | 5.477225575 | 0 | 3.8729833 | 2 |
| clomiphene | 9.3274 | 8.42615 | 7.874 | 6.245 | 2.645751311 | 3.8729833 | 0 | 4.358899 |
| raloxifene | 8 | 6.32456 | 4.899 | 3.31662 | 6.480740698 | 2.236068 | 4.3588989 | 0 |
| ICI 182,780 | 8.4261 | 5.19615 | 4.472 | 2.23607 | 9.433981132 | 5.3851648 | 7.2111026 | 4.123106 |
| 16α-OH estrone | 7.2801 | 4.3589 | 3.162 | 1 | 8.306623863 | 4.1231056 | 6.164414 | 3 |
| DES | 6.3246 | 4.69042 | 3.317 | 2.44949 | 8.602325267 | 4.6904158 | 6.5574385 | 3.741657 |
| progesterone | 2 | 8.48528 | 5.916 | 6.78233 | 9.797958971 | 7.2111026 | 8.3066239 | 6.63325 |

5B contd

ER Alpha

| ICI 182,780 | 16α-OH estrone | DES | progesterone |
|---|---|---|---|
| 1 | 2 | 2 | 1 |
| 1 | 2 | 2 | 6 |
| 1 | 2 | 1 | 2 |
| 0 | 1 | 1 | 1 |
| 1 | 1 | 1 | 1 |
| 7 | 6 | 6 | 6 |
| 6 | 5 | 4 | 1 |
| 2 | 2 | 3 | 4 |

| | | | |
|---|---|---|---|
| 19.79899 | 19.79898987 | 19.8 | 19.79898987 |
| 17.406895 | 15.26433752 | 15 | 16.03121954 |
| | | | |
| 8.4261498 | 7.280109889 | 6.32 | 2 |
| 5.1961524 | 4.358898944 | 4.69 | 8.485281374 |
| 4.472136 | 3.16227766 | 3.32 | 5.916079783 |
| 2.236068 | 1 | 2.45 | 6.782329983 |
| 9.4339811 | 8.306623863 | 8.6 | 9.797958971 |
| 5.3851648 | 4.123105626 | 4.69 | 7.211102551 |
| 7.2111026 | 6.164414003 | 6.56 | 8.306623863 |
| 4.2426407 | 3.464101615 | 4.12 | 6.708203932 |
| 0 | 2.449489743 | 3 | 7.549834435 |
| 2.4494897 | 0 | 1.73 | 6.08276253 |
| 3 | 1.732050808 | 0 | 5.291502622 |
| 7.5498344 | 6.08276253 | 5.29 | 0 |

Fig. 5C

**Scaled alpha**

| | buffer | estradiol | estriol | premarin | 4-OH Tam | nafoxidene | clomiphen | raloxifene |
|---|---|---|---|---|---|---|---|---|
| buffer | 1 | 0.53709 | 0.668799 | 0.602303 | 0.460726 | 0.571429 | 0.528896 | 0.595939 |
| estradiol | 0.53709 | 1 | 0.848477 | 0.785714 | 0.484921 | 0.650073 | 0.574415 | 0.680562 |
| estriol | 0.668799 | 0.848477 | 1 | 0.832485 | 0.507713 | 0.692774 | 0.602303 | 0.728008 |
| premarin | 0.602303 | 0.785714 | 0.832485 | 1 | 0.577423 | 0.774123 | 0.68458 | 0.825036 |
| 4-OH Tamoxifen | 0.460726 | 0.484921 | 0.507713 | 0.577423 | 1 | 0.723358 | 0.866369 | 0.672673 |
| nafoxidene | 0.571429 | 0.650073 | 0.692774 | 0.774123 | 0.723358 | 1 | 0.804385 | 0.898985 |
| clomiphene | 0.528896 | 0.574415 | 0.602303 | 0.68458 | 0.866369 | 0.804385 | 1 | 0.779842 |
| raloxifene | 0.595939 | 0.680562 | 0.752564 | 0.832485 | 0.672673 | 0.887062 | 0.779842 | 1 |
| ICI 182,780 | 0.574415 | 0.737555 | 0.774123 | 0.887062 | 0.523512 | 0.728008 | 0.635784 | 0.791752 |
| 16α-OH estrone | 0.632299 | 0.779842 | 0.840281 | 0.949492 | 0.580452 | 0.791752 | 0.68865 | 0.848477 |
| DES | 0.680562 | 0.763098 | 0.832485 | 0.876282 | 0.565517 | 0.763098 | 0.668799 | 0.811018 |
| progesterone | 0.898985 | 0.571429 | 0.701193 | 0.657441 | 0.505128 | 0.635784 | 0.580452 | 0.66497 |

5C contd

**Scaled alpha**

| ICI 182,78( | 16α-OH es | DES | progesterone |
|---|---|---|---|
| 0.574415 | 0.632299 | 0.680562 | 0.898985 |
| 0.737555 | 0.779842 | 0.763098 | 0.571429 |
| 0.774123 | 0.840281 | 0.832485 | 0.701193 |
| 0.887062 | 0.949492 | 0.876282 | 0.657441 |
| 0.523512 | 0.580452 | 0.565517 | 0.505128 |
| 0.728008 | 0.791752 | 0.763098 | 0.635784 |
| 0.635784 | 0.68865 | 0.668799 | 0.580452 |
| 0.785714 | 0.825036 | 0.791752 | 0.661185 |
| 1 | 0.876282 | 0.848477 | 0.618676 |
| 0.876282 | 1 | 0.912518 | 0.692774 |
| 0.848477 | 0.912518 | 1 | 0.732739 |
| 0.618676 | 0.692774 | 0.732739 | 1 |

Fig. 5D

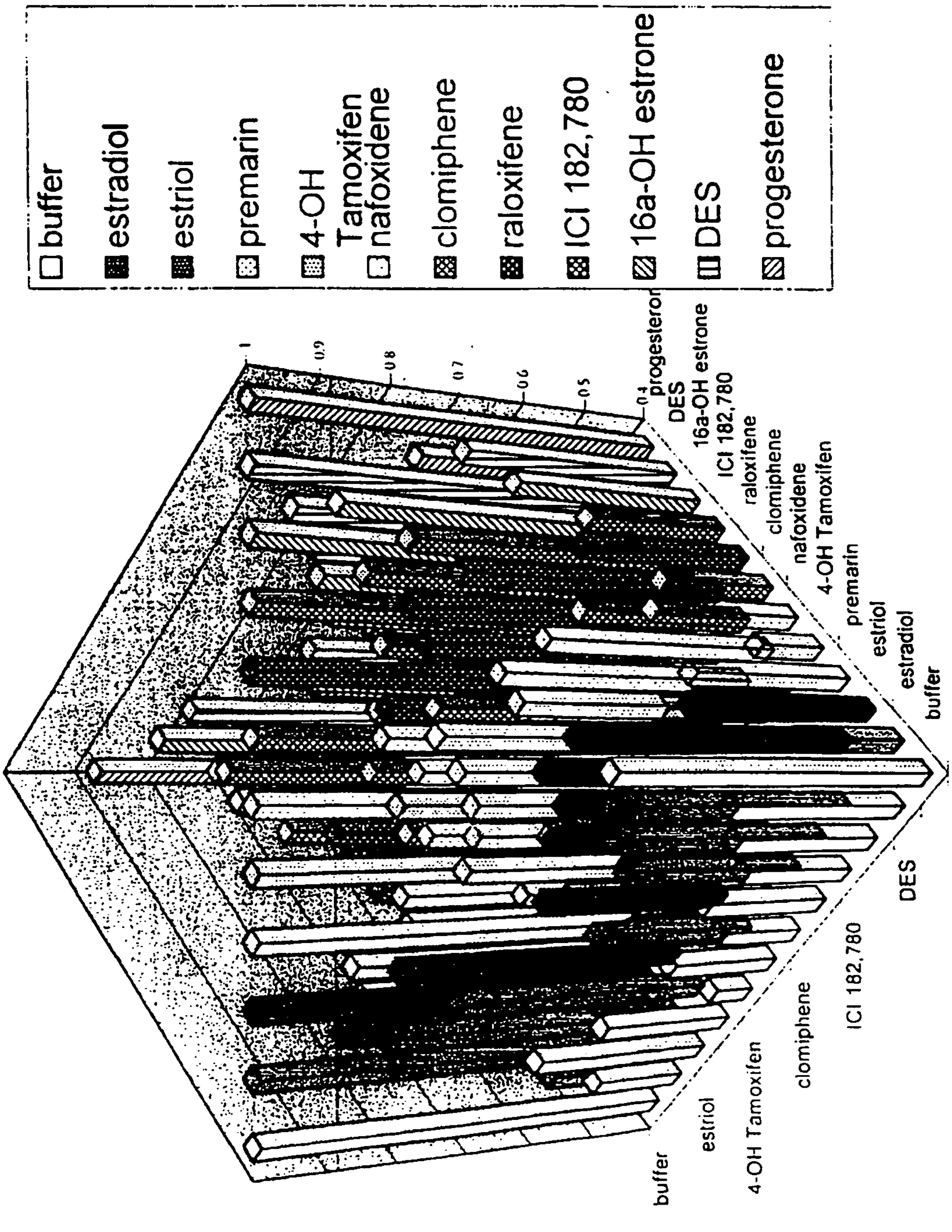
buffer
estradiol
estriol
premarin
4-OH Tamoxifen
nafoxidene
clomiphene
raloxifene
ICI 182,780
16a-OH estrone
DES
progesterone
1
0.9
0.8
0.7
0.6
0.5
0.4
progesterone
DES
16a-OH estrone
ICI 182,780
raloxifene
clomiphene
nafoxidene
4-OH Tamoxifen
premarin
estriol
estradiol
buffer
buffer
estriol
4-OH Tamoxifen
clomiphene
ICI 182,780
DES

Fig. 5E

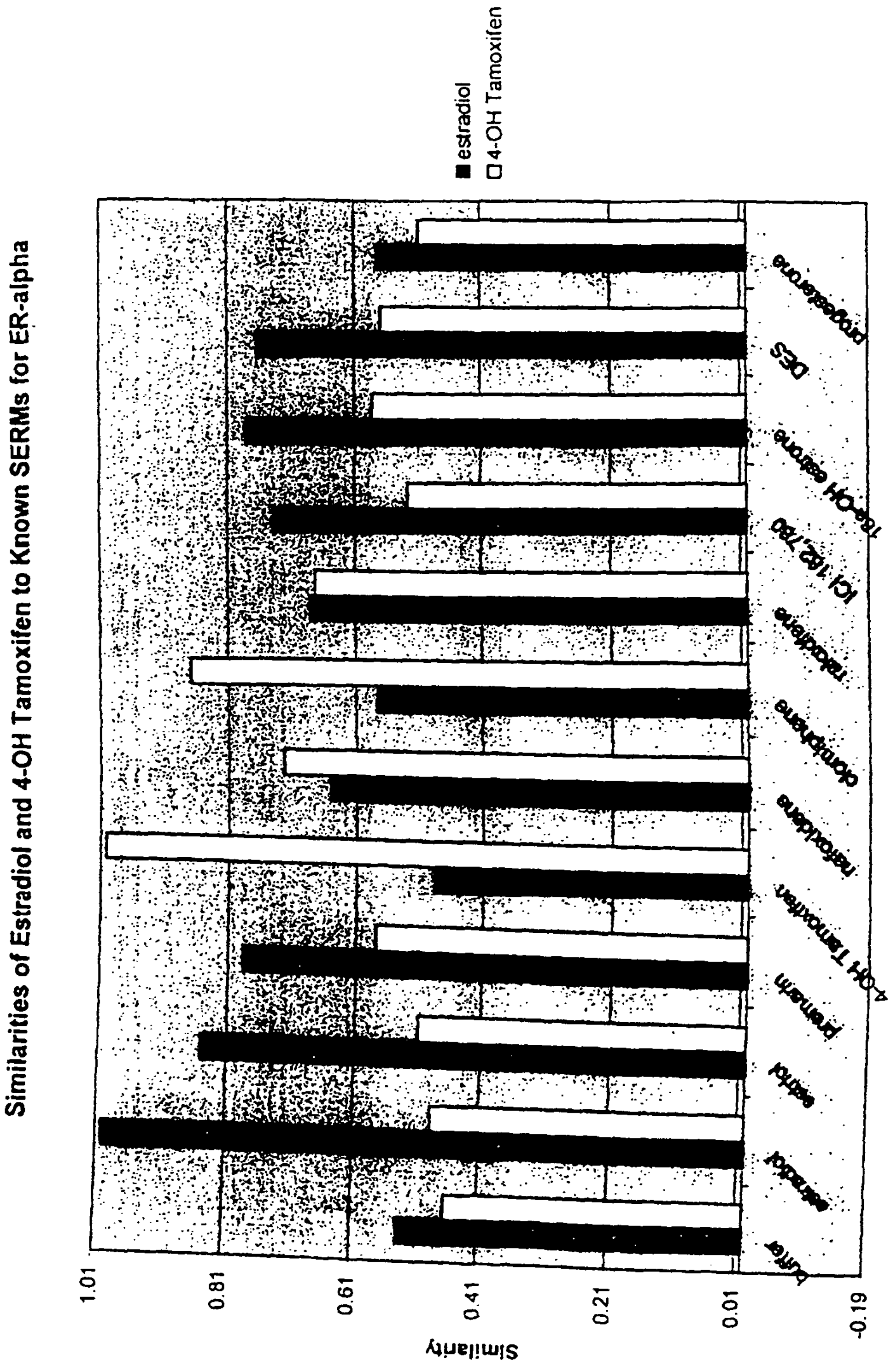
Similarities of Estradiol and 4-OH Tamoxifen to Known SERMs for ER-alpha
estradiol
4-OH Tamoxifen
Similarity
1.01
0.81
0.61
0.41
0.21
0.01
-0.19
buffer
estradiol
estriol
premarin
4-OH Tamoxifen
nafoxidene
clomiphene
raloxifene
ICI 182,780
16α-OH estrone
DES
progesterone
SERMs

Fig. 6A

ER Beta

| | buffer | estradiol | estriol | premarin | 4-OH Tamoxifen | nafoxidene | clomiphene |
|---|---|---|---|---|---|---|---|
| αβ I | 2 | 7 | 7 | 6 | 0 | 1 | 0 |
| αβ II | 7 | 2 | 6 | 4 | 1 | 4 | 1 |
| αβ III | 2 | 1 | 1 | 1 | 7 | 3 | 5 |
| β I | 2 | 1 | 1 | 1 | 7 | 5 | 5 |
| β II | 1 | 1 | 1 | 1 | 7 | 3 | 5 |
| β III | 6 | 3 | 2 | 7 | 7 | 4 | 3 |
| β VI | 1 | 5 | 6 | 4 | 0 | 0 | 0 |
| β V | 7 | 7 | 7 | 7 | 1 | 5 | 3 |

| | | | | | | | |
|---|---|---|---|---|---|---|---|
| Universal Max | | | 19.79899 | | | | |
| Max | 16.76305 | 16.49242 | 17.4069 | 16.55295 | 19.13112647 | 14.106736 | 15.524175 |
| buffer | 0 | 8.774964 | 8.306624 | 6.082763 | 12.80624847 | 5.7445626 | 10 |
| estradiol | 8.774964 | 0 | 4.242641 | 4.690416 | 15.32970972 | 9.6953597 | 11.789826 |
| estriol | 8.306624 | 4.242641 | 0 | 5.830952 | 16.70329309 | 10.392305 | 13.228757 |
| premarin | 6.082763 | 4.690416 | 5.830952 | 0 | 14.31782106 | 8.8317609 | 11.874342 |
| 4-OH Tamoxifen | 12.80625 | 15.32971 | 16.70329 | 14.31782 | 0 | 8.4261498 | 5.6568542 |
| nafoxidene | 5.744563 | 9.69536 | 10.3923 | 8.831761 | 8.426149773 | 0 | 4.7958315 |
| clomiphene | 10 | 11.78983 | 13.22876 | 11.87434 | 5.656854249 | 4.7958315 | 0 |
| raloxifene | 7.416198 | 11.40175 | 9.949874 | 8.774964 | 7.615773106 | 4 | 4.5825757 |
| ICI 182,780 | 10.24695 | 10.95445 | 11.91638 | 11.91638 | 13.07669683 | 8.1240384 | 8.4261498 |
| 16α-OH estrone | 6.855655 | 3.162278 | 4.690416 | 5.291503 | 14.38749457 | 7.6157731 | 10.148892 |
| DES | 5.91608 | 3.162278 | 5.09902 | 3.464102 | 13.82027496 | 7.3484692 | 10.148892 |
| progesterone | 3.162278 | 9.219544 | 9.219544 | 7.28011 | 12.40967365 | 5.3851648 | 9.0553851 |

Fig. 6A cont'd

ER Beta

| raloxifene | ICI 182,780 | 16α-OH estrone | DES | progesterone |
|---|---|---|---|---|
| 0 | 0 | 5 | 5 | 1 |
| 4 | 2 | 3 | 3 | 6 |
| 6 | 1 | 1 | 1 | 1 |
| 4 | 1 | 1 | 1 | 1 |
| 2 | 0 | 1 | 1 | 1 |
| 4 | 0 | 2 | 4 | 5 |
| 0 | 0 | 3 | 3 | 0 |
| 3 | 1 | 7 | 7 | 5 |

| | | | | |
|---|---|---|---|---|
| 14.93318 | 18.1383571 | 15.45962483 | 15.16575 | 16.70329309 |
| 7.416198 | 10.2469508 | 6.8556546 | 5.91608 | 3.16227766 |
| 11.40175 | 10.9544512 | 3.16227766 | 3.162278 | 9.219544457 |
| 12 | 11.9163753 | 4.69041576 | 5.09902 | 9.219544457 |
| 10.58301 | 11.9163753 | 5.291502622 | 3.464102 | 7.280109889 |
| 7.549834 | 13.0766968 | 14.38749457 | 13.82027 | 12.40967365 |
| 4 | 8.1240384 | 7.615773106 | 7.348469 | 5.385164807 |
| 4.582576 | 8.42614977 | 10.14889157 | 10.14889 | 9.055385138 |
| 0 | 7.87400787 | 9.486832981 | 9.273618 | 6.708203932 |
| 7.874008 | 0 | 8.717797887 | 9.380832 | 7.681145748 |
| 9.486833 | 8.71779789 | 0 | 2 | 6.8556546 |
| 9.273618 | 9.38083152 | 2 | 0 | 6.244997998 |
| 6.708204 | 7.68114575 | 6.8556546 | 6.244998 | 0 |

Fig. 6B scaled beta

| | buffer | estradiol | estriol | premarin | 4-OH Tam | nafoxidene | clomiphene |
|---|---|---|---|---|---|---|---|
| buffer | 1 | 0.556797 | 0.580452 | 0.692774098 | 0.353187 | 0.709856 | 0.494924 |
| estradiol | 0.556797 | 1 | 0.785714 | 0.763098229 | 0.225733 | 0.51031 | 0.404524 |
| estriol | 0.580452 | 0.785714 | 1 | 0.705492455 | 0.156356 | 0.475109 | 0.331847 |
| premarin | 0.692774 | 0.763098 | 0.705492 | 1 | 0.276841 | 0.553929 | 0.400255 |
| 4-OH Tamoxifen | 0.353187 | 0.225733 | 0.156356 | 0.276840831 | 1 | 0.574415 | 0.714286 |
| nafoxidene | 0.709856 | 0.51031 | 0.475109 | 0.553928714 | 0.574415 | 1 | 0.757774 |
| clomiphene | 0.494924 | 0.404524 | 0.331847 | 0.400255156 | 0.714286 | 0.757774 | 1 |
| raloxifene | 0.625425 | 0.424124 | 0.497455 | 0.55679737 | 0.615345 | 0.797969 | 0.768545 |
| ICI 182,780 | 0.482451 | 0.446717 | 0.398132 | 0.398132159 | 0.339527 | 0.589674 | 0.574415 |
| 16α-OH estrone | 0.653737 | 0.840281 | 0.763098 | 0.732738758 | 0.273322 | 0.615345 | 0.487404 |
| DES | 0.701193 | 0.840281 | 0.742461 | 0.825036447 | 0.301971 | 0.628846 | 0.487404 |
| progesterone | 0.840281 | 0.534343 | 0.534343 | 0.632298924 | 0.373217 | 0.728008 | 0.542634 |

Fig. 6B contd scaled beta

| raloxifene | ICI 182,78 | 16α-OH es | DES | progesterone |
|---|---|---|---|---|
| 0.625425 | 0.482451 | 0.653737 | 0.701193 | 0.840281 |
| 0.424124 | 0.446717 | 0.840281 | 0.840281 | 0.534343 |
| 0.393908 | 0.398132 | 0.763098 | 0.742461 | 0.534343 |
| 0.465478 | 0.398132 | 0.732739 | 0.825036 | 0.632299 |
| 0.618676 | 0.339527 | 0.273322 | 0.301971 | 0.373217 |
| 0.797969 | 0.589674 | 0.615345 | 0.628846 | 0.728008 |
| 0.768545 | 0.574415 | 0.487404 | 0.487404 | 0.542634 |
| 1 | 0.602303 | 0.520843 | 0.531612 | 0.661185 |
| 0.602303 | 1 | 0.559685 | 0.526196 | 0.612044 |
| 0.520843 | 0.559685 | 1 | 0.898985 | 0.653737 |
| 0.531612 | 0.526196 | 0.898985 | 1 | 0.68458 |
| 0.661185 | 0.612044 | 0.653737 | 0.68458 | 1 |

Fig. 6C

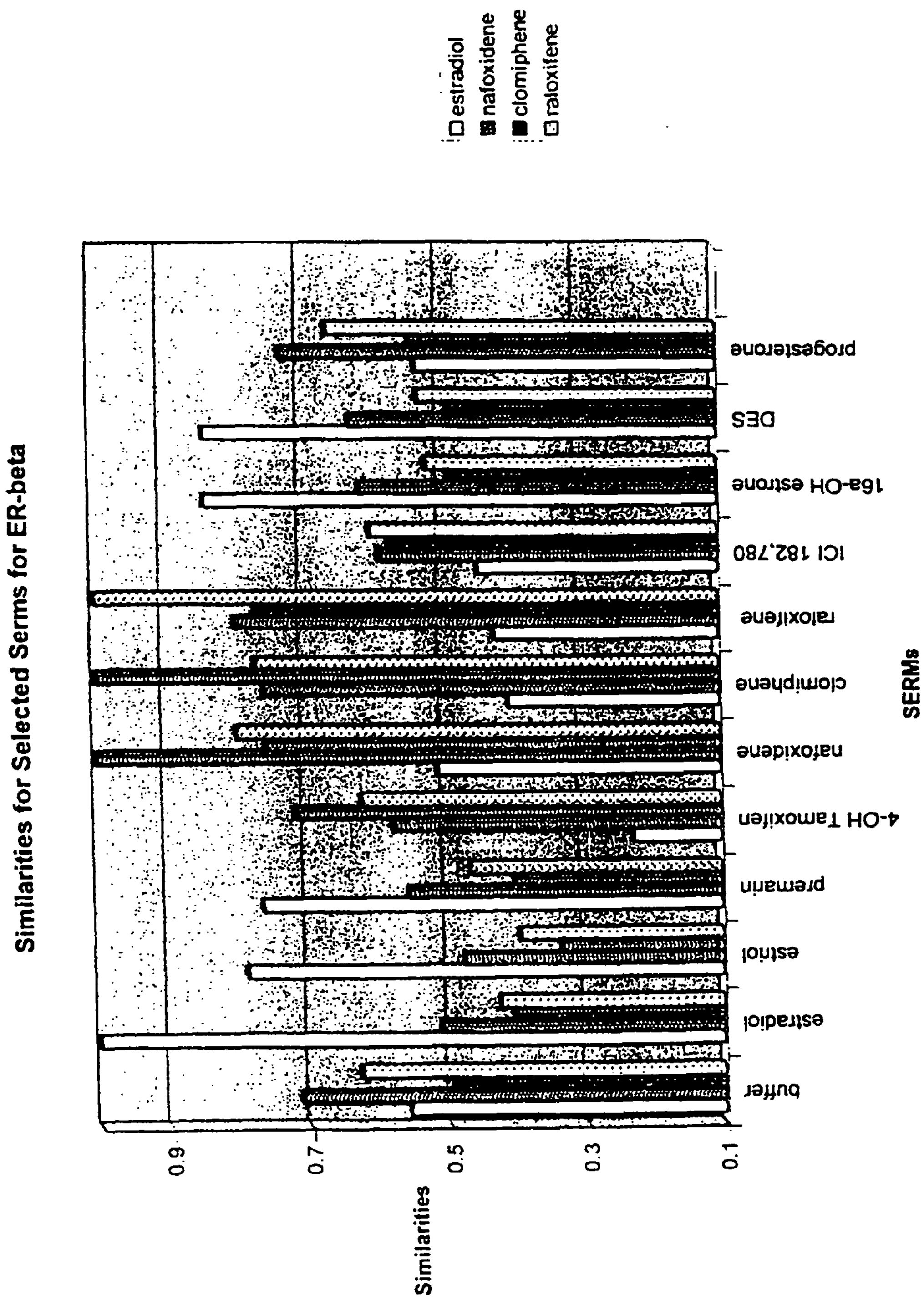
Similarities for Selected Serms for ER-beta
Similarities
0.1
0.3
0.5
0.7
0.9
buffer
estradiol
estriol
premarin
4-OH Tamoxifen
nafoxidene
clomiphene
raloxifene
ICI 182,780
16a-OH estrone
DES
progesterone
SERMs
estradiol
nafoxidene
clomiphene
raloxifene

Fig. 7

Analysis of ER-peptide interactions
using the mammalian two hybrid system

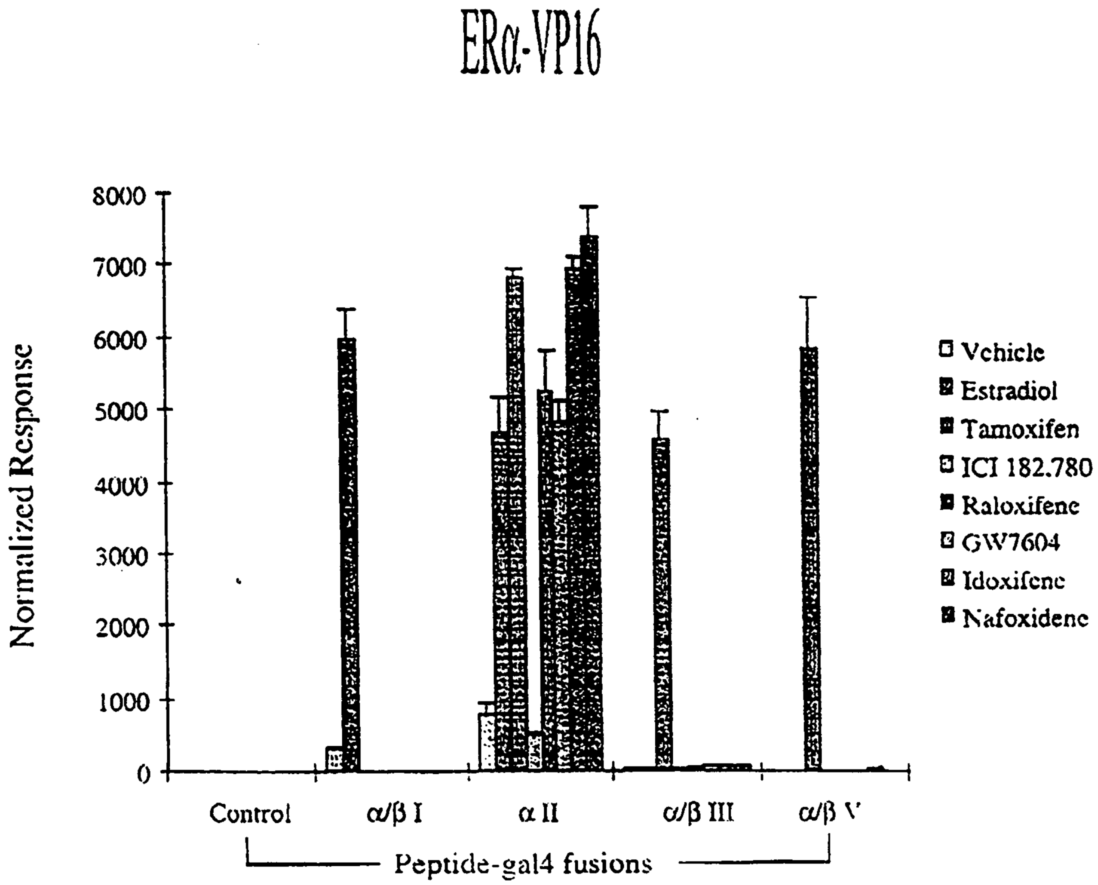


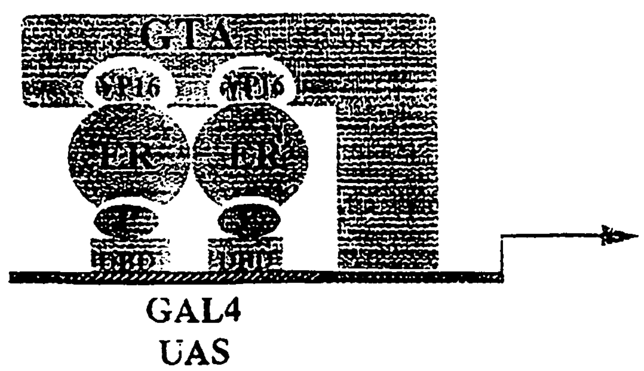

Figure 8
Analysis of the specificty of
interaction between peptides
and nuclear receptor family members

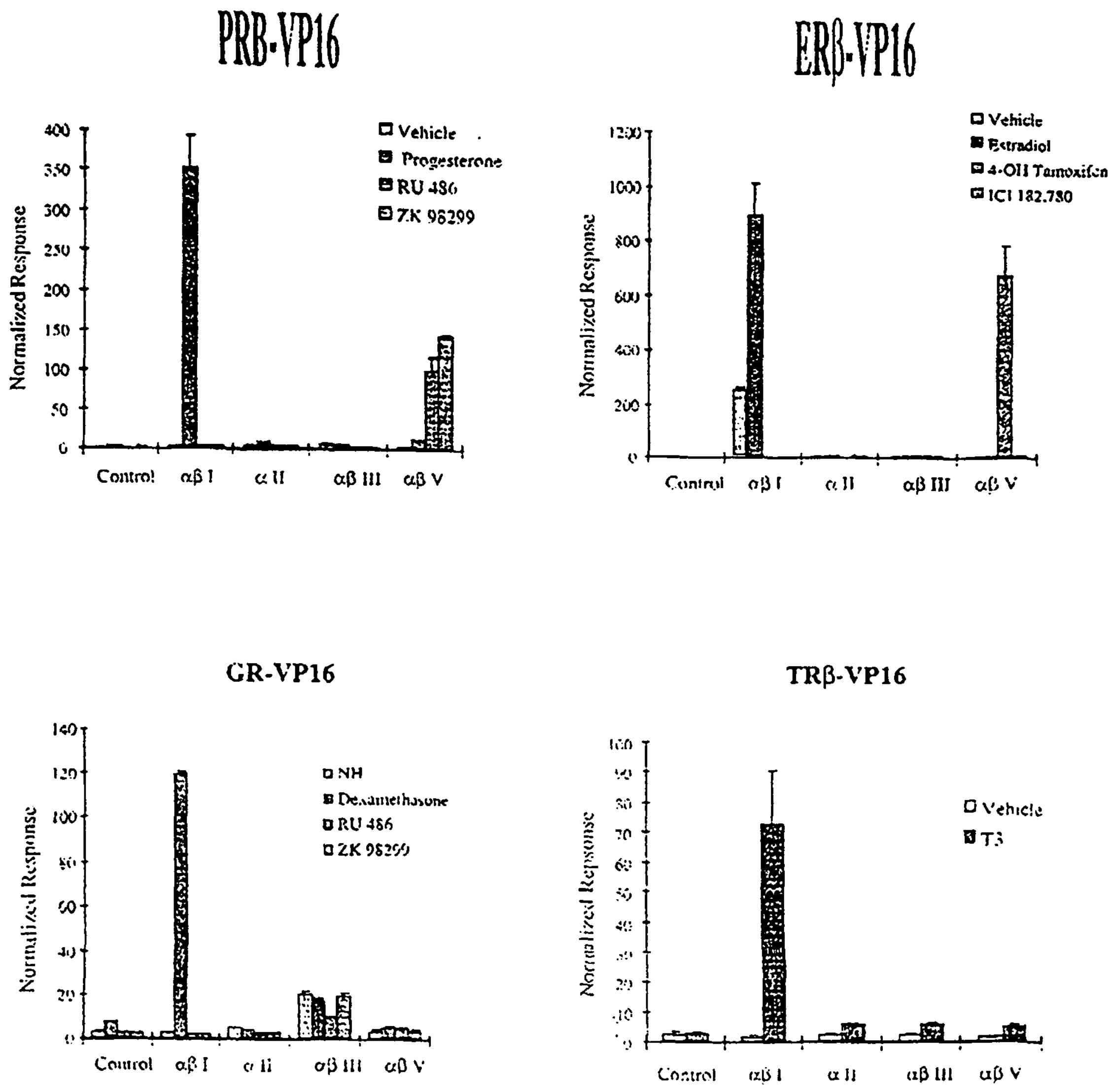

Figure 9 Peptides which interact with the Tamoxifen activated ER do not require AF-2 (helix 12)

A

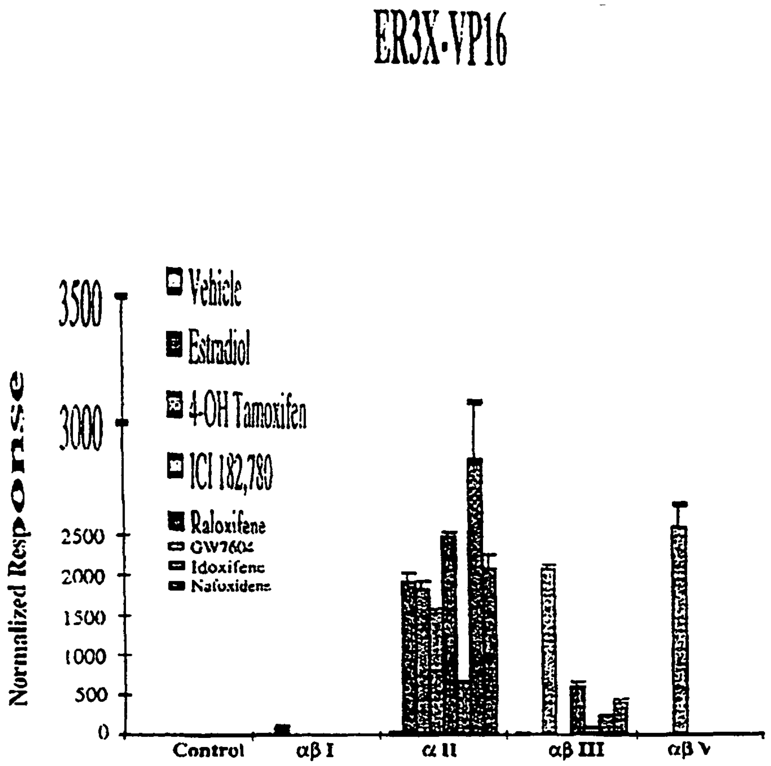


B

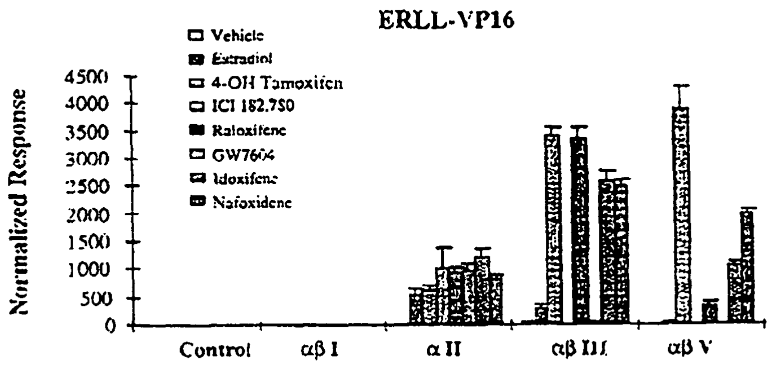


C

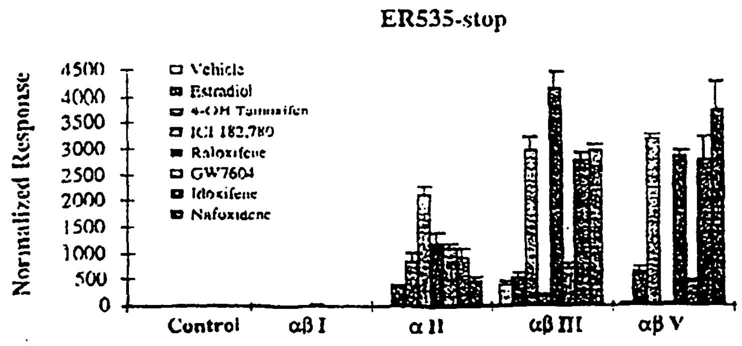

Figure 10 Disruption of ER mediated transcriptional activity by Gal 4-peptide fusion proteins

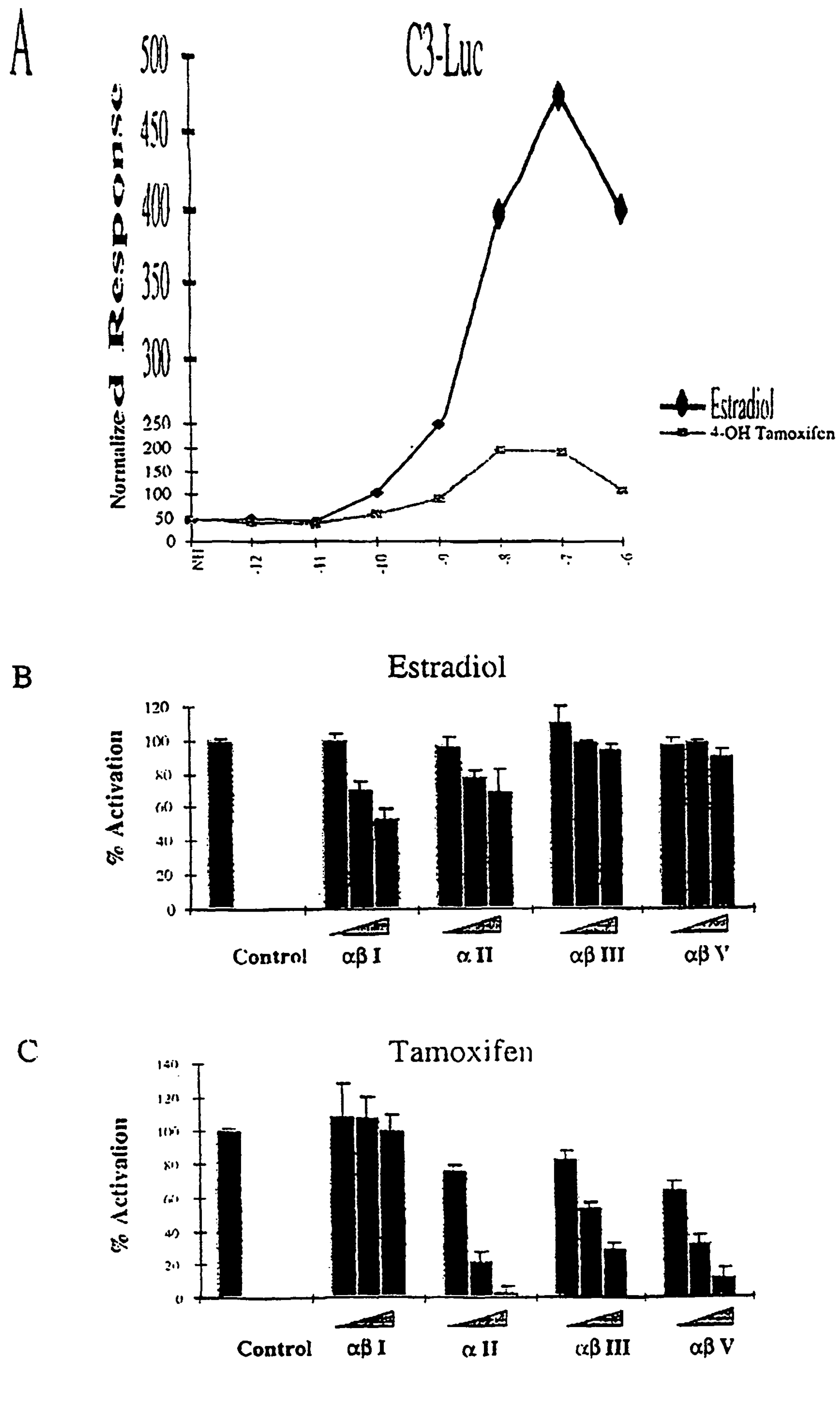

Fig. 11
Disruption of Tamoxifen Activated
ER Transcriptional Activity by
a II peptide is not Promoter Dependent

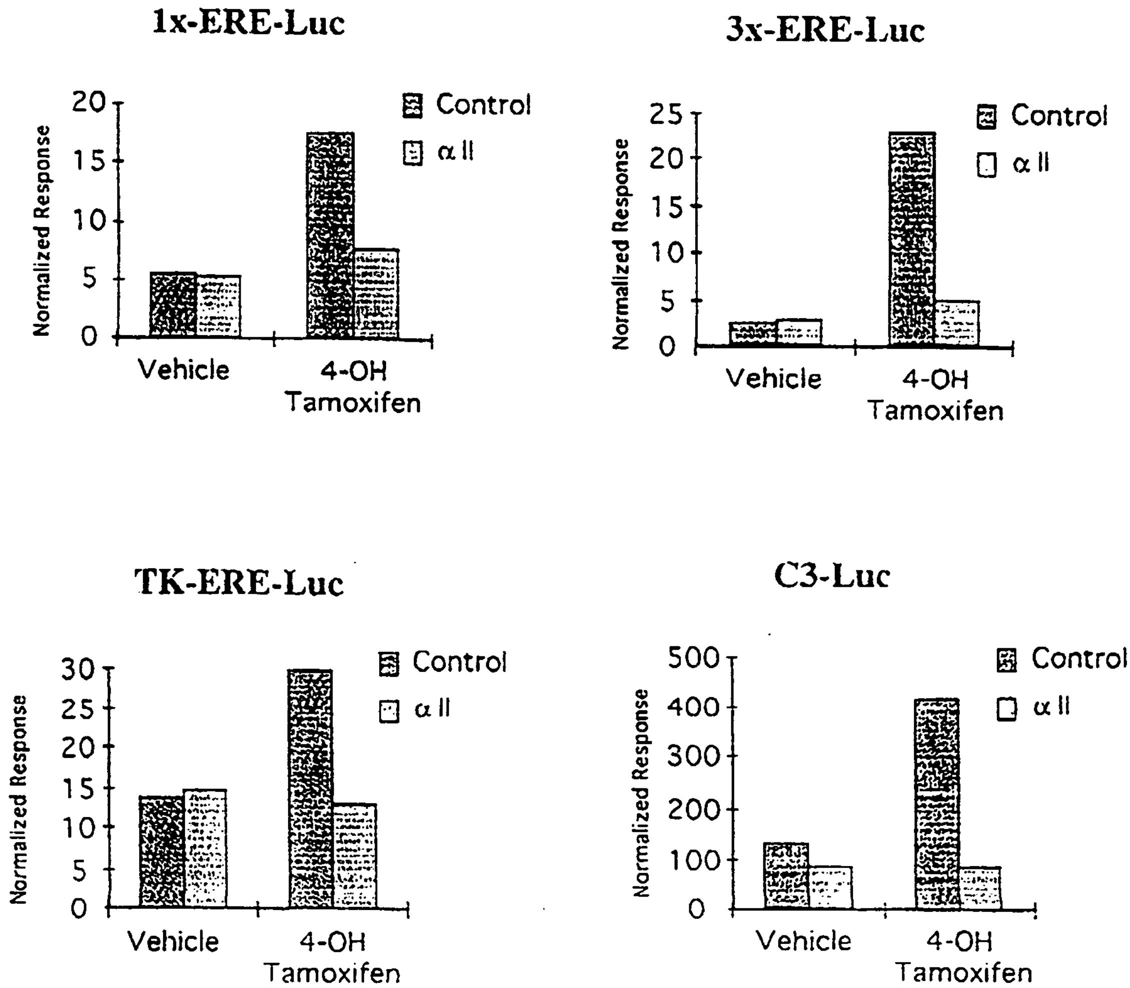

Fig. 12
Disruption of ER mediated transcriptional activity through the AP-I pathway by gal 4 peptide fusion proteins

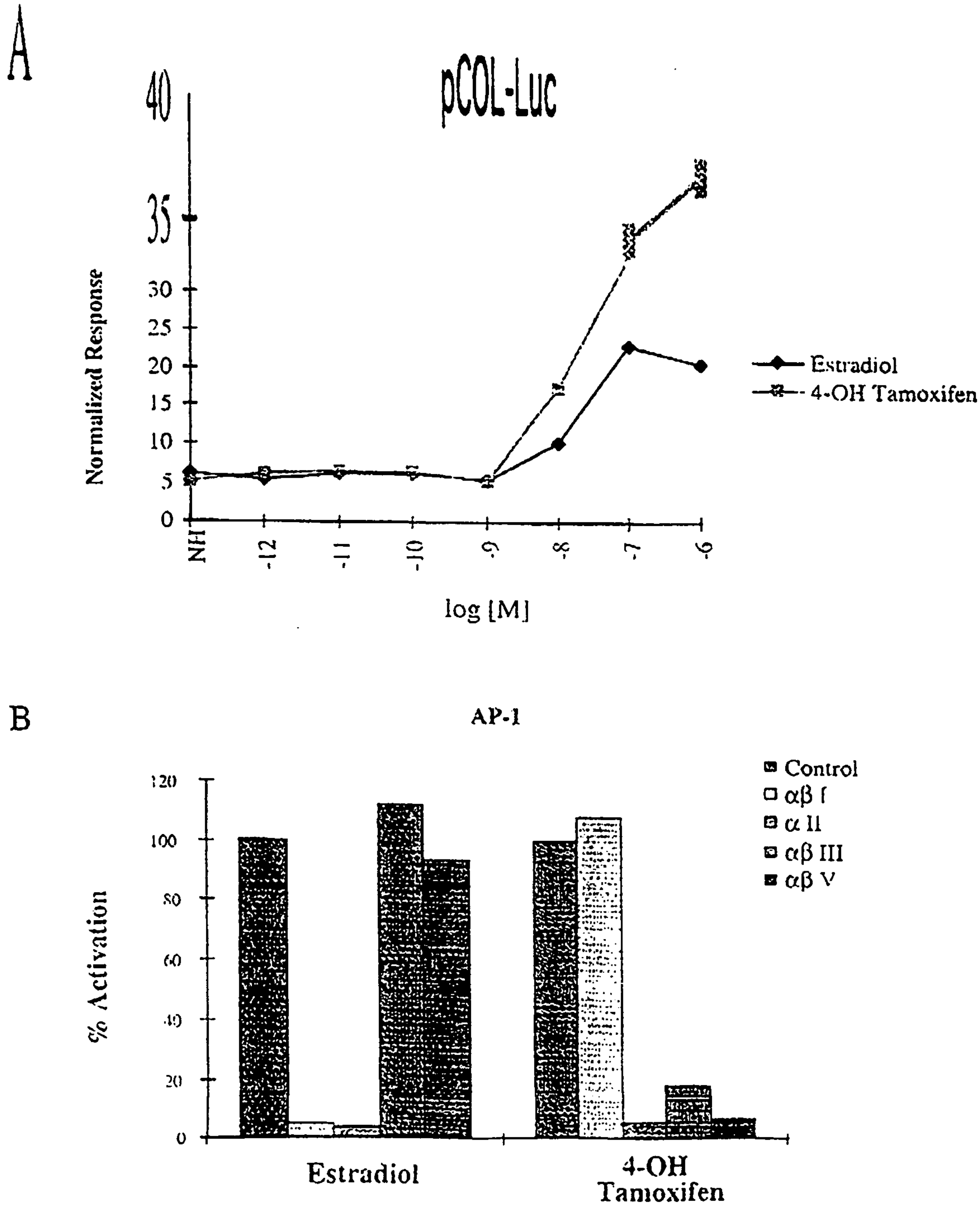

FIG. 13 Potential mechanisms to exp' in the activity of peptides which block tamoxifen partial agonist activity

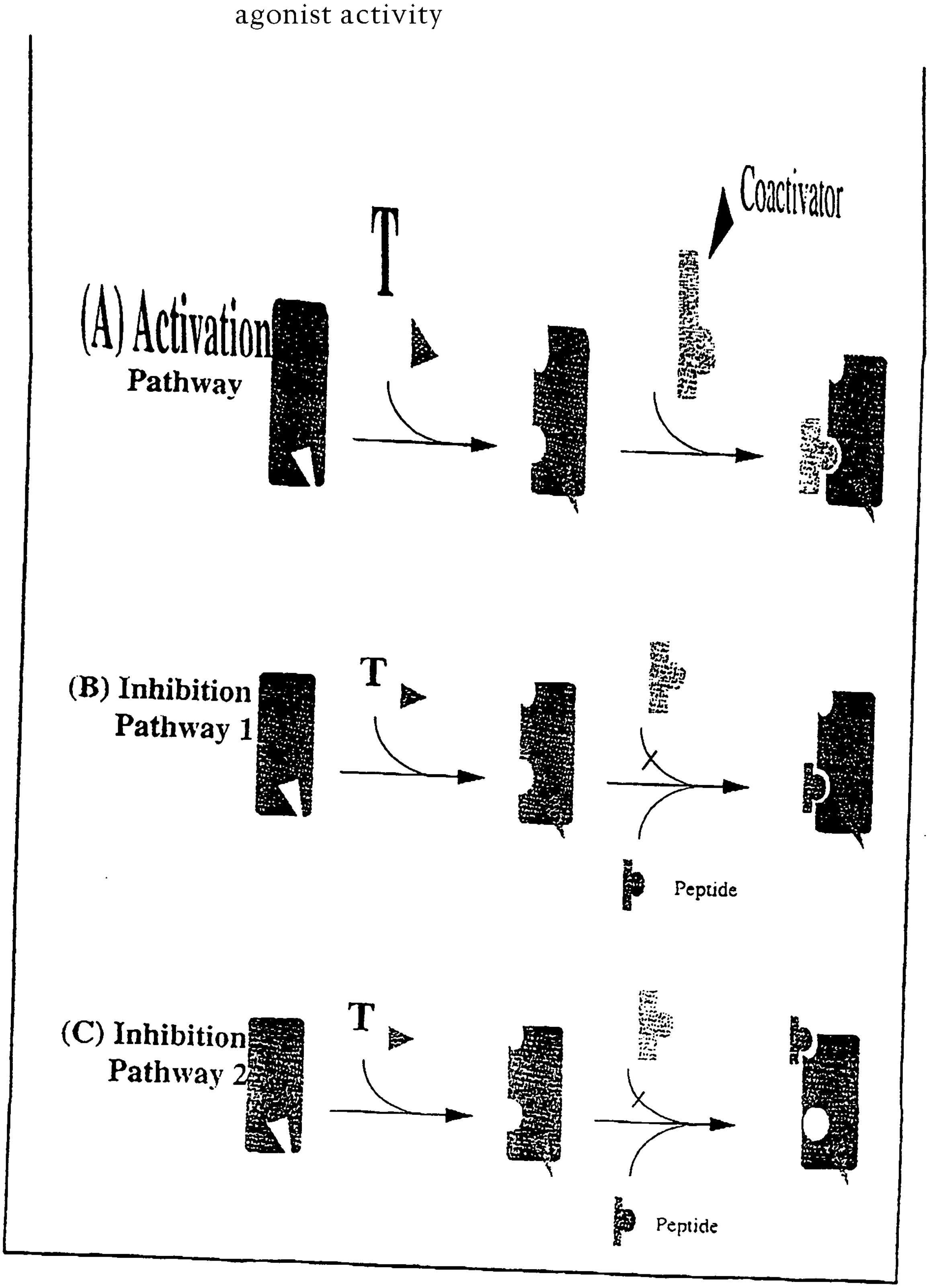

Figure 14 LXXLL containing peptides are sensitive probes for ER AF2 activation

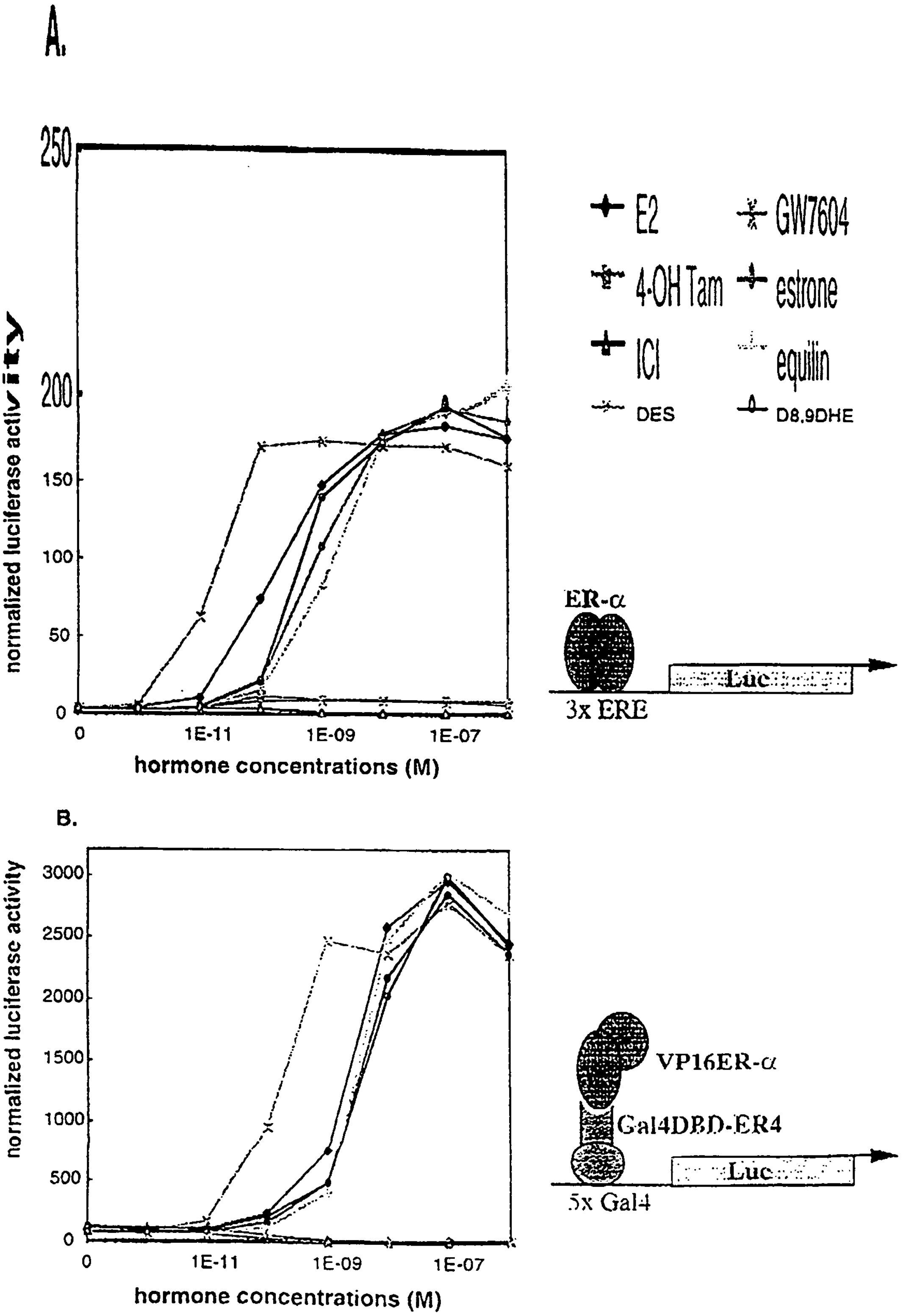

Fig. 14 (cont'd) Both partial agonists and pure antagonists disrupt ER-LXXLL motif interaction

C

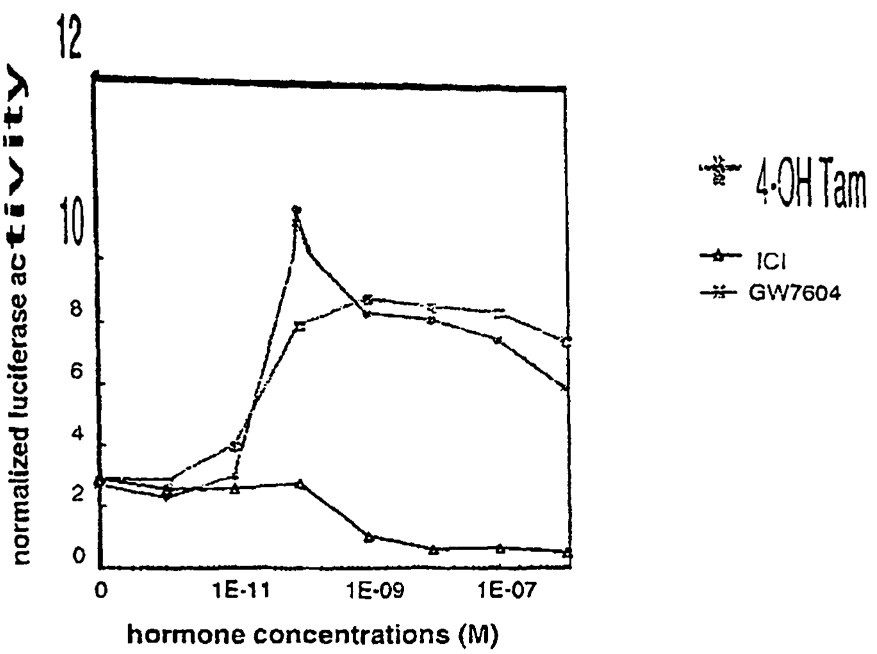


D

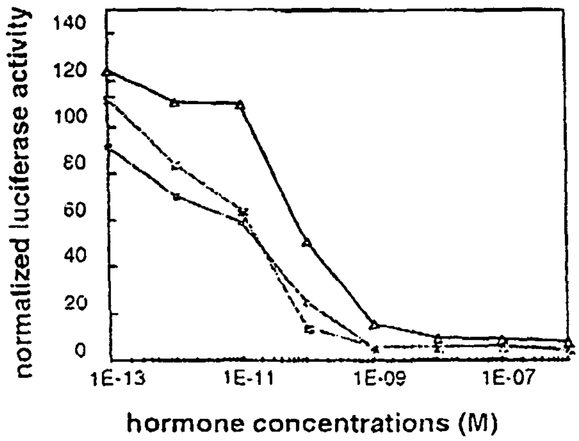

Fig. 15

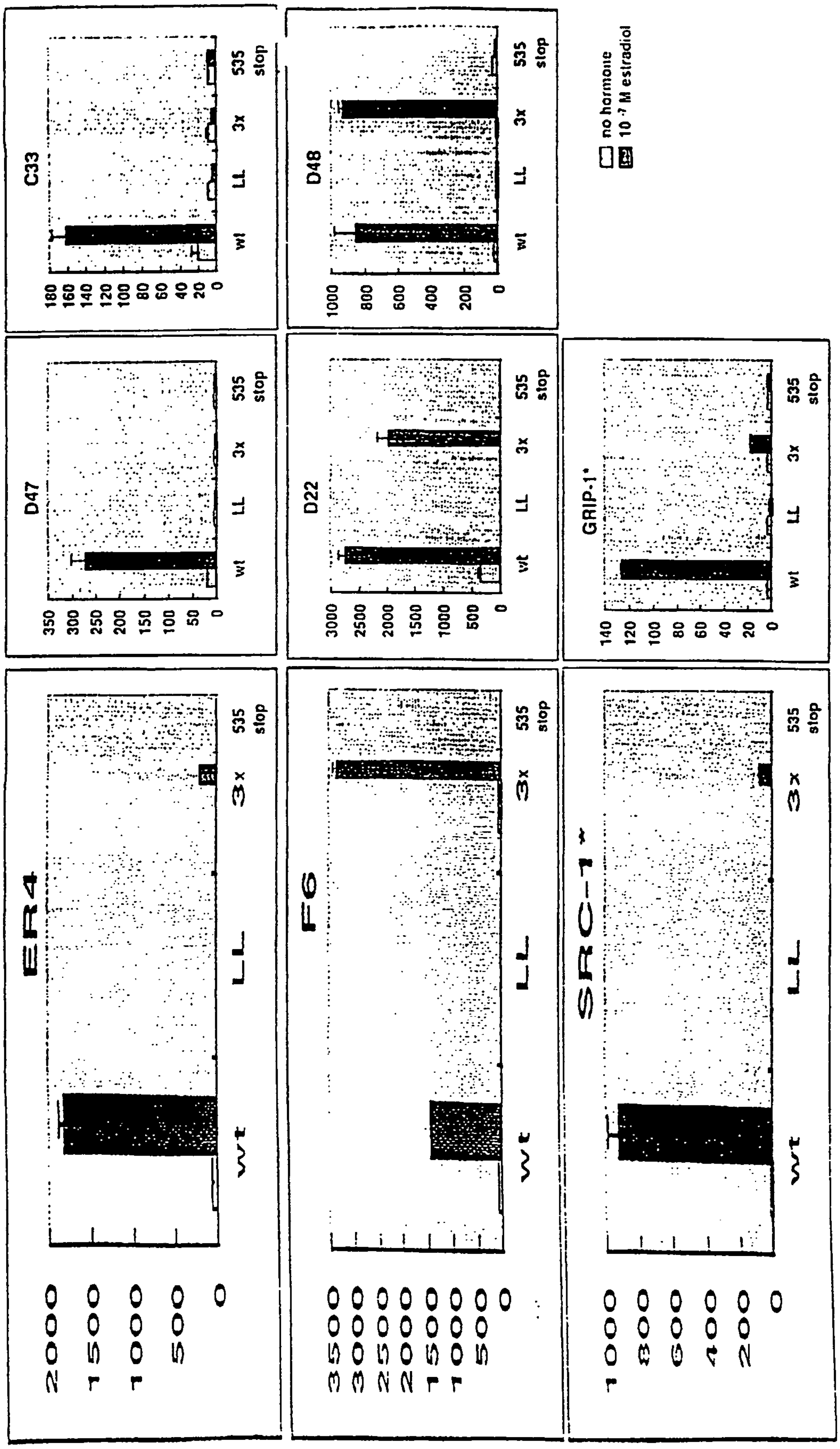
ER4
F6
SRC-1*
D47
D22
GRIP-1*
C33
D48
wt
LL
3x
535 stop
no hormone
10⁻⁷ M estradiol

**Figure 16 - LXXLL containing peptides disrupt ER-α transcriptional activation function in mammalian cells**

(A)

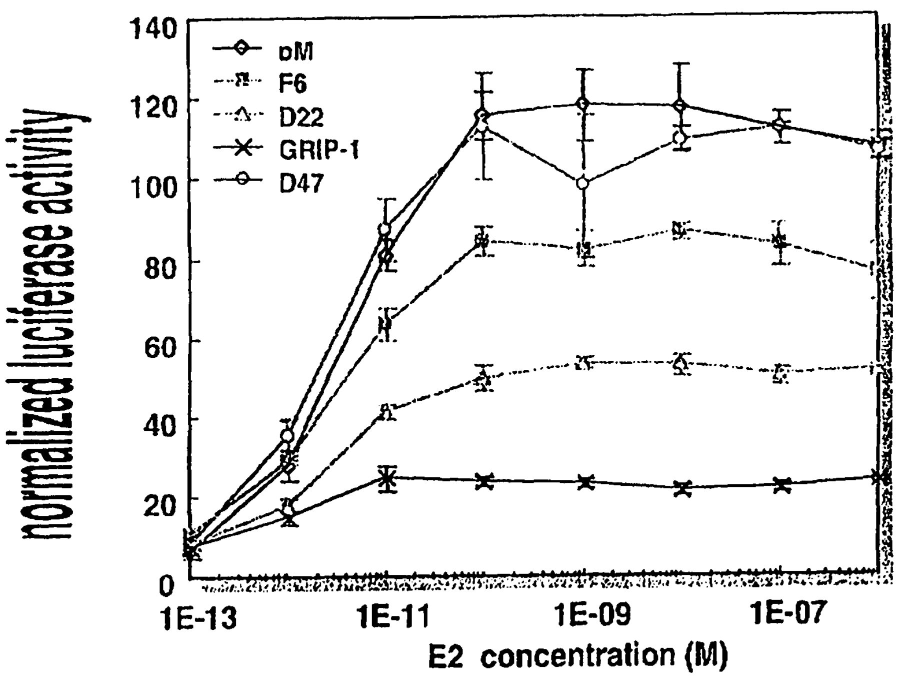

Fig. 16(B)

**Two copies of LXXLL motif function synergistically to compete with endogenous coactivators**

(B)

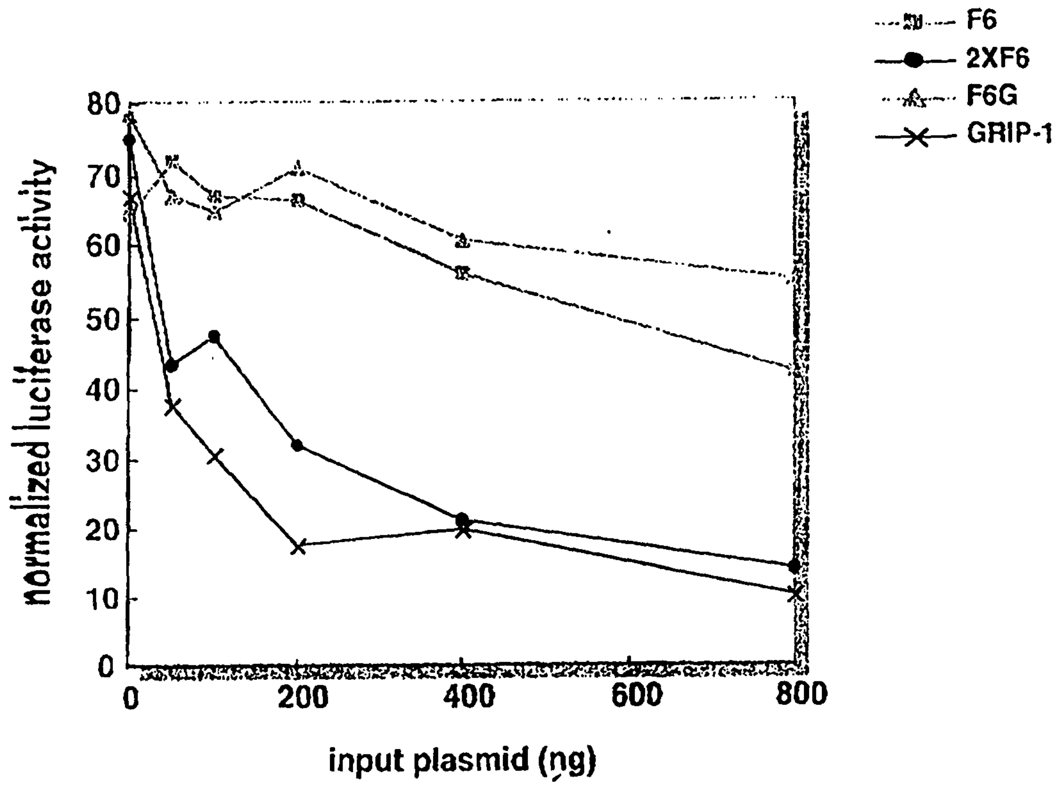

Figure 17 - LXXLL containing peptides disrupt AF2 functions in HepG2 cells

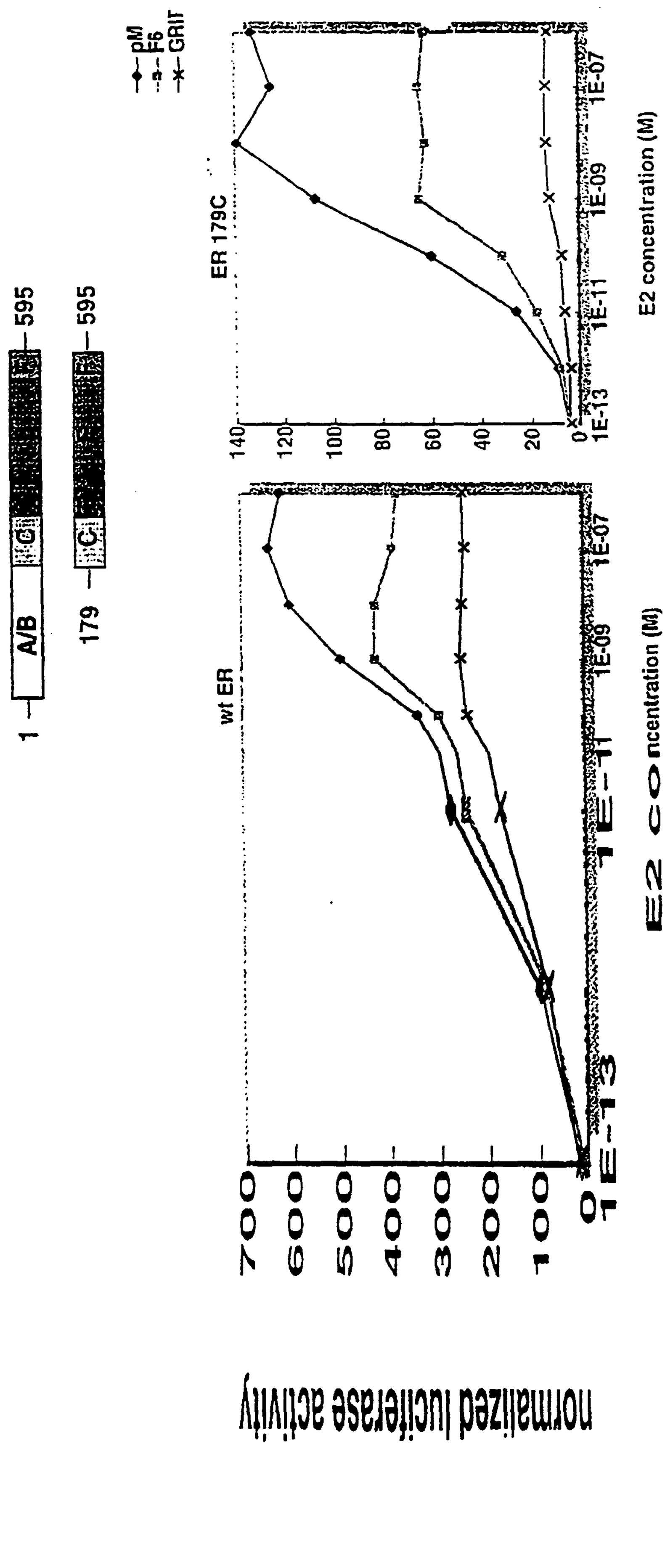

Fig. 18. Nuclear receptors have distinct preferences for different LXXLL motifs

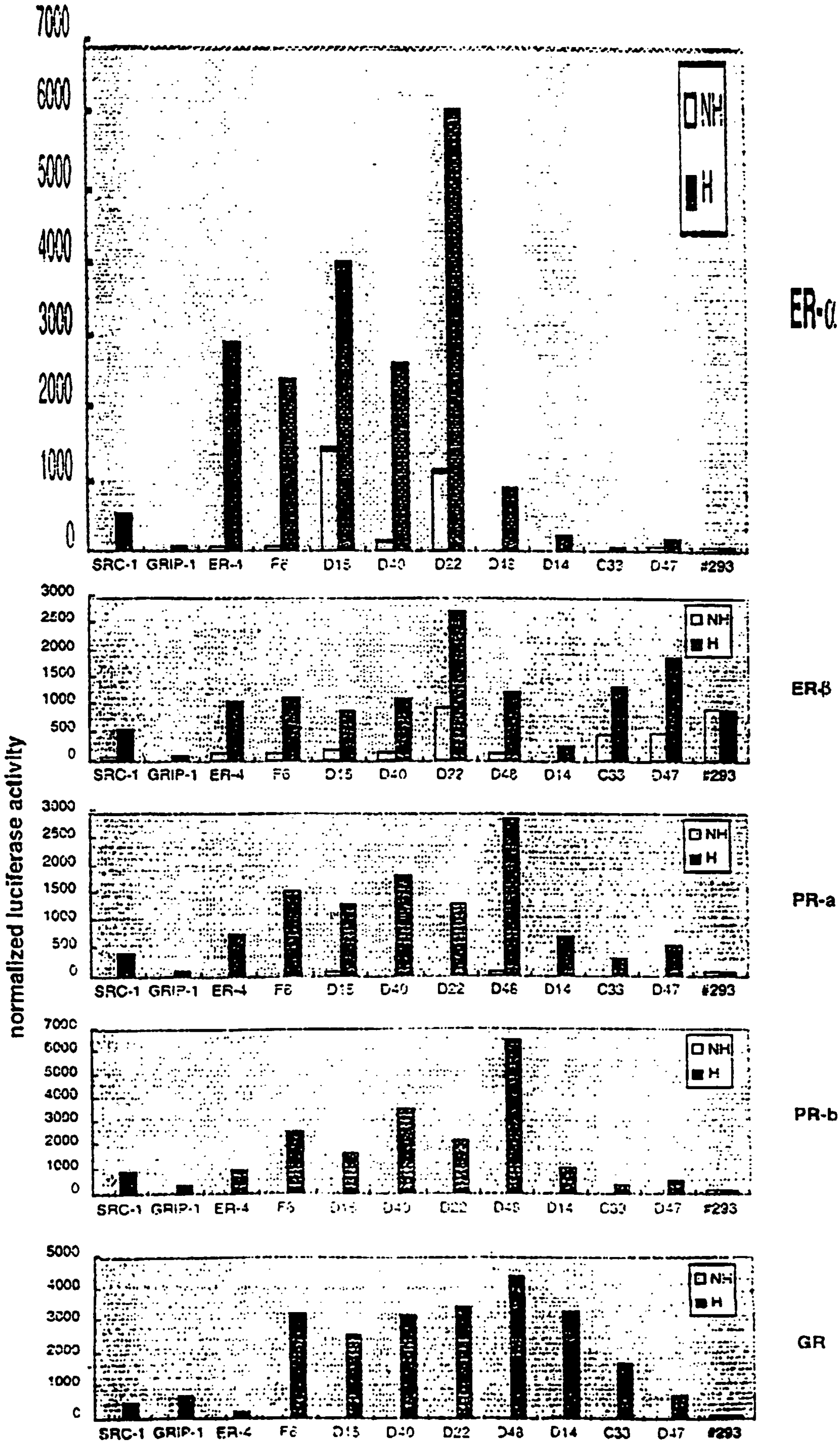

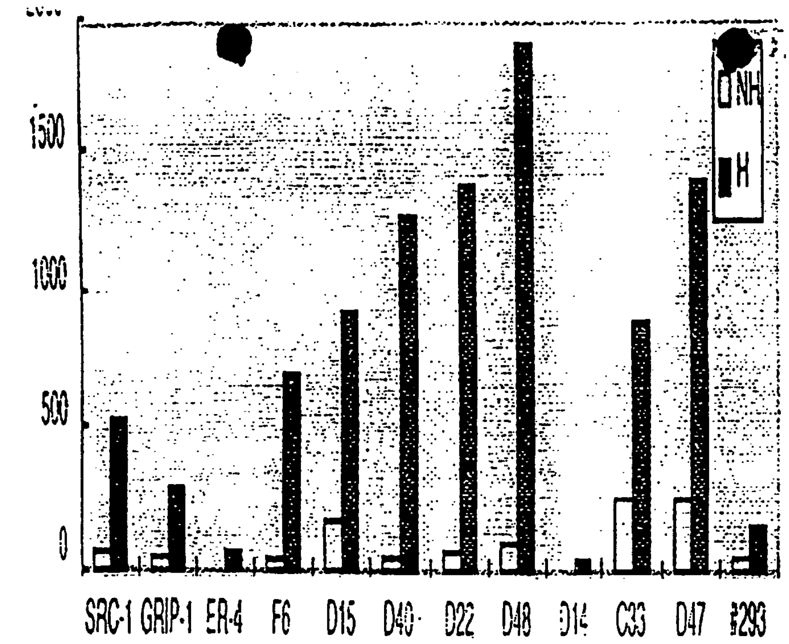


Fig. 18 (cont'd)

RAR

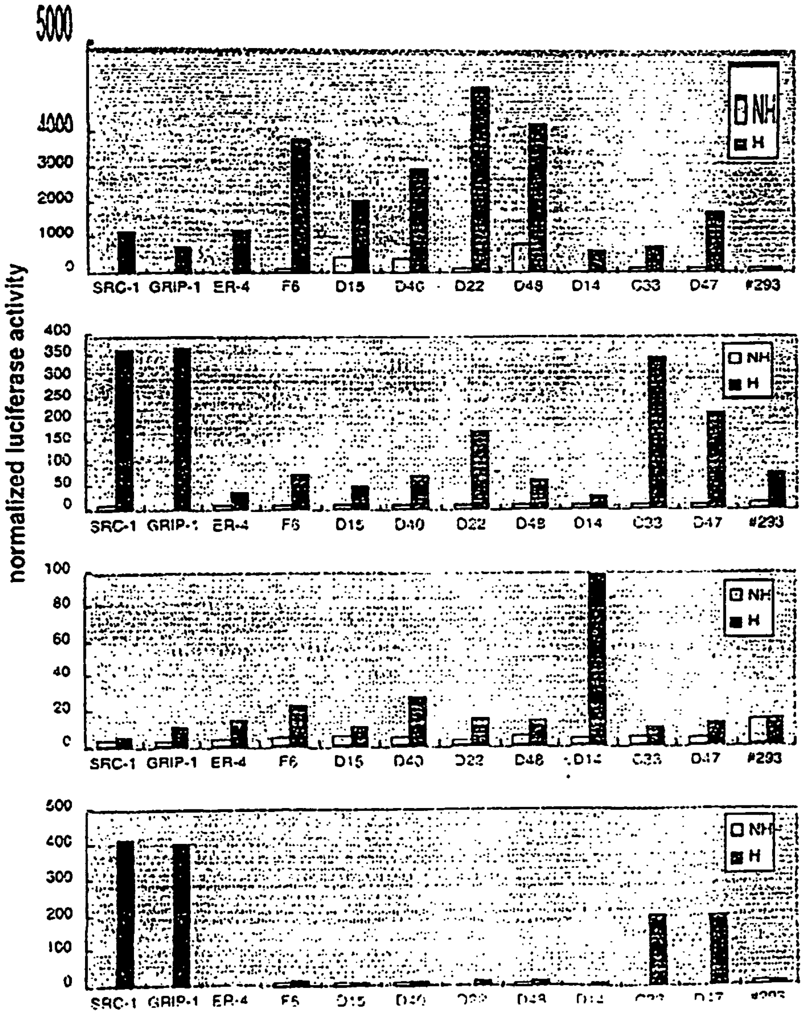


RXR

TR

AR

VDR

Fig. 19

| | NH | E2 | OT | ICI | Ralox | 7804 | Idox | Nafox |
|---|---|---|---|---|---|---|---|---|
| itrol | 1.341 | 1.621 | 3.076 | 0.846 | 2.031 | 1.414 | 2.294 | 2.053 |
| :1 | 293.5 | 5962 | 8.54 | 1.359 | 6.449 | 16.08 | 4.256 | 3.324 |
| | 752.3 | 4663 | 6821 | 507.3 | 5245 | 4805 | 6926 | 7397 |
| 3 | 32.93 | 34.67 | 4696 | 33.15 | 56.18 | 64.21 | 60.2 | 63.84 |
| 5 | 2.901 | 9.313 | 5637 | 7.16 | 5.059 | 13.2 | 21.75 | 8.434 |
| l dist | 0 | 6886.618 | 9627.63 | 381.3001 | 4501.922 | 4062.318 | 6179.562 | 6651.107 |
| dist | 6886.618 | 0 | 9787.266 | 7266.298 | 5983.981 | 5947.69 | 6372.767 | 6556.022 |
| dist | 9627.63 | 9787.266 | 0 | 9777.12 | 7617.299 | 7709.374 | 7437.648 | 7467.322 |
| dist | 381.3001 | 7266.298 | 9777.12 | 0 | 4737.759 | 4297.842 | 6417.774 | 6889.769 |
| lox dist | 4501.922 | 5983.981 | 7617.299 | 4737.759 | 0 | 440.2544 | 1680.089 | 2152.019 |
| )4 dist | 4062.318 | 5947.69 | 7709.374 | 4297.842 | 440.2544 | 0 | 2120.054 | 2592.036 |
| x dist | 6179.562 | 6372.767 | 7437.648 | 6417.774 | 1680.089 | 2120.054 | 0 | 472.2028 |
| fox dist | 6651.107 | 6556.022 | 7467.322 | 6889.769 | 2152.019 | 2592.036 | 472.2028 | 0 |
| l sim | 1.00 | 0.58 | 0.42 | 0.98 | 0.73 | 0.75 | 0.63 | 0.60 |
| sim | 0.58 | 1.00 | 0.41 | 0.56 | 0.64 | 0.64 | 0.61 | 0.60 |
| sim | 0.42 | 0.41 | 1.00 | 0.41 | 0.54 | 0.53 | 0.55 | 0.55 |
| sim | 0.98 | 0.56 | 0.41 | 1.00 | 0.71 | 0.74 | 0.61 | 0.58 |
| lox sim | 0.73 | 0.64 | 0.54 | 0.71 | 1.00 | 0.97 | 0.90 | 0.87 |
| )4 sim | 0.75 | 0.64 | 0.53 | 0.74 | 0.97 | 1.00 | 0.87 | 0.84 |
| x sim | 0.63 | 0.61 | 0.55 | 0.61 | 0.90 | 0.87 | 1.00 | 0.97 |
| 'ox sim | 0.60 | 0.60 | 0.55 | 0.58 | 0.87 | 0.84 | 0.97 | 1.00 |

Yax dist = SQRT (7397*7397*5) = 16540.19

$$SIM = \frac{\text{max dist} - \text{ligand dist}}{\text{max dist}}$$

SEQUENCE LISTING

<110> CHRISTENSEN, Dale J.
MCDONNELL, Donald P.
BARNETT, Tom
BUEHRER, Benjamin
FOWLKES, Dana M.
HAMILTON, Paul T.
PAIGE, Lisa A.
NOVALON PHARMACEUTICAL CORPORATION

<120> METHOD OF PREDICTING THE ABILITY OF COMPOUNDS TO MODULATE THE BIOLOGICAL ACTIVITY OF RECEPTORS

<130> PAIGE1CPCT

<140> PCT/US99/06664
<141> 1999-03-26

<150> 60/082,756
<151> 1998-04-23

<150> 60/099,656
<151> 1998-09-09

<150> 60/115,345
<151> 1999-01-08

<160> 284

<170> PatentIn Ver. 2.0

<210> 1
<211> 20
<212> PRT
<213> Artificial Sequence

<220>
<223> Description of Artificial Sequence:Arbitrary peptide

<400> 1

```
Ser Ser Asn His Gln Ser Ser Arg Leu Ile Glu Leu Leu Ser Arg Ser
 1               5                  10                  15

Gly Ser Gly Lys
            20
```

<210> 2
<211> 5
<212> PRT
<213> Artificial Sequence

<220>
<223> Description of Artificial Sequence:Arbitrary peptide

<400> 2

```
Leu Xaa Xaa Leu Leu
 1               5
```

<210> 3
<211> 23
<212> DNA
<213> Artificial Sequence

<220>
<223> Description of Artificial Sequence:Arbitrary peptide

<400> 3
gatctaggtc acagtgacct gcg 23

<210> 4
<211> 23
<212> DNA
<213> Artificial Sequence

<220>
<223> Description of Artificial Sequence:Arbitrary peptide

<400> 4
gatccgcagg tcactgtgac cta 23

<210> 5
<211> 85
<212> DNA
<213> Artificial Sequence

<220>
<223> Description of Artificial Sequence:Arbitrary peptide

<400> 5

gactgtgcga attcggtcat gaaccattaa ctttattaga aagattatta atggatgata 60
aacaagctgt tctcgagcgt gtcag 85

<210> 6
<211> 73
<212> DNA
<213> Artificial Sequence

<220>
<223> Description of Artificial Sequence:Arbitrary peptide

<400> 6

gactgtgcga attctcttct ttaacttcta gagattttgg ttcttggtat gcttctagac 60
tcgagcgtgt cag 73

<210> 7
<211> 73
<212> DNA
<213> Artificial Sequence

<220>
<223> Description of Artificial Sequence:Arbitrary peptide

<400> 7

gactgtgcga attctcttct tgggatatgc atcaattttt ttgggaaggt gtttctagac 60
tcgagcgtgt cag 73

<210> 8
<211> 73
<212> DNA
<213> Artificial Sequence

<220>
<223> Description of Artificial Sequence:Arbitrary peptide

<400> 8

gactgtgcga attctcttct ccaggttcta gagaatggtt taaagatatg ttatctagac 60
tcgagcgtgt cag 73

<210> 9
<211> 14
<212> DNA
<213> Artificial Sequence

<220>
<223> Description of Artificial Sequence:Arbitrary peptide

<400> 9
ctgacacgct cgag 14

<210> 10
<211> 19
<212> PRT
<213> Artificial Sequence

<220>
<223> Description of Artificial Sequence:Arbitrary peptide

<400> 10

Gly His Glu Pro Leu Thr Leu Leu Glu Arg Leu Leu Met Asp Asp Lys
1 5 10 15

Gln Ala Val

<210> 11
<211> 15
<212> PRT
<213> Artificial Sequence

<220>
<223> Description of Artificial Sequence:Arbitrary peptide

<400> 11

Ser Ser Trp Asp Met His Gln Phe Phe Trp Glu Gly Val Ser Arg
1 5 10 15

<210> 12
<211> 15
<212> PRT
<213> Artificial Sequence

<220>
<223> Description of Artificial Sequence:Arbitrary peptide

<400> 12

Ser Ser Pro Gly Ser Arg Glu Trp Phe Lys Asp Met Leu Ser Arg
1 5 10 15

<210> 13
<211> 15
<212> PRT
<213> Artificial Sequence

<220>
<223> Description of Artificial Sequence:Arbitrary peptide

<400> 13

Ser Ser Leu Thr Ser Arg Asp Phe Gly Ser Trp Tyr Ala Ser Arg
1 5 10 15

<210> 14
<211> 14
<212> PRT
<213> Artificial Sequence

<220>
<223> Description of Artificial Sequence:Arbitrary peptide

<400> 14

Ser Arg Trp Glu Ser Pro Leu Gly Thr Trp Glu Trp Ser Arg
1 5 10

<210> 15
<211> 14
<212> PRT
<213> Artificial Sequence

<220>

<223> Description of Artificial Sequence:Arbitrary peptide

<400> 15

Ser Ala Ala Pro Arg Thr Ile Ser His Tyr Leu Met Gly Gly
1 5 10

<210> 16
<211> 15
<212> PRT
<213> Artificial Sequence

<220>
<223> Description of Artificial Sequence:Arbitrary peptide

<400> 16

Ser Ser Trp Val Arg Leu Ser Asp Phe Pro Trp Gly Val Ser Arg
1 5 10 15

<210> 17
<211> 15
<212> PRT
<213> Artificial Sequence

<220>
<223> Description of Artificial Sequence:Arbitrary peptide

<400> 17

Ser Ser Trp Asp Arg Leu Ser Asp Phe Pro Trp Gly Val Ser Arg
1 5 10 15

<210> 18
<211> 15
<212> PRT
<213> Artificial Sequence

<220>
<223> Description of Artificial Sequence:Arbitrary peptide

<400> 18

Ser Ser Trp Ile Arg Leu Arg Asp Leu Pro Trp Gly Glu Ser Arg
1 5 10 15

<210> 19
<211> 14
<212> PRT
<213> Artificial Sequence

<220>
<223> Description of Artificial Sequence:Arbitrary peptide

<400> 19

Ser Ser Trp Val Leu Leu Arg Asp Leu Pro Trp Gly Ser Arg
1 5 10

<210> 20
<211> 14
<212> PRT
<213> Artificial Sequence

<220>
<223> Description of Artificial Sequence:Arbitrary peptide

<400> 20

Ser Ser Trp Val Val Leu Arg Asp Leu Pro Trp Gly Ser Arg
1 5 10

<210> 21
<211> 15
<212> PRT
<213> Artificial Sequence

<220>
<223> Description of Artificial Sequence:Arbitrary peptide

<400> 21

Ser Ser Cys Lys Trp Tyr Glu Lys Cys Ser Gly Leu Trp Ser Arg
1 5 10 15

<210> 22
<211> 15
<212> PRT
<213> Artificial Sequence

<220>
<223> Description of Artificial Sequence:Arbitrary peptide

<400> 22

Ser Ser Gly Ile Cys Phe Phe Trp Asp Gly Cys Phe Glu Ser Arg
1 5 10 15

<210> 23
<211> 15
<212> PRT

<213> Artificial Sequence

<220>
<223> Description of Artificial Sequence:Arbitrary peptide

<400> 23

Ser Arg Asn Leu Cys Phe Phe Trp Asp Asp Glu Tyr Cys Ser Arg
1 5 10 15

<210> 24
<211> 14
<212> PRT
<213> Artificial Sequence

<220>
<223> Description of Artificial Sequence:Arbitrary peptide

<400> 24

His His His Arg His Pro Ala His Pro His Thr Tyr Gly Gly
1 5 10

<210> 25
<211> 15
<212> PRT
<213> Artificial Sequence

<220>
<223> Description of Artificial Sequence:Arbitrary peptide

<400> 25

Ser Arg Ala Gly Leu Leu Ser Asp Leu Leu Glu Gly Lys Ser Arg
1 5 10 15

<210> 26
<211> 15
<212> PRT
<213> Artificial Sequence

<220>
<223> Description of Artificial Sequence:Arbitrary peptide

<400> 26

Ser Ser Arg Ser Leu Leu Arg Asp Leu Leu Met Val Asp Ser Arg
1 5 10 15

<210> 27
<211> 15

<212> PRT
<213> Artificial Sequence

<220>
<223> Description of Artificial Sequence:Arbitrary peptide

<400> 27

Ser Ser Asn Lys Leu Leu Tyr Asn Leu Leu Lys Met Glu Ser Arg
1 5 10 15

<210> 28
<211> 15
<212> PRT
<213> Artificial Sequence

<220>
<223> Description of Artificial Sequence:Arbitrary peptide

<400> 28

Ser Ser Lys Ser Leu Leu Leu Asn Leu Leu Ser Thr Pro Ser Arg
1 5 10 15

<210> 29
<211> 16
<212> PRT
<213> Artificial Sequence

<220>
<223> Description of Artificial Sequence:Arbitrary peptide

<400> 29

His Ser Phe Pro Arg Glu Ser Leu Leu Val Arg Leu Leu Gln Gly Gly
1 5 10 15

<210> 30
<211> 15
<212> PRT
<213> Artificial Sequence

<220>
<223> Description of Artificial Sequence:Arbitrary peptide

<400> 30

Ser Arg Leu Glu Met Leu Leu Arg Ser Glu Thr Asp Phe Ser Arg
1 5 10 15

<210> 31
<211> 15
<212> PRT
<213> Artificial Sequence

<220>
<223> Description of Artificial Sequence:Arbitrary peptide

<400> 31

Ser Arg Leu Glu Glu Leu Leu Lys Trp Gly Ser Val Thr Ser Arg
1 5 10 15

<210> 32
<211> 15
<212> PRT
<213> Artificial Sequence

<220>
<223> Description of Artificial Sequence:Arbitrary peptide

<400> 32

Ser Arg Leu Glu Gln Leu Leu Lys Glu Glu Phe Ser Tyr Ser Arg
1 5 10 15

<210> 33
<211> 15
<212> PRT
<213> Artificial Sequence

<220>
<223> Description of Artificial Sequence:Arbitrary peptide

<400> 33

Ser Arg Leu Glu Gln Leu Leu Arg Ser Glu Pro Asp Phe Ser Arg
1 5 10 15

<210> 34
<211> 15
<212> PRT
<213> Artificial Sequence

<220>
<223> Description of Artificial Sequence:Arbitrary peptide

<400> 34

Ser Arg Leu Glu Asp Leu Leu Arg Ala Pro Phe Thr Thr Ser Arg
1 5 10 15

<210> 35
<211> 15
<212> PRT
<213> Artificial Sequence

<220>
<223> Description of Artificial Sequence:Arbitrary peptide

<400> 35

Ser Arg Leu Glu Ser Leu Leu Arg Phe Gly Gln Leu Asp Ser Arg
1 5 10 15

<210> 36
<211> 15
<212> PRT
<213> Artificial Sequence

<220>
<223> Description of Artificial Sequence:Arbitrary peptide

<400> 36

Ser Ser Arg Leu Leu Ser Leu Leu Val Gly Asp Phe Asn Ser Arg
1 5 10 15

<210> 37
<211> 15
<212> PRT
<213> Artificial Sequence

<220>
<223> Description of Artificial Sequence:Arbitrary peptide

<400> 37

Ser Arg Leu Glu Glu Leu Leu Leu Gly Thr Asn Arg Asp Ser Arg
1 5 10 15

<210> 38
<211> 15
<212> PRT
<213> Artificial Sequence

<220>
<223> Description of Artificial Sequence:Arbitrary peptide

<400> 38

Ser Arg Leu Lys Glu Leu Leu Leu Leu Pro Thr Asp Leu Ser Arg
1 5 10 15

<210> 39
<211> 15
<212> PRT
<213> Artificial Sequence

<220>
<223> Description of Artificial Sequence:Arbitrary peptide

<400> 39

Ser Arg Leu Glu Cys Leu Leu Glu Gly Arg Leu Asn Cys Ser Arg
1 5 10 15

<210> 40
<211> 15
<212> PRT
<213> Artificial Sequence

<220>
<223> Description of Artificial Sequence:Arbitrary peptide

<400> 40

Ser Ser Lys Leu Tyr Cys Leu Leu Asp Glu Ser Tyr Cys Ser Arg
1 5 10 15

<210> 41
<211> 15
<212> PRT
<213> Artificial Sequence

<220>
<223> Description of Artificial Sequence:Arbitrary peptide

<400> 41

Ser Arg Leu Ser Cys Leu Leu Met Gly Phe Glu Asp Cys Ser Arg
1 5 10 15

<210> 42
<211> 16
<212> PRT
<213> Artificial Sequence

<220>
<223> Description of Artificial Sequence:Arbitrary peptide

<400> 42

Ser Ser Lys Leu Ile Arg Leu Leu Thr Ser Asp Glu Glu Leu Ser Arg
1 5 10 15

<210> 43
<211> 16
<212> PRT
<213> Artificial Sequence

<220>
<223> Description of Artificial Sequence:Arbitrary peptide

<400> 43

Ser Ser Arg Leu Met Glu Leu Leu Gln Glu Gly Gln Gly Trp Ser Arg
1 5 10 15

<210> 44
<211> 15
<212> PRT
<213> Artificial Sequence

<220>
<223> Description of Artificial Sequence:Arbitrary peptide

<400> 44

Ser Ser Asn His Gln Ser Ser Arg Leu Ile Glu Leu Leu Ser Arg
1 5 10 15

<210> 45
<211> 15
<212> PRT
<213> Artificial Sequence

<220>
<223> Description of Artificial Sequence:Arbitrary peptide

<400> 45

Ser Ser Arg Leu Trp Gln Leu Leu Ala Ser Thr Asp Thr Ser Arg
1 5 10 15

<210> 46
<211> 15
<212> PRT
<213> Artificial Sequence

<220>
<223> Description of Artificial Sequence:Arbitrary peptide

<400> 46

Ser Ser Asn Ser Met Leu Trp Lys Leu Leu Ala Ala Pro Ser Arg
1 5 10 15

<210> 47
<211> 15
<212> PRT
<213> Artificial Sequence

<220>
<223> Description of Artificial Sequence:Arbitrary peptide

<400> 47

Ser Ser Lys Thr Leu Trp Arg Leu Leu Glu Gly Glu Arg Ser Arg
1 5 10 15

<210> 48
<211> 15
<212> PRT
<213> Artificial Sequence

<220>
<223> Description of Artificial Sequence:Arbitrary peptide

<400> 48

Ser Arg Ala Gly Pro Val Leu Trp Gly Leu Leu Ser Glu Ser Arg
1 5 10 15

<210> 49
<211> 15
<212> PRT
<213> Artificial Sequence

<220>
<223> Description of Artificial Sequence:Arbitrary peptide

<400> 49

Ser Ser Leu Thr Ser Arg Asp Phe Gly Ser Trp Tyr Ala Ser Arg
1 5 10 15

<210> 50
<211> 15
<212> PRT
<213> Artificial Sequence

<220>
<223> Description of Artificial Sequence:Arbitrary peptide

<400> 50

Ser Ser Trp Val Arg Leu Ser Asp Phe Pro Trp Gly Val Ser Arg
1 5 10 15

<210> 51
<211> 14
<212> PRT
<213> Artificial Sequence

<220>
<223> Description of Artificial Sequence:Arbitrary peptide

<400> 51

Ser Ser Glu Tyr Cys Phe Tyr Asp Ser Ala His Cys Ser Arg
1 5 10

<210> 52
<211> 15
<212> PRT
<213> Artificial Sequence

<220>
<223> Description of Artificial Sequence:Arbitrary peptide

<400> 52

Ser Arg Ser Leu Leu Glu Cys His Leu Met Gly Asn Cys Ser Arg
1 5 10 15

<210> 53
<211> 15
<212> PRT
<213> Artificial Sequence

<220>
<223> Description of Artificial Sequence:Arbitrary peptide

<400> 53

Ser Ser Glu Leu Leu Arg Trp His Leu Thr Arg Asp Thr Ser Arg
1 5 10 15

<210> 54
<211> 15
<212> PRT
<213> Artificial Sequence

<220>
<223> Description of Artificial Sequence:Arbitrary peptide

<400> 54

Ser Arg Leu Glu Tyr Trp Leu Lys Trp Glu Pro Gly Pro Ser Arg
1 5 10 15

<210> 55
<211> 15
<212> PRT
<213> Artificial Sequence

<220>
<223> Description of Artificial Sequence:Arbitrary peptide

<400> 55

Ser Arg Ser Asp Ser Ile Leu Trp Arg Met Leu Ser Glu Ser Arg
1 5 10 15

<210> 56
<211> 16
<212> PRT
<213> Artificial Sequence

<220>
<223> Description of Artificial Sequence:Arbitrary peptide

<400> 56

Ser Ser Lys Gly Val Leu Trp Arg Met Leu Ala Glu Pro Val Ser Arg
1 5 10 15

<210> 57
<211> 16
<212> PRT
<213> Artificial Sequence

<220>
<223> Description of Artificial Sequence:Arbitrary peptide

<400> 57

His Ser His Gly Pro Leu Thr Leu Asn Leu Leu Arg Ser Ser Gly Gly
1 5 10 15

<210> 58
<211> 15
<212> PRT

<213> Artificial Sequence

<220>
<223> Description of Artificial Sequence:Arbitrary peptide

<400> 58

Ser Ser Ala Gly Gly Gly Ala Pro Ala Gly Ser Thr Pro Ser Arg
1 5 10 15

<210> 59
<211> 15
<212> PRT
<213> Artificial Sequence

<220>
<223> Description of Artificial Sequence:Arbitrary peptide

<400> 59

Ser Ser Lys Tyr Ser Tyr Ser Arg Ser Ser Glu Gly His Ser Arg
1 5 10 15

<210> 60
<211> 15
<212> PRT
<213> Artificial Sequence

<220>
<223> Description of Artificial Sequence:Arbitrary peptide

<400> 60

Ser Ser Tyr Gln Trp Glu Thr His Ser Asp Lys Trp Arg Ser Arg
1 5 10 15

<210> 61
<211> 15
<212> PRT
<213> Artificial Sequence

<220>
<223> Description of Artificial Sequence:Arbitrary peptide

<400> 61

Ser Ser Val Thr Lys Lys Ala Leu Thr Ile Ala Lys Asp Ser Arg
1 5 10 15

<210> 62

<211> 15
<212> PRT
<213> Artificial Sequence

<220>
<223> Description of Artificial Sequence:Arbitrary peptide

<400> 62

Ser Ser Asn His Gln Ser Ser Arg Leu Ile Glu Leu Leu Ser Arg
1 5 10 15

<210> 63
<211> 15
<212> PRT
<213> Artificial Sequence

<220>
<223> Description of Artificial Sequence:Arbitrary peptide

<400> 63

Ser Arg Leu Lys Glu Leu Leu Leu Leu Pro Thr Asp Leu Ser Arg
1 5 10 15

<210> 64
<211> 15
<212> PRT
<213> Artificial Sequence

<220>
<223> Description of Artificial Sequence:Arbitrary peptide

<400> 64

Ser Ser Lys Leu Tyr Cys Leu Leu Asp Glu Ser Tyr Cys Ser Arg
1 5 10 15

<210> 65
<211> 14
<212> PRT
<213> Artificial Sequence

<220>
<223> Description of Artificial Sequence:Arbitrary peptide

<400> 65

His Gly Pro Leu Thr Leu Asn Leu Leu Arg Ser Ser Gly Gly
1 5 10

<210> 66
<211> 15
<212> PRT
<213> Artificial Sequence

<220>
<223> Description of Artificial Sequence:Arbitrary peptide

<400> 66

Ser Arg Leu Glu Tyr Trp Leu Lys Trp Glu Pro Gly Pro Ser Arg
1 5 10 15

<210> 67
<211> 15
<212> PRT
<213> Artificial Sequence

<220>
<223> Description of Artificial Sequence:Arbitrary peptide

<400> 67

Ser Ser Cys Lys Trp Tyr Glu Lys Cys Ser Gly Leu Trp Ser Arg
1 5 10 15

<210> 68
<211> 15
<212> PRT
<213> Artificial Sequence

<220>
<223> Description of Artificial Sequence:Arbitrary peptide

<400> 68

Ser Ser Glu Tyr Cys Phe Tyr Trp Asp Ser Ala His Cys Ser Arg
1 5 10 15

<210> 69
<211> 14
<212> PRT
<213> Artificial Sequence

<220>
<223> Description of Artificial Sequence:Arbitrary peptide

<400> 69

Ser Ser Trp Val Leu Leu Arg Asp Leu Pro Trp Gly Ser Arg
1 5 10

<210> 70
<211> 15
<212> PRT
<213> Artificial Sequence

<220>
<223> Description of Artificial Sequence:Arbitrary peptide

<400> 70

Ser Ser Trp Val Arg Leu Ser Asp Phe Pro Trp Gly Val Ser Arg
1 5 10 15

<210> 71
<211> 15
<212> PRT
<213> Artificial Sequence

<220>
<223> Description of Artificial Sequence:Arbitrary peptide

<400> 71

Ser Ser Leu Thr Ser Arg Asp Phe Gly Ser Trp Tyr Ala Ser Arg
1 5 10 15

<210> 72
<211> 15
<212> PRT
<213> Artificial Sequence

<220>
<223> Description of Artificial Sequence:Arbitrary peptide

<400> 72

Ser Arg Thr Trp Glu Ser Pro Leu Gly Thr Trp Glu Trp Ser Arg
1 5 10 15

<210> 73
<211> 14
<212> PRT
<213> Artificial Sequence

<220>
<223> Description of Artificial Sequence:Arbitrary peptide

<400> 73

Ser Ala Ala Cys Ala Thr Ile Ser His Tyr Leu Met Gly Gly
1 5 10

<210> 74
<211> 15
<212> PRT
<213> Artificial Sequence

<220>
<223> Description of Artificial Sequence:Arbitrary peptide

<400> 74

Ser Arg Asn Leu Cys Phe Phe Trp Asp Asp Glu Tyr Cys Ser Arg
1 5 10 15

<210> 75
<211> 14
<212> PRT
<213> Artificial Sequence

<220>
<223> Description of Artificial Sequence:Arbitrary peptide

<400> 75

Ser Trp Asp Met His Gln Phe Phe Trp Glu Gly Val Ser Arg
1 5 10

<210> 76
<211> 15
<212> PRT
<213> Artificial Sequence

<220>
<223> Description of Artificial Sequence:Arbitrary peptide

<400> 76

Ser Arg Trp His Gly Thr Leu Phe Trp Gln Asp Glu Gln Ser Arg
1 5 10 15

<210> 77
<211> 15
<212> PRT
<213> Artificial Sequence

<220>

<223> Description of Artificial Sequence:Arbitrary peptide

<400> 77

Ser Ser Cys Lys Trp Tyr Glu Lys Cys Ser Gly Leu Trp Ser Arg
1 5 10 15

<210> 78
<211> 15
<212> PRT
<213> Artificial Sequence

<220>
<223> Description of Artificial Sequence:Arbitrary peptide

<400> 78

Ser Ser Arg Met Gly His Val Trp Tyr Asp Trp Thr Phe Ser Arg
1 5 10 15

<210> 79
<211> 15
<212> PRT
<213> Artificial Sequence

<220>
<223> Description of Artificial Sequence:Arbitrary peptide

<400> 79

Ser Ser Arg Leu Leu Gly Asp Phe Gly Gly Ser Val Val Ser Arg
1 5 10 15

<210> 80
<211> 15
<212> PRT
<213> Artificial Sequence

<220>
<223> Description of Artificial Sequence:Arbitrary peptide

<400> 80

Ser Ser Lys Tyr Val Phe Gly Phe Gln Val Ala Gly Gly Ser Arg
1 5 10 15

<210> 81
<211> 15
<212> PRT
<213> Artificial Sequence

<220>
<223> Description of Artificial Sequence:Arbitrary peptide

<400> 81

Ser Ser Trp Ala Gly Ile Lys Phe Gly Lys Pro Pro His Ser Arg
1 5 10 15

<210> 82
<211> 15
<212> PRT
<213> Artificial Sequence

<220>
<223> Description of Artificial Sequence:Arbitrary peptide

<400> 82

Ser Ser Ser Trp Ser Tyr Gly Lys Pro Thr Phe Leu Ser Ser Arg
1 5 10 15

<210> 83
<211> 15
<212> PRT
<213> Artificial Sequence

<220>
<223> Description of Artificial Sequence:Arbitrary peptide

<400> 83

Ser Arg Asp Thr Gly Asp Met Trp Trp Gly Arg Gly Gly Ser Arg
1 5 10 15

<210> 84
<211> 15
<212> PRT
<213> Artificial Sequence

<220>
<223> Description of Artificial Sequence:Arbitrary peptide

<400> 84

Ser Ser Gly Arg Tyr Asp Pro Phe Val Leu Asn Ala Ala Ser Arg
1 5 10 15

<210> 85
<211> 15

<212> PRT
<213> Artificial Sequence

<220>
<223> Description of Artificial Sequence:Arbitrary peptide

<400> 85

Ser Ser Ser Pro Trp Trp Ser Phe Asn Leu Arg Asp Met Ser Arg
1 5 10 15

<210> 86
<211> 15
<212> PRT
<213> Artificial Sequence

<220>
<223> Description of Artificial Sequence:Arbitrary peptide

<400> 86

Ser Ser Trp Pro Tyr Leu Pro Lys Arg Glu Glu Trp Ala Ser Arg
1 5 10 15

<210> 87
<211> 15
<212> PRT
<213> Artificial Sequence

<220>
<223> Description of Artificial Sequence:Arbitrary peptide

<400> 87

Ser Ser Gly Trp Ile Glu Gln Lys Leu Arg Gly Ser Phe Ser Arg
1 5 10 15

<210> 88
<211> 15
<212> PRT
<213> Artificial Sequence

<220>
<223> Description of Artificial Sequence:Arbitrary peptide

<400> 88

Ser Ser Ser Ala Thr Ser Ile Lys Val Gln Tyr Gln Ile Ser Arg
1 5 10 15

<210> 89
<211> 15
<212> PRT
<213> Artificial Sequence

<220>
<223> Description of Artificial Sequence:Arbitrary peptide

<400> 89

Ser Ser Tyr Leu Thr Leu Gly Lys Ser Met Met Ala Ile Ser Arg
1 5 10 15

<210> 90
<211> 15
<212> PRT
<213> Artificial Sequence

<220>
<223> Description of Artificial Sequence:Arbitrary peptide

<400> 90

Ser Ser Trp His Ser Arg Trp Asp Leu Ala Leu Gly Phe Ser Arg
1 5 10 15

<210> 91
<211> 15
<212> PRT
<213> Artificial Sequence

<220>
<223> Description of Artificial Sequence:Arbitrary peptide

<400> 91

Ser Ser Gly Tyr Trp Gly Gly Trp Asp Tyr Gly Ala Gly Ser Arg
1 5 10 15

<210> 92
<211> 15
<212> PRT
<213> Artificial Sequence

<220>
<223> Description of Artificial Sequence:Arbitrary peptide

<400> 92

Ser Arg Asp Asn Cys Gly Ala Gly Leu Trp Ala Gly Cys Ser Arg
1 5 10 15

<210> 93
<211> 15
<212> PRT
<213> Artificial Sequence

<220>
<223> Description of Artificial Sequence:Arbitrary peptide

<400> 93

Ser Ser Ser Thr Pro Gly Trp Trp Glu Trp Asp Trp Ala Ser Arg
1 5 10 15

<210> 94
<211> 19
<212> PRT
<213> Artificial Sequence

<220>
<223> Description of Artificial Sequence:Arbitrary peptide

<400> 94

Ser Ser Tyr Trp Asp Gly Ser Trp Arg Arg Lys Glu Thr Cys Val Ser
1 5 10 15

Cys Ser Arg

<210> 95
<211> 19
<212> PRT
<213> Artificial Sequence

<220>
<223> Description of Artificial Sequence:Arbitrary peptide

<400> 95

Ser Ser Arg Thr Ala Glu Asp Tyr Cys Phe Phe Ala Asp Asp Tyr Trp
1 5 10 15

Cys Ser Arg

<210> 96
<211> 15
<212> PRT
<213> Artificial Sequence

<220>
<223> Description of Artificial Sequence:Arbitrary peptide

<400> 96

Ser Ser Arg Ala Leu Ala Leu Phe Pro Val Gly Met Glu Ser Arg
1 5 10 15

<210> 97
<211> 19
<212> PRT
<213> Artificial Sequence

<220>
<223> Description of Artificial Sequence:Arbitrary peptide

<400> 97

Ser Ser Asp Cys Glu Ser Leu Thr Ser Tyr Pro His Leu Lys Ala Leu
1 5 10 15

Cys Ser Arg

<210> 98
<211> 15
<212> PRT
<213> Artificial Sequence

<220>
<223> Description of Artificial Sequence:Arbitrary peptide

<400> 98

Ser Ser Thr Ala Thr Ala Leu Arg Asp Arg Leu Ala Tyr Ser Arg
1 5 10 15

<210> 99
<211> 15
<212> PRT
<213> Artificial Sequence

<220>
<223> Description of Artificial Sequence:Arbitrary peptide

<400> 99

Ser Ser Gly Lys Thr Arg Glu His Tyr Arg Glu Gly Thr Ser Arg
1 5 10 15

<210> 100

<211> 16
<212> PRT
<213> Artificial Sequence

<220>
<223> Description of Artificial Sequence:Arbitrary peptide

<400> 100

His Ser His Asn His His Ser Pro Trp Leu Phe Arg Leu Leu Gly Gly
1 5 10 15

<210> 101
<211> 16
<212> PRT
<213> Artificial Sequence

<220>
<223> Description of Artificial Sequence:Arbitrary peptide

<400> 101

His Ser His Pro His His Ser His Leu Leu Tyr Lys Leu Met Gly Gly
1 5 10 15

<210> 102
<211> 16
<212> PRT
<213> Artificial Sequence

<220>
<223> Description of Artificial Sequence:Arbitrary peptide

<400> 102

His Ser His Pro Leu Pro Pro Leu Leu Ser Arg Leu Leu Thr Gly Gly
1 5 10 15

<210> 103
<211> 19
<212> PRT
<213> Artificial Sequence

<220>
<223> Description of Artificial Sequence:Arbitrary peptide

<400> 103

Ser Arg Leu Thr Cys Leu Leu Gln Ser Asn Gly Trp Asp Ser Glu Gln
1 5 10 15

Cys Ser Arg

<210> 104
<211> 15
<212> PRT
<213> Artificial Sequence

<220>
<223> Description of Artificial Sequence:Arbitrary peptide

<400> 104

Ser Ser Leu Thr Ser Arg Asp Phe Gly Ser Trp Tyr Ala Ser Arg
1 5 10 15

<210> 105
<211> 14
<212> PRT
<213> Artificial Sequence

<220>
<223> Description of Artificial Sequence:Arbitrary peptide

<400> 105

Ser Arg Thr Leu Gln Leu Asp Trp Gly Thr Leu Tyr Ser Arg
1 5 10

<210> 106
<211> 19
<212> PRT
<213> Artificial Sequence

<220>
<223> Description of Artificial Sequence:Arbitrary peptide

<400> 106

Ser Arg Leu Pro Pro Ser Val Phe Ser Met Cys Gly Ser Glu Val Cys
1 5 10 15

Leu Ser Arg

<210> 107
<211> 28
<212> PRT

<213> Artificial Sequence

<220>
<223> Description of Artificial Sequence:Arbitrary peptide

<400> 107

Ser Arg Phe Glu Ile Trp Lys Pro Glu Pro Gly Cys Val Ser Ser Leu
1 5 10 15

Glu Asn Trp Glu Pro Gly Lys Arg Val Cys Ser Arg
20 25

<210> 108
<211> 19
<212> PRT
<213> Artificial Sequence

<220>
<223> Description of Artificial Sequence:Arbitrary peptide

<400> 108

Ser Arg Val Phe Gly Val Ser Gly Gly Glu Val Val Leu Ile Asn Gly
1 5 10 15

Ser Ser Arg

<210> 109
<211> 19
<212> PRT
<213> Artificial Sequence

<220>
<223> Description of Artificial Sequence:Arbitrary peptide

<400> 109

Ser Arg Leu Cys Phe Gly Asp Trp Cys Met Leu Gly Gly Val Asp Val
1 5 10 15

Leu Ser Arg

<210> 110
<211> 19
<212> PRT
<213> Artificial Sequence

<220>
<223> Description of Artificial Sequence:Arbitrary peptide

<400> 110

Ser Ser Leu Asn Met Val Val Asp Thr Pro Trp Cys Gly Lys Trp Val
1               5                   10                  15

Cys Ser Arg

<210> 111
<211> 15
<212> PRT
<213> Artificial Sequence

<220>
<223> Description of Artificial Sequence:Arbitrary peptide

<400> 111

Ser Ser Arg Pro Asp Ala Ala Phe Phe Gly Ala Lys Leu Ser Arg
1               5                   10                  15

<210> 112
<211> 15
<212> PRT
<213> Artificial Sequence

<220>
<223> Description of Artificial Sequence:Arbitrary peptide

<400> 112

Ser Ser Arg Pro Ser Pro Ser Phe Trp Glu Lys Gln Leu Ser Arg
1               5                   10                  15

<210> 113
<211> 15
<212> PRT
<213> Artificial Sequence

<220>
<223> Description of Artificial Sequence:Arbitrary peptide

<400> 113

Ser Ser Arg Pro Thr Ala Glu Trp Phe Arg Glu Asn Leu Ser Arg
1               5                   10                  15

<210> 114
<211> 15
<212> PRT
<213> Artificial Sequence

<220>
<223> Description of Artificial Sequence:Arbitrary peptide

<400> 114

Ser Arg Trp Trp Asp Thr Ser Trp Trp Leu Glu Glu Leu Ser Arg
1 5 10 15

<210> 115
<211> 15
<212> PRT
<213> Artificial Sequence

<220>
<223> Description of Artificial Sequence:Arbitrary peptide

<400> 115

Ser Ser Arg Ile Ala Asp Leu Phe Trp Arg Leu Glu Pro Ser Arg
1 5 10 15

<210> 116
<211> 15
<212> PRT
<213> Artificial Sequence

<220>
<223> Description of Artificial Sequence:Arbitrary peptide

<400> 116

Ser Arg Ser Tyr His Gly Glu Trp Gly Val Trp Thr Leu Ser Arg
1 5 10 15

<210> 117
<211> 19
<212> PRT
<213> Artificial Sequence

<220>
<223> Description of Artificial Sequence:Arbitrary peptide

<400> 117

Ser Ser Asp Trp Cys Phe Gly Trp Gly Gly Trp Cys Ala Ser Glu Ala
1 5 10 15

Val Ser Arg

<210> 118
<211> 19
<212> PRT
<213> Artificial Sequence

<220>
<223> Description of Artificial Sequence:Arbitrary peptide

<400> 118

Ser Arg Asn Trp Asp Trp Ala Ala Leu Glu Leu Leu Pro Tyr Pro His
1 5 10 15

Pro Ser Arg

<210> 119
<211> 15
<212> PRT
<213> Artificial Sequence

<220>
<223> Description of Artificial Sequence:Arbitrary peptide

<400> 119

Ser Ser Leu Thr Ser Arg Asp Phe Gly Ser Trp Tyr Ala Ser Arg
1 5 10 15

<210> 120
<211> 15
<212> PRT
<213> Artificial Sequence

<220>
<223> Description of Artificial Sequence:Arbitrary peptide

<400> 120

Ser Arg Ser Pro Ile Leu Thr His Leu Leu Ser Leu Gly Ser Arg
1 5 10 15

<210> 121
<211> 15
<212> PRT
<213> Artificial Sequence

<220>
<223> Description of Artificial Sequence:Arbitrary peptide

<400> 121

Ser Ser Thr Gly Ile Leu Trp Lys Leu Leu Thr Ala Glu Ser Arg
1 5 10 15

<210> 122
<211> 15
<212> PRT
<213> Artificial Sequence

<220>
<223> Description of Artificial Sequence:Arbitrary peptide

<400> 122

Ser Ser His Gly Ile Leu Trp Arg Leu Leu Ser Glu Gly Ser Arg
1 5 10 15

<210> 123
<211> 15
<212> PRT
<213> Artificial Sequence

<220>
<223> Description of Artificial Sequence:Arbitrary peptide

<400> 123

Ser Arg Ser Asp Ser Ile Leu Trp Arg Met Leu Ser Glu Ser Arg
1 5 10 15

<210> 124
<211> 15
<212> PRT
<213> Artificial Sequence

<220>
<223> Description of Artificial Sequence:Arbitrary peptide

<400> 124

Ser Arg Leu Val Ala Leu Leu Lys Ser Pro Trp Ser Val Ser Arg
1 5 10 15

<210> 125
<211> 15
<212> PRT
<213> Artificial Sequence

<220>
<223> Description of Artificial Sequence:Arbitrary peptide

<400> 125

Ser Arg Leu Glu Glu Leu Leu Leu Met Asp Phe Trp Arg Ser Arg
1 5 10 15

<210> 126
<211> 15
<212> PRT
<213> Artificial Sequence

<220>
<223> Description of Artificial Sequence:Arbitrary peptide

<400> 126

Ser Ser Lys Leu Trp Gln Leu Leu Ser Ser Pro Ile Asp Ser Arg
1 5 10 15

<210> 127
<211> 15
<212> PRT
<213> Artificial Sequence

<220>
<223> Description of Artificial Sequence:Arbitrary peptide

<400> 127

Ser Ser Lys Leu Tyr Cys Leu Leu Asp Glu Ser Tyr Cys Ser Arg
1 5 10 15

<210> 128
<211> 19
<212> PRT
<213> Artificial Sequence

<220>
<223> Description of Artificial Sequence:Arbitrary peptide

<400> 128

Ser Arg Ser Leu Leu Met Asp Met Leu Met Ser Asp Asp Tyr Val Thr
1 5 10 15

Val Ser Arg

<210> 129
<211> 19
<212> PRT

<213> Artificial Sequence

<220>
<223> Description of Artificial Sequence:Arbitrary peptide

<400> 129

Ser Ser Arg Leu Leu Ala Cys Glu Leu Met Tyr Glu Asp Ala Asp Val
1 5 10 15

Cys Ser Arg

<210> 130
<211> 16
<212> PRT
<213> Artificial Sequence

<220>
<223> Description of Artificial Sequence:Arbitrary peptide

<400> 130

His Ser His Ser Pro Leu Leu Met Ala Leu Leu Ala Pro Pro Gly Gly
1 5 10 15

<210> 131
<211> 15
<212> PRT
<213> Artificial Sequence

<220>
<223> Description of Artificial Sequence:Arbitrary peptide

<400> 131

Ser Arg Leu Glu Tyr Tyr Leu Arg Leu Gly Thr Tyr Glu Ser Arg
1 5 10 15

<210> 132
<211> 15
<212> PRT
<213> Artificial Sequence

<220>
<223> Description of Artificial Sequence:Arbitrary peptide

<400> 132

Ser Ser Cys Leu Arg Glu Ile Leu Leu Tyr Gly Ala Cys Ser Arg
1 5 10 15

<210> 133
<211> 19
<212> PRT
<213> Artificial Sequence

<220>
<223> Description of Artificial Sequence:Arbitrary peptide

<400> 133

Ser Ser Arg Thr Ala Glu Asp Tyr Cys Phe Phe Ala Asp Asp Tyr Trp
1 5 10 15

Cys Ser Arg

<210> 134
<211> 19
<212> PRT
<213> Artificial Sequence

<220>
<223> Description of Artificial Sequence:Arbitrary peptide

<400> 134

Ser Ser Leu Arg Cys Tyr Leu Ser Ser Ser Lys Val Asp Gln Trp Ala
1 5 10 15

Cys Ser Arg

<210> 135
<211> 19
<212> PRT
<213> Artificial Sequence

<220>
<223> Description of Artificial Sequence:Arbitrary peptide

<400> 135

Ser Ser Tyr Lys Pro His Ser Leu Leu Glu Trp His Leu Leu Gly Gly
1 5 10 15

Thr Ser Arg

<210> 136

<211> 15
<212> PRT
<213> Artificial Sequence

<220>
<223> Description of Artificial Sequence:Arbitrary peptide

<400> 136

Ser Arg Leu His Cys Leu Leu Asp Ser Ser Tyr Cys Ser Ser Arg
1 5 10 15

<210> 137
<211> 15
<212> PRT
<213> Artificial Sequence

<220>
<223> Description of Artificial Sequence:Arbitrary peptide

<400> 137

Ser Arg Leu His Cys Leu Leu Asp Ser Ser Tyr Cys Ser Ser Arg
1 5 10 15

<210> 138
<211> 15
<212> PRT
<213> Artificial Sequence

<220>
<223> Description of Artificial Sequence:Arbitrary peptide

<400> 138

Ser Ser Trp Pro Asn Pro Thr Phe Trp Glu Arg Gln Leu Ser Arg
1 5 10 15

<210> 139
<211> 14
<212> PRT
<213> Artificial Sequence

<220>
<223> Description of Artificial Sequence:Arbitrary peptide

<400> 139

Ser Tyr Ser Lys Glu Trp Phe Glu Glu Arg Leu Asn Ser Arg
1 5 10

<210> 140
<211> 15
<212> PRT
<213> Artificial Sequence

<220>
<223> Description of Artificial Sequence:Arbitrary peptide

<400> 140

Ser Ser Ser Met Met Arg Glu Phe Phe Glu Arg Glu Leu Ser Arg
1 5 10 15

<210> 141
<211> 15
<212> PRT
<213> Artificial Sequence

<220>
<223> Description of Artificial Sequence:Arbitrary peptide

<400> 141

Ser Ser Gly Leu Pro Pro Asn Phe Glu Arg Met Leu Lys Ser Arg
1 5 10 15

<210> 142
<211> 15
<212> PRT
<213> Artificial Sequence

<220>
<223> Description of Artificial Sequence:Arbitrary peptide

<400> 142

Ser Ser Gly Pro Trp Leu Met His Tyr Leu Gly Gly Gly Ser Arg
1 5 10 15

<210> 143
<211> 15
<212> PRT
<213> Artificial Sequence

<220>
<223> Description of Artificial Sequence:Arbitrary peptide

<400> 143

Ser Ser Thr Ser Trp Leu His His Tyr Leu Met Gly Thr Ser Arg
1 5 10 15

<210> 144
<211> 15
<212> PRT
<213> Artificial Sequence

<220>
<223> Description of Artificial Sequence:Arbitrary peptide

<400> 144

Ser Arg Gly Gly Gly Glu Cys Leu Gly Pro Trp Cys Leu Ser Arg
1 5 10 15

<210> 145
<211> 19
<212> PRT
<213> Artificial Sequence

<220>
<223> Description of Artificial Sequence:Arbitrary peptide

<400> 145

Ser Ser Glu Ala Cys Val Gly Arg Trp Met Leu Cys Glu Gln Leu Gly
1 5 10 15

Val Ser Arg

<210> 146
<211> 15
<212> PRT
<213> Artificial Sequence

<220>
<223> Description of Artificial Sequence:Arbitrary peptide

<400> 146

Ser Ser Gln Val Trp Pro Gly Pro Trp Arg Leu Val Glu Ser Arg
1 5 10 15

<210> 147
<211> 15
<212> PRT

<213> Artificial Sequence

<220>
<223> Description of Artificial Sequence:Arbitrary peptide

<400> 147

Ser Ser Ser Leu Gly Pro Trp Arg Leu Ser Glu Leu Glu Ser Arg
1 5 10 15

<210> 148
<211> 15
<212> PRT
<213> Artificial Sequence

<220>
<223> Description of Artificial Sequence:Arbitrary peptide

<400> 148

Ser Ser Ser Gly Pro Trp Arg Trp Gly Leu Ser Ile Glu Ser Arg
1 5 10 15

<210> 149
<211> 15
<212> PRT
<213> Artificial Sequence

<220>
<223> Description of Artificial Sequence:Arbitrary peptide

<400> 149

Ser Arg Glu Cys Val Gly Gly Trp Cys Leu Ala Glu Leu Ser Arg
1 5 10 15

<210> 150
<211> 15
<212> PRT
<213> Artificial Sequence

<220>
<223> Description of Artificial Sequence:Arbitrary peptide

<400> 150

Ser Ser Ile Pro Pro Arg Ser Trp Trp Leu Ser Gln Leu Ser Arg
1 5 10 15

<210> 151

<211> 15
<212> PRT
<213> Artificial Sequence

<220>
<223> Description of Artificial Sequence:Arbitrary peptide

<400> 151

Ser Ser Trp Pro Gly Ala Glu Trp Phe Lys Glu Gln Leu Ser Arg
1 5 10 15

<210> 152
<211> 15
<212> PRT
<213> Artificial Sequence

<220>
<223> Description of Artificial Sequence:Arbitrary peptide

<400> 152

Ser Ser Lys Leu Tyr Cys Leu Leu Asp Glu Ser Tyr Cys Ser Arg
1 5 10 15

<210> 153
<211> 16
<212> PRT
<213> Artificial Sequence

<220>
<223> Description of Artificial Sequence:Arbitrary peptide

<400> 153

His Ser Tyr Ser Ser His Pro Leu Leu Leu Ser Tyr Leu Trp Gly Gly
1 5 10 15

<210> 154
<211> 16
<212> PRT
<213> Artificial Sequence

<220>
<223> Description of Artificial Sequence:Arbitrary peptide

<400> 154

His Ser Trp Leu Gly Pro Trp Arg Leu Ser Ser Ile Asp Leu Gly Gly
1 5 10 15

<210> 155
<211> 16
<212> PRT
<213> Artificial Sequence

<220>
<223> Description of Artificial Sequence:Arbitrary peptide

<400> 155

His Ser Thr Asp Met Gly Trp Leu Arg Pro Trp Arg Leu Leu Gly Gly
1 5 10 15

<210> 156
<211> 15
<212> PRT
<213> Artificial Sequence

<220>
<223> Description of Artificial Sequence:Arbitrary peptide

<400> 156

Ser Ser Val Phe Thr Ile Met Asp Gly Lys Val Ala Leu Ser Arg
1 5 10 15

<210> 157
<211> 15
<212> PRT
<213> Artificial Sequence

<220>
<223> Description of Artificial Sequence:Arbitrary peptide

<400> 157

Ser Arg Pro Tyr Cys Leu Gly Asp Val Trp Cys Leu Asp Ser Arg
1 5 10 15

<210> 158
<211> 15
<212> PRT
<213> Artificial Sequence

<220>
<223> Description of Artificial Sequence:Arbitrary peptide

<400> 158

Ser Arg Glu Trp Glu Asp Gly Phe Gly Gly Arg Trp Leu Ser Arg
1 5 10 15

<210> 159
<211> 15
<212> PRT
<213> Artificial Sequence

<220>
<223> Description of Artificial Sequence:Arbitrary peptide

<400> 159

Ser Ser Trp Asn Ser Arg Glu Phe Phe Leu Ser Gln Leu Ser Arg
1 5 10 15

<210> 160
<211> 15
<212> PRT
<213> Artificial Sequence

<220>
<223> Description of Artificial Sequence:Arbitrary peptide

<400> 160

Ser Ser Thr Thr Met Phe Asp Phe Phe Tyr Glu Arg Leu Ser Arg
1 5 10 15

<210> 161
<211> 15
<212> PRT
<213> Artificial Sequence

<220>
<223> Description of Artificial Sequence:Arbitrary peptide

<400> 161

Ser Ser Ala Arg Pro Trp Trp Leu Gln Phe Glu Gly Ser Ser Arg
1 5 10 15

<210> 162
<211> 15
<212> PRT
<213> Artificial Sequence

<220>
<223> Description of Artificial Sequence:Arbitrary peptide

<400> 162

Ser Ser Gln Glu Glu Trp Leu Leu Pro Trp Arg Leu Ala Ser Arg
1 5 10 15

<210> 163
<211> 19
<212> PRT
<213> Artificial Sequence

<220>
<223> Description of Artificial Sequence:Arbitrary peptide

<400> 163

Ser Arg Leu Pro Pro Ser Val Phe Ser Met Cys Gly Ser Glu Val Cys
1 5 10 15

Leu Ser Arg

<210> 164
<211> 19
<212> PRT
<213> Artificial Sequence

<220>
<223> Description of Artificial Sequence:Arbitrary peptide

<400> 164

Ser Ser Gly Pro Phe Tyr Val Gly Gly Met Leu Trp Pro Ala Asp Cys
1 5 10 15

Leu Ser Arg

<210> 165
<211> 15
<212> PRT
<213> Artificial Sequence

<220>
<223> Description of Artificial Sequence:Arbitrary peptide

<400> 165

Ser Arg Glu Gly Trp Met Gly Pro Trp Arg Leu Ala Asp Ser Arg
1 5 10 15

<210> 166

<211> 15
<212> PRT
<213> Artificial Sequence

<220>
<223> Description of Artificial Sequence:Arbitrary peptide

<400> 166

```
Ser Arg Asn Glu Cys Ile Gly Pro Trp Cys Leu Thr Ile Ser Arg
1               5                   10                  15
```

<210> 167
<211> 15
<212> PRT
<213> Artificial Sequence

<220>
<223> Description of Artificial Sequence:Arbitrary peptide

<400> 167

```
Ser Ser Pro Gly Ser Arg Glu Trp Phe Lys Asp Met Leu Ser Arg
1               5                   10                  15
```

<210> 168
<211> 15
<212> PRT
<213> Artificial Sequence

<220>
<223> Description of Artificial Sequence:Arbitrary peptide

<400> 168

```
Ser Ser Val Ala Ser Arg Glu Trp Trp Val Arg Glu Leu Ser Arg
1               5                   10                  15
```

<210> 169
<211> 16
<212> PRT
<213> Artificial Sequence

<220>
<223> Description of Artificial Sequence:Arbitrary peptide

<400> 169

```
Ser Arg Met Phe Gln Val Cys Gly Asp Glu Val Cys Leu Arg Ser Arg
```

1 5 10 15

<210> 170
<211> 16
<212> PRT
<213> Artificial Sequence

<220>
<223> Description of Artificial Sequence:Arbitrary peptide

<400> 170

Ser Ser Asp Leu His Arg Asp Cys Leu Gly Val Trp Cys Leu Ser Arg
1 5 10 15

<210> 171
<211> 19
<212> PRT
<213> Artificial Sequence

<220>
<223> Description of Artificial Sequence:Arbitrary peptide

<400> 171

Ser Arg Leu Asn Gly Val Phe Cys His Asp Ser Ser Asp Leu Trp Val
1 5 10 15

Cys Ser Arg

<210> 172
<211> 19
<212> PRT
<213> Artificial Sequence

<220>
<223> Description of Artificial Sequence:Arbitrary peptide

<400> 172

Ser Arg Pro Gly Cys Leu Arg Gly Val Trp Cys Leu Ala Asp Thr Pro
1 5 10 15

Pro Ser Arg

<210> 173
<211> 19
<212> PRT
<213> Artificial Sequence

<220>
<223> Description of Artificial Sequence:Arbitrary peptide

<400> 173

Ser Ser Arg Leu Val Pro His Ser Phe Trp Leu Asp Gly Leu Met His
1 5 10 15

Gly Ser Arg

<210> 174
<211> 19
<212> PRT
<213> Artificial Sequence

<220>
<223> Description of Artificial Sequence:Arbitrary peptide

<400> 174

Ser Ser Ile Ser Thr Tyr His Met Gly Glu Trp Phe Tyr Ala Met Leu
1 5 10 15

Ser Ser Arg

<210> 175
<211> 18
<212> PRT
<213> Artificial Sequence

<220>
<223> Description of Artificial Sequence:Arbitrary peptide

<400> 175

Ser Ser Asp Leu Tyr Ser Gln Met Arg Glu Phe Phe Gln Ile Asn Leu

1 5 10 15

Ser Arg

<210> 176
<211> 15
<212> PRT
<213> Artificial Sequence

<220>
<223> Description of Artificial Sequence:Arbitrary peptide

<400> 176

Ser Ser Arg Gly Leu Leu Trp Asp Leu Leu Thr Lys Asp Ser Arg
1 5 10 15

<210> 177
<211> 15
<212> PRT
<213> Artificial Sequence

<220>
<223> Description of Artificial Sequence:Arbitrary peptide

<400> 177

Ser Arg His Gly Ile Leu Trp Asp Leu Leu Gln Gly Asp Ser Arg
1 5 10 15

<210> 178
<211> 15
<212> PRT
<213> Artificial Sequence

<220>
<223> Description of Artificial Sequence:Arbitrary peptide

<400> 178

Ser Arg Leu His Asp Leu Leu Leu Arg Asp Glu Ser Pro Ser Arg
1 5 10 15

<210> 179
<211> 15
<212> PRT
<213> Artificial Sequence

<220>
<223> Description of Artificial Sequence:Arbitrary peptide

<400> 179

Ser Arg Asp Trp Arg Ser Gly Phe Leu Tyr Glu Leu Leu Ser Arg
1 5 10 15

<210> 180
<211> 19
<212> PRT
<213> Artificial Sequence

<220>
<223> Description of Artificial Sequence:Arbitrary peptide

<400> 180

Ser Ser Asp Thr Arg Ser Arg Leu Tyr Glu Leu Leu Ser Ser Ser Tyr
1 5 10 15

Thr Ser Arg

<210> 181
<211> 15
<212> PRT
<213> Artificial Sequence

<220>
<223> Description of Artificial Sequence:Arbitrary peptide

<400> 181

Ser Arg Leu Glu Glu Leu Leu Arg Val Gly Val Leu Thr Ser Arg
1 5 10 15

<210> 182
<211> 16
<212> PRT
<213> Artificial Sequence

<220>
<223> Description of Artificial Sequence:Arbitrary peptide

<400> 182

Ser Arg Leu Glu Asp Leu Leu Arg Gly Asp Ser Lys Pro Gln Ser Arg
1 5 10 15

<210> 183
<211> 19
<212> PRT
<213> Artificial Sequence

<220>
<223> Description of Artificial Sequence:Arbitrary peptide

<400> 183

Ser Ser Pro Thr Gly His Arg Leu Leu Glu Ser Leu Leu Leu Asn Ser
1 5 10 15

Asn Ser Arg

<210> 184

<211> 16
<212> PRT
<213> Artificial Sequence

<220>
<223> Description of Artificial Sequence:Arbitrary peptide

<400> 184

Ser Ser Ile Leu Glu Arg Leu Leu Gly Gly Gly Ser Ala Glu Thr Val
1 5 10 15

<210> 185
<211> 15
<212> PRT
<213> Artificial Sequence

<220>
<223> Description of Artificial Sequence:Arbitrary peptide

<400> 185

Ser Arg Ser Pro Ile Leu Trp His Leu Leu Gln Asp Gly Ser Arg
1 5 10 15

<210> 186
<211> 15
<212> PRT
<213> Artificial Sequence

<220>
<223> Description of Artificial Sequence:Arbitrary peptide

<400> 186

Ser Ser Arg Thr Pro Ile Leu Phe Ser Leu Leu Glu Thr Ser Arg
1 5 10 15

<210> 187
<211> 15
<212> PRT
<213> Artificial Sequence

<220>
<223> Description of Artificial Sequence:Arbitrary peptide

<400> 187

Ser Ser Ile Lys Asp Phe Pro Asn Leu Ile Ser Leu Leu Ser Arg
1 5 10 15

<210> 188
<211> 19
<212> PRT
<213> Artificial Sequence

<220>
<223> Description of Artificial Sequence:Arbitrary peptide

<400> 188

Ser Ser Gly Ser Ser Ala Gly Arg Leu Met Met Leu Leu Gln Asp Gly
1 5 10 15

Val Ser Arg

<210> 189
<211> 15
<212> PRT
<213> Artificial Sequence

<220>
<223> Description of Artificial Sequence:Arbitrary peptide

<400> 189

Ser Arg Glu Gly Leu Leu Met Arg Leu Leu Ile Gly Asp Ser Arg
1 5 10 15

<210> 190
<211> 15
<212> PRT
<213> Artificial Sequence

<220>
<223> Description of Artificial Sequence:Arbitrary peptide

<400> 190

Ser Ser His Cys His Thr Arg Leu Cys Ser Leu Leu Thr Ser Arg
1 5 10 15

<210> 191
<211> 15
<212> PRT
<213> Artificial Sequence

<220>
<223> Description of Artificial Sequence:Arbitrary peptide

<400> 191

Ser Ser Arg Leu Leu Cys Leu Leu Asp Ala Gly Gln Cys Ser Arg
1 5 10 15

<210> 192
<211> 15
<212> PRT
<213> Artificial Sequence

<220>
<223> Description of Artificial Sequence:Arbitrary peptide

<400> 192

Ser Arg Asn Leu Leu Cys Leu Leu Asp Gln Glu Ala Cys Ser Arg
1 5 10 15

<210> 193
<211> 14
<212> PRT
<213> Artificial Sequence

<220>
<223> Description of Artificial Sequence:Arbitrary peptide

<400> 193

Ser Ser Leu Lys Cys Leu Leu Asn Ser Asn Phe Cys Ser Arg
1 5 10

<210> 194
<211> 19
<212> PRT
<213> Artificial Sequence

<220>
<223> Description of Artificial Sequence:Arbitrary peptide

<400> 194

Ser Ser Leu Lys Cys Leu Leu Gln Ser Ser Pro Gln Lys Gln Pro Phe
1 5 10 15

Cys Ser Arg

<210> 195

<211> 15
<212> PRT
<213> Artificial Sequence

<220>
<223> Description of Artificial Sequence:Arbitrary peptide

<400> 195

```
Ser Ser Arg Thr Leu Leu Glu His Tyr Leu Leu Gly Gly Ser Arg
 1               5                  10                  15
```

<210> 196
<211> 15
<212> PRT
<213> Artificial Sequence

<220>
<223> Description of Artificial Sequence:Arbitrary peptide

<400> 196

```
Ser Ser Ala Gly Leu Leu Glu Asp Met Leu Arg Ser Arg Ser Arg
 1               5                  10                  15
```

<210> 197
<211> 19
<212> PRT
<213> Artificial Sequence

<220>
<223> Description of Artificial Sequence:Arbitrary peptide

<400> 197

```
Ser Ser Arg Cys Ser Ser Leu Leu Cys Glu Met Leu Ile Gln Thr Lys
 1               5                  10                  15

Glu Ser Arg
```

<210> 198
<211> 19
<212> PRT
<213> Artificial Sequence

<220>
<223> Description of Artificial Sequence:Arbitrary peptide

<400> 198

Ser Ser Leu Gln Ala Gly Ser Trp Leu Met His Tyr Leu Arg Gly Gly
1 5 10 15

Asp Ser Arg

<210> 199
<211> 15
<212> PRT
<213> Artificial Sequence

<220>
<223> Description of Artificial Sequence:Arbitrary peptide

<400> 199

Ser Arg Pro Glu Gly Ser Ser Trp Leu Leu His Tyr Leu Ser Arg
1 5 10 15

<210> 200
<211> 15
<212> PRT
<213> Artificial Sequence

<220>
<223> Description of Artificial Sequence:Arbitrary peptide

<400> 200

Ser Ser Arg Thr Leu Leu Glu His Tyr Leu Leu Gly Gly Ser Arg
1 5 10 15

<210> 201
<211> 16
<212> PRT
<213> Artificial Sequence

<220>
<223> Description of Artificial Sequence:Arbitrary peptide

<400> 201

Ser Arg Trp Trp Leu Asp Asp His Glu Leu Leu Leu Tyr Ser Ser Arg
1 5 10 15

<210> 202
<211> 19
<212> PRT
<213> Artificial Sequence

<220>

<223> Description of Artificial Sequence:Arbitrary peptide

<400> 202

Ser Ser Arg Thr Leu Tyr Cys His Leu Thr Ser Ser Asn Pro Glu Trp
1 5 10 15

Cys Ser Arg

<210> 203
<211> 19
<212> PRT
<213> Artificial Sequence

<220>
<223> Description of Artificial Sequence:Arbitrary peptide

<400> 203

Ser Ser Thr Arg Leu Met Cys Trp Leu Gly Ser Ala Asp Thr Ser His
1 5 10 15

Cys Ser Arg

<210> 204
<211> 19
<212> PRT
<213> Artificial Sequence

<220>
<223> Description of Artificial Sequence:Arbitrary peptide

<400> 204

Ser Ser Tyr Asp Trp Gln Cys Pro Ser Trp Tyr Cys Pro Ala Pro Pro
1 5 10 15

Ser Ser Arg

<210> 205
<211> 15
<212> PRT
<213> Artificial Sequence

<220>
<223> Description of Artificial Sequence:Arbitrary peptide

<400> 205

Ser Ser Thr Thr Trp Arg Cys Pro Glu Trp Tyr Cys Gly Ser Arg
1 5 10 15

<210> 206
<211> 15
<212> PRT
<213> Artificial Sequence

<220>
<223> Description of Artificial Sequence:Arbitrary peptide

<400> 206

Ser Ser Trp Asp Phe Arg Val Pro Trp Trp Tyr Asn Asn Ser Arg
1 5 10 15

<210> 207
<211> 15
<212> PRT
<213> Artificial Sequence

<220>
<223> Description of Artificial Sequence:Arbitrary peptide

<400> 207

Ser Ser Gln Trp Gln Ala Pro Trp Trp Tyr Ile Asp Ala Ser Arg
1 5 10 15

<210> 208
<211> 19
<212> PRT
<213> Artificial Sequence

<220>
<223> Description of Artificial Sequence:Arbitrary peptide

<400> 208

Ser Ser Arg Pro Ser Phe Thr Ile Pro Trp Trp Phe Asp Asp Pro Ser
1 5 10 15

Arg Ser Arg

<210> 209
<211> 15
<212> PRT
<213> Artificial Sequence

<220>
<223> Description of Artificial Sequence:Arbitrary peptide

<400> 209

Ser Ser Tyr Glu Ile Pro Lys Trp Ala Leu Gln Trp Leu Ser Arg
1 5 10 15

<210> 210
<211> 19
<212> PRT
<213> Artificial Sequence

<220>
<223> Description of Artificial Sequence:Arbitrary peptide

<400> 210

Ser Ser Leu Asp Leu Ser Gln Phe Pro Met Thr Ala Ser Phe Leu Arg
1 5 10 15

Glu Ser Arg

<210> 211
<211> 15
<212> PRT
<213> Artificial Sequence

<220>
<223> Description of Artificial Sequence:Arbitrary peptide

<400> 211

Ser Ser Asn His Gln Ser Ser Arg Leu Ile Glu Leu Leu Ser Arg
1 5 10 15

<210> 212
<211> 14
<212> PRT
<213> Artificial Sequence

<220>
<223> Description of Artificial Sequence:Arbitrary peptide

<400> 212

Ser Ala Pro Arg Ala Thr Ile Ser His Tyr Leu Met Gly Gly
1 5 10

<210> 213

<211> 15
<212> PRT
<213> Artificial Sequence

<220>
<223> Description of Artificial Sequence:Arbitrary peptide

<400> 213

Ser Ser Trp Asp Met His Gln Phe Phe Trp Glu Gly Val Ser Arg
1 5 10 15

<210> 214
<211> 19
<212> PRT
<213> Artificial Sequence

<220>
<223> Description of Artificial Sequence:Arbitrary peptide

<400> 214

Ser Arg Leu Pro Pro Ser Val Phe Ser Met Cys Gly Ser Glu Val Cys
1 5 10 15

Leu Ser Arg

<210> 215
<211> 15
<212> PRT
<213> Artificial Sequence

<220>
<223> Description of Artificial Sequence:Arbitrary peptide

<400> 215

Ser Ser Pro Gly Ser Arg Glu Trp Phe Lys Asp Met Leu Ser Arg
1 5 10 15

<210> 216
<211> 15
<212> PRT
<213> Artificial Sequence

<220>
<223> Description of Artificial Sequence:Arbitrary peptide

<400> 216

Ser Ser Glu Tyr Cys Phe Tyr Trp Asp Ser Ala His Cys Ser Arg
1 5 10 15

<210> 217
<211> 15
<212> PRT
<213> Artificial Sequence

<220>
<223> Description of Artificial Sequence:Arbitrary peptide

<400> 217

Ser Ser Leu Thr Ser Arg Asp Phe Gly Ser Trp Tyr Ala Ser Arg
1 5 10 15

<210> 218
<211> 15
<212> PRT
<213> Artificial Sequence

<220>
<223> Description of Artificial Sequence:Arbitrary peptide

<400> 218

Ser Arg Thr Trp Glu Ser Pro Leu Gly Thr Trp Glu Trp Ser Arg
1 5 10 15

<210> 219
<211> 15
<212> PRT
<213> Artificial Sequence

<220>
<223> Description of Artificial Sequence:Arbitrary peptide

<400> 219

Ser Arg Glu Trp Glu Asp Gly Phe Gly Gly Arg Trp Leu Ser Arg
1 5 10 15

<210> 220
<211> 19
<212> PRT
<213> Artificial Sequence

<220>
<223> Description of Artificial Sequence:Arbitrary peptide

<400> 220

Ser Ser Leu Asp Leu Ser Gln Phe Pro Met Thr Ala Ser Phe Leu Arg
1 5 10 15

Glu Ser Arg

<210> 221
<211> 19
<212> PRT
<213> Artificial Sequence

<220>
<223> Description of Artificial Sequence:Arbitrary peptide

<400> 221

Ser Ser Glu Ala Cys Val Gly Arg Trp Met Leu Cys Glu Gln Leu Gly
1 5 10 15

Val Ser Arg

<210> 222
<211> 15
<212> PRT
<213> Artificial Sequence

<220>
<223> Description of Artificial Sequence:Arbitrary peptide

<400> 222

Ser Arg Ala Gly Leu Leu Ser Asp Leu Leu Glu Gly Lys Ser Arg
1 5 10 15

<210> 223
<211> 15
<212> PRT
<213> Artificial Sequence

<220>
<223> Description of Artificial Sequence:Arbitrary peptide

<400> 223

Ser Ser Arg Ser Leu Leu Arg Asp Leu Leu Met Val Asp Ser Arg
1 5 10 15

<210> 224
<211> 15
<212> PRT
<213> Artificial Sequence

<220>
<223> Description of Artificial Sequence:Arbitrary peptide

<400> 224

Ser Ser Asn Lys Leu Leu Tyr Asn Leu Leu Lys Met Glu Ser Arg
1 5 10 15

<210> 225
<211> 15
<212> PRT
<213> Artificial Sequence

<220>
<223> Description of Artificial Sequence:Arbitrary peptide

<400> 225

Ser Ser Lys Ser Leu Leu Leu Asn Leu Leu Ser Thr Pro Ser Arg
1 5 10 15

<210> 226
<211> 16
<212> PRT
<213> Artificial Sequence

<220>
<223> Description of Artificial Sequence:Arbitrary peptide

<400> 226

His Ser Phe Pro Arg Glu Ser Leu Leu Val Arg Leu Leu Gln Gly Gly
1 5 10 15

<210> 227
<211> 15
<212> PRT
<213> Artificial Sequence

<220>
<223> Description of Artificial Sequence:Arbitrary peptide

<400> 227

Ser Arg Leu Glu Met Leu Leu Arg Ser Glu Thr Asp Phe Ser Arg
1 5 10 15

<210> 228
<211> 15
<212> PRT
<213> Artificial Sequence

<220>
<223> Description of Artificial Sequence:Arbitrary peptide

<400> 228

Ser Arg Leu Glu Glu Leu Leu Lys Trp Gly Ser Val Thr Ser Arg
1 5 10 15

<210> 229
<211> 15
<212> PRT
<213> Artificial Sequence

<220>
<223> Description of Artificial Sequence:Arbitrary peptide

<400> 229

Ser Arg Leu Glu Gln Leu Leu Lys Glu Glu Phe Ser Tyr Ser Arg
1 5 10 15

<210> 230
<211> 15
<212> PRT
<213> Artificial Sequence

<220>
<223> Description of Artificial Sequence:Arbitrary peptide

<400> 230

Ser Arg Leu Glu Gln Leu Leu Arg Ser Glu Pro Asp Phe Ser Arg
1 5 10 15

<210> 231
<211> 15
<212> PRT
<213> Artificial Sequence

<220>
<223> Description of Artificial Sequence:Arbitrary peptide

<400> 231

Ser Arg Leu Glu Asp Leu Leu Arg Ala Pro Phe Thr Thr Ser Arg
1 5 10 15

<210> 232
<211> 15
<212> PRT
<213> Artificial Sequence

<220>
<223> Description of Artificial Sequence:Arbitrary peptide

<400> 232

Ser Arg Leu Glu Ser Leu Leu Arg Phe Gly Gln Leu Asp Ser Arg
1 5 10 15

<210> 233
<211> 15
<212> PRT
<213> Artificial Sequence

<220>
<223> Description of Artificial Sequence:Arbitrary peptide

<400> 233

Ser Ser Arg Leu Leu Ser Leu Leu Val Gly Asp Phe Asn Ser Arg
1 5 10 15

<210> 234
<211> 15
<212> PRT
<213> Artificial Sequence

<220>
<223> Description of Artificial Sequence:Arbitrary peptide

<400> 234

Ser Arg Leu Glu Glu Leu Leu Leu Gly Thr Asn Arg Asp Ser Arg
1 5 10 15

<210> 235
<211> 15
<212> PRT
<213> Artificial Sequence

<220>

<223> Description of Artificial Sequence:Arbitrary peptide

<400> 235

Ser Arg Leu Glu Glu Leu Leu Leu Met Asp Phe Trp Arg Ser Arg
1 5 10 15

<210> 236
<211> 15
<212> PRT
<213> Artificial Sequence

<220>
<223> Description of Artificial Sequence:Arbitrary peptide

<400> 236

Ser Arg Leu Lys Glu Leu Leu Leu Leu Pro Thr Asp Leu Ser Arg
1 5 10 15

<210> 237
<211> 15
<212> PRT
<213> Artificial Sequence

<220>
<223> Description of Artificial Sequence:Arbitrary peptide

<400> 237

Ser Arg Leu Glu Cys Leu Leu Glu Gly Arg Leu Asn Cys Ser Arg
1 5 10 15

<210> 238
<211> 15
<212> PRT
<213> Artificial Sequence

<220>
<223> Description of Artificial Sequence:Arbitrary peptide

<400> 238

Ser Ser Lys Leu Tyr Cys Leu Leu Asp Glu Ser Tyr Cys Ser Arg
1 5 10 15

<210> 239
<211> 15
<212> PRT

<213> Artificial Sequence

<220>
<223> Description of Artificial Sequence:Arbitrary peptide

<400> 239

Ser Arg Leu Ser Cys Leu Leu Met Gly Phe Glu Asp Cys Ser Arg
1 5 10 15

<210> 240
<211> 16
<212> PRT
<213> Artificial Sequence

<220>
<223> Description of Artificial Sequence:Arbitrary peptide

<400> 240

Ser Ser Lys Leu Ile Arg Leu Leu Thr Ser Asp Glu Glu Leu Ser Arg
1 5 10 15

<210> 241
<211> 16
<212> PRT
<213> Artificial Sequence

<220>
<223> Description of Artificial Sequence:Arbitrary peptide

<400> 241

Ser Ser Arg Leu Met Glu Leu Leu Gln Glu Gly Gln Gly Trp Ser Arg
1 5 10 15

<210> 242
<211> 15
<212> PRT
<213> Artificial Sequence

<220>
<223> Description of Artificial Sequence:Arbitrary peptide

<400> 242

Ser Ser Asn His Gln Ser Ser Arg Leu Ile Glu Leu Leu Ser Arg
1 5 10 15

<210> 243
<211> 15
<212> PRT
<213> Artificial Sequence

<220>
<223> Description of Artificial Sequence:Arbitrary peptide

<400> 243

Ser Ser Arg Leu Trp Gln Leu Leu Ala Ser Thr Asp Thr Ser Arg
1 5 10 15

<210> 244
<211> 15
<212> PRT
<213> Artificial Sequence

<220>
<223> Description of Artificial Sequence:Arbitrary peptide

<400> 244

Ser Ser Lys Leu Trp Gln Leu Leu Ser Ser Pro Ile Asp Ser Arg
1 5 10 15

<210> 245
<211> 15
<212> PRT
<213> Artificial Sequence

<220>
<223> Description of Artificial Sequence:Arbitrary peptide

<400> 245

Ser Arg Leu Val Ala Leu Leu Lys Ser Pro Trp Ser Val Ser Arg
1 5 10 15

<210> 246
<211> 15
<212> PRT
<213> Artificial Sequence

<220>
<223> Description of Artificial Sequence:Arbitrary peptide

<400> 246

Ser Ser Asn Ser Met Leu Trp Lys Leu Leu Ala Ala Pro Ser Arg
1 5 10 15

<210> 247
<211> 15
<212> PRT
<213> Artificial Sequence

<220>
<223> Description of Artificial Sequence:Arbitrary peptide

<400> 247

Ser Ser Lys Thr Leu Trp Arg Leu Leu Glu Gly Glu Arg Ser Arg
1 5 10 15

<210> 248
<211> 15
<212> PRT
<213> Artificial Sequence

<220>
<223> Description of Artificial Sequence:Arbitrary peptide

<400> 248

Ser Arg Ala Gly Pro Val Leu Trp Gly Leu Leu Ser Glu Ser Arg
1 5 10 15

<210> 249
<211> 15
<212> PRT
<213> Artificial Sequence

<220>
<223> Description of Artificial Sequence:Arbitrary peptide

<400> 249

Ser Arg Ser Pro Ile Leu Thr His Leu Leu Ser Leu Gly Ser Arg
1 5 10 15

<210> 250
<211> 15
<212> PRT
<213> Artificial Sequence

<220>

<223> Description of Artificial Sequence:Arbitrary peptide

<400> 250

Ser Ser Thr Gly Ile Leu Trp Lys Leu Leu Thr Ala Glu Ser Arg
1 5 10 15

<210> 251
<211> 15
<212> PRT
<213> Artificial Sequence

<220>
<223> Description of Artificial Sequence:Arbitrary peptide

<400> 251

Ser Ser His Gly Ile Leu Trp Arg Leu Leu Ser Glu Gly Ser Arg
1 5 10 15

<210> 252
<211> 11
<212> PRT
<213> Artificial Sequence

<220>
<223> Description of Artificial Sequence:Arbitrary peptide

<400> 252

Lys Leu Val Gln Leu Leu Thr Thr Thr Ala Glu
1 5 10

<210> 253
<211> 11
<212> PRT
<213> Artificial Sequence

<220>
<223> Description of Artificial Sequence:Arbitrary peptide

<400> 253

Ile Leu His Arg Leu Leu Gln Glu Gly Ser Pro
1 5 10

<210> 254
<211> 11
<212> PRT

<213> Artificial Sequence

<220>
<223> Description of Artificial Sequence:Arbitrary peptide

<400> 254

Leu Leu Arg Tyr Leu Leu Asp Lys Asp Glu Lys
1 5 10

<210> 255
<211> 8
<212> PRT
<213> Artificial Sequence

<220>
<223> Description of Artificial Sequence:Arbitrary peptide

<400> 255

Leu Leu Gln Gln Leu Leu Thr Glu
1 5

<210> 256
<211> 11
<212> PRT
<213> Artificial Sequence

<220>
<223> Description of Artificial Sequence:Arbitrary peptide

<400> 256

Gln Leu Ser Glu Leu Leu Arg Gly Gly Ser Gly
1 5 10

<210> 257
<211> 11
<212> PRT
<213> Artificial Sequence

<220>
<223> Description of Artificial Sequence:Arbitrary peptide

<400> 257

Gln Leu Val Leu Leu Leu His Ala His Lys Cys
1 5 10

<210> 258

<211> 11
<212> PRT
<213> Artificial Sequence

<220>
<223> Description of Artificial Sequence:Arbitrary peptide

<400> 258

Tyr Leu Glu Gly Leu Leu Met His Gln Ala Ala
1 5 10

<210> 259
<211> 11
<212> PRT
<213> Artificial Sequence

<220>
<223> Description of Artificial Sequence:Arbitrary peptide

<400> 259

Leu Leu Ala Ser Leu Leu Gln Ser Glu Ser Ser
1 5 10

<210> 260
<211> 11
<212> PRT
<213> Artificial Sequence

<220>
<223> Description of Artificial Sequence:Arbitrary peptide

<400> 260

His Leu Lys Thr Leu Leu Lys Lys Ser Lys Val
1 5 10

<210> 261
<211> 11
<212> PRT
<213> Artificial Sequence

<220>
<223> Description of Artificial Sequence:Arbitrary peptide

<400> 261

Gln Leu Ala Leu Leu Leu Ser Ser Glu Ala His
1 5 10

<210> 262
<211> 11
<212> PRT
<213> Artificial Sequence

<220>
<223> Description of Artificial Sequence:Arbitrary peptide

<400> 262

Leu Leu Leu His Leu Leu Lys Ser Gln Thr Ile
1 5 10

<210> 263
<211> 11
<212> PRT
<213> Artificial Sequence

<220>
<223> Description of Artificial Sequence:Arbitrary peptide

<400> 263

Leu Leu Gln Leu Leu Leu Gly His Lys Asn Glu
1 5 10

<210> 264
<211> 11
<212> PRT
<213> Artificial Sequence

<220>
<223> Description of Artificial Sequence:Arbitrary peptide

<400> 264

Val Leu Gln Leu Leu Leu Gly Asn Pro Lys Gly
1 5 10

<210> 265
<211> 11
<212> PRT
<213> Artificial Sequence

<220>
<223> Description of Artificial Sequence:Arbitrary peptide

<400> 265

Leu Leu Ser Arg Leu Leu Arg Gln Asn Gln Asp
1 5 10

<210> 266
<211> 11
<212> PRT
<213> Artificial Sequence

<220>
<223> Description of Artificial Sequence:Arbitrary peptide

<400> 266

Val Leu Lys Gln Leu Leu Leu Ser Glu Asn Cys
1 5 10

<210> 267
<211> 14
<212> PRT
<213> Artificial Sequence

<220>
<223> Description of Artificial Sequence:Arbitrary peptide

<400> 267

Ser Ser Asn His Gln Ser Arg Leu Ile Glu Leu Leu Ser Arg
1 5 10

<210> 268
<211> 19
<212> PRT
<213> Artificial Sequence

<220>
<223> Description of Artificial Sequence:Arbitrary peptide

<400> 268

His Val Tyr Gln His Pro Leu Leu Leu Ser Leu Leu Ser Ser Glu His
1 5 10 15

Glu Ser Gly

<210> 269
<211> 19
<212> PRT
<213> Artificial Sequence

<220>
<223> Description of Artificial Sequence:Arbitrary peptide

<400> 269

His Val Glu Met His Pro Leu Leu Met Gly Leu Leu Met Glu Ser Gln
1 5 10 15

Trp Gly Ala

<210> 270
<211> 19
<212> PRT
<213> Artificial Sequence

<220>
<223> Description of Artificial Sequence:Arbitrary peptide

<400> 270

Gln Glu Ala His Gly Pro Leu Leu Trp Asn Leu Leu Ser Arg Ser Asp
1 5 10 15

Thr Asp Trp

<210> 271
<211> 19
<212> PRT
<213> Artificial Sequence

<220>
<223> Description of Artificial Sequence:Arbitrary peptide

<400> 271

Gly His Glu Pro Leu Thr Leu Leu Glu Arg Leu Leu Met Asp Asp Lys
1 5 10 15

Gln Ala Val

<210> 272
<211> 19
<212> PRT
<213> Artificial Sequence

<220>
<223> Description of Artificial Sequence:Arbitrary peptide

<400> 272

Leu Pro Tyr Glu Gly Ser Leu Leu Leu Lys Leu Leu Arg Ala Pro Val
1 5 10 15

Glu Glu Val

<210> 273
<211> 19
<212> PRT
<213> Artificial Sequence

<220>
<223> Description of Artificial Sequence:Arbitrary peptide

<400> 273

Ser Gly Trp Glu Asn Ser Ile Leu Tyr Ser Leu Leu Ser Asp Arg Val
1 5 10 15

Ser Leu Asp

<210> 274
<211> 19
<212> PRT
<213> Artificial Sequence

<220>
<223> Description of Artificial Sequence:Arbitrary peptide

<400> 274

Ala His Gly Glu Ser Ser Leu Leu Ala Trp Leu Leu Ser Gly Glu Tyr
1 5 10 15

Ser Ser Ala

<210> 275
<211> 19
<212> PRT
<213> Artificial Sequence

<220>
<223> Description of Artificial Sequence:Arbitrary peptide

<400> 275

Gly Val Phe Cys Asp Ser Ile Leu Cys Gln Leu Leu Ala His Asp Asn
1 5 10 15

Ala Arg Leu

<210> 276
<211> 19
<212> PRT
<213> Artificial Sequence

<220>
<223> Description of Artificial Sequence:Arbitrary peptide

<400> 276

His His Asn Gly His Ser Ile Leu Tyr Gly Leu Leu Ala Gly Ser Asp
1 5 10 15

Ala Pro Ser

<210> 277
<211> 19
<212> PRT
<213> Artificial Sequence

<220>
<223> Description of Artificial Sequence:Arbitrary peptide

<400> 277

Leu Gly Glu Arg Ala Ser Leu Leu Asp Met Leu Leu Arg Gln Glu Asn
1 5 10 15

Pro Ala Trp

<210> 278
<211> 19
<212> PRT
<213> Artificial Sequence

<220>
<223> Description of Artificial Sequence:Arbitrary peptide

<400> 278

Ser Gly Trp Asn Glu Ser Thr Leu Tyr Arg Leu Leu Gln Ala Asp Ala
1 5 10 15

Phe Asp Val

<210> 279
<211> 19
<212> PRT
<213> Artificial Sequence

<220>
<223> Description of Artificial Sequence:Arbitrary peptide

<400> 279

Pro Ser Gly Gly Ser Ser Val Leu Glu Tyr Leu Leu Thr His Asp Thr
1 5 10 15

Ser Ile Leu

<210> 280
<211> 19
<212> PRT
<213> Artificial Sequence

<220>
<223> Description of Artificial Sequence:Arbitrary peptide

<400> 280

Gly Ser Glu Pro Lys Ser Arg Leu Leu Glu Leu Leu Ser Ala Pro Val
1 5 10 15

Thr Asp Val

<210> 281
<211> 19
<212> PRT
<213> Artificial Sequence

<220>
<223> Description of Artificial Sequence:Arbitrary peptide

<400> 281

His Pro Thr His Ser Ser Arg Leu Trp Glu Leu Leu Met Glu Ala Thr
1 5 10 15

Pro Thr Met

<210> 282
<211> 19
<212> PRT
<213> Artificial Sequence

<220>
<223> Description of Artificial Sequence:Arbitrary peptide

<400> 282

Val Glu Ser Gly Ser Ser Arg Leu Met Gln Leu Leu Met Ala Asn Asp
1 5 10 15

Leu Leu Thr

<210> 283
<211> 19
<212> PRT
<213> Artificial Sequence

<220>
<223> Description of Artificial Sequence:Arbitrary peptide

<400> 283

Trp Glu Glu His Ser Gln Met Leu Leu His Leu Leu Asp Thr Gly Glu
1 5 10 15

Ala Val Trp

<210> 284
<211> 19
<212> PRT
<213> Artificial Sequence

<220>
<223> Description of Artificial Sequence:Arbitrary peptide

<400> 284

Pro Val Gly Glu Pro Gly Leu Leu Trp Arg Leu Leu Ser Ala Pro Val
1 5 10 15

Glu Arg Glu